# EUROPEAN PATENT SPECIFICATION

(11) **EP 4 041 231 B1**
(45) Date of publication and mention of the grant of the patent: **02.09.2026**
(21) Application number: 20789952.7
(22) Date of filing: 09.10.2020
(51) Int. Cl.: A61K 31/443, C07D 401/04, A61P 35/00, C07D 401/14, C07D 491/107

(54) **ISOINDOLINONE COMPOUNDS**
ISOINDOLINONVERBINDUNGEN
COMPOSÉS D'ISO-INDOLINONE

(30) Priority: 09.10.2019 CH 12812019
(43) Date of publication of application: 17.08.2022
(73) Proprietor: Monte Rosa Therapeutics AG, 4057 Basel (CH)
(72) Inventor: FLOHR, Alexander, 4057 Basel (CH); EIDAM, Oliv, 4057 Basel (CH); FASCHING, Bernhard, 4057 Basel (CH); MENICONI, Mirco, Sutton SM2 5NG (GB); SADOK, Amine, 4057 Basel (CH); CHOPRA, Rajesh, Putney, London (GB); ZHU`AI WANG, Hannah, Sutton SM2 5NG (GB); CALDWELL, John Jamieson, Sutton SM2 5NG (GB); COLLINS, Ian, Sutton SM2 5NG (GB); RYCKMANS, Thomas, 68680 Kembs (FR)
(74) Representative: Carpmaels & Ransford LLP
(86) International application number: PCT/EP2020/078483
(87) International publication number: WO 2021/069705

(56) References cited:
- WO-A1-02/059106
- WO-A1-2019/148055
- WO-A2-2008/027542
- PHILIP P. CHAMBERLAIN ET AL: "Cereblon modulators: Low molecular weight inducers of protein degradation", DRUG DISCOVERY TODAY: TECHNOLOGIES, vol. 31, 1 April 2019 (2019-04-01), AMSTERDAM, NL, pages 29 - 34, XP055758408, ISSN: 1740-6749, DOI: 10.1016/j.ddtec.2019.02.004
- GANG LU ET AL: "A novel cereblon modulator recruits GSPT1 to the CRL4CRBN ubiquitin ligase (inlcudes methods)", vol. 535, 14 July 2016 (2016-07-14), pages 252 - 257, 17pp, XP002784903, ISSN: 0028-0836, Retrieved from the Internet <URL:https://www.nature.com/articles/nature18611> [retrieved on 20160622], DOI: 10.1038/NATURE18611
- DANIEL P. BONDESON ET AL: "Targeted Protein Degradation by Small Molecules", ANNUAL REVIEW OF PHARMACOLOGY AND TOXICOLOGY., vol. 57, no. 1, 12 October 2016 (2016-10-12), US, pages 107 - 123, XP055588533, ISSN: 0362-1642, DOI: 10.1146/annurev-pharmtox-010715-103507

## Description

### Field of disclosure

The present disclosure relates to new compounds as modulators of cereblon. The disclosure also relates to methods of preparation of these compounds, compositions comprising these compounds, and methods of using them in the treatment of abnormal cell growth in mammals, especially humans.

### Background, prior art

The ubiquitin proteasome system can be manipulated with different small molecules to trigger targeted degradation of specific proteins of interest. Promoting the targeted degradation of pathogenic proteins using small molecule degraders is emerging as a new modality in the treatment of diseases. One such modality relies on redirecting the activity of E3 ligases such as cereblon (a phenomenon known as E3 reprogramming) using low molecular weight compounds, which have been termed molecular glues (Tan et al. Nature 2007, 446, 640-645 and Sheard et al. Nature 2010, 468, 400-405) to promote the poly-ubiquitination and ultimately proteasomal degradation of new protein substrates involved in the development of diseases. The molecular glues bind to both the E3 ligase and the target protein, thereby mediating an alteration of the ligase surface and enabling an interaction with the target protein. Particular relevant compounds for the E3 ligase cereblon are the IMiD (immunomodulatory imide drugs) class including Thalidomide, Lenalidomide and Pomalidomide. These IMiDs have been approved by the FDA for use in hematological cancers. However, compounds for efficiently targeting other diseases, in particular other types of cancers, are still required.

WO2019148055 relates to imide-based modulators of proteolysis and methods of use thereof.

Philip P. Chamberlain et al.: "Cereblon modulators: Low molecular weight inducers of protein degradation", Drug Discovery Today: Technologies, vol. 31, ist April 2019, pages 29-34 relates to cereblon modulators as inducers of protein degradation.

WO2008/027542 relates to 5-substituted isoindoline compounds.

WO02/059106 relates to isoindole-imide compounds, compositions, and uses thereof.

Gang Lu et al.: "A novel cereblon modulator recruits GSPT1 to the CRL4CRBN ubiquitin ligase (includes methods)", Nature, Macmillan Journals Ltd, vol. 535 14th July 2016, pages 252-257 relates to the identification of a cereblon modulator, CC-885, with potent anti-tumour activity.

Daniel P. Bondeson et al.: "Targeted Protein Degredation by Small Molecules", Annual Review of Pharmacology and Toxicology, vol. 57, no. 1, 12th October 2016, peages 107-123 relates to advances in the use of small molecules to degrade proteins in a selective manner.

### Summary of disclosure

It is therefore an object of the present disclosure to advance the state of the art of cereblon modulators and preferably provide modulators for novel use in different diseases, in particular in different cancers.

In favorable embodiments, compounds are provided which efficiently target c-Myc dependent cells over non c-Myc dependent cells.

In some favorable embodiments, compounds are provided for use in therapy of solid tumors, in particular for use in the therapy of breast cancer.

The present disclosure is in a first aspect directed towards a compound or pharmaceutically acceptable salts or stereoisomers thereof of formula I: 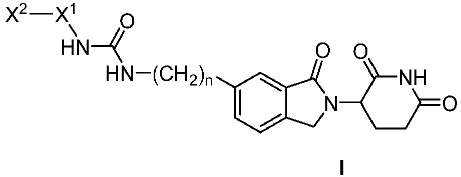 wherein
- X¹: is linear or branched C₁₋₆ alkyl, C₃₋₆ cycloalkyl, -C₁₋₆ alkyl C₃₋₆ cycloalkyl, C₆₋₁₀ aryl, 5-10 membered heteroaryl, C₁₋₆ alkyl C₆₋₁₀ aryl, C₁₋₆ alkyl 5-10 membered heteroaryl, wherein X¹ is unsubstituted or substituted with one or more of halogen, linear or branched C₁₋₆ alkyl, C₁₋₆ heteroalkyl, CF₃, CHF₂, -O-CHF₂, -O-(CH₂)₂-OMe, OCF₃, C₁₋₆ alkylamino, -CN, -N(H)C(O)-C₁₋₆alkyl, -OC(O)-C₁₋₆alkyl, -OC(O)-C₁₋₄alkylamino, - C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy;
- X²: is hydrogen, C₆₋₁₀ aryl, 5-10 membered heteroaryl, -0-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein X² is unsubstituted or substituted with one or more of linear or branched C₁₋₆ alkyl, NH₂, NMe₂ or 5-6 membered heterocycloalkyl;
- n: is 0, 1 or 2.

In certain embodiments, X¹ is linear or branched C₁₋₄ alkyl, C₃₋₆ cycloalkyl, -C₁₋₄ alkyl C₃₋₆ cycloalkyl, C₆ aryl, 5-10 membered heteroaryl, C₁₋₆ alkyl C₆ aryl, C₁₋₆ alkyl 5-10 membered heteroaryl, wherein X¹ is unsubstituted or substituted with one or more of halogen, linear or branched C₁₋₄ alkyl, C₁₋₆ heteroalkyl, CF₃, CHF₂, -O-CHF₂, -O-(CH₂)₂-OMe, OCF₃, C₁₋₆ alkylamino, -CN, -N(H)C(O)-C₁₋₆alkyl, -OC(O)-C₁₋₆alkyl, -OC(O)-C₁₋₄alkylamino, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; and X² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein X² is unsubstituted or substituted with one or more of linear or branched C₁₋₆ alkyl, NH₂, NMe₂ or 5-6 membered heterocycloalkyl; and wherein n is 0, 1, or 2.

In some embodiments, n is 1. In some embodiments, n is 2.

More specifically, the present disclosure is directed towards a compound or pharmaceutically acceptable salts or stereoisomers thereof of formula II 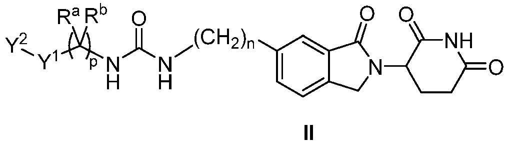 wherein
- n: is 1 or 2;
- p: is 0, 1, 2, 3, 4, 5, 6;
- Y¹: is hydrogen, C₆ aryl, 5- 10 membered heteroaryl, C₃₋₆ cycloalkyl, wherein Y¹ is unsubstituted or substituted with one or more of halogen, preferably F, Cl or Br, linear or branched C₁₋₄ alkyl, C₁₋₆ heteroalkyl, CF₃, CHF₂, -O-CHF₂, -O-(CH₂)₂-OMe, OCF₃, -CN, -N(H)C(O)-C₁₋₆alkyl, -OC(O)-C₁₋₆alkyl, -OC(O)-C₁₋₄heteroalkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; with the proviso that when p is 0, Y¹ is not hydrogen;
- Y²: is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y² is unsubstituted or substituted with one or more of linear or branched C₁₋₆ alkyl, NH₂, NMe₂ or 5-6 membered heterocycloalkyl.
- R^{a}, R^{b}: each are independently selected from hydrogen and linear or branched C₁₋₄ alkyl, preferably hydrogen and methyl.

In some embodiments of a compound or pharmaceutically acceptable salts or stereoisomers thereof of formula II, n is 1.

More specifically, the present disclosure is directed towards a compound or pharmaceutically acceptable salts or stereoisomers thereof of formula Illa, Illb or IIIc 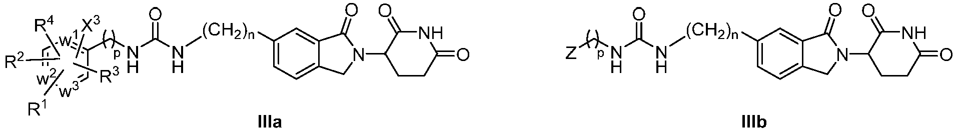 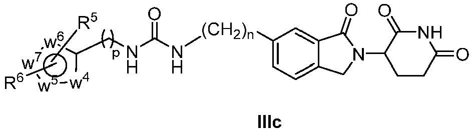 wherein
n is 1 or 2
p is 0, 1 or 2
one of w¹, w² or w³ is selected from C and N, and the other two of w¹, w² or w³ are C;
one or two of w⁴, w⁵, w⁶, w⁷ is selected from C, O, N, NMe, NH, or S while two or three of w⁴, w⁵, w⁶ and w⁷ are C;
R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, - O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl;
R⁵, R⁶ each are independently selected from hydrogen, linear or branched C₁₋₄ alkyl, CF₃, CHF₂, halogen, preferably F, Cl, Br, more preferably F or Cl;
X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl;
Z is linear or branched C₁₋₆ alkyl or C₃₋₆ cycloalkyl or C₁₋₄ alkoxy, wherein Z is unsubstituted or substituted with C₁₋₄ alkyl.

In some embodiments of a compound or pharmaceutically acceptable salts or stereoisomers thereof of formula Illa, Illb or Illc, n is 1.

In certain embodiments of a compound or pharmaceutically acceptable salts or stereoisomers thereof of formula Illa, Illb or Illc, p is 0 or 1.

In certain embodiments of a compound or pharmaceutically acceptable salts or stereoisomers thereof of formula Illa, Illb or Illc, p is 0.

In other embodiments of a compound or pharmaceutically acceptable salts or stereoisomers thereof of formula Illa, Illb or Illc, p is 1.

In some embodiments of a compound or pharmaceutically acceptable salts or stereoisomers thereof of formula Illa, R¹, R², R³ are defined as above and R⁴ is hydrogen such that the aromatic ring contains 4 or 5 substituents which are not hydrogen.

In some embodiments of a compound or pharmaceutically acceptable salts or stereoisomers thereof of formula Illa, R¹ and R² are defined as above and R³ and R⁴ each are hydrogen, such that the aromatic ring contains 3 or 4 substituents which are not hydrogen.

More specifically, the present disclosure is directed towards a compound or pharmaceutically acceptable salts or stereoisomers thereof of formula IV, V, VI and VII 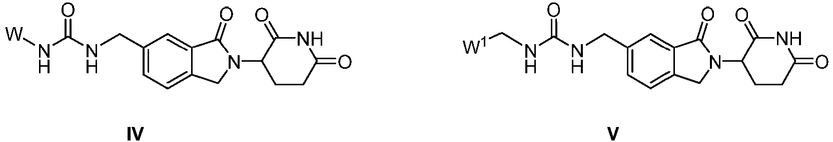 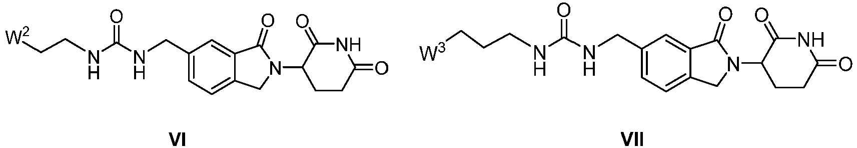 wherein
W, W¹, W², W³ are independently selected from

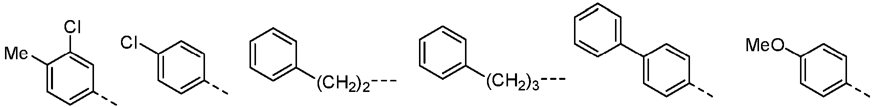

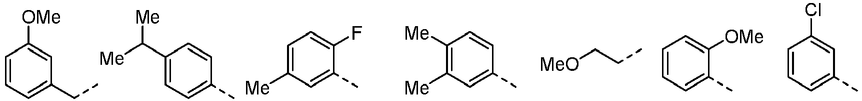

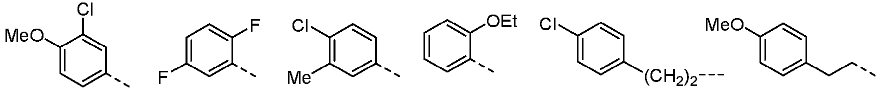

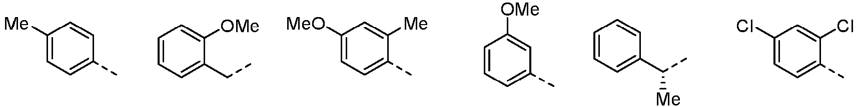

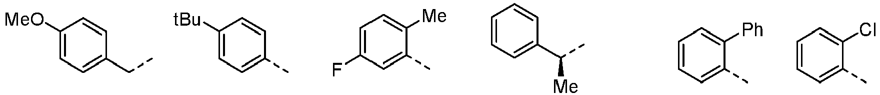

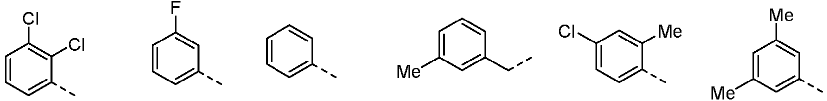

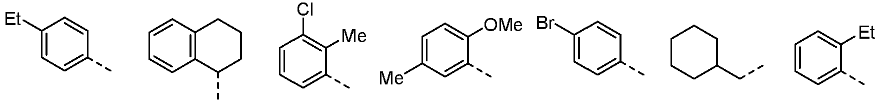

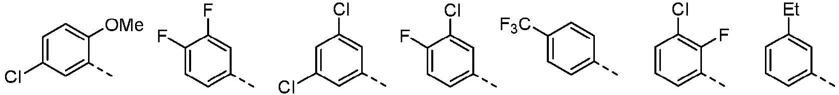

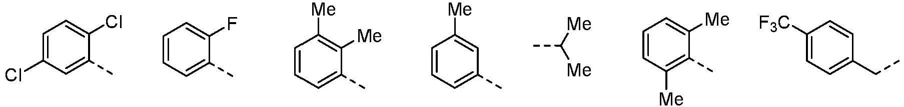

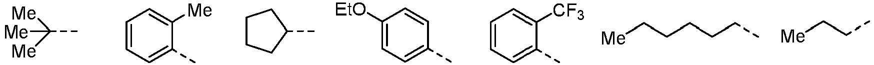

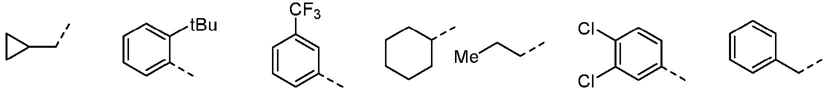

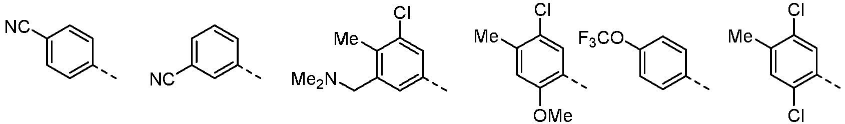

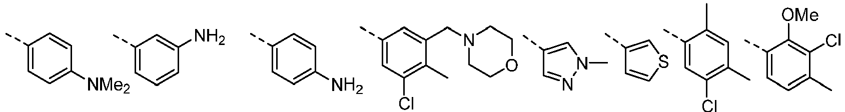

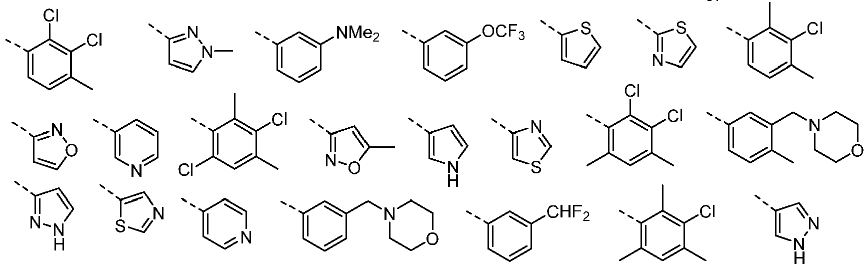

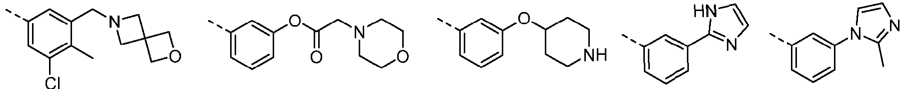

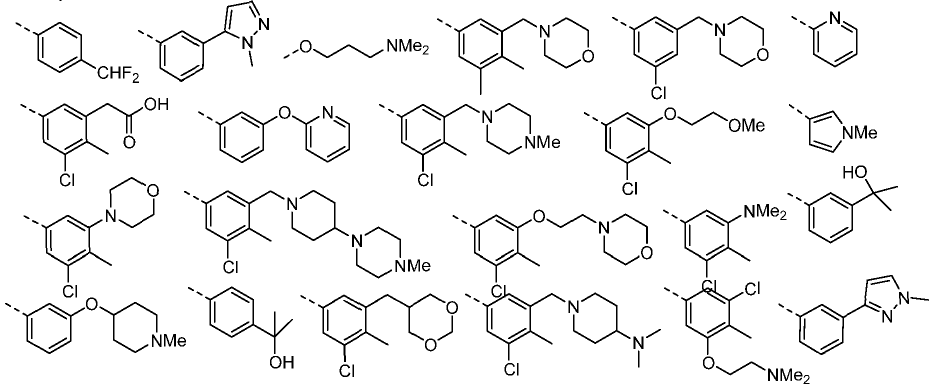

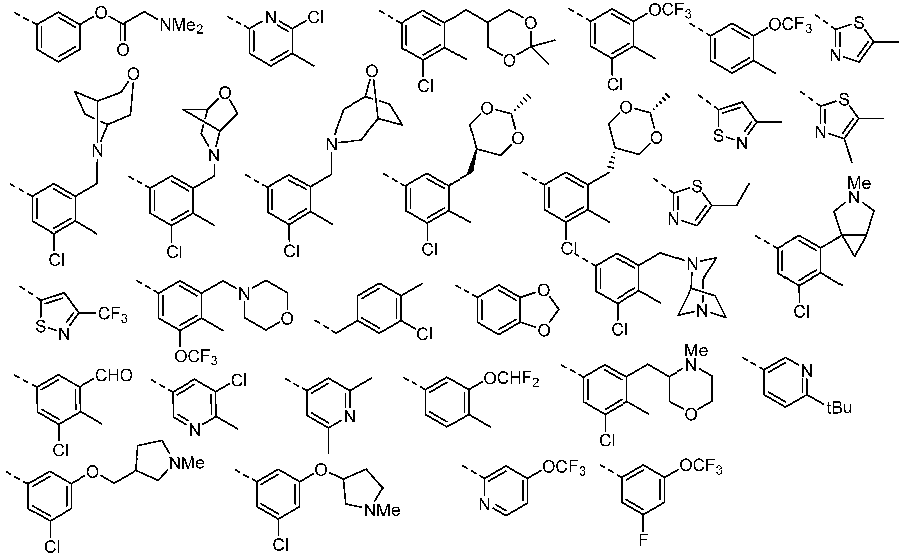

In a second aspect, the disclosure is directed to a composition comprising a compound according to any one of the embodiments or pharmaceutically acceptable salts or stereoisomers thereof described herein.

In some embodiments, the composition further comprises a pharmaceutically acceptable carrier.

In yet further embodiments, the composition further comprises a second therapeutically active agent.

In a third aspect, the disclosure is directed to a composition according to any of the embodiments described herein, for use in therapy.

In a fourth aspect, certain embodiments comprise a compound or pharmaceutically acceptable salts or stereoisomers thereof of formula I-IV, a pharmaceutical acceptable salt thereof or a composition described herein for use in treatment of diseases associated or caused by GSPT1, in particular the treatment of cancer associated with GSPT1, such as glioma, thyroid cancer, lung cancer, colorectal cancer, head and neck cancer, stomach cancer, liver cancer, pancreatic cancer, renal cancer, urothelial cancer, prostate cancer, testis cancer, breast cancer, cervical cancer, endometrial cancer, ovarian cancer, melanoma and multiple myeloma.

Some embodiments comprise the compound or the composition according to any of the embodiments described herein for use in the treatment of breast cancer.

In a fifth aspect, the disclosure is directed to a use of a compound or the composition according to any of the embodiments described herein for binding to cereblon comprising administering to a subject a therapeutically-effective amount of the composition.

Some embodiments comprise the use of a compound or the composition according to any of the embodiments described herein for treating cancer in particular breast cancer. Certain embodiments comprise the use of a composition according to any of the embodiments described herein for treating cancer associated with GSPT1, such as glioma, thyroid cancer, lung cancer, colorectal cancer, head and neck cancer, stomach cancer, liver cancer, pancreatic cancer, renal cancer, urothelial cancer, prostate cancer, testis cancer, breast cancer, cervical cancer, endometrial cancer, ovarian cancer, melanoma and multiple myeloma.

In a sixth aspect, the disclosure is directed to the compound or the composition of any of the embodiments as described herein for use in a method of treating cancer.

In some embodiments, the compound is a compound according to any of the embodiments as described herein or pharmaceutically acceptable salts or stereoisomers thereof that binds to cereblon.

### Detailed description of the disclosure

Unless specified otherwise the following general definitions apply to all compounds of the disclosure according to the description.

The term "compound of the disclosure," as used herein, refers to compounds represented by formulae I to IV and any of the specific examples disclosed herein.

It is understood that "independently of each other" means that when a group is occurring more than one time in any compound, its definition on each occurrence is independent from any other occurrence.

It is further understood that a dashed line (or a wave being transverse to a bond) depicts the site of attachment of a residue (i.e. a partial formula).

It is further understood that the abbreviations "C" and "N" are representative for all possible degrees of saturation, which typically do not result in radicals, nitrenes or carbenes, i.e. N includes -NH- and -N=, C includes -CH₂- and =CH-. In addition, "C" as an atom in an aromatic or heteroaromatic ring which has a substituent R^{x} at any suitable position, includes =CH- as well as =CR^{x}-.

The term "C₆₋₁₀ aryl" refers to a fully or partially aromatic ring system having 6, 7, 8, 9, 10 ring atoms and includes monocycles and fused bicycles. Examples of C₆₋₁₀ aryl include e.g. phenyl, indenyl, naphthyl, 1, 2, 3, 4-tetrahydronaphthyl.

The term "5 to 10 membered heteroaryl", refers to a fully or partially aromatic ring system having 5, 6, 7, 8, 9, 10 ring atoms selected from C, N, O, or S, preferably C, N, or O, more preferably C, N, with the number of N atoms preferably being 0, 1, 2 or 3 and the number of O and S atoms each being 0, 1 or 2. The term "5-10 membered heteroaryl" comprises monocycles and fused bicycles. Fully aromatic ring systems are preferably monocycles with 5 or 6 ring atoms, selected from C, N, O, or S, preferably C, N, or O, more preferably C, N, with the number of N atoms preferably being 0, 1, 2 or 3 and the number of O and S atoms each being 0, 1 or 2. Partially aromatic ring systems are preferably fused bicycles with 8 or 9 ring atoms, selected from C, N, O, or S, preferably C, N, or O, more preferably C, O. Examples of "5 to 10 membered heteroaryl" include furyl, imidazolyl, isoxazolyl, oxazolyl, pyrazinyl, pyrazolyl (pyrazyl), pyridazinyl, pyridinyl, pyrimidinyl, pyrrolyl, thiazolyl, thienyl, indolyl, quinazolinyl, oxazolinyl, isoxazolinyl, indazolinyl, isothiazolyl, 1, 3-benzodioxolyl and the like. Preferred examples of "heteroaryl" include pyridinyl, isothiazolyl, thiazolyl, pyrazolyl, thienyl, prolyl, isoxazolinyl and 1,3-benzodioxolyl.

The term "C₃₋₆ cycloalkyl" refers to a saturated alkyl ring system containing 3, 4, 5 or 6 carbon atoms, such as cyclopropyl, cyclobutyl, cyclopentyl, cyclohexyl.

The term "4-8 membered heterocycloalkyl" refers to a ring system having 4, 5, 6, 7 or 8 ring atoms selected from C, N, O, or S, preferably C, N, or O, the number of N atoms being 0, 1, 2 and the number of O and S atoms each being 0, 1, 2. The term "4-8 membered heterocycloalkyl" preferably comprises fully saturated monocycles, fused bicycles, bridged bicycles or spirobicycles. Examples of 5-6 membered heterocycloalkyl groups include pyrrolidinyl, tetrahydrofuranyl, tetrahydrothiopyranyl, dihydropyranyl, tetrahydropyranyl, 1,3-dioxolanyl, 1,4-dioxanyl, 1,4-oxathianyl 1,4-dithianyl, 1,3-dioxane, 1,3-dithianyl, piperazinyl, thiomorpholinyl, piperidinyl, and morpholinyl. Preferred 4-8 membered heterocycloalkyl include 5-membered heterocycloalkyl having 1 or 2 N-atoms, such as pyrrolidinyl, 6-membered heterocycloalkyl having N and O-atoms, such as morpholinyl, piperidinyl, piperazyinyl , dioxanyl, 7-membered heterocycloalkyl having N and O-atoms, such as 1 N- and 1 O-atom, such as 2-oxa-5-azabicyclo[2.2.1]heptan-5-yl, 1,4-diazabicyclo[3.2.1]octan-4-yl, 3-methyl-3-azabicyclo[3.1.0]hexan-1-yl; 8-membered heterocycloalkyl having N and O-atoms, such as 1 N- and 1 O-atom, such as 8-oxa-3-azabicyclo[3.2.1]octan-3-yl. The term "C₁₋₄ alkyl 4-8 membered heterocycloalkyl" refers to an alkyl as defined below with 1 to 4 carbon atoms, which is bound to a 4-8 membered heterocycloalkyl as defined above. Preferably, the C₁₋₄ alkyl may be C₁, resulting in -(CH₂)-(4-8 membered heterocycloalkyl) or C₂, resulting in -(CH₂)₂-(4-8 membered heterocycloalkyl) or C₃, resulting in -(CH₂)₃-(4-8 membered heterocycloalkyl). Examples include - (CH₂)-morpholinyl, -(CH₂)₂-morpholinyl, -(CH₂)₃-morpholinyl, -(CH₂)₄-morpholinyl, - (CH₂)-piperazinyl, -(CH₂)₂-N-methyl-piperazinyl, -(CH₂)₃-piperazinyl or -(CH₂)₄-piperazinyl. The term "C₁₋₄ alkoxy 4-8 membered heterocycloalkyl" refers to a 4-7 membered heterocycloalkyl as described above, which is linked via a C₁₋₄ alkoxy group to its neighbouring group. Preferably, the
C₁₋₄ alkoxy may be C₁, resulting in -(O-CH₂)-(4-8 membered heterocycloalkyl) or C₂, resulting in - (O-CH₂)₂-(4-8 membered heterocycloalkyl) or C₃, resulting in -(O-CH₂)₃-(4-8 membered heterocycloalkyl). Examples include -(O-CH₂)-(N-morpholinyl), -(O-CH₂)₂-(N-morpholinyl). The term "-O-(4-8 membered heterocycloalkyl)" refers to a 4-8 membered heterocycloalkyl as described above, which is linked via a -O-group to its neighbouring group. Examples include -O-morpholinyl, -O-piperazinyl, and -O-pyrrolidinyl. The term "-O(CO)-C₁₋₄ alkyl 4-7 membered heterocycloalkyl" refers to a 4-8 membered heterocycloalkyl as described above, which is linked via a -O(CO)-C₁₋₄ alkyl group to its neighbouring group. Preferably, the "-O(CO)-C₁₋₄ alkyl may be C₁, resulting in -(O(CO)-CH₂)-(4-8 membered heterocycloalkyl) or C₂, resulting in -(O(CO)-CH₂)₂-(4-8 membered heterocycloalkyl) or C₃, resulting in -(O(CO)-CH₂)₃-(4-8 membered heterocycloalkyl). Examples include -(O(CO)-CH₂)-(N-morpholinyl) or -(O(CO)-CH₂-CH₂)-(N-morpholinyl). The term "halogen" or "hal" as used herein may be fluoro, chloro, bromo or iodo preferably fluoro, chloro or bromo, more preferably fluoro or chloro.

The term "alkyl" as used herein refers to a fully saturated branched or unbranched hydrocarbon moiety. The terms "C₁₋₄alkyl" and "C₁₋₆alkyl" refer to a fully saturated branched or unbranched hydrocarbon moiety having 1, 2, 3 or 4 and 1, 2, 3, 4, 5 or 6 carbon atoms, respectively. Representative examples of alkyl include, but are not limited to, methyl, ethyl, n-propyl, isopropyl, n-butyl, sec-butyl, iso-butyl, tert-butyl, n-pentyl, iso-pentyl, neopentyl, n-hexyl, iso-hexyl or neohexyl.

The term "C₁₋₄ alkoxy" refers to an unsubstituted or substituted alkyl chain linked to the remainder of the molecule through an oxygen atom, and in particular to methoxy, ethoxy, n-propoxy, iso-propoxy, n-butoxy, iso-butoxy, and t-butoxy.

The term "C₁₋₄ alkyl-C₁₋₄ alkoxy" refers to a C₁₋₄ alkyl group functionalized with a C₁₋₄ alkoxy group, such as e.g. -CH₂-O-CH₃, -(CH₂)₂-O-CH₃, -(CH₂)₃-O-CH₃, -(CH₂)₄-O-CH₃, -CH₂-O- CH₂-CH₃, -CH₂-O-(CH₂)₂-CH₃, -CH₂-O-(CH₂)₃-CH₃, and branched isomers thereof.

The term "C₁₋₆ heteroalkyl" refers to an alkyl as defined below with 1, 2, 3, 4, 5 or 6 carbon atoms in which at least one, or at least two, carbon atoms are substituted with a heteroatom, such as N, O, S, preferably N, O. It is understood that the heteroatom may further be substituted with one or two C₁₋₆ alkyl. The skilled person also knows that the term "substituted" includes substitutions at one or more C-atoms (e.g. - CH₂-CH₂-) with a heteroatom (e.g. - CH₂-CH(Het)-) and/or substitutions of one or more C-atoms (e.g. -CH₂- CH₂-CH₂) within the alkyl chain (to obtain e.g. - CH₂-Het-CH₂-) Examples include -(CH₂)₂-O-Me, -(CH₂)₃-O-Me, -(CH)(OMe)(CH₃), -(CH₂)₂-O-CH₂Me, -(CH₂)₂-NMe₂,-(CH₂)-NMe₂, -(CH₂)₂-NEt₂,-(CH₂)-NEt₂, and -O-(CH₂)₃-NMe₂.

The term "C₁₋₄alkylamino" refers to a fully saturated branched or unbranched C₁₋₄ alkyl, which is substituted with at least one, preferably only one, amino group, alkylamino group or dialkylaminogroup, such as NH₂, HN(C₁₋₄alkyl) or N(C₁₋₄alkyl)₂. Thus, a C₁₋₄alkylamino refers to C₁₋₄alkylamino, C₁₋₄alkyl-(C₁₋₄alkyl)amino, C₁₋₄alkyl-(C₁₋₄dialkyl)amino. Examples include but are not limited to dimethylamino, methylaminomethyl, dimethylamonimethyl, aminomethyl, dimethylaminoethyl, aminoethyl, methylaminoethyl, n-propylamino, iso-propylamino, n-butylamino, sec-butylamino, iso-butylamino, tert-butylamino.

Based on the definitions given throughout the application the skilled person knows which combinations are synthetically feasible and realistic, e.g. typically combinations of groups leading to heteroatoms directly linked to each other are not contemplated.

In a first aspect directed towards a compound or pharmaceutically acceptable salts or stereoisomers thereof of formula I: 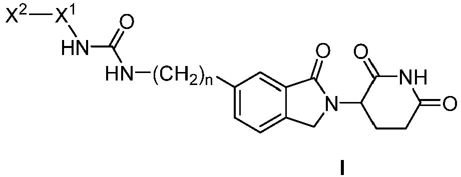 wherein
- X¹: is linear or branched C₁₋₆ alkyl, C₃₋₆ cycloalkyl, -C₁₋₆ alkyl C₃₋₆ cycloalkyl, C₆₋₁₀ aryl, 5-10 membered heteroaryl, C₁₋₆ alkyl C₆₋₁₀ aryl, C₁₋₆ alkyl 5-10 membered heteroaryl, wherein X¹ is unsubstituted or substituted with one or more of halogen, linear or branched C₁₋₆ alkyl, linear or branched C₁₋₆ heteroalkyl, CF₃, CHF₂, -O-CHF₂, -O-(CH₂)₂-OMe, OCF₃, C₁₋₆ alkylamino, -CN, -N(H)C(O)-C₁₋₆alkyl, -OC(O)-C₁₋₆alkyl, -OC(O)-C₁₋₄alkylamino, -C(O)O-C₁₋₆alkyl, -COOH, - CHO, -C₁₋₆alkylC(O)OH, -C₁₋₆alkylC(O)O-C₁₋₆alkyl, NH₂, C₁₋₆ alkoxy or C₁₋₆ alkylhydroxy;
- X²: is hydrogen, C₆₋₁₀ aryl, 5-10 membered heteroaryl, -0-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein X² is unsubstituted or substituted with one or more of linear or branched C₁₋₆ alkyl, NH₂, NMe₂ or 5-6 membered heterocycloalkyl;
- n: is 0, 1 or 2.

In a preferred embodiment of a compound of formula I, n is 1 or 2.

In further embodiments of a compound of formula I, n is 1.

In further embodiments of a compound of formula I, n is 2.

In yet specific embodiments of a compound of formula I, X¹ is linear or branched C₁₋₆ alkyl, C₃₋₆ cycloalkyl, -C₁₋₆ alkyl C₃₋₆ cycloalkyl, C₆ aryl, 5-10 membered heteroaryl, C₁₋₆ alkyl C₆₋₁₀ aryl, C₁₋₆ alkyl 5-10 membered heteroaryl, wherein X¹ is unsubstituted or substituted with one or more of halogen, linear or branched C₁₋₆ alkyl, linear or branched C₁₋₆ heteroalkyl, CF₃, CHF₂, -O-CHF₂, -O-(CH₂)₂-OMe, OCF₃, C₁₋₆ alkylamino, -CN, -N(H)C(O)-C₁₋₆alkyl, -OC(O)-C₁₋₆alkyl, -OC(O)-C₁₋₄alkylamino, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; and X² is hydrogen, C₆₋₁₀ aryl, 5-10 membered heteroaryl, -O-(5-10 membered heteroaryl), 4-8 membered heterocycloalkyl, C₁₋₆ alkyl 4-8 membered heterocycloalkyl, -O-(4-8 membered heterocycloalkyl), -O-C₁₋₆ alkyl-(4-8 membered heterocycloalkyl), -OC(O)-C₁₋₆alkyl-4-8 membered heterocycloalkyl or C₆ aryloxy, wherein X² is unsubstituted or substituted with one or more of linear or branched C₁₋₆ alkyl, NH₂, NMe₂ or 5-6 membered heterocycloalkyl; and wherein n is 0, 1, or 2.

In more specific embodiments of a compound of formula I, X¹ is linear or branched C₁₋₆ alkyl, C₃₋₆ cycloalkyl, -C₁₋₆ alkyl C₃₋₆ cycloalkyl, C₆₋₁₀ aryl, 5-10 membered heteroaryl, C₁₋₆ alkyl C₆ aryl, wherein X¹ is unsubstituted or substituted with one or more of halogen, linear or branched C₁₋₆ alkyl, linear or branched C₁₋₆ heteroalkyl, CF₃, CHF₂, -O-CHF₂, -O-(CH₂)₂-OMe, OCF₃, -CN, -OC(O)-C₁₋₆alkylamino, -CHO, -C₁₋₆alkylC(O)OH, NH₂, C₁₋₆ alkoxy or C₁₋₆ alkylhydroxy; and X² is hydrogen, C₆₋₁₀ aryl, 5-10 membered heteroaryl, -O-(5-10 membered heteroaryl), 4-8 membered heterocycloalkyl, C₁₋₆ alkyl 4-8 membered heterocycloalkyl, -O-(4-8 membered heterocycloalkyl), -O-C₁₋₆ alkyl-(4-8 membered heterocycloalkyl) or -OC(O)-C₁₋₆alkyl-4-8 membered heterocycloalkyl, wherein X² is unsubstituted or substituted with one or more of linear or branched C₁₋₆ alkyl, NH₂, NMe₂ or 5-6 membered heterocycloalkyl; and wherein n is 0, 1, or 2.

In yet specific embodiments of a compound of formula I, X¹ is linear or branched C₁₋₄ alkyl, C₃₋₆ cycloalkyl, -C₁₋₄ alkyl C₃₋₆ cycloalkyl, C₆ aryl, 5-10 membered heteroaryl, C₁₋₆ alkyl C₆ aryl, C₁₋₆ alkyl 5-10 membered heteroaryl, wherein X¹ is unsubstituted or substituted with one or more of halogen, linear or branched C₁₋₄ alkyl, linear or branched C₁₋₆ heteroalkyl, CF₃, CHF₂, -O-CHF₂, -O-(CH₂)₂-OMe, OCF₃, C₁₋₆ alkylamino, -CN, -N(H)C(O)-C₁₋₆alkyl, -OC(O)-C₁₋₆alkyl, -OC(O)-C₁₋₄alkylamino, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; and X² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein X² is unsubstituted or substituted with one or more of linear or branched C₁₋₆ alkyl, NH₂, NMe₂ or 5-6 membered heterocycloalkyl; and wherein n is 0, 1, or 2.

In more specific embodiments of a compound of formula I, X¹ is linear or branched C₁₋₄ alkyl, C₃₋₆ cycloalkyl, -C₁₋₄ alkyl C₃₋₆ cycloalkyl, C₆ aryl, 5-10 membered heteroaryl, C₁₋₆ alkyl C₆ aryl, wherein X¹ is unsubstituted or substituted with one or more of halogen, linear or branched C₁₋₄ alkyl, linear or branched C₁₋₆ heteroalkyl, CF₃, CHF₂, -O-CHF₂, -O-(CH₂)₂-OMe, OCF₃, -CN, -OC(O)-C₁₋₄alkylamino, -CHO, -C₁₋₆alkylC(O)OH, NH₂, C₁₋₆ alkoxy or C₁₋₆ alkylhydroxy; and X² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl) or -OC(O)-C₁₋₄alkyl-4-8 membered heterocycloalkyl, wherein X² is unsubstituted or substituted with one or more of linear or branched C₁₋₆ alkyl, NH₂, NMe₂ or 5-6 membered heterocycloalkyl; and wherein n is 0, 1, or 2.

In yet specific embodiments of a compound of formula I, X¹ is linear or branched C₁₋₆ alkyl, C₃₋₆ cycloalkyl, -C₁₋₆ alkyl C₃₋₆ cycloalkyl, C₆ aryl, 5-10 membered heteroaryl, C₁₋₆ alkyl C₆₋₁₀ aryl, C₁₋₆ alkyl 5-10 membered heteroaryl, wherein X¹ is unsubstituted or substituted with one or more of halogen, linear or branched C₁₋₆ alkyl, linear or branched C₁₋₆ heteroalkyl, CF₃, CHF₂, -O-CHF₂, -O-(CH₂)₂-OMe, OCF₃, C₁₋₆ alkylamino, -CN, -N(H)C(O)-C₁₋₆alkyl, -OC(O)-C₁₋₆alkyl, -OC(O)-C₁₋₄alkylamino, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; and X² is hydrogen, C₆₋₁₀ aryl, 5-10 membered heteroaryl, -O-(5-10 membered heteroaryl), 4-8 membered heterocycloalkyl, C₁₋₆ alkyl 4-8 membered heterocycloalkyl, -O-(4-8 membered heterocycloalkyl), -O-C₁₋₆ alkyl-(4-8 membered heterocycloalkyl), -OC(O)-C₁₋₆alkyl-4-8 membered heterocycloalkyl or C₆ aryloxy, wherein X² is unsubstituted or substituted with one or more of linear or branched C₁₋₆ alkyl, NH₂, NMe₂ or 5-6 membered heterocycloalkyl; and wherein n is 1.

In more specific embodiments of a compound of formula I, X¹ is linear or branched C₁₋₆ alkyl, C₃₋₆ cycloalkyl, -C₁₋₆ alkyl C₃₋₆ cycloalkyl, C₆₋₁₀ aryl, 5-10 membered heteroaryl, C₁₋₆ alkyl C₆ aryl, wherein X¹ is unsubstituted or substituted with one or more of halogen, linear or branched C₁₋₆ alkyl, linear or branched C₁₋₆ heteroalkyl, CF₃, CHF₂, -O-CHF₂, -O-(CH₂)₂-OMe, OCF₃, -CN, -OC(O)-C₁₋₆alkylamino, -CHO, -C₁₋₆alkylC(O)OH, NH₂, C₁₋₆ alkoxy or C₁₋₆ alkylhydroxy; and X² is hydrogen, C₆₋₁₀ aryl, 5-10 membered heteroaryl, -O-(5-10 membered heteroaryl), 4-8 membered heterocycloalkyl, C₁₋₆ alkyl 4-8 membered heterocycloalkyl, -O-(4-8 membered heterocycloalkyl), -O-C₁₋₆ alkyl-(4-8 membered heterocycloalkyl) or -OC(O)-C₁₋₆alkyl-4-8 membered heterocycloalkyl, wherein X² is unsubstituted or substituted with one or more of linear or branched C₆ alkyl, NH₂, NMe₂ or 5-6 membered heterocycloalkyl; and wherein n is 1.

In yet specific embodiments of a compound of formula I, X¹ is linear or branched C₁₋₄ alkyl, C₃₋₆ cycloalkyl, -C₁₋₄ alkyl C₃₋₆ cycloalkyl, C₆ aryl, 5-10 membered heteroaryl, C₁₋₆ alkyl C₆ aryl, C₁₋₆ alkyl 5-10 membered heteroaryl, wherein X¹ is unsubstituted or substituted with one or more of halogen, linear or branched C₁₋₄ alkyl, linear or branched C₁₋₆ heteroalkyl, CF₃, CHF₂, -O-CHF₂, -O-(CH₂)₂-OMe, OCF₃, C₁₋₆ alkylamino, -CN, -N(H)C(O)-C₁₋₆alkyl, -OC(O)-C₁₋₆alkyl, -OC(O)-C₁₋₄alkylamino, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; and X² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein X² is unsubstituted or substituted with one or more of linear or branched C₁₋₆ alkyl, NH₂, NMe₂ or 5-6 membered heterocycloalkyl; and wherein n is 1.

In more specific embodiments of a compound of formula I, X¹ is linear or branched C₁₋₄ alkyl, C₃₋₆ cycloalkyl, -C₁₋₄ alkyl C₃₋₆ cycloalkyl, C₆ aryl, 5-10 membered heteroaryl, C₁₋₆ alkyl C₆ aryl, wherein X¹ is unsubstituted or substituted with one or more of halogen, linear or branched C₁₋₄ alkyl, linear or branched C₁₋₆ heteroalkyl, CF₃, CHF₂, -O-CHF₂, -O-(CH₂)₂-OMe, OCF₃, -CN, -OC(O)-C₁₋₄alkylamino, -CHO, -C₁₋₆alkylC(O)OH, NH₂, C₁₋₆ alkoxy or C₁₋₆ alkylhydroxy; and X² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl) or -OC(O)-C₁₋₄alkyl-4-8 membered heterocycloalkyl, wherein X² is unsubstituted or substituted with one or more of linear or branched C₁₋₆ alkyl, NH₂, NMe₂ or 5-6 membered heterocycloalkyl; and wherein n is 1.

In yet more specific embodiments the compounds of formula I are compounds or pharmaceutically acceptable salts or stereoisomers thereof of formula I': 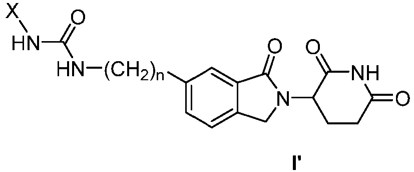 wherein
- X: is linear or branched C₁₋₆ alkyl, C₃₋₆ cycloalkyl, C₁₋₆ alkyl C₃₋₆ cycloalkyl, C₆₋₁₀ aryl, 5-10 membered heteroaryl, C₁₋₆ alkylaryl, C₁₋₆ alkyl 5-10 membered heteroaryl, wherein X is unsubsituted or substituted with one or more of halogen, linear or branched C₁₋₆ alkyl, C₆₋₁₀ aryl, 5-10 membered heteroaryl, C₁₋₆ heteroalkyl, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, -OC(O)-C₁₋₆alkyl, -N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₆ alkoxy, C₁₋₆ alkylhydroxy or C₆ aryloxy;
- n: is 0, 1 or 2.

In a preferred embodiment of a compound of formula I', n is 1 or 2, more preferably n is 1.

In further embodiments of a compound of formula I', n is 1.

In yet specific embodiments of a compound of formula I', X is linear or branched C₁₋₆ alkyl, C₃₋₆ cycloalkyl, -C₁₋₆ alkyl C₃₋₆ cycloalkyl, C₆₋₁₀ aryl, wherein X is unsubsituted or substituted with one or more of halogen, in particular F, Cl, Br, linear or branched C₁₋₆ alkyl, C₆₋₁₀ aryl, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₆ alkylhydroxy,C₁₋₆ alkoxy or C₁₋₆ hydroxy.

In yet specific embodiments of a compound of formula I', X is linear or branched C₁₋₆ alkyl, C₃₋₆ cycloalkyl, -C₁₋₆ alkyl C₃₋₆ cycloalkyl, C₆₋₁₀ aryl, wherein X is unsubstituted or substituted with one or more of halogen, in particular F, Cl, Br, linear or branched C₁₋₆ alkyl, C₆₋₁₀ aryl, CF₃, OCF₃, C₁₋₆ alkylamino, -CN, C₁₋₆ alkoxy.

In further embodiments of a compound of formula I', n is 1 and X is linear or branched C₁₋₆ alkyl, C₃₋₆ cycloalkyl, -C₁₋₆ alkyl C₃₋₆ cycloalkyl, C₆₋₁₀ aryl, wherein X is unsubstituted or substituted with one or more of halogen, in particular F, Cl, Br, linear or branched C₁₋₆ alkyl, C₆₋₁₀ aryl, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, - C(O)O-C₁₋₆alkyl, -COOH, -C₁₋₆alkylC(O)OH, -C₁₋₆alkylC(O)O-C₁₋₆alkyl, NH₂, C₁₋₆ alkylhydroxy,C₁₋₆ alkoxy.

In further embodiments of a compound of formula I', n is 1 and X is linear or branched C₁₋₆ alkyl, C₃₋₆ cycloalkyl, -C₁₋₆ alkyl C₃₋₆ cycloalkyl, C₆₋₁₀ aryl, wherein X is unsubstituted or substituted with one or more of halogen, in particular F, Cl, Br, linear or branched C₁₋₆ alkyl, C₆₋₁₀ aryl, CF₃, OCF₃, C₁₋₆ alkylamino, -CN, C₁₋₆ alkoxy.

In further specific embodiments of a compound of formula I', X is linear or branched C₁₋₄ alkyl, C₃₋₄ cycloalkyl, C₁₋₄ alkyl C₃₋₆ cycloalkyl, C₆ aryl, 5-10 membered heteroaryl, C₁₋₆ alkylaryl, C₁₋₆ alkyl 5-10 membered heteroaryl, wherein X is optionally substituted with one or more of halogen, in particular F, Cl, Br, linear or branched C₁₋₄ alkyl, C₆ aryl, 5-10 membered heteroaryl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, - C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy and n is 0, 1 or 2.

In further specific embodiments of a compound of formula I', X is linear or branched C₁₋₄ alkyl, C₃₋₄ cycloalkyl, C₁₋₄ alkyl C₃₋₆ cycloalkyl, C₆ aryl, 5-10 membered heteroaryl, C₁₋₆ alkylaryl, C₁₋₆ alkyl 5-10 membered heteroaryl, wherein X is optionally substituted with one or more of halogen, in particular F, Cl, Br, linear or branched C₁₋₄ alkyl, C₆ aryl, 5-10 membered heteroaryl, CF₃, OCF₃, C₁₋₄ alkylamino, -CN, C₁₋₄ alkoxy and n is 0, 1 or 2.

In further specific embodiments of a compound of formula I', X is linear or branched C₁₋₄ alkyl, C₃₋₄ cycloalkyl, C₁₋₄ alkyl C₃₋₆ cycloalkyl, C₆ aryl, 5-10 membered heteroaryl, C₁₋₆ alkylaryl, C₁₋₆ alkyl 5-10 membered heteroaryl, wherein X is optionally substituted with one or more of halogen, in particular F, Cl, Br, linear or branched C₁₋₄ alkyl, C₆ aryl, 5-10 membered heteroaryl, morpholinyl, -(CH₂)-morpholinyl, piperazinyl, -(CH₂)-piperazinyl, CF₃, CHF₂, -O-(CH₂)₂-OMe, OCF₃, C₁₋₆ alkylamino, -CN, -OC(O)-C₁₋₆alkyl, -N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy and n is 0, 1 or 2.

In certain specific embodiments of a compound of formula I', X is linear or branched C₁₋₄ alkyl, C₃₋₄ cycloalkyl, C₁₋₄ alkyl C₃₋₆ cycloalkyl, C₆ aryl, 5-10 membered heteroaryl, C₁₋₆ alkylaryl, C₁₋₆ alkyl 5-10 membered heteroaryl, wherein X is optionally substituted with one or more of halogen, in particular F, Cl, Br, linear or branched C₁₋₄ alkyl, C₆ aryl, 5-10 membered heteroaryl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, - C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy,C₁₋₄ alkoxy and n is 1.

In certain specific embodiments of a compound of formula I', X is linear or branched C₁₋₄ alkyl, C₃₋₄ cycloalkyl, C₁₋₄ alkyl C₃₋₆ cycloalkyl, C₆ aryl, 5-10 membered heteroaryl, C₁₋₆ alkylaryl, C₁₋₆ alkyl 5-10 membered heteroaryl, wherein X is optionally substituted with one or more of halogen, in particular F, Cl, Br, linear or branched C₁₋₄ alkyl, C₆ aryl, 5-10 membered heteroaryl, morpholinyl, -(CH₂)-morpholinyl, piperazinyl, -(CH₂)-piperazinyl, CF₃, CHF₂, -O-(CH₂)₂-OMe, OCF₃, C₁₋₆ alkylamino, -CN, -OC(O)-C₁₋₆alkyl, -N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy,C₁₋₄ alkoxy and n is 1

In certain specific embodiments of a compound of formula I', X is linear or branched C₁₋₄ alkyl, C₃₋₄ cycloalkyl, C₁₋₄ alkyl C₃₋₆ cycloalkyl, C₆ aryl, 5-10 membered heteroaryl, C₁₋₆ alkylaryl, C₁₋₆ alkyl 5-10 membered heteroaryl, wherein X is optionally substituted with one or more of halogen, in particular F, Cl, Br, linear or branched C₁₋₄ alkyl, C₆ aryl, 5-10 membered heteroaryl, CF₃, OCF₃, C₁₋₄ alkylamino, -CN, C₁₋₄ alkoxy and n is 1.

In other embodiments of a compound of formula I', X is linear or branched C₁₋₄ alkyl, C₃₋₄ cycloalkyl, C₁₋₄ alkyl C₃₋₆ cycloalkyl, C₆ aryl, wherein X is optionally substituted with one or more of halogen, in particular F, Cl, Br, linear or branched C₁₋₄ alkyl, C₆ aryl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, - COOH, -C₁₋₆alkylC(O)OH, -C₁₋₆alkylC(O)O-C₁₋₆alkyl, NH₂, C₁₋₄ alkylhydroxy, C₁₋₄ alkoxy and n is 1.

In other embodiments of a compound of formula I', X is linear or branched C₁₋₄ alkyl, C₃₋₄ cycloalkyl, C₁₋₄ alkyl C₃₋₆ cycloalkyl, C₆ aryl, wherein X is optionally substituted with one or more of halogen, in particular F, Cl, Br, linear or branched C₁₋₄ alkyl, C₆ aryl, morpholinyl, - (CH₂)-morpholinyl, piperazinyl, -(CH₂)-piperazinyl, CF₃, CHF₂, -O-(CH₂)₂-OMe, OCF₃, C₁₋₆ alkylamino, -CN, -OC(O)-C₁₋₆alkyl, -N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy and n is 1.

In other embodiments of a compound of formula I', X is linear or branched C₁₋₄ alkyl, C₃₋₄ cycloalkyl, C₁₋₄ alkyl C₃₋₆ cycloalkyl, C₆ aryl, wherein X is optionally substituted with one or more of halogen, in particular F, Cl, Br, linear or branched C₁₋₄ alkyl, C₆ aryl, CF₃, OCF₃, C₁₋₄ alkylamino, -CN, C₁₋₄ alkoxy and n is 1.

More specifically, the present disclosure is directed towards a compound or pharmaceutically acceptable salts or stereoisomers thereof of formula II 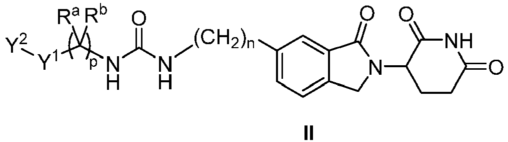 wherein
- n: is 1 or 2;
- p: is 0, 1, 2, 3, 4, 5, 6;
- Y¹: is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, -N(H)C(O)-C₁₋₆alkyl, -OC(O)-C₁₋₆alkyl, - OC(O)-C₁₋₄heteroalkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; with the proviso that when p is 0, Y¹ is not hydrogen;
- Y²: is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y² is unsubstituted or substituted with one or more of linear or branched C₁₋₆ alkyl, NH₂, NMe₂ or 5-6 membered heterocycloalkyl.

R^{a}, R^{b} each are independently selected from hydrogen and linear or branched C₁₋₄ alkyl, preferably hydrogen or methyl.

It is understood for compounds of formula II that if Y¹ is hydrogen, Y² is absent.

In some specific embodiments of the compound of formula II, n is 1.

In further specific embodiments of the compound of formula II, p is 0, 1 or 2.

In some specific embodiments of the compound of formula II, p is 0 or 1.

In other embodiments of the compound of formula II, n is 1 and p is 0, 1 or 2.

In some specific embodiments of the compound of formula II, n is 1 and p is 0 or 1.

In yet specific embodiments of a compound of formula II, Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, -C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y² is unsubstituted or substituted with one or more of linear or branched C₁₋₆ alkyl, NH₂, NMe₂ or 5-6 membered heterocycloalkyl.

In more specific embodiments, of the compound of formula II, n is 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, -N(H)C(O)-C₁₋₆alkyl, -OC(O)-C₁₋₆alkyl, -OC(O)-C₁₋₄heteroalkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y² is unsubstituted or substituted with one or more of linear or branched C₁₋₆ alkyl, NH₂, NMe₂ or 5-6 membered heterocycloalkyl.

In more specific embodiments, of the compound of formula II, n is 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, - C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y² is unsubstituted or substituted with one or more of linear or branched C₁₋₆ alkyl, NH₂, NMe₂ or 5-6 membered heterocycloalkyl.

In more specific embodiments, of the compound of formula II, p is 0, 1, or 2, and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, - N(H)C(O)-C₁₋₆alkyl, -OC(O)-C₁₋₆alkyl, -OC(O)-C₁₋₄heteroalkyl, -C(O)O-C₁₋₆alkyl, -COOH, - CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y² is unsubstituted or substituted with one or more of linear or branched C₁₋₆ alkyl, NH₂, NMe₂ or 5-6 membered heterocycloalkyl.

In more specific embodiments, of the compound of formula II, p is 0, 1, 2 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, -C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y²is unsubstituted or substituted with one or more of linear or branched C₁₋₆ alkyl, NH₂, NMe₂ or 5-6 membered heterocycloalkyl.

In more specific embodiments, of the compound of formula II, p is 0 or 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, - N(H)C(O)-C₁₋₆alkyl, -OC(O)-C₁₋₆alkyl, -OC(O)-C₁₋₄heteroalkyl, -C(O)O-C₁₋₆alkyl, -COOH, - CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y²is unsubstituted or substituted with one or more of linear or branched C₁₋₆ alkyl, NH₂, NMe₂ or 5-6 membered heterocycloalkyl.

In more specific embodiments, of the compound of formula II, n is 1, p is 0, 1, or 2, and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, - N(H)C(O)-C₁₋₆alkyl, -OC(O)-C₁₋₆alkyl, -OC(O)-C₁₋₄heteroalkyl, -C(O)O-C₁₋₆alkyl, -COOH, - CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y² is unsubstituted or substituted with one or more of linear or branched C₁₋₆ alkyl, NH₂, NMe₂ or 5-6 membered heterocycloalkyl.

In more specific embodiments, of the compound of formula II, n is 1, p is 0, 1, 2 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, -C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y² is unsubstituted or substituted with one or more of linear or branched C₁₋₆ alkyl, NH₂, NMe₂ or 5-6 membered heterocycloalkyl.

In more specific embodiments, of the compound of formula II, n is 1, p is 0 or 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, - N(H)C(O)-C₁₋₆alkyl, -OC(O)-C₁₋₆alkyl, -OC(O)-C₁₋₄heteroalkyl, -C(O)O-C₁₋₆alkyl, -COOH, - CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y²is unsubstituted or substituted with one or more of linear or branched C₁₋₆ alkyl, NH₂, NMe₂ or 5-6 membered heterocycloalkyl.

In more specific embodiments, of the compound of formula II, n is 1, p is 0 or 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, -C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y²is unsubstituted or substituted with one or more of linear or branched C₁₋₆ alkyl, NH₂, NMe₂ or 5-6 membered heterocycloalkyl.

In some other specific embodiments of the compound of formula II, R^{a} and R^{b} each are independently selected from hydrogen, methyl and ethyl.

In some embodiments of the compound of formula II only a single R^{a} and a single R^{b} is C₁₋₄ alkyl, preferably methyl or ethyl and all other R^{a} and R^{b} are hydrogen.

In more specific embodiments, of the compound of formula II, and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, -N(H)C(O)-C₁₋₆alkyl, - OC(O)-C₁₋₆alkyl, -OC(O)-C₁₋₄heteroalkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y²is unsubstituted or substituted with one or more of linear or branched C₁₋₆ alkyl, NH₂, NMe₂ or 5-6 membered heterocycloalkyl; and R^{a} and R^{b} each are independently selected from hydrogen, methyl and ethyl.

In more specific embodiments, of the compound of formula II, and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, -C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y²is unsubstituted or substituted with one or more of linear or branched C₁₋₆ alkyl, NH₂, NMe₂ or 5-6 membered heterocycloalkyl; R^{a} and R^{b} each are independently selected from hydrogen, methyl and ethyl.

In more specific embodiments, of the compound of formula II, n is 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, -N(H)C(O)-C₁₋₆alkyl, -OC(O)-C₁₋₆alkyl, -OC(O)-C₁₋₄heteroalkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y²is unsubstituted or substituted with one or more of linear or branched C₁₋₆ alkyl, NH₂, NMe₂ or 5-6 membered heterocycloalkyl; and R^{a} and R^{b} each are independently selected from hydrogen, methyl and ethyl.

In more specific embodiments, of the compound of formula II, n is 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, - C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y²is unsubstituted or substituted with one or more of linear or branched C₁₋₆ alkyl, NH₂, NMe₂ or 5-6 membered heterocycloalkyl; R^{a} and R^{b} each are independently selected from hydrogen, methyl and ethyl.

In more specific embodiments, of the compound of formula II, p is 0, 1, 2 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, - N(H)C(O)-C₁₋₆alkyl, -OC(O)-C₁₋₆alkyl, -OC(O)-C₁₋₄heteroalkyl, -C(O)O-C₁₋₆alkyl, -COOH, - CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -0-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y²is unsubstituted or substituted with one or more of linear or branched C₁₋₆ alkyl, NH₂, NMe₂ or 5-6 membered heterocycloalkyl; and R^{a} and R^{b} each are independently selected from hydrogen, methyl and ethyl.

In more specific embodiments, of the compound of formula II, p is 0, 1, 2 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, -C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y²is unsubstituted or substituted with one or more of linear or branched C₁₋₆ alkyl, NH₂, NMe₂ or 5-6 membered heterocycloalkyl; R^{a} and R^{b} each are independently selected from hydrogen, methyl and ethyl.

In more specific embodiments, of the compound of formula II, p is 0 or 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, - N(H)C(O)-C₁₋₆alkyl, -OC(O)-C₁₋₆alkyl, -OC(O)-C₁₋₄heteroalkyl, -C(O)O-C₁₋₆alkyl, -COOH, - CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y² is unsubstituted or substituted with one or more of linear or branched C₁₋₆ alkyl, NH₂, NMe₂ or 5-6 membered heterocycloalkyl; and R^{a} and R^{b} each are independently selected from hydrogen, methyl and ethyl.

In more specific embodiments, of the compound of formula II, p is 0 or 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, -C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y²is unsubstituted or substituted with one or more of linear or branched C₁₋₆ alkyl, NH₂, NMe₂ or 5-6 membered heterocycloalkyl; R^{a} and R^{b} each are independently selected from hydrogen, methyl and ethyl.

In more specific embodiments, of the compound of formula II, n is 1, p is 0, 1, 2 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, - N(H)C(O)-C₁₋₆alkyl, -OC(O)-C₁₋₆alkyl, -OC(O)-C₁₋₄heteroalkyl, -C(O)O-C₁₋₆alkyl, -COOH, - CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y²is unsubstituted or substituted with one or more of linear or branched C₁₋₆ alkyl, NH₂, NMe₂ or 5-6 membered heterocycloalkyl; and R^{a} and R^{b} each are independently selected from hydrogen, methyl and ethyl.

In more specific embodiments, of the compound of formula II, n is 1, p is 0, 1, 2 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, -C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y²is unsubstituted or substituted with one or more of linear or branched C₁₋₆ alkyl, NH₂, NMe₂ or 5-6 membered heterocycloalkyl; R^{a} and R^{b} each are independently selected from hydrogen, methyl and ethyl.

In more specific embodiments, of the compound of formula II, n is 1, p is 0 or 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, - N(H)C(O)-C₁₋₆alkyl, -OC(O)-C₁₋₆alkyl, -OC(O)-C₁₋₄heteroalkyl, -C(O)O-C₁₋₆alkyl, -COOH, - CHO, -C₁₋₆alkylC(O)OH, -C₁₋₆alkylC(O)O-C₁₋₆alkyl, NH₂, C₁₋₆ alkoxy or C₁₋₆ alkylhydroxy; and Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y² is unsubstituted or substituted with one or more of linear or branched C₁₋₆ alkyl, NH₂, NMe₂ or 5-6 membered heterocycloalkyl; and R^{a} and R^{b} each are independently selected from hydrogen, methyl and ethyl.

In more specific embodiments, of the compound of formula II, n is 1, p is 0 or 1 and Y¹ is hydrogen, is C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, OCF₃, -CN, -OC(O)-C₁₋₄heteroalkyl, -CHO, -C₁₋₆alkylC(O)OH, NH₂, or C₁₋₆ alkylhydroxy; and Y² is hydrogen C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl, wherein Y²is unsubstituted or substituted with one or more of linear or branched C₁₋₆ alkyl, NH₂, NMe₂ or 5-6 membered heterocycloalkyl; R^{a} and R^{b} each are independently selected from hydrogen, methyl and ethyl.

In more specific embodiments, the present disclosure is directed towards a compound or pharmaceutically acceptable salts or stereoisomers thereof of formula II' 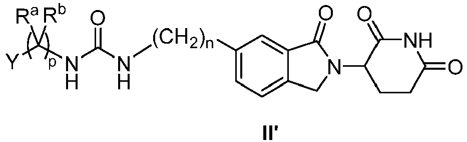 wherein
- n: is 1 or 2;
- p: is 0, 1, 2, 3, 4, 5, 6;
- Y: is hydrogen, C₆ aryl, 5-10 membered heteroaryl, C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, - OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br;
- R^{a}, R^{b}: each are independently selected from hydrogen and linear or branched C₁₋₄ alkyl, preferably hydrogen and methyl.

In some specific embodiments of the compound of formula II', n is 1.

In further specific embodiments of the compound of formula II', p is 0, 1 or 2.

In some specific embodiments of the compound of formula II', p is 0 or 1.

In other embodiments of the compound of formula II', n is 1 and p is 0, 1 or 2.

In some specific embodiments of the compound of formula II', n is 1 and p is 0 or 1.

In some specific embodiments of a compound of formula II', Y is hydrogen, C₆ aryl, 5-10 membered heteroaryl or C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, - N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br

In some specific embodiments of a compound of formula II', Y is hydrogen, C₆ aryl, 5-10 membered heteroaryl or C₃₋₅ cycloalkyl, wherein Y is unsubstituted or substituted with one or more of linear or branched C₁₋₄ alkyl, morpholinyl, -(CH₂)-morpholinyl, piperazinyl, -(CH₂)-piperazinyl, CF₃, CHF₂, -O-(CH₂)₂-OMe, OCF₃, C₁₋₆ alkylamino, -CN, -N(H)C(O)- C₁₋₆alkyl, - OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br

In yet other specific embodiments of a compound of formula II', Y is hydrogen, C₆ aryl or C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, - C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl.

In yet other specific embodiments of a compound of formula II', Y is hydrogen, C₆ aryl or C₃₋₅ cycloalkyl, wherein Y is unsubstituted or substituted with one or more of linear or branched C₁₋₄ alkyl, morpholinyl, -(CH₂)-morpholinyl, piperazinyl, -(CH₂)-piperazinyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl.

In yet other specific embodiments of a compound of formula II', Y is hydrogen, C₆ aryl or C₃₋₅ cycloalkyl, wherein Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl.

In more specific embodiments, of the compound of formula II', n is 1 and Y is hydrogen, C₆ aryl or C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl.

In more specific embodiments, of the compound of formula II', n is 1 and Y is hydrogen, C₆ aryl or C₃₋₅ cycloalkyl, wherein Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl.

In yet further specific embodiments of the compound of formula II', p is 0, 1 or 2 and Y is hydrogen, C₆ aryl or C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl.

In yet further specific embodiments of the compound of formula II', p is 0, 1 or 2 and Y is hydrogen, C₆ aryl or C₃₋₅ cycloalkyl, wherein Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl.

In yet further specific embodiments of the compound of formula II', p is 0 or 1 and Y is hydrogen, C₆ aryl or C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl.

In yet further specific embodiments of the compound of formula II', p is 0 or 1 and Y is hydrogen, C₆ aryl or C₃₋₅ cycloalkyl, wherein Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl.

In yet more specific embodiments of the compound of formula II', n is 1 and p is 0, 1 or 2 and Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, G₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl.

In yet more specific embodiments of the compound of formula II', n is 1 and p is 0, 1 or 2 and Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl.

In yet more specific embodiments of the compound of formula II', n is 1 and p is 0 or 1 and Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(0)O-C₁₋₆alkyl, NH₂, C₁₋₄ alkylhydroxy, C₁₋₄ alkoxy, halogen, preferably F, Cl, Br, more preferably F or Cl.

In yet more specific embodiments of the compound of formula II', n is 1 and p is 0 or 1 and Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl.

In some other specific embodiments of the compound of formula II', R^{a} and R^{b} each are independently selected from hydrogen, methyl and ethyl, preferably hydrogen and methyl.

In some embodiments of the compound of formula II' only a single R^{a} and a single R^{b} is C₁₋₄ alkyl, preferably methyl and all other R^{a} and R^{b} are hydrogen.

In yet other specific embodiments of a compound of formula II', Y is hydrogen, C₆ aryl, C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, - C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl and R^{a} and R^{b} each are independently selected from hydrogen, methyl and ethyl.

In yet other specific embodiments of a compound of formula II', Y is hydrogen, C₆ aryl, C₃₋₅ cycloalkyl, wherein Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl and R^{a} and R^{b} each are independently selected from hydrogen, methyl and ethyl.

In more specific embodiments, of the compound of formula II', n is 1 and Y is hydrogen, C₆ aryl, C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl and R^{a} and R^{b} each are independently selected from hydrogen, methyl and ethyl.

In more specific embodiments, of the compound of formula II', n is 1 and Y is hydrogen, C₆ aryl, C₃₋₅ cycloalkyl, wherein Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl and R^{a} and R^{b} each are independently selected from hydrogen, methyl and ethyl.

In yet further specific embodiments of the compound of formula II', p is 0, 1 or 2 and Y is hydrogen, C₆ aryl, C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl and R^{a} and R^{b} each are independently selected from hydrogen, methyl and ethyl.

In yet further specific embodiments of the compound of formula II', p is 0, 1 or 2 and Y is hydrogen, C₆ aryl, C₃₋₅ cycloalkyl, wherein Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl and R^{a} and R^{b} each are independently selected from hydrogen, methyl and ethyl.

In yet further specific embodiments of the compound of formula II', p is 0 or 1 and Y is hydrogen, C₆ aryl, C₃₋₅ cycloalkyl, wherein Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl and R^{a} and R^{b} each are independently selected from hydrogen, methyl and ethyl.

In yet further specific embodiments of the compound of formula II', p is 0 or 1 and Y is hydrogen, C₆ aryl, C₃₋₅ cycloalkyl, wherein Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl and R^{a} and R^{b} each are independently selected from hydrogen, methyl and ethyl.

In yet more specific embodiments of the compound of formula II', n is 1 and p is 0, 1 or 2 and Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl and R^{a} and R^{b} each are independently selected from hydrogen, methyl and ethyl.

In yet more specific embodiments of the compound of formula II', n is 1 and p is 0, 1 or 2 and Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl and R^{a} and R^{b} each are independently selected from hydrogen, methyl and ethyl.

In yet more specific embodiments of the compound of formula II', n is 1 and p is 0 or 1 and Y is unsubstituted or substituted with one or more of linear or branched C₁₋₄ alkyl, 4-6 membered heterocycloalkyl, C₁₋₄ alkyl 4-6 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, C₁₋₄ alkoxy, halogen, preferably F, Cl, Br, more preferably F or Cl and R^{a} and R^{b} each are independently selected from hydrogen, methyl and ethyl.

In yet more specific embodiments of the compound of formula II', n is 1 and p is 0 or 1 and Y is unsubstituted or substituted with one or more of linear or branched C₁₋₄ alkyl, CF₃, OCF₃, C₁₋₄ alkylamino, -CN, C₁₋₄ alkoxy, halogen, preferably F, Cl, Br, more preferably F or Cl and R^{a} and R^{b} each are independently selected from hydrogen, methyl and ethyl.

More specifically, the present disclosure is directed towards a compound or pharmaceutically acceptable salts or stereoisomers thereof of formula Illa, Illb or IIIc 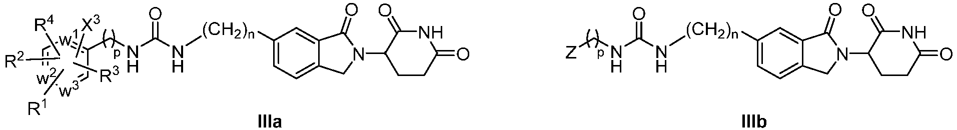 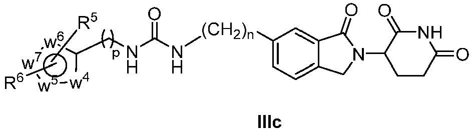 wherein
n is 1 or 2
p is 0, 1 or 2
one of w¹, w² or w³ is selected from C and N, and the other two of w¹, w² or w³ are C;
one or two of w⁴, w⁵, w⁶, w⁷ is selected from C, O, N, NMe, NH, or S while two or three of w⁴, w⁵, w⁶ and w⁷ are C;
R¹, R², R³, R⁴ each are independently selected from hydrogen, linear or branched C₁₋₆ alkyl, linear or branched C₁₋₆heteroalkyl, C₆ aryl, preferably phenyl, CF₃, CHF₂, - O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl;
R⁵, R⁶ each are independently selected from hydrogen, linear or branched C₁₋₄ alkyl, CF₃, CHF₂, halogen, preferably F, Cl, Br, more preferably F or Cl
X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl;
Z is linear or branched C₁₋₆ alkyl or C₃₋₆ cycloalkyl, C₁₋₄ alkoxy or C₁₋₄ alkyl-C₁₋₄ alkoxy, wherein Z is unsubstituted or substituted with C₁₋₄ alkyl.

More specifically, the present disclosure is directed towards a compound or pharmaceutically acceptable salts or stereoisomers thereof of formula IIIa, 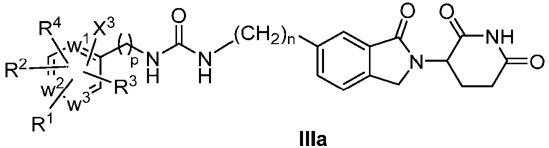 wherein
n is 1 or 2
p is 0, 1 or 2
one of w¹, w² or w³ is selected from C and N, and the other two of w¹, w² or w³ are C;
R¹, R², R³, R⁴ each are independently selected from hydrogen, linear or branched C₁₋₆ alkyl, linear or branched C₁₋₆heteroalkyl, C₆ aryl, preferably phenyl, CF₃, CHF₂, - O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; and
X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

More specifically, the present disclosure is directed towards a compound or pharmaceutically acceptable salts or stereoisomers thereof of formula IIIb 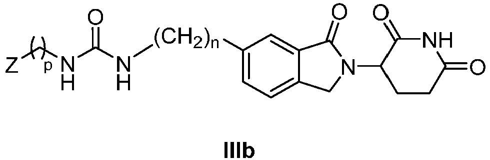 wherein
- n: is 1 or 2
- p: is 0, 1 or 2; and
- Z: is linear or branched C₁₋₆ alkyl or C₃₋₆ cycloalkyl, C₁₋₄ alkoxy or C₁₋₄ alkyl-C₁₋₄ alkoxy, wherein Z is unsubstituted or substituted with C₁₋₄ alkyl.

More specifically, the present disclosure is directed towards a compound or pharmaceutically acceptable salts or stereoisomers thereof of formula IIIc 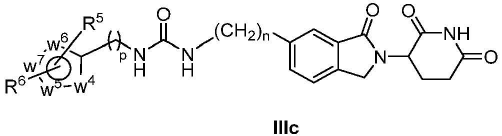 wherein
n is 1 or 2
p is 0, 1 or 2
one of w¹, w² or w³ is selected from C and N, and the other two of w¹, w² or w³ are C;
one or two of w⁴, w⁵, w⁶, w⁷ is selected from C, O, N, NMe, NH, or S while two or three of w⁴, w⁵, w⁶ and w⁷ are C;
R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, - O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl;
R⁵, R⁶ each are independently selected from hydrogen, linear or branched C₁₋₄ alkyl, CF₃, CHF₂, halogen, preferably F, Cl, Br, more preferably F or Cl
X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl;
Z is linear or branched C₁₋₆ alkyl or C₃₋₆ cycloalkyl, C₁₋₄ alkoxy or C₁₋₄ alkyl-C₁₋₄ alkoxy, wherein Z is unsubstituted or substituted with C₁₋₄ alkyl.

In some embodiments of a compound or pharmaceutically acceptable salts or stereoisomers thereof of formula Illa, Illb or Illc, n is 1.

In certain embodiments of a compound or pharmaceutically acceptable salts or stereoisomers thereof of formula Illa, Illb or Illc, p is 0 or 1.

In certain embodiments of a compound or pharmaceutically acceptable salts or stereoisomers thereof of formula Illa, Illb or Illc, p is 0.

In other embodiments of a compound or pharmaceutically acceptable salts or stereoisomers thereof of formula Illa, Illb or Illc, p is 1.

In some embodiments of a compound or pharmaceutically acceptable salts or stereoisomers thereof of formula Illa, Illb or Illc, n is 1 and p is 0 or 1.

In some embodiments of a compound or pharmaceutically acceptable salts or stereoisomers thereof of formula Illa, Illb or Illc, n is 1 and p is 0.

In some embodiments of a compound or pharmaceutically acceptable salts or stereoisomers thereof of formula IIIa, R¹, R², R³ are defined as above and R⁴ is hydrogen such that the aromatic ring contains 4 or 5 substituents which are not hydrogen.

In some embodiments of a compound or pharmaceutically acceptable salts or stereoisomers thereof of formula IIIa, R¹ and R² are defined as above and R³ and R⁴ each are hydrogen, such that the aromatic ring contains 3 or 4 substituents which are not hydrogen.

In specific compounds of Illa, Illb or Illc, R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄alkyl and CF₃; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In some embodiments of a compound of formula Illa, Illb or Illc, n is 1 and R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂ -O-(CH₂)₂-OMe, OCF₃, -CN, - N(H)C(O)-C₁₋₆alkyl, -OC(O)-C₁₋₄alkylamino, -OC(O)-C₁₋₆alkyl, -C(O)O-C₁₋₆alkyl, -COOH, - CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl, CF₃, CHF₂, halogen, preferably F, Cl, Br, more preferably F or Cl; and X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -0-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In specific compounds of Illa, Illb or Illc, n is 1 and R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl and CF₃; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -0-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In some embodiments of a compound of formula IIIa, Illb or Illc, p is 0, 1 or 2 and R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl, CF₃, CHF₂, halogen, preferably F, Cl, Br, more preferably F or Cl; and X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -0-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In specific compounds of IIIa, Illb or Illc, p is 0, 1 or 2and R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl and CF₃; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -0-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In some embodiments of a compound of formula Illa, Illb or Illc, p is 0 or 1 and R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl, CF₃, CHF₂, halogen, preferably F, Cl, Br, more preferably F or Cl; and X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In specific compounds of IIIa, Illb or Illc, p is 0 or 1 and R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl and CF₃; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -0-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In some embodiments of a compound of formula Illa, Illb or Illc, n is 1, p is 0, 1 or 2 and R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl, CF₃, CHF₂, halogen, preferably F, Cl, Br, more preferably F or Cl; and X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In specific compounds of Illa, Illb or Illc, n is 1, p is 0, 1 or 2 and R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl and CF₃; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -0-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In some embodiments of a compound of formula Illa, Illb or Illc, n is 1, p is 0 or 1 and R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl, CF₃, CHF₂, halogen, preferably F, Cl, Br, more preferably F or Cl; and X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In specific compounds of Illa, Illb or Illc, n is 1, p is 0 or 1 and R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; R⁵, R⁶ each are independently selected from hydrogen, linear or branched C₁₋₄ alkyl and CF₃; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -0-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In some embodiments of the compound of formula Illb, C₁₋₆ alkyl is selected from methyl, ethyl, n-propyl, iso-propyl, n-butyl, iso-butyl, t-butyl, n-pentyl, iso-pentyl, neopentyl, n-hexyl, iso-hexyl, and neohexyl.

In some embodiments of the compound of formula Illb, C₃₋₆ cycloalkyl is selected from cyclopropyl, cyclobutyl, cyclopentyl, and cyclohexyl.

In some embodiments of the compound of formula Illb, C₁₋₄ alkoxy is selected from methoxy, ethoxy, n-propoxy, iso-propoxy, n-butoxy, iso-butoxy, and t-butoxy.

In some embodiments of the compound of formula Illb, C₁₋₄ alkyl-C₁₋₄ alkoxy" is selected from methyl-methoxy, methyl-ethoxy, methyl-n-propoxy, methyl-iso-propoxy, methyl-n-butoxy, methyl-iso-butoxy, methyl-t-butoxy, ethyl-methoxy, ethyl-ethoxy, ethyl-n-propoxy, ethyl-iso-propoxy, ethyl-n-butoxy, ethyl-iso-butoxy, ethyl-t-butoxy, propyl-methoxy, propyl-ethoxy, propyl-n-propoxy, propyl-iso-propoxy, propyl-n-butoxy, propyl- iso-butoxy, and propyl-t-butoxy.

In specific embodiments of the compound of formula IIIb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl.

In specific embodiments of the compound of formula Illb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy.

In more specific embodiments of the compound of formula Illb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl and n is 1.

In specific embodiments of the compound of formula IIIb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy and n is 1.

In further specific embodiments of the compound of formula IIIb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl and p is 0, 1 or 2.

In further specific embodiments of the compound of formula IIIb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl and p is 0 or 1.

In yet further specific embodiments of the compound of formula Illb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl and n is 1 and p is 0, 1 or 2.

In yet further specific embodiments of the compound of formula Illb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl and n is 1 and p is 0 or 1.

In more specific embodiments, the present disclosure is directed to compounds or pharmaceutically acceptable salts or stereoisomers thereof of formula Illa-1 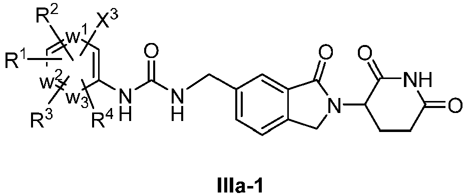 wherein
one of w¹, w² or w³ is selected from C and N, and the other two of w¹, w² or w³ are C;
R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, - C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl;
X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In specific compounds of IIIa-1 R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; X³ is absent, hydrogen or 4-8 membered heterocycloalkyl, C₁₋₄ alkyl 4-8 membered heterocycloalkyl, -O-(4-8 membered heterocycloalkyl), -C₁₋₄alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In some embodiments of a compound or pharmaceutically acceptable salts or stereoisomers thereof of formula Illa-1, R¹, R², R³ are defined as above and R⁴ is hydrogen such that the aromatic ring contains 4 or 5 substituents which are not hydrogen.

In some embodiments of a compound or pharmaceutically acceptable salts or stereoisomers thereof of formula Illa-1, R¹ and R² are defined as above and R³ and R⁴ each are hydrogen, such that the aromatic ring contains 3 or 4 substituents which are not hydrogen.

In more specific embodiments, the present disclosure is directed to compounds or pharmaceutically acceptable salts or stereoisomers thereof of formula Illa-2, Illa-3, IIIa-4 or Illa-5 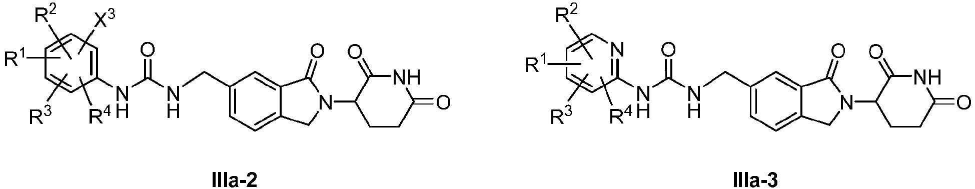 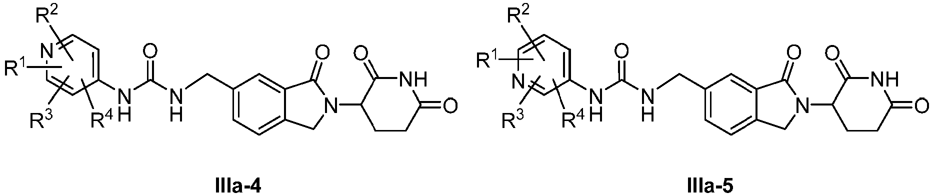 wherein
R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, - C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl;
X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In specific compounds of formula Illa-2, Illa-3, IIIa-4 or Illa-5 R¹, R², R³ and R⁴ each are independently selected from hydrogen, linear or branched C₁₋₆ alkyl, linear or branched C₁₋₆heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In yet further specific embodiments of formula Illa-2, R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl; while in compounds of formula Illa-3, Illa-4 or Illa-5 R¹ and R² each are independently selected from hydrogen, linear or branched C₁₋₆ alkyl, and halogen, preferably Cl, while R³ and R⁴ are hydrogen.

In more specific embodiments, the compound of formula IIIa, Illa-1, IIIa-2, Illa-3, IIIa-4 or IIIa-5 is given by a compound of formula Illa-2a, Illa-2b, Illa-2c, IIIa-3a, Illa-4a or Illa-5a or or pharmaceutically acceptable salts or stereoisomers thereof 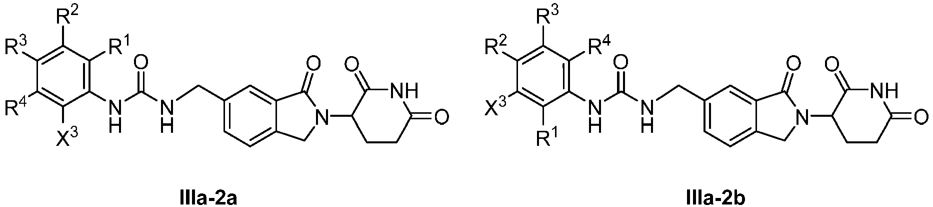 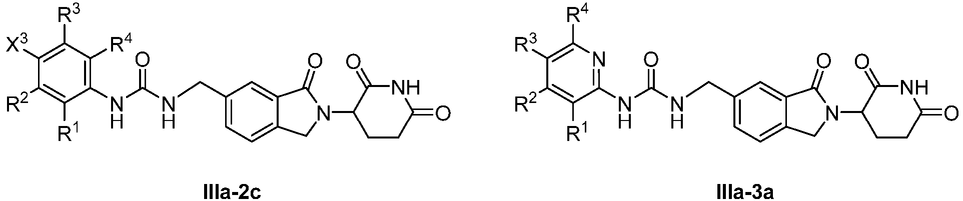 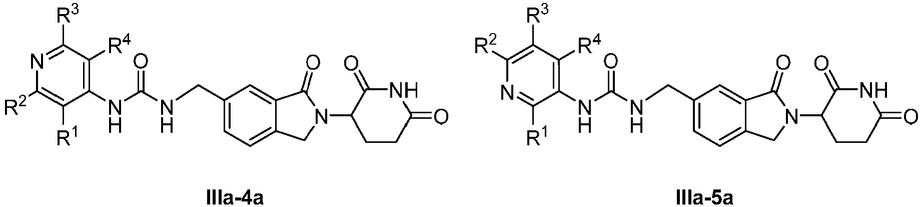 wherein
R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, - C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ formtogether a 5-6 membered heterocycloalkyl or a 5-6 membered heteroaryl;
X³ is hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In specific compounds of formula IIIa-2a, IIIa-2b, IIIa-2c, IIIa-3a, IIIa-4a or IIIa-5a R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In yet further specific embodiments of formula IIIa-2a, IIIa-2b, IIIa-2c, R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; X³ is hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl; while in compounds of formula IIIa-3a, IIIa-4a or IIIa two of R¹, R², R³ and R⁴ each are independently selected from hydrogen, linear or branched C₁₋₆ alkyl, and halogen, preferably Cl, while R³ and R⁴ are hydrogen; while the other two of R¹, R², R³ and R⁴ are hydrogen.

In some embodiments of the compound of formula IIIa-2a, IIIa-2b, IIIa-2c, IIIa-3a, IIIa-4a or IIIa-5a three of R¹, R², R³, R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl, -COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; while one of R¹, R², R³, R⁴ is hydrogen; and X³ is hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -0-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In some embodiments of the compound of formula Illa-2a, Illa-2b, Illa-2c, Illa-3a, Illa-4a or Illa-5a three of R¹, R², R³ and R⁴ each are independently selected from hydrogen, linear or branched C₁₋₆ alkyl, linear or branched C₁₋₆ heteroalkyl, C₆ aryl, preferably phenyl, CF₃, CHF₂, -O-CHF₂, OCF₃, -CN, -CHO, -C₁₋₆alkylC(O)OH, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl; while one of R¹, R², R³ and R⁴ is hydrogen; X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl), -OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl.

In some embodiments the compound of formula Illb has formula Illb-1 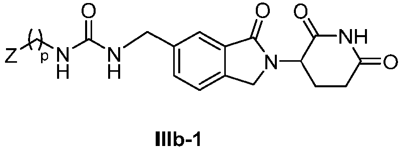 wherein
- p: is 0, 1 or 2; and
- Z: is linear or branched C₁₋₆ alkyl or C₃₋₆ cycloalkyl, C₁₋₄ alkoxy or C₁₋₄ alkyl-C₁₋₄ alkoxy, wherein Z is unsubstituted or substituted with C₁₋₄ alkyl.

In some embodiments of the compound of formula Illb, C₁₋₆ alkyl is selected from methyl, ethyl, n-propyl, iso-propyl, n-butyl, iso-butyl, t-butyl, n-pentyl, iso-pentyl, neopentyl, n-hexyl, iso-hexyl, and neohexyl.

In some embodiments of the compound of formula Illb, C₃₋₆ cycloalkyl is selected from cyclopropyl, cyclobutyl, cyclopentyl, and cyclohexyl.

In some embodiments of the compound of formula Illb, C₁₋₄ alkoxy is selected from methoxy, ethoxy, n-propoxy, iso-propoxy, n-butoxy, iso-butoxy, and t-butoxy.

In some embodiments of the compound of formula Illb, C₁₋₄ alkyl-C₁₋₄ alkoxy" is selected from methyl-methoxy, methyl-ethoxy, methyl-n-propoxy, methyl-iso-propoxy, methyl-n-butoxy, methyl-iso-butoxy, methyl-t-butoxy, ethyl-methoxy, ethyl-ethoxy, ethyl-n-propoxy, ethyl-iso-propoxy, ethyl-n-butoxy, ethyl-iso-butoxy, ethyl-t-butoxy, propyl-methoxy, propyl-ethoxy, propyl-n-propoxy, propyl-iso-propoxy, propyl-n-butoxy, propyl- iso-butoxy, and propyl-t-butoxy.

In further specific embodiments of the compound of formula Illb-1, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl and p is 0.

In further specific embodiments of the compound of formula IIIb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl and p is 1.

In yet further specific embodiments of the compound of formula Illb, Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl and p is 2.

In some embodiments, the compounds of formula IIIc are of formula Illc-1 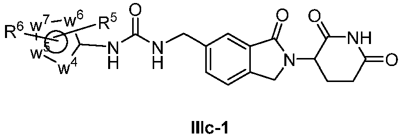 wherein
one or two of w⁴, w⁵, w⁶, w⁷ is selected from C, O, N, NMe, NH, or S while two or three of w⁴, w⁵, w⁶ and w⁷ are C;
R⁵, R⁶ each are independently selected from hydrogen, linear or branched C₁₋₄ alkyl, CF₃, CHF₂, halogen, preferably F, Cl, Br, more preferably F or Cl.

In yet specific embodiments of compounds of formula Illc-1 or pharmaceutically acceptable salts or stereoisomers thereof, R⁵, R⁶ each are independently selected from hydrogen, methyl, ethyl and CF₃.

In some embodiments of compounds of formula Illc-1 or pharmaceutically acceptable salts or stereoisomers thereof, w⁵ is N, w⁷ is NMe, w⁶ and w⁴ are C; or w⁵ is C, w⁷ is S, w⁶ and w⁴ are C; or w⁵ is C, w⁷ is NMe, w⁶ is N and w⁴ is C; or w⁵ is C, w⁷ is C, w⁶ is C and w⁴ is S; or w⁵ is C, w⁷ is C, w⁶ is N and w⁴ is N; or w⁵ is O, w⁷ is C, w⁶ is C and w⁴ is S; or w⁵ is NH, w⁷ is C, w⁶ is C and w⁴ is C; or w⁵ is C, w⁷ is S, w⁶ is C and w⁴ is N; or w⁵ is NH, w⁷ is C, w⁶ is C and w⁴ is N; or w⁵ is C, w⁷ is N, w⁶ is C and w⁴ is S; or w⁵ is NH, w⁷ is N, w⁶ is C and w⁴ is C; or w⁵ is C, w⁷ is NMe, w⁶ is C and w⁴ is C; or w⁵ is N, w⁷ is C, w⁶ is C and w⁴ is S; or w⁵ is C, w⁷ is C, w⁶ is S and w⁴ is N.

In some embodiments of compounds of formula Illc-1 or pharmaceutically acceptable salts or stereoisomers thereof, R⁵, R⁶ each are independently selected from hydrogen, methyl, ethyl and CF₃ and w⁵ is N, w⁷ is NMe, w⁶ and w⁴ are C; or w⁵ is C, w⁷ is S, w⁶ and w⁴ are C; or w⁵ is C, w⁷ is NMe, w⁶ is N and w⁴ is C; or w⁵ is C, w⁷ is C, w⁶ is C and w⁴ is S; or w⁵ is C, w⁷ is C, w⁶ is N and w⁴ is N; or w⁵ is O, w⁷ is C, w⁶ is C and w⁴ is S; or w⁵ is NH, w⁷ is C, w⁶ is C and w⁴ is C; or w⁵ is C, w⁷ is S, w⁶ is C and w⁴ is N; or w⁵ is NH, w⁷ is C, w⁶ is C and w⁴ is N; or w⁵ is C, w⁷ is N, w⁶ is C and w⁴ is S; or w⁵ is NH, w⁷ is N, w⁶ is C and w⁴ is C; or w⁵ is C, w⁷ is NMe, w⁶ is C and w⁴ is C; or w⁵ is N, w⁷ is C, w⁶ is C and w⁴ is S; or w⁵ is C, w⁷ is C, w⁶ is Sand w⁴ is N.

In more specific embodiments, the present disclosure, particularly compounds of formula Illa and Illb, is directed towards a compound or pharmaceutically acceptable salts or stereoisomers thereof of formula IIIa' or IIIb' 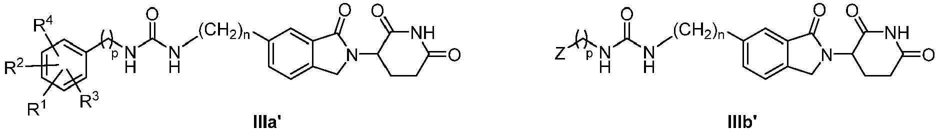 wherein
- n: is 1 or 2
- p: is 0, 1 or 2
- R¹, R², R³, R⁴: each are independently selected from hydrogen, CF₃, linear or branched C₁₋₄ alkyl, C₁₋₄ alkoxy, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, - OC(O)-C₁₋₆alkyl, -N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl, and C₆ aryl, preferably phenyl
- Z: is linear or branched C₁₋₆ alkyl, C₃₋₆ cycloalkyl or C₁₋₄ alkoxy, wherein Z is unsubstituted or substituted with C₁₋₄ alkyl or C₆ aryl.

In some specific embodiments of the compound of formula IIIa' or IIIb', n is 1.

In further specific embodiments of the compound of formula IIIa' or IIIb', p is 0, 1 or 2.

In other embodiments of the compound of formula IIIa' or IIIb', p is 0 or 1.

In yet other embodiments of the compound of formula IIIa' or Illb', n is 1 and p is 0, 1 or 2.

In certain other embodiments of the compound of formula IIIa' or Illb', n is 1 and p is 0 or 1.

In some embodiments of a compound or pharmaceutically acceptable salts or stereoisomers thereof of formula IIIa', R¹, R², R³ are defined as above and R⁴ is hydrogen such that the aromatic ring contains 4 substituents which are not hydrogen.

In some embodiments of a compound or pharmaceutically acceptable salts or stereoisomers thereof of formula IIIa', R¹ and R² are defined as above and R³ and R⁴ each are hydrogen, such that the aromatic ring contains 3 substituents which are not hydrogen.

In some specific embodiments of the compound of formula IIIa' R¹ and R² are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, -OC(O)-C₁₋₆alkyl, - N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl.

In some specific embodiments of the compound of formula IIIa' R¹ and R² are independently selected from hydrogen, morpholinyl, -(CH₂)-morpholinyl, piperazinyl, -(CH₂)-piperazinyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl.

In some specific embodiments of the compound of formula IIIa' R¹ and R² are independently selected from hydrogen, CF₃, OCF₃, C₁₋₄ alkylamino, -CN, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl.

In more specific embodiments of the compound of formula IIIa' R¹ and R² are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, -OC(O)-C₁₋₆alkyl, - N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and n is 1.

In more specific embodiments of the compound of formula IIIa' R¹ and R² are independently selected from hydrogen, morpholinyl, -(CH₂)-morpholinyl, piperazinyl, -(CH₂)-piperazinyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and n is 1.

In more specific embodiments of the compound of formula IIIa' R¹ and R² are independently selected from hydrogen, CF₃, OCF₃, C₁₋₄ alkylamino, -CN, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and n is 1.

In some other specific embodiments of the compound of formula IIIa' R¹ and R² are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, - N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and p is 0, 1 or 2.

In some other specific embodiments of the compound of formula IIIa' R¹ and R² are independently selected from hydrogen, morpholinyl, -(CH₂)-morpholinyl, piperazinyl, - (CH₂)-piperazinyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and p is 0, 1 or 2.

In some other specific embodiments of the compound of formula IIIa' R¹ and R² are independently selected from hydrogen, CF₃, OCF₃, C₁₋₄ alkylamino, -CN, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and p is 0, 1 or 2.

In some other specific embodiments of the compound of formula IIIa' R¹ and R² are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, - OC(O)-C₁₋₆alkyl, -N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and p is 0 or 1.

In some other specific embodiments of the compound of formula IIIa' R¹ and R² are independently selected from hydrogen, morpholinyl, -(CH₂)-morpholinyl, piperazinyl, - (CH₂)-piperazinyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and p is 0 or 1.

In some other specific embodiments of the compound of formula IIIa' R¹ and R² are independently selected from hydrogen, CF₃, OCF₃, C₁₋₄ alkylamino, -CN, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and p is 0 or 1 .

In yet further specific embodiments of the compound of formula Illa' R¹ and R² are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, - N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and n is 1 and p is 0, 1 or 2.

In yet further specific embodiments of the compound of formula Illa' R¹ and R² are independently selected from hydrogen, morpholinyl, -(CH₂)-morpholinyl, piperazinyl, - (CH₂)-piperazinyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and n is 1 and p is 0, 1 or 2.

In yet further specific embodiments of the compound of formula Illa' R¹ and R² are independently selected from hydrogen, CF₃, OCF₃, C₁₋₄ alkylamino, -CN, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and n is 1 and p is 0, 1 or 2.

In yet further specific embodiments of the compound of formula Illa' R¹ and R² are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, - OC(O)-C₁₋₆alkyl, -N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and n is 1 and p is 0 or 1.

In yet further specific embodiments of the compound of formula IIIa' R¹ and R² are independently selected from hydrogen, morpholinyl, -(CH₂)-morpholinyl, piperazinyl, - (CH₂)-piperazinyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and n is 1 and p is 0 or 1 .

In yet further specific embodiments of the compound of formula IIIa' R¹ and R² are independently selected from hydrogen, CF₃, OCF₃, C₁₋₄ alkylamino, -CN, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl and n is 1 and p is 0 or 1.

In specific embodiments of the compound of formula Illb', Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl or C₆ aryl.

In more specific embodiments of the compound of formula IIIb', Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl or C₆ aryl and n is 1.

In further specific embodiments of the compound of formula IIIb', Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl or C₆ aryl and p is 0, 1 or 2.

In further specific embodiments of the compound of formula Illb', Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl or C₆ aryl and p is 0 or 1.

In yet further specific embodiments of the compound of formula Illb', Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl or C₆ aryl and n is 1 and p is 0, 1 or 2.

In yet further specific embodiments of the compound of formula Illb', Z is methyl, ethyl, n-propyl, iso-propyl, n-butyl, iso-butyl, t-butyl, n-pentyl, iso-pentyl, neopentyl, n-hexyl, iso-hexyl, neohexyl or cyclopropyl, cyclobutyl, cyclopentyl, cyclohexyl, methoxy, ethoxy, n-propoxy, iso-propoxy, n-butoxy, iso-butoxy, t-butoxy, wherein Z is unsubstituted or substituted with methyl, ethyl, n-propyl, iso-propyl, n-butyl, iso-butyl, t-butyl or C₆ aryl and n is 1 and p is 0 or 1.

In more specific embodiments, the present disclosure is directed to compounds or pharmaceutically acceptable salts or stereoisomers thereof of formula IIIa'-1 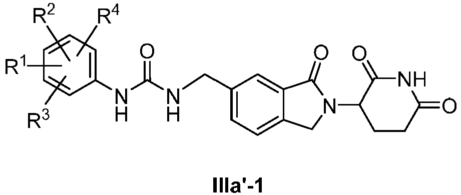 wherein
R¹, R², R³, R⁴ each are independently selected from hydrogen, 4-7 membered heterocycloalkyl, C₁₋₄ alkyl 4-7 membered heterocycloalkyl, CF₃, linear or branched C₁₋₄ alkyl, C₁₋₄ alkoxy, CHF₂, -O-(CH₂)₂-OMe, OCF₃, C₁₋₆ alkylamino, -CN, -OC(O)-C₁₋₆alkyl, -N(H)C(O)- C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl, and C₆ aryl, preferably phenyl.

In some specific embodiments of the compound of formula Illa'-1 R¹,R² R³ and R⁴ are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, - N(H)C(O)- C₁₋₆alkyl, -OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br or phenyl, preferably from hydrogen, CF₃, OCF₃, C₁₋₄ alkylamino, -CN, methyl, ethyl, t-butyl, methoxy, F, Cl and phenyl, more preferably from hydrogen, CF₃, methyl, ethyl, t-butyl, methoxy, F, Cl and phenyl.

In some embodiments of a compound or pharmaceutically acceptable salts or stereoisomers thereof of formula Illa'-1, R¹, R², R³ are defined as above and R⁴ is hydrogen such that the aromatic ring contains 4 substituents which are not hydrogen.

In some embodiments of a compound or pharmaceutically acceptable salts or stereoisomers thereof of formula Illa'-1, R¹ and R² are defined as above and R³ and R⁴ each are hydrogen, such that the aromatic ring contains 3 substituents which are not hydrogen.

In some embodiments the compound of formula Illa'-1 is defined by formula IIIa'-1a, IIIa'-1b, IIIa'-1c or IIIa'-1d 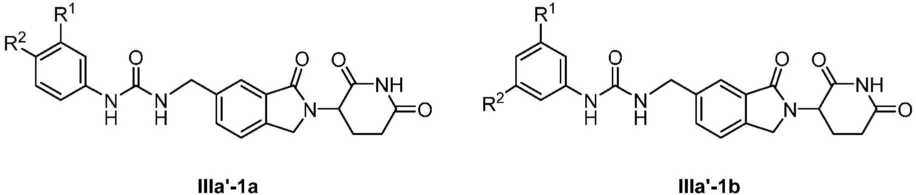 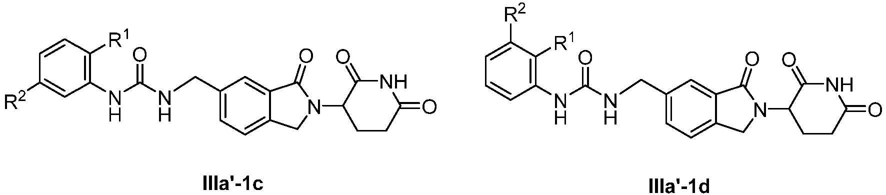 wherein R¹, R² each are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, -OC(O)-C₁₋₆alkyl, -N(H)C(O)- C₁₋₆alkyl, -C(O)O-C₁₋₆alkyl, - COOH, -C₁₋₆alkylC(O)OH, -C₁₋₆alkylC(O)O-C₁₋₆alkyl, NH₂, C₁₋₄ alkylhydroxy, linear or branched C₁₋₄ alkyl, C₁₋₄ alkoxy, halogen, preferably F, Cl, Br, more preferably F or Cl, and C₆ aryl, preferably phenyl.

In some specific embodiments of the compound of formula IIIa'-1a, IIIa'-1b, IIIa'-1c or IIIa'-1d R¹ and R² are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, - CN, -N(H)C(O)- C₁₋₆alkyl, -OC(O)-C₁₋₆alkyl, -C(O)O-C₁₋₆alkyl, -COOH, -C₁₋₆alkylC(O)OH, - C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl, preferably from hydrogen, CF₃, methyl, ethyl, t-butyl, methoxy, F, Cl or phenyl.

In some embodiments, of the compound of formula IIIa'-1a, IIIa'-1b, IIIa'-1c or IIIa'-1d R¹ and R² are independently selected from hydrogen, CF₃, OCF₃, C₁₋₄ alkylamino, -CN, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl, preferably from hydrogen, CF₃, methyl, ethyl, t-butyl, methoxy, F, Cl or phenyl.

In some embodiments the compound of formula IIIa'-1 is defined by formula IIIa'-1e, IIIa'-1f, IIIa'-1g, IIIa'-1h, IIIa'-1i or IIIa'-1j, in particular by formula IIIa'-1h or IIIa'-1g 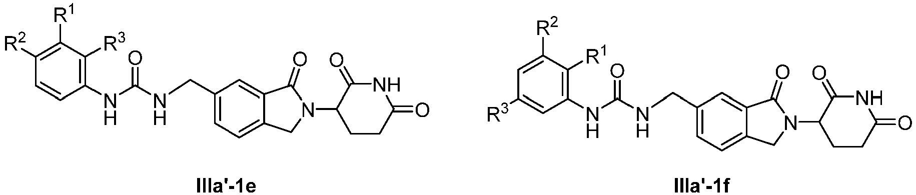 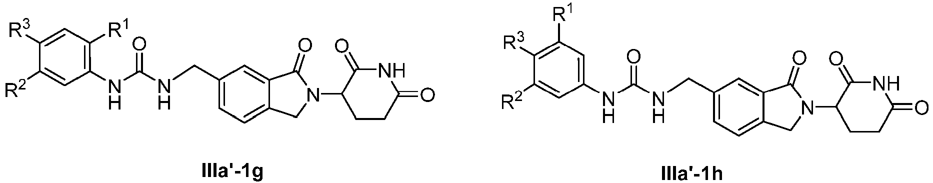 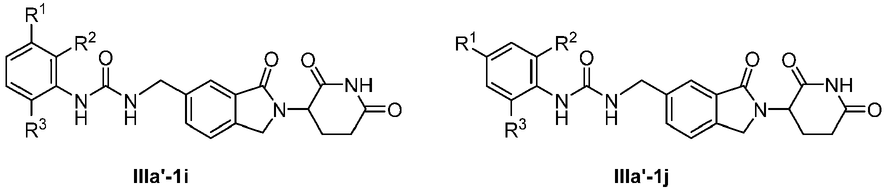 wherein R¹, R² and R³ each are independently selected from hydrogen, 4-7 membered heterocycloalkyl, C₁₋₄ alkyl 4-7 membered heterocycloalkyl, CF₃, linear or branched C₁₋₄ alkyl, C₁₋₄ alkoxy, CHF₂, -O-(CH₂)₂-OMe, OCF₃, C₁₋₆ alkylamino, -CN, -N(H)C(O)- C₁₋₆alkyl, - OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl, or C₆ aryl, preferably phenyl.

In some specific embodiments of the compound of formula IIIa'-1e, IIIa'-1f, IIIa'-1g, IIIa'-1h, IIIa'-1i or Illa'-1j R¹, R² and R³ are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, -CN, -N(H)C(O)- C₁₋₆alkyl, -OC(O)-C₁₋₆alkyl, -C(O)O-C₁₋₆alkyl, - COOH, -C₁₋₆alkylC(O)OH, -C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl, preferably from hydrogen, CF₃, methyl, ethyl, t-butyl, methoxy, F, Cl and phenyl.

In some embodiments, of the compound of formula IIIa'-1e, IIIa'-1f, IIIa'-1g, IIIa'-1h, IIIa'-1i or IIIa'-1j R¹, R² and R³ are independently selected from hydrogen, CF₃, OCF₃, C₁₋₄ alkylamino, -CN, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl, preferably from hydrogen, CF₃, methyl, ethyl, t-butyl, methoxy, F, Cl and phenyl.

In some embodiments the compound of formula Illa'-1 is defined by formula IIIa'-1l, IIIa'-1m or Illa'-1n 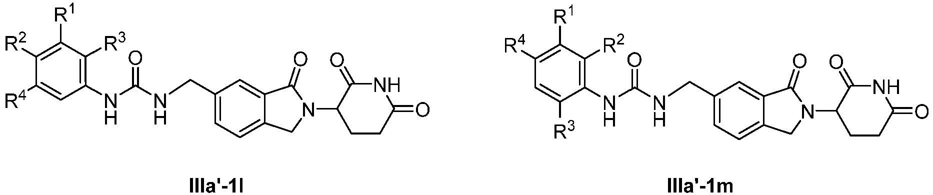 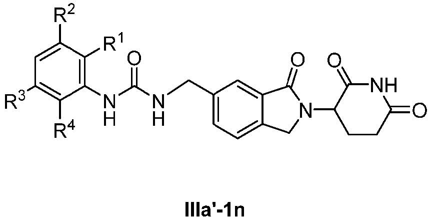 wherein R¹, R², R³ and R⁴ each are independently selected from hydrogen, 4-7 membered heterocycloalkyl, C₁₋₄ alkyl 4-7 membered heterocycloalkyl, CF₃, linear or branched C₁₋₄ alkyl, C₁₋₄ alkoxy, CHF₂, -O-(CH₂)₂-OMe, OCF₃, C₁₋₆ alkylamino, -CN, -N(H)C(O)- C₁₋₆alkyl, - OC(O)-C₁₋₆alkyl, -C(O)O-C₁₋₆alkyl, -COOH, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl, and C₆ aryl, preferably phenyl.

In some specific embodiments of the compound of formula IIIa'-1l, IIIa'-1 m or IIIa'-1 n R¹, R², R³ and R⁴ are independently selected from hydrogen, 4-7 membered heterocycloalkyl, C₁₋₄ alkyl 4-7 membered heterocycloalkyl, CF₃, CHF₂, -O-(CH₂)₂-OMe, OCF₃, C₁₋₆ alkylamino, - CN, -N(H)C(O)- C₁₋₆alkyl, -OC(O)-C₁₋₆alkyl, -C(O)O-C₁₋₆alkyl, -COOH, -C₁₋₆alkylC(O)OH, - C₁₋₆alkylC(O)O-C₁₋₆alkyl, NH₂, C₁₋₄ alkylhydroxy, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl, preferably from hydrogen, CF₃, methyl, ethyl, t-butyl, methoxy, F, Cl and phenyl.

In some embodiments, of the compound of formula IIIa'-1l, IIIa'-1 m or IIIa'-1n R¹, R², R³ and R⁴ are independently selected from hydrogen, CF₃, OCF₃, C₁₋₄ alkylamino, -CN, methyl, ethyl, n-propyl, iso-propyl, n-butyl, iso-butyl, t-butyl, methoxy, ethoxy, n-propoxy, iso-propoxy, n-butoxy, iso-butoxy, t-butoxy, F, Cl, Br and phenyl, preferably from hydrogen, CF₃, methyl, ethyl, t-butyl, methoxy, F, Cl and phenyl.

In more specific embodiments, the present disclosure is directed towards a compound or pharmaceutically acceptable salts or stereoisomers thereof of formula IV 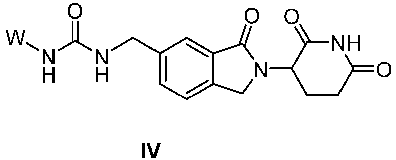 wherein
W is selected from

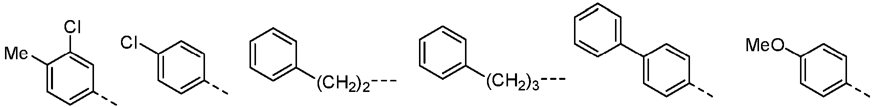

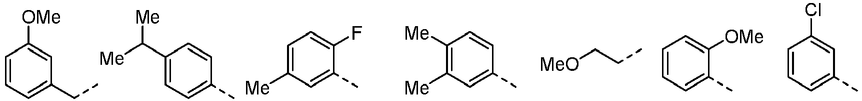

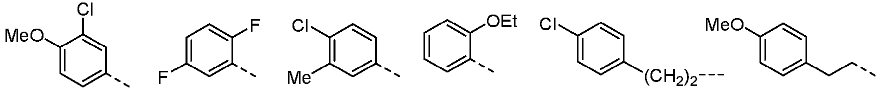

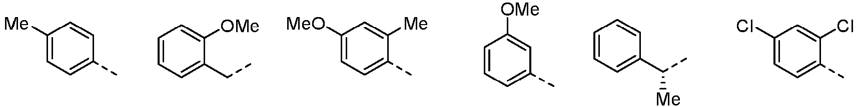

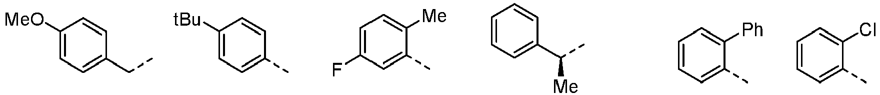

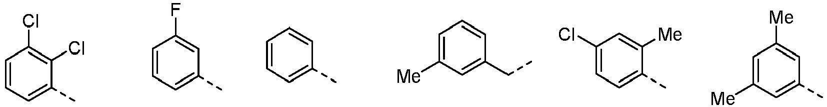

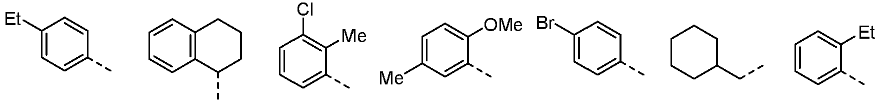

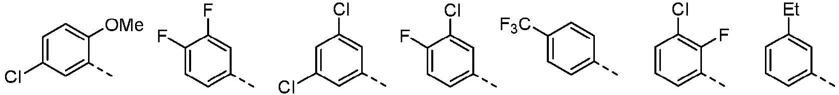

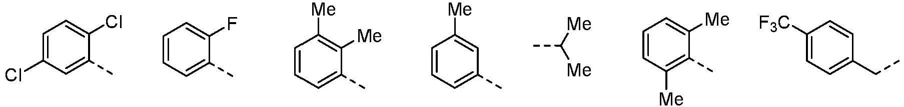

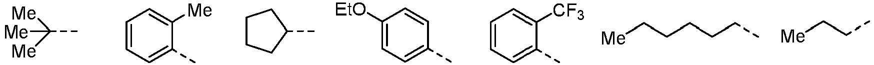

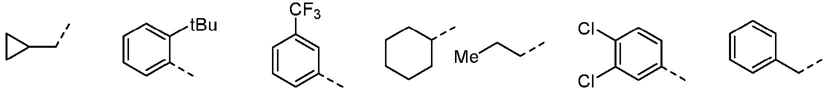

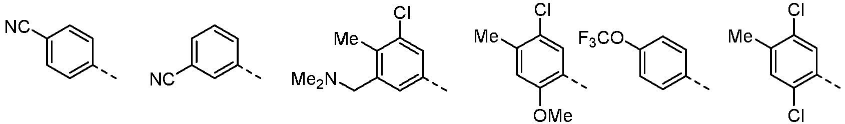

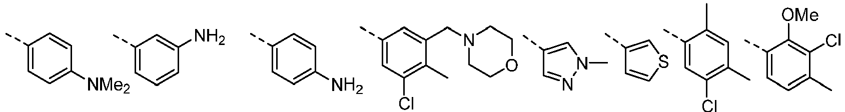

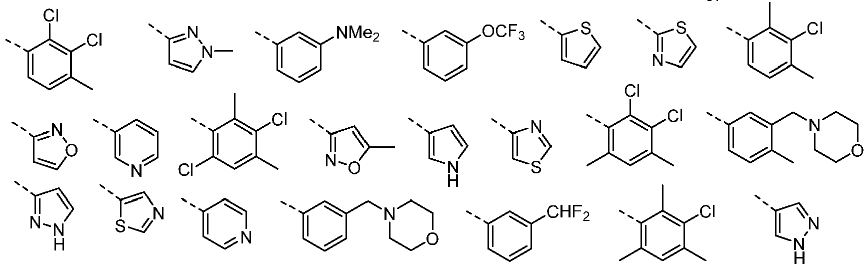

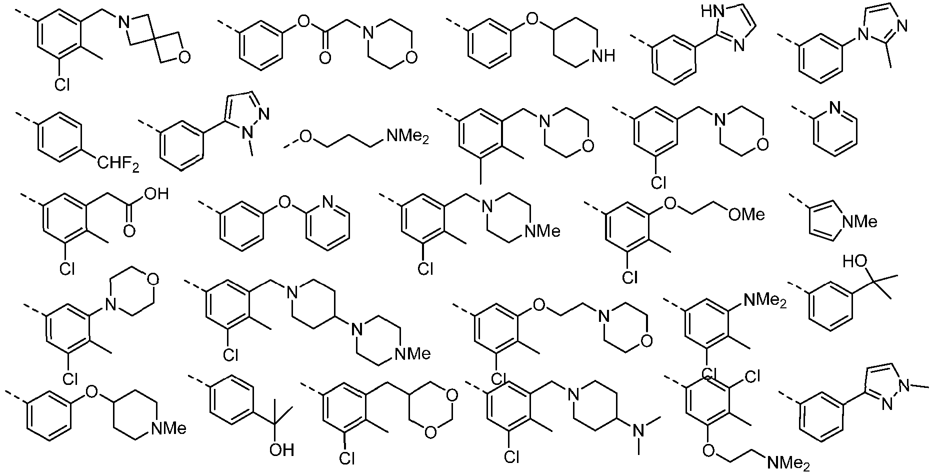

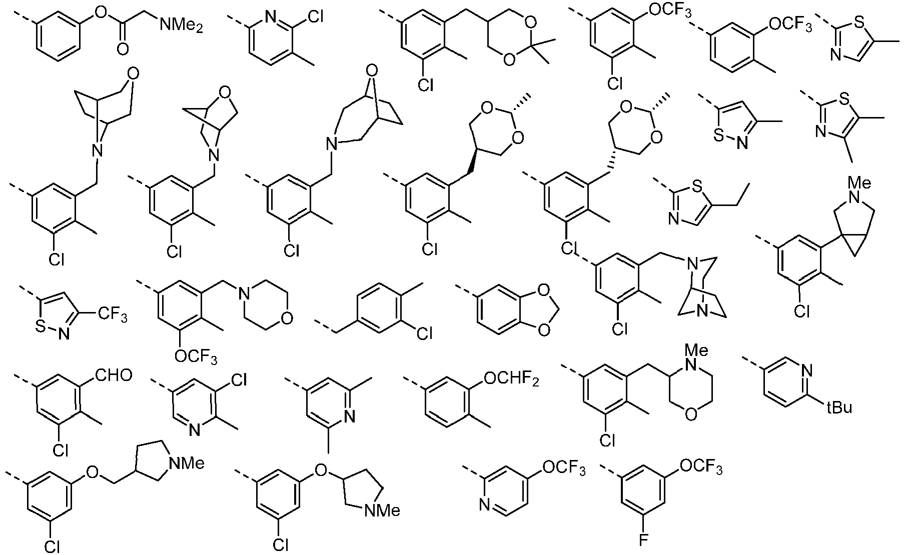

In specific embodiments of a compound of formula IV, W is

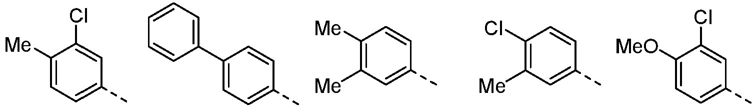

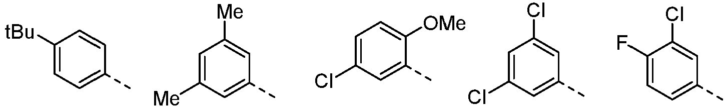

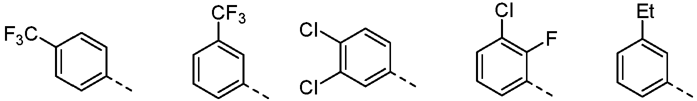

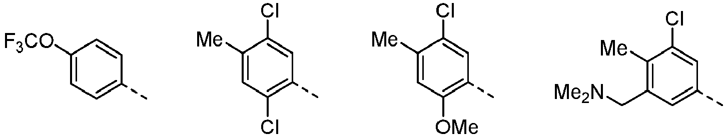

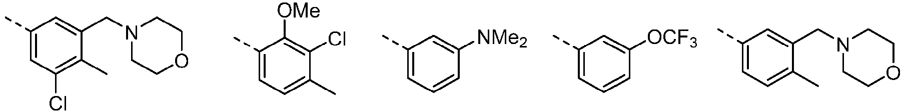

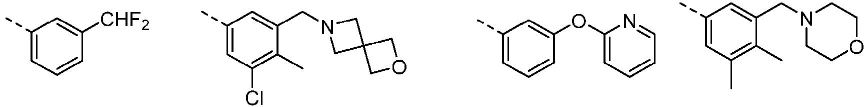

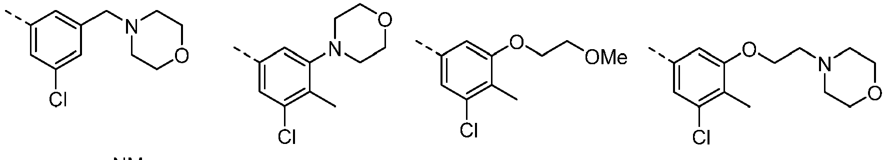

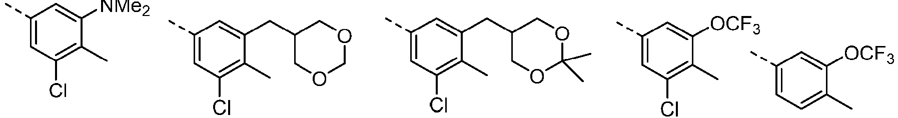

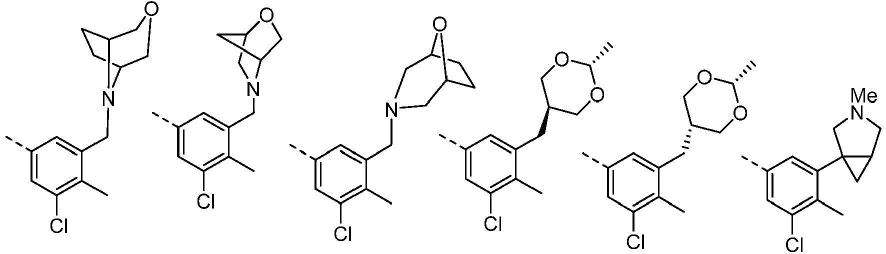

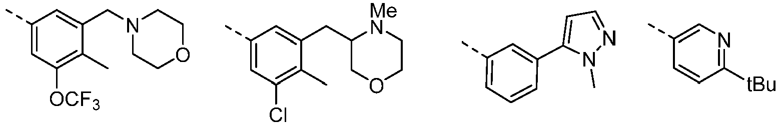

In more specific embodiments, the present disclosure is directed towards a compound or pharmaceutically acceptable salts or stereoisomers thereof of formula IV' 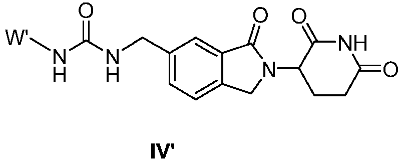 wherein
W' is selected from

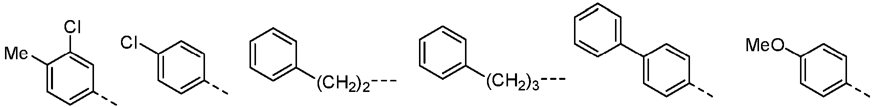

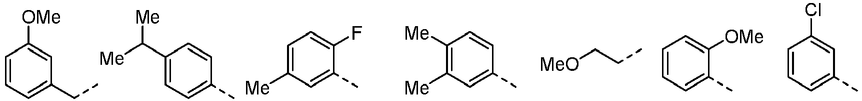

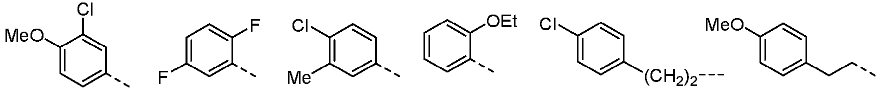

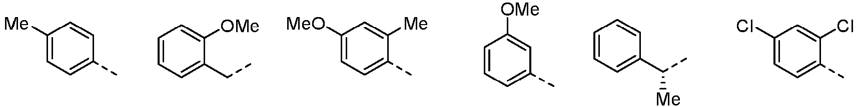

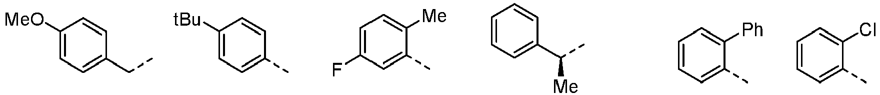

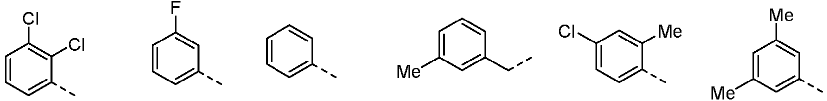

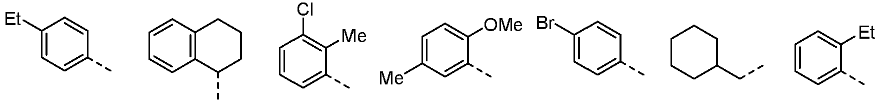

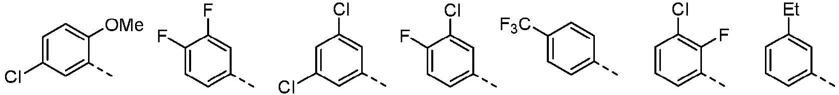

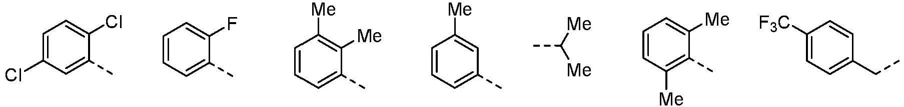

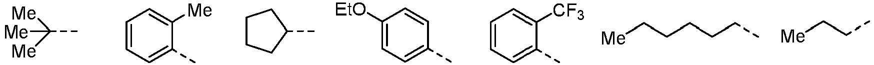

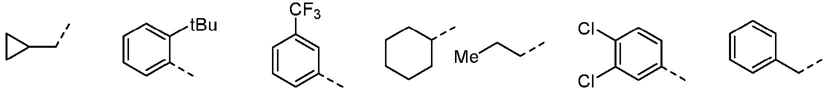

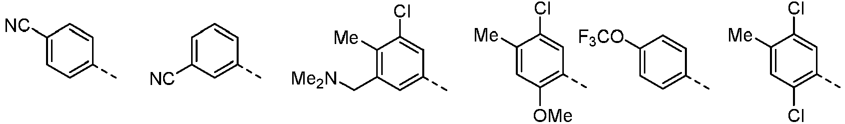

In specific embodiments of a compound of formula IV', W' is

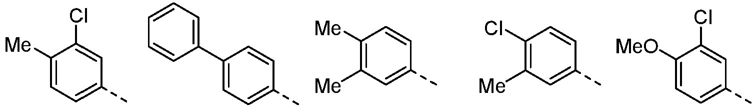

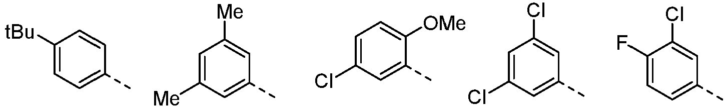

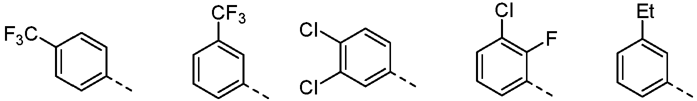

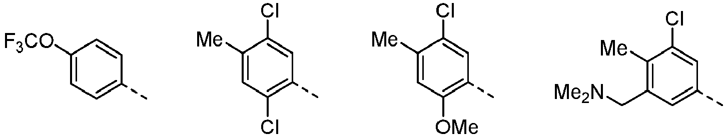

In more specific embodiments, the present disclosure is directed towards a compound or pharmaceutically acceptable salts or stereoisomers thereof of formula V 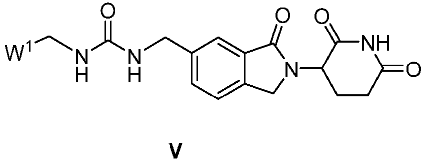 wherein
W¹ is selected from

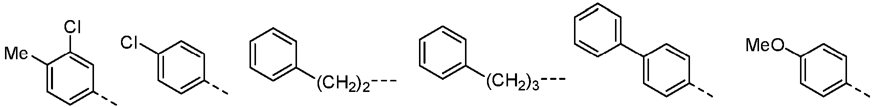

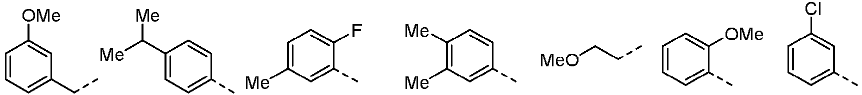

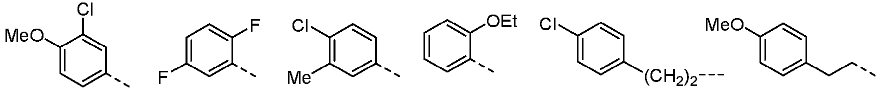

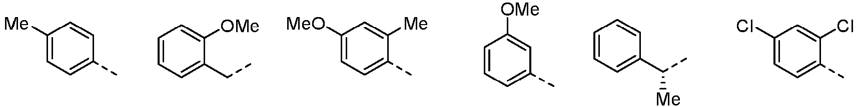

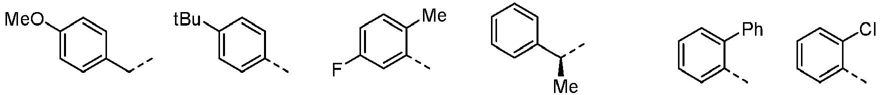

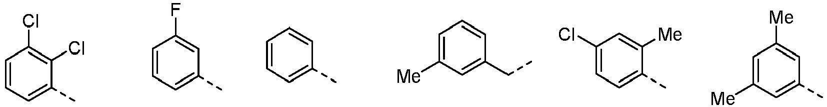

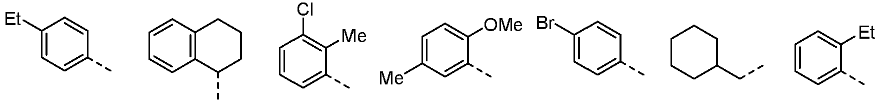

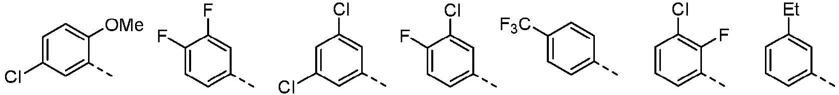

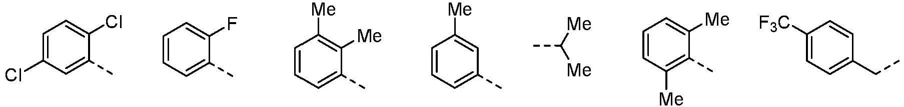

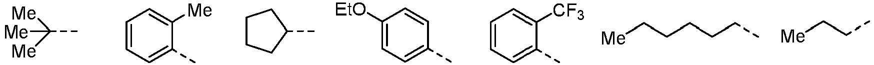

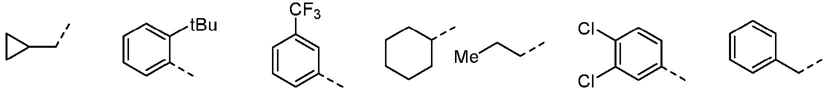

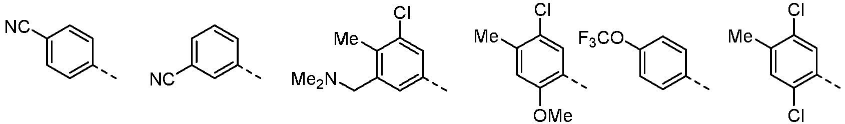

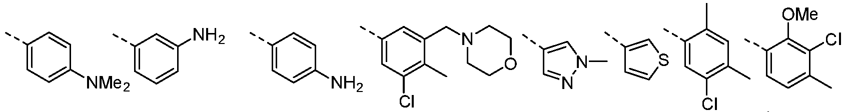

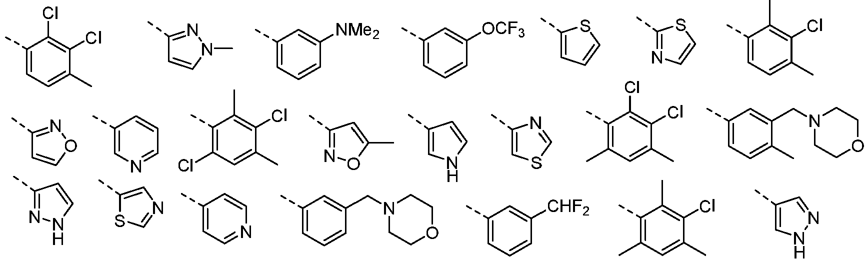

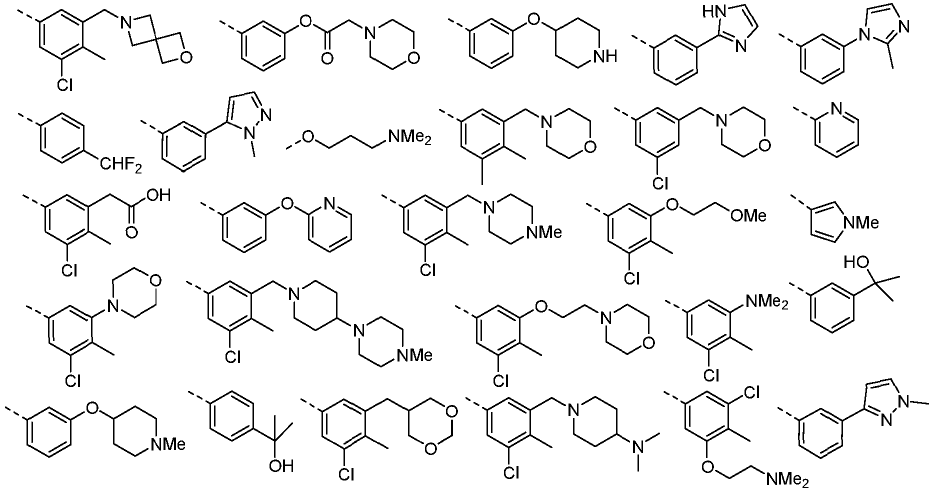

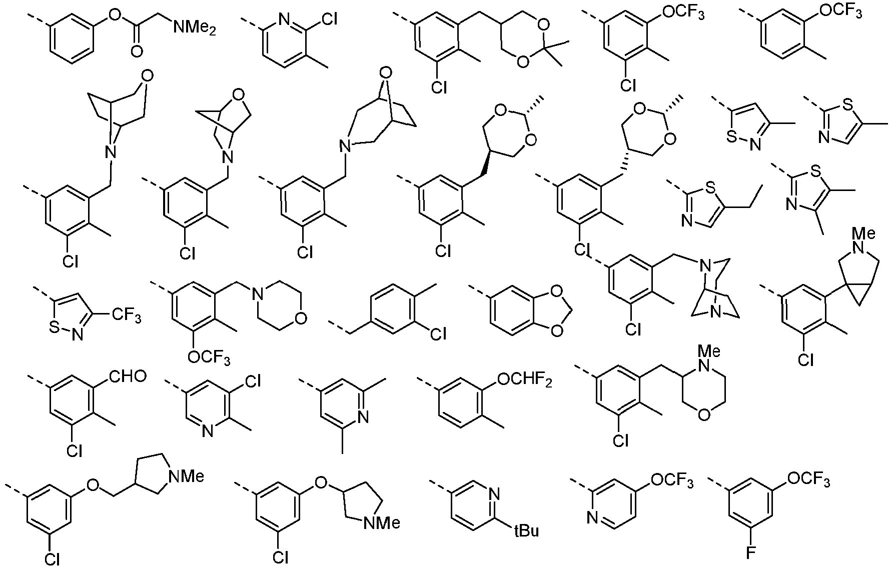

In specific embodiments of a compound of formula V, W¹ is selected from

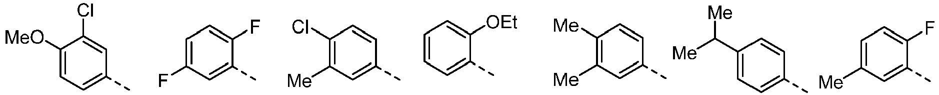

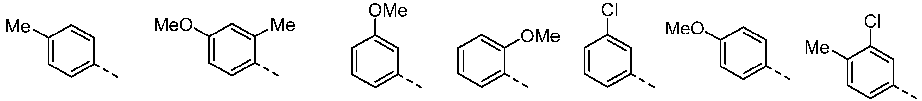

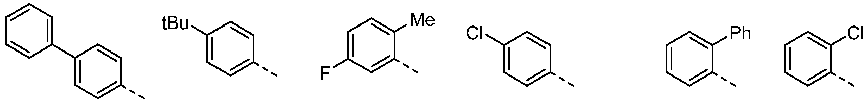

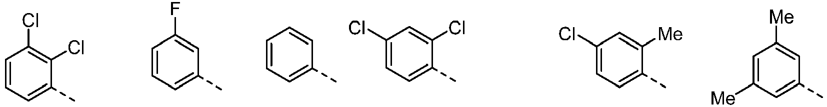

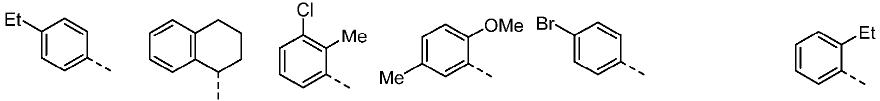

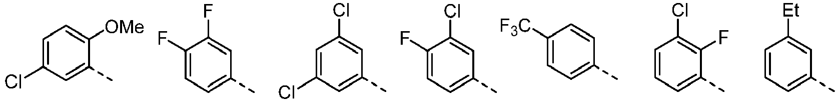

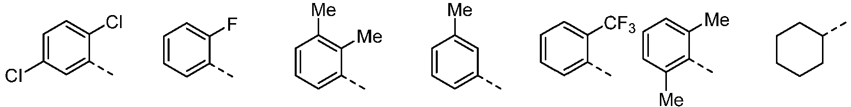

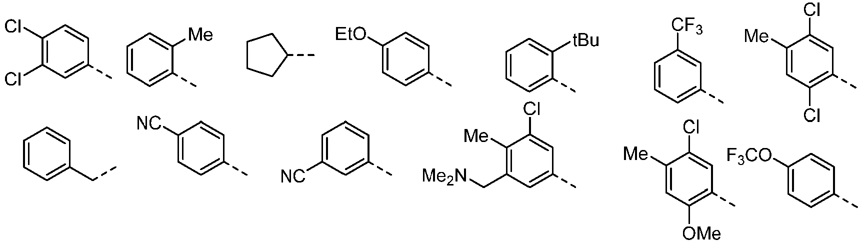

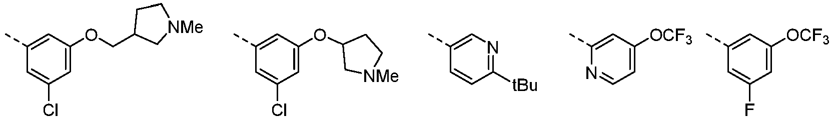

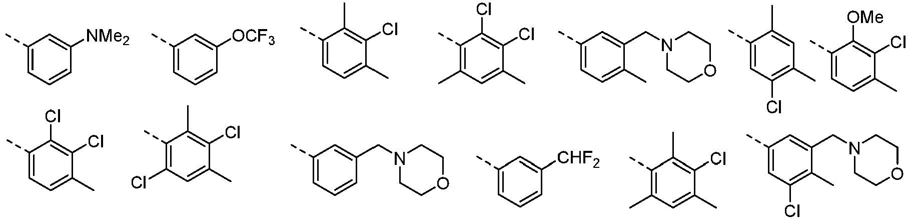

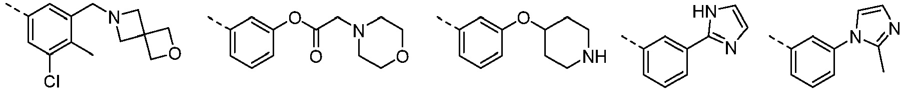

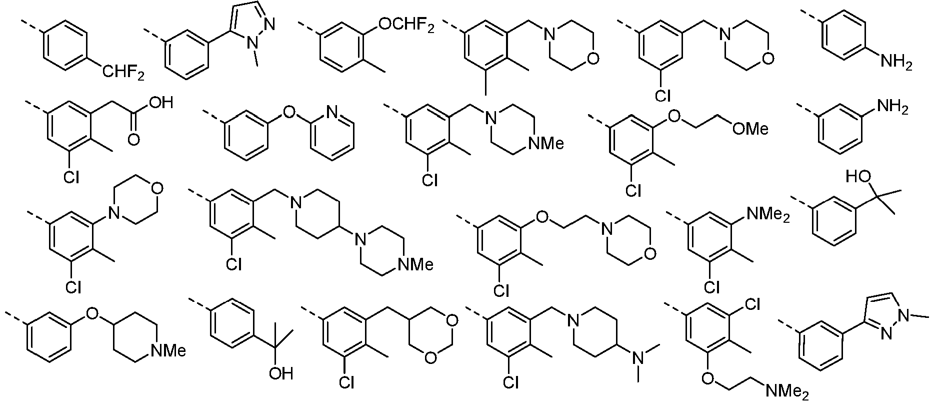

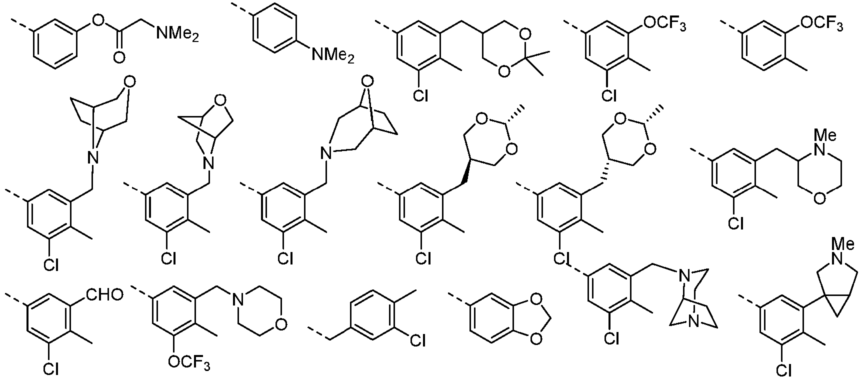

In more specific embodiments, the present disclosure is directed towards a compound or pharmaceutically acceptable salts or stereoisomers thereof of formula VI 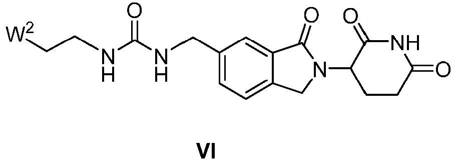 wherein
W² is selected from

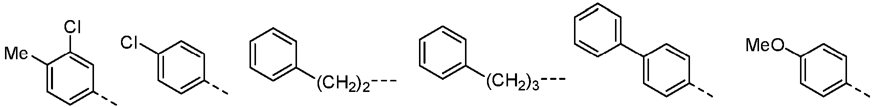

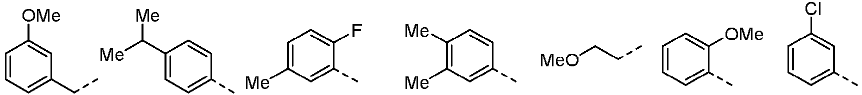

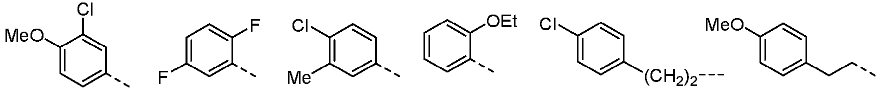

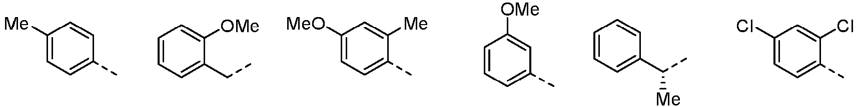

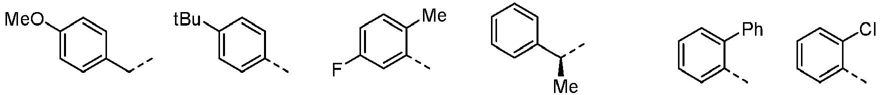

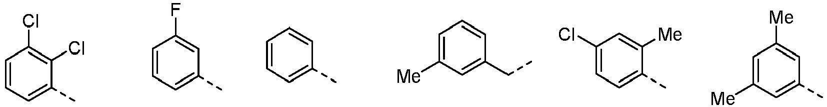

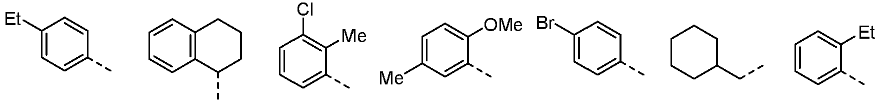

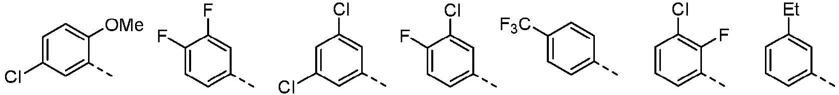

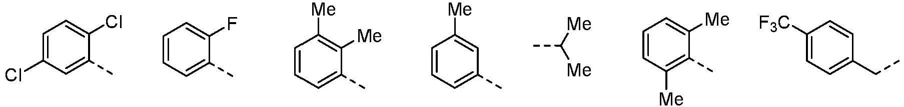

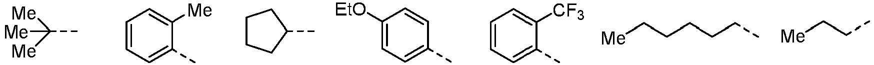

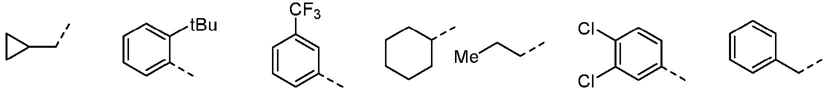

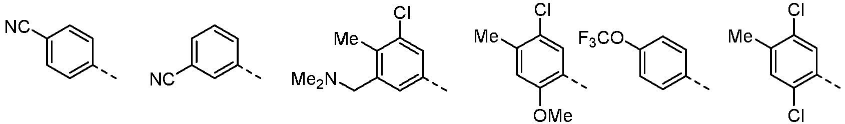

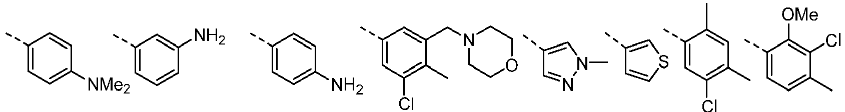

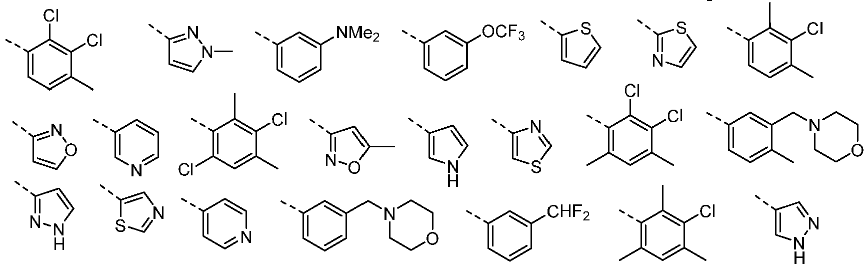

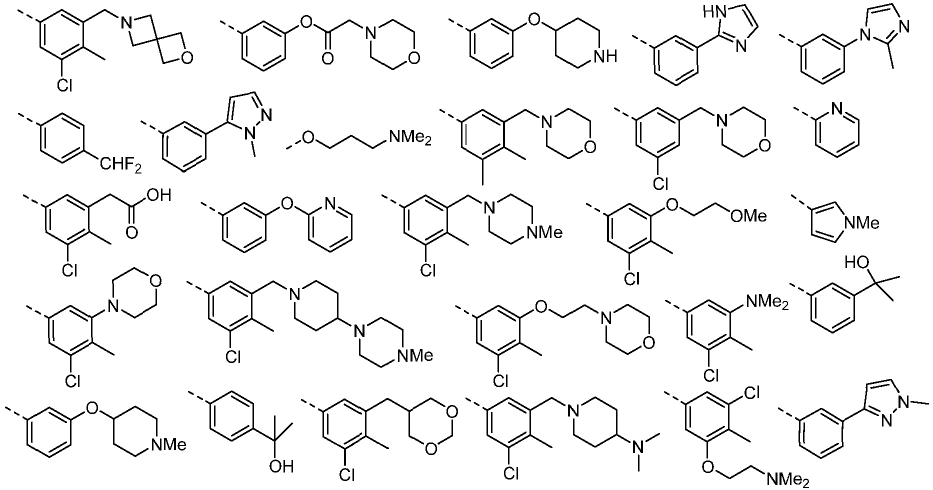

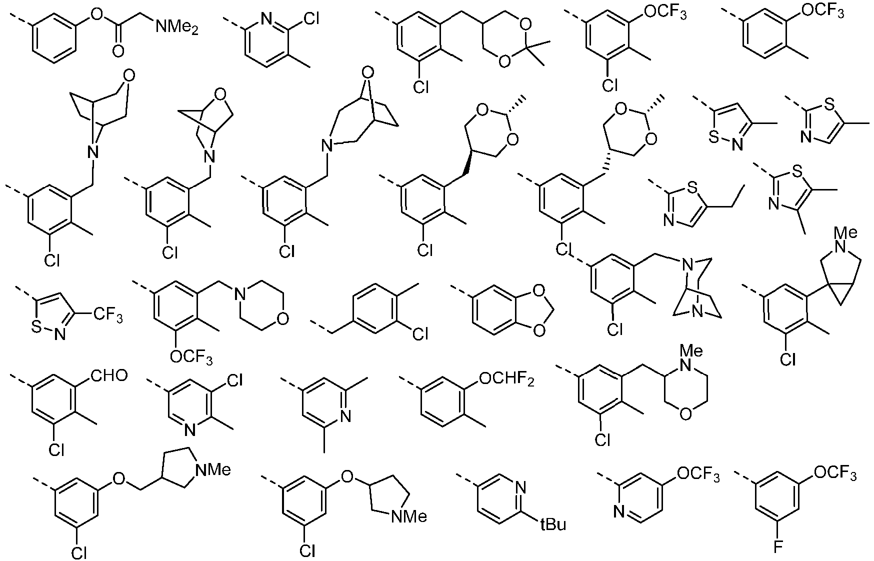

In more specific embodiments of a compound of formula VI, W² is selected from:

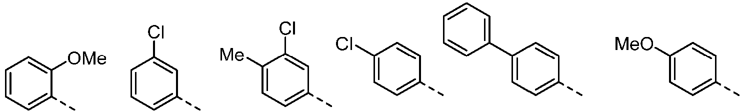

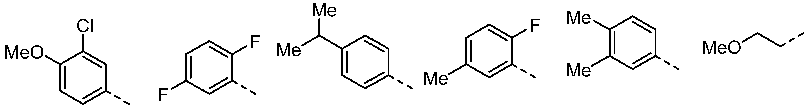

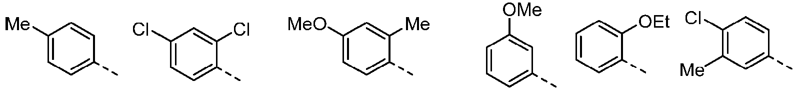

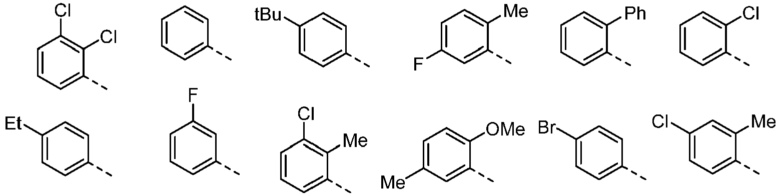

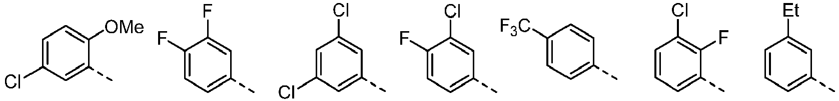

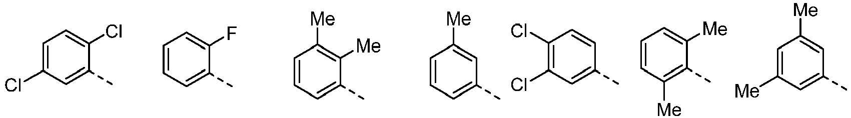

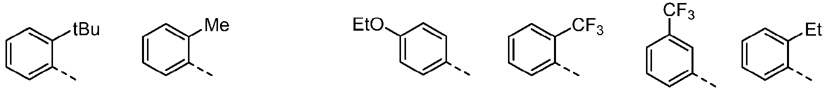

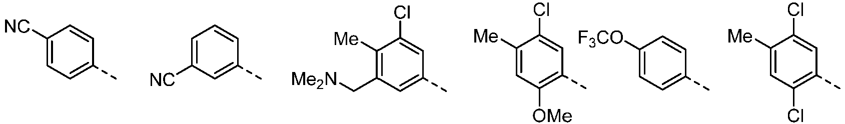

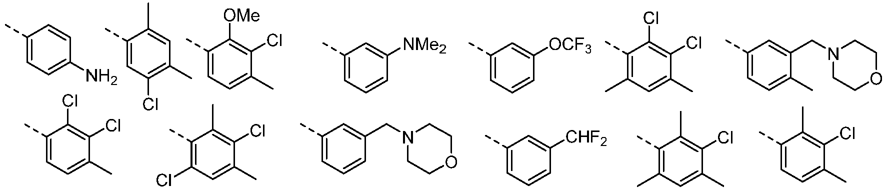

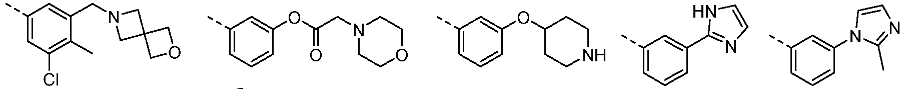

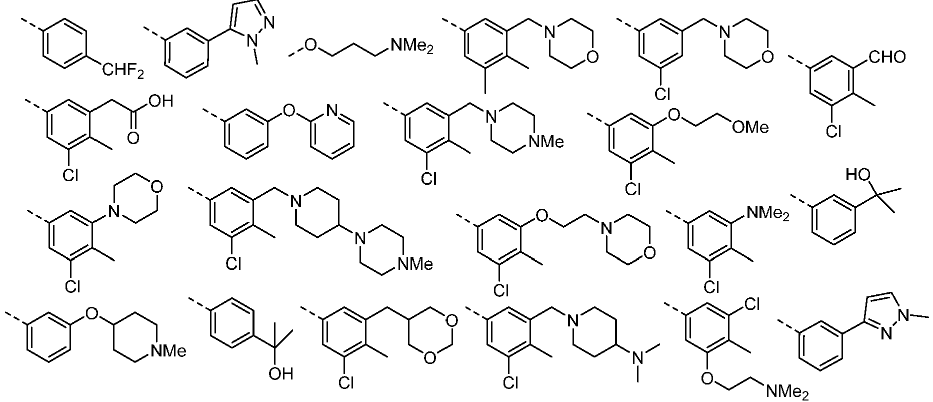

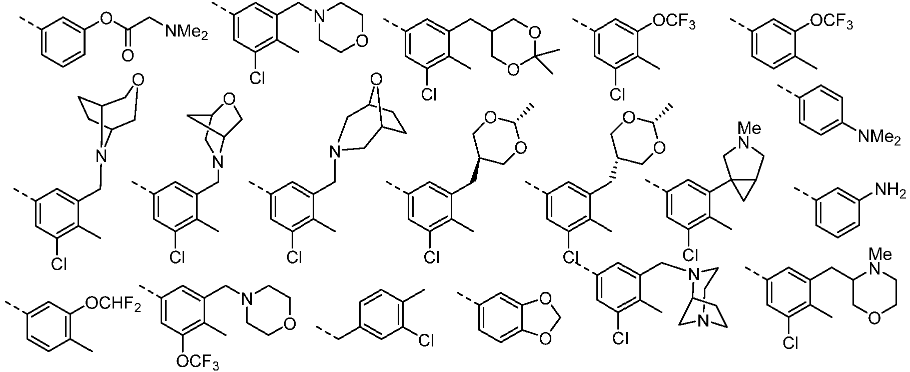

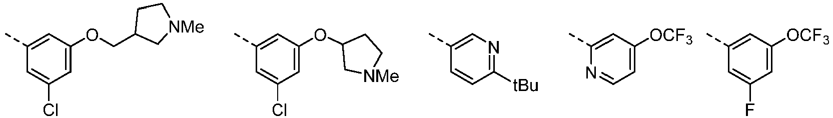

In more specific embodiments, the present disclosure is directed towards a compound or pharmaceutically acceptable salts or stereoisomers thereof of formula VII 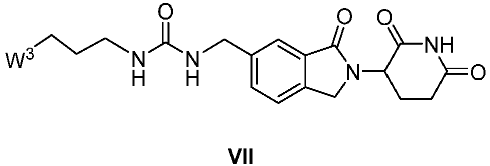 wherein
W³ is selected from

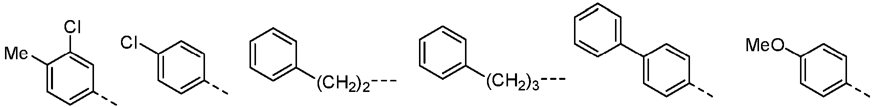

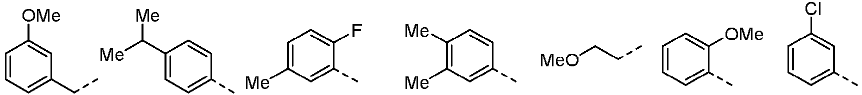

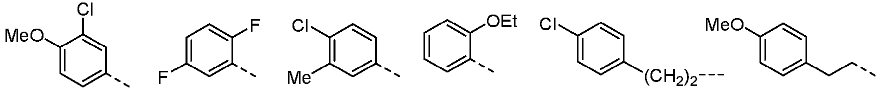

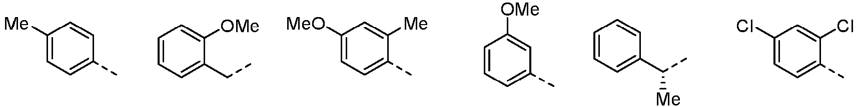

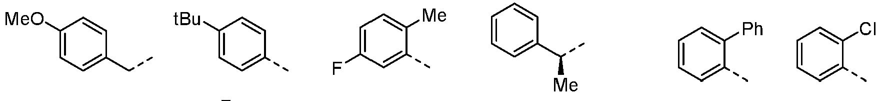

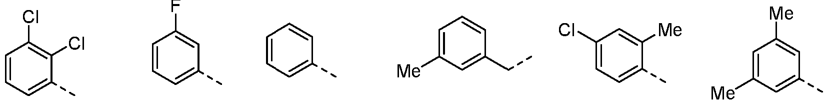

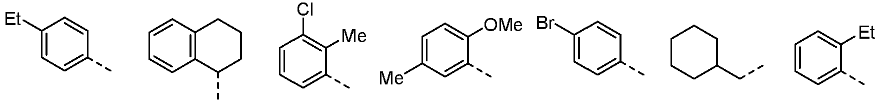

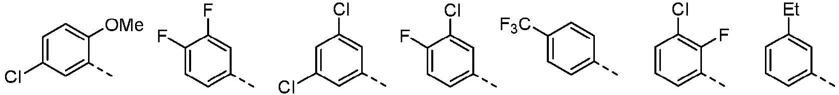

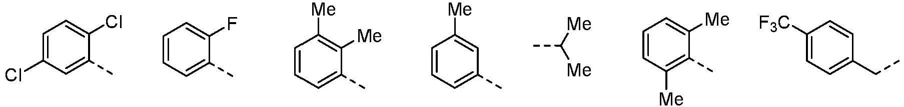

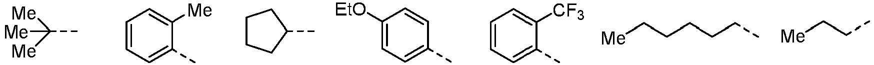

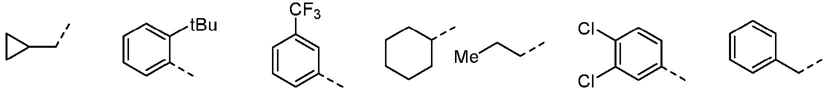

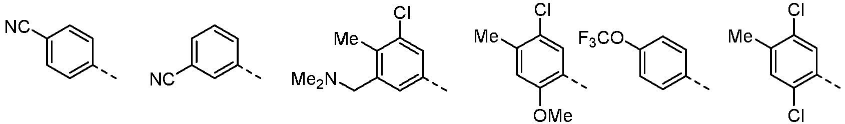

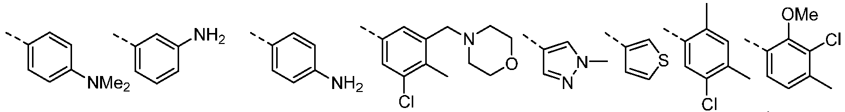

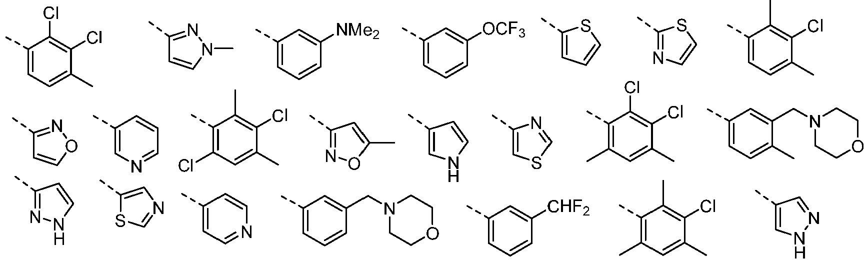

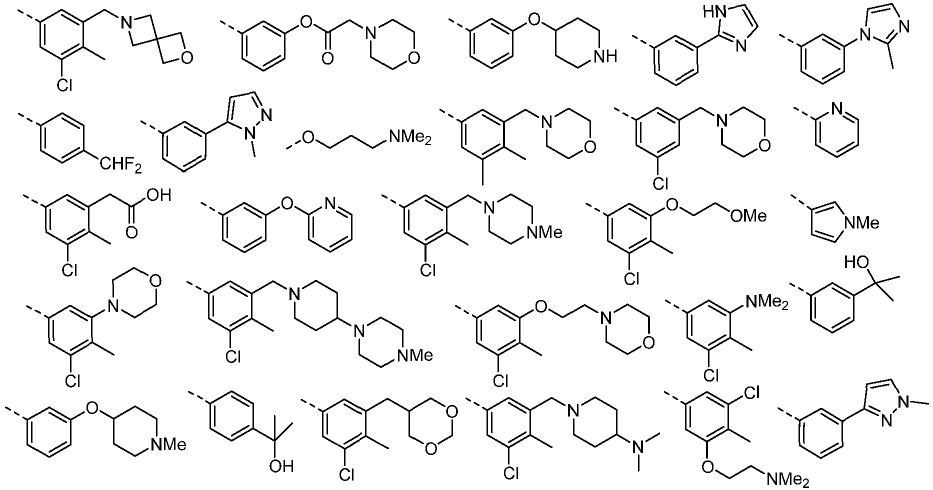

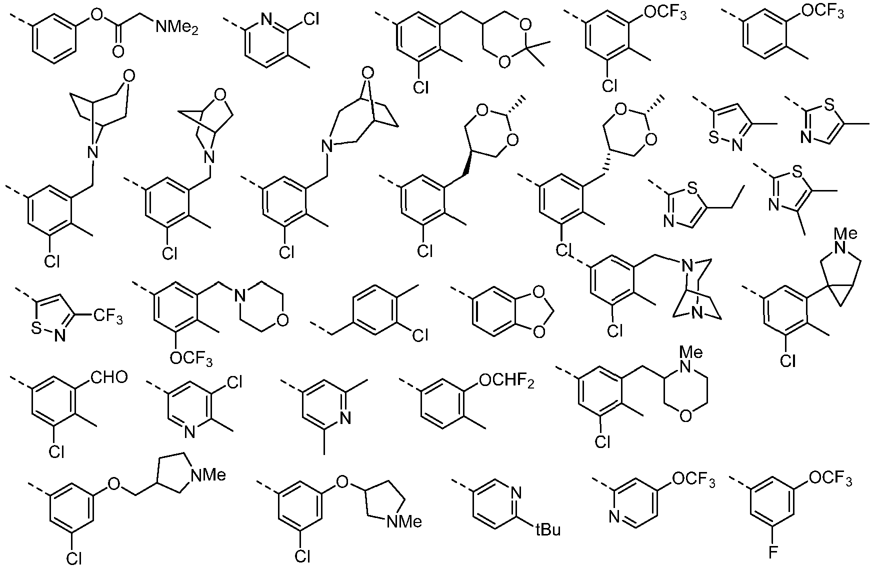

In more specific embodiments of compounds of formula VII, W³ is selected from:

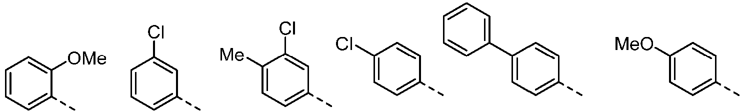

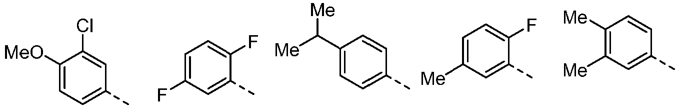

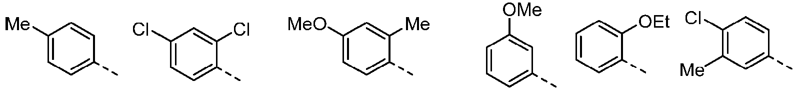

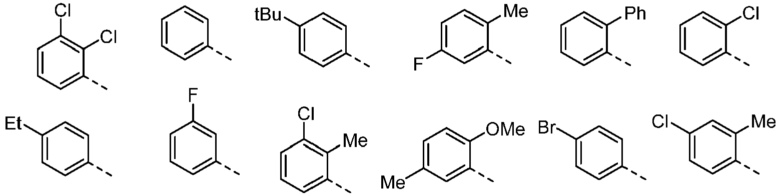

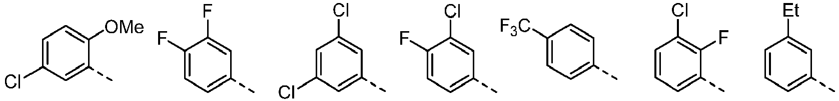

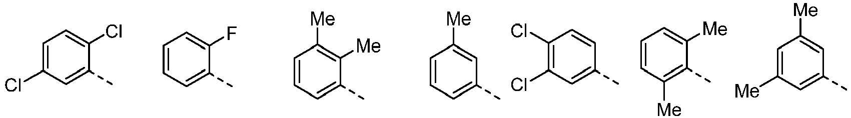

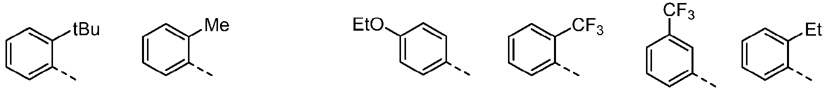

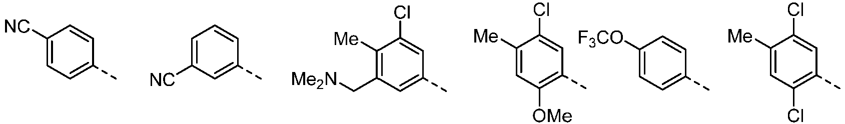

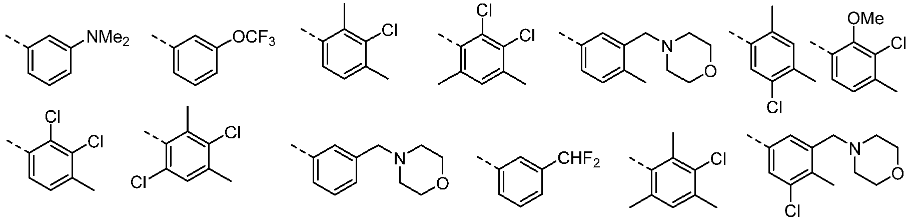

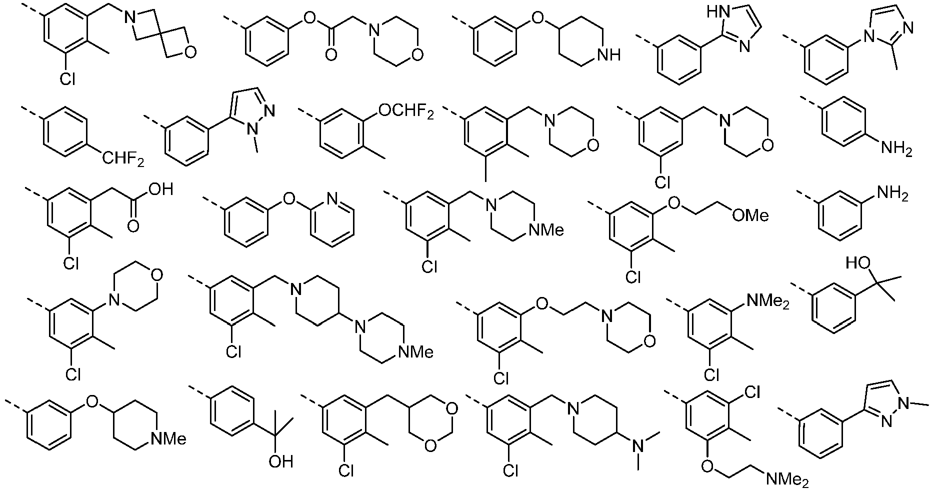

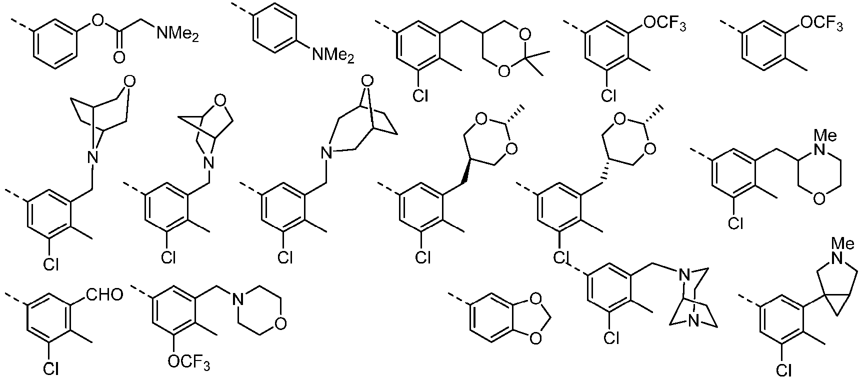

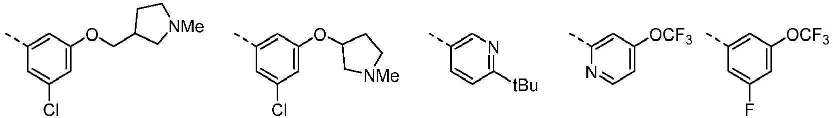

In further specific embodiments, the disclosure is directed to the specific examples disclosed in Table 1.

In some embodiments, the disclosure is directed to the (S) enantiomer of the compounds of any of formula I-VII.

In some embodiments, the disclosure is directed to the (R) enantiomer of the compounds of any of formula I-VII.

In some embodiments, the disclosure is directed to the racemate of the compounds of any of formula I-VII.

In a further aspect, the disclosure is directed to a method for producing a compound of any of formula I-VII according to the general procedure A.

The compounds of the disclosure can contain one or more asymmetric centers in the molecule. A compound without designation of the stereochemistry is to be understood to include all the optical isomers (e.g., diastereomers and enantiomers) in pure or substantially pure form, as well as mixtures thereof (e.g. a racemic mixture, or an enantiomerically enriched mixture). It is well known in the art how to prepare such optically active forms (e.g. by resolution of the racemic form by recrystallization techniques, by synthesis from optically-active starting materials, by chiral synthesis, by chromatographic separation using a chiral stationary phase, and other methods).

The compounds can be isotopically-labeled compounds, for example, compounds including various isotopes of hydrogen, carbon, nitrogen, oxygen, phosphorous, fluorine, iodine, or chlorine. The disclosed compounds may exist in tautomeric forms and mixtures and separate individual tautomers are contemplated. In addition, some compounds may exhibit polymorphism.

The compounds of the disclosure include the free form as well as the pharmaceutically acceptable salts and stereoisomers thereof. The pharmaceutically acceptable salts include all the typical pharmaceutically acceptable salts. The pharmaceutically acceptable salts of the present compounds can be synthesized from the compounds of this disclosure which contain a basic or acidic moiety by conventional chemical methods, see e.g. Berge et al, "Pharmaceutical Salts," J. Pharm. ScL, 1977:66:1-19. Furthermore, the compounds of the disclosure also include lyophilized and polymorphs of the free form.

For example, conventional pharmaceutically acceptable salts for a basic compound include those derived from inorganic acids such as hydrochloric, hydrobromic, sulfuric, sulfamic, phosphoric, and nitric, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, and trifluoroacetic. Conventional pharmaceutically acceptable salts for an acidic compound include those derived from inorganic bases include aluminum, ammonium, calcium, copper, ferric, ferrous, lithium, magnesium, manganic salts, manganous, potassium, sodium, and zinc. Salts derived from pharmaceutically acceptable organic bases include salts of primary, secondary and tertiary amines, substituted amines including naturally occurring substituted amines, cyclic amines and basic ion exchange resins, such as arginine, betaine caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, and tromethamine.

The compounds of the disclosure may exist in solid, i.e. crystalline or noncrystalline form (optionally as solvates) or liquid form. In the solid state, it may exist in, or as a mixture thereof.

In crystalline solvates, solvent molecules are incorporated into the crystalline lattice during crystallization. The formation of solvates may include non-aqueous solvents such as, but not limited to, ethanol, isopropanol, DMSO, acetic acid, ethanolamine, or ethyl acetate, or aqueous solvents such as water (also called "hydrates"). It is common knowledge that crystalline forms (and solvates thereof) may exhibit polymorphism, i.e. exist in different crystalline structures known as "polymorphs", that have the same chemical composition but differ in packing, geometrical arrangement, and other descriptive properties of the crystalline solid state. Polymorphs, therefore, may have different physical properties such as shape, density, hardness, deformability, stability, and dissolution properties, and may display different melting points, IR spectra, and X-ray powder diffraction patterns, which may be used for identification. Such different polymorphs may be produced, for example, by changing or adjusting the reaction conditions or reagents, during preparation of the compound of the disclosure.

In a further aspect, the disclosure also provides methods of preparation of the compounds of the disclosure. Typically, they are prepared according to the syntheses shown in the experimental section.

In yet another aspect, the disclosure further provides a pharmaceutical composition comprising a therapeutically-effective amount of one or more of the compounds of the disclosure or pharmaceutically acceptable salt thereof and one or more pharmaceutically acceptable carriers and/or excipients (also referred to as diluents). The excipients are acceptable in the sense of being compatible with the other ingredients of the formulation and not deleterious to the recipient thereof (i.e., the patient). The term "therapeutically-effective amount" as used herein refers to the amount of a compound (as such or in form of a pharmaceutical composition) of the present disclosure which is effective for producing some desired therapeutic effect.

Pharmaceutical compositions may be in unit dose form containing a predetermined amount of a compound of the disclosure per unit dose. Such a unit may contain a therapeutically effective dose of a compound of the disclosure or salt thereof or a fraction of a therapeutically effective dose such that multiple unit dosage forms might be administered at a given time to achieve the desired therapeutically effective dose. Preferred unit dosage formulations are those containing a daily dose or sub-dose, or an appropriate fraction thereof, of a compound of the disclosure or salt thereof.

The compounds of the disclosure may be administered by any acceptable means in solid or liquid form, including (1) oral administration, for example, drenches (aqueous or non-aqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; (8) nasally; (9) pulmonary; or (10) intrathecally.

The phrase "pharmaceutically-acceptable carrier" as used herein means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical compositions.

Such compositions may contain further components conventional in pharmaceutical preparations, e.g. wetting agents, emulsifiers and lubricants, such as sodium lauryl sulfate and magnesium stearate, as well as coloring agents, release agents, coating agents, sweetening, flavoring and perfuming agents, preservatives and antioxidants, pH modifiers, bulking agents, and further active agents. Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Such compositions may be prepared by any method known in the art, for example, by bringing into association the active ingredient with one or more carriers and/or excipients. Different compositions and examples of carriers and/or excipients are well known to the skilled person and are described in detail in, e.g., Remington: The Science and Practice of Pharmacy. Pharmaceutical Press, 2013; Rowe, Sheskey, Quinn: Handbook of Pharmaceutical Excipients.Pharmaceutical Press, 2009. Excipients that may be used in the preparation of the pharmaceutical compositions may include one or more of buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide a composition suitable for an administration of choice.

As indicated above, the compounds of the present disclosure may be in solid or liquid form and administered by various routes in any convenient administrative form, e.g., tablets, powders, capsules, solutions, dispersions, suspensions, syrups, sprays, suppositories, gels, emulsions, patches, etc.

In solid dosage forms of the disclosure for oral administration (capsules, tablets, pills, dragees, powders, granules, trouches and the like), a compound is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like. A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent. The tablets, and other solid dosage forms of the pharmaceutical compositions of the present disclosure, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose **in** varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the disclosure include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. An oral composition can also include adjuvants such as wetting agents, emulsifying and suspending agents, sweetening, flavoring, coloring, perfuming and preservative agents.

In form of suspensions, a compound may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Dosage forms for rectal or vaginal administration of a compound of the disclosure include a suppository, which may be prepared by mixing one or more compounds of the disclosure with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound. Other suitable forms include pessaries, tampons, creams, gels, pastes, foams or spray formulations containing such carriers as are known in the art to be appropriate.

Dosage forms for the topical or transdermal administration of a compound of the disclosure include powders, sprays, ointments, pastes, creams, lotions, gels, solutions, patches and inhalants. The active compound may be mixed under sterile conditions with a pharmaceutically-acceptable carrier, and with any preservatives, buffers, or propellants which may be required. Such ointments, pastes, creams and gels may contain, in addition to a compound of the disclosure, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Dosage forms such as powders and sprays for administration of a compound of the disclosure, may contain excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Dosage forms such as transdermal patches for administration of a compound of the disclosure may include absorption enhancers or retarders to increase or decrease the flux of the compound across the skin. The rate of such flux can be controlled by either providing a rate controlling membrane or dispersing the compound in a polymer matrix or gel. Other dosage forms contemplated include ophthalmic formulations, eye ointments, powders, solutions and the like. It is understood that all contemplated compositions must be stable under the conditions of manufacture and storage, and preserved against the contaminating action of microorganisms, such as bacteria and fungi.

The dosage levels of a compound of the disclosure in the pharmaceutical compositions of the disclosure may be adjusted in order to obtain an amount of a compound of the disclosure which is effective to achieve the desired therapeutic response for a particular patient, composition, and mode of administration, without being deleterious to the patient. The dosage of choice will depend upon a variety of factors including the nature of the particular compound of the present disclosure used, the route of administration, the time of administration, the rate of excretion or metabolism of the particular compound used, the rate and extent of absorption, the duration of the treatment, other drugs, compounds and/or materials used in combination with the particular compound, the age, sex, weight, condition, general health and prior medical history of the patient being treated, and like factors well known in the medical arts. A medical practitioner having ordinary skill in the art can readily determine and prescribe the effective amount of the pharmaceutical composition required.

Typically, a suitable daily dose of a compound of the disclosure will be that amount of the compound which is the lowest dose effective to produce a therapeutic effect. Such an effective dose will generally depend upon the factors described above. Generally, oral, intravenous, intracerebroventricular and subcutaneous doses of the compounds of this disclosure for a patient, when used for the indicated analgesic effects, will range from about 0.0001 to about 100 mg, more usual 0.1 to 100 mg/kg per kilogram of body weight of recipient (patient, mammal) per day. Acceptable daily dosages may be from about 1 to about 1000 mg/day, and for example, from about 1 to about 100 mg/day.

The effective dose of a compound of the disclosure may be administered as two, three, four, five, six or more sub-doses administered separately at appropriate intervals throughout a specified period (per day or per week or per month), optionally, in unit dosage forms. Preferred dosing also depends on factors as indicated above, e.g. on the administration, and can be readily arrived at by one skilled in medicine or the pharmacy art.

The compounds of the disclosure modulate the activity of cereblon. Thus, the compounds and compositions of the disclosure can be useful as a medicament, i.e. as a medicament in therapy, more specifically for the treatment of cancer, as detailed below. Therefore, in a further aspect, the present disclosure provides a compound or composition of the disclosure for use in a method of treatment of a mammal, for example, a human, suffering from cancer, as detailed below. The term "treatment" is intended to encompass prophylaxis, therapy and cure. Such treatment comprises the step of administering a therapeutically effective amount of a compound of Formula I or salt thereof (or of a pharmaceutical composition containing a compound of Formula I or salt thereof) to said mammal, for example, a human.

Thus, the disclosure is directed towards the use of the compounds of the disclosure or pharmaceutically acceptable salts or stereoisomers thereof or a pharmaceutical composition thereof for use in the treatment of a disease associated or caused with GSPT1, in particular the treatment of cancer, as detailed below, in a mammal, for example a human.

In particular embodiments, the compounds of the disclosure or pharmaceutically acceptable salts or stereoisomers thereof or a pharmaceutical composition thereof are for use in the treatment of cancer associated with GSPT1, such as glioma, thyroid cancer, lung cancer, colorectal cancer, head and neck cancer, stomach cancer, liver cancer, pancreatic cancer, renal cancer, urothelial cancer, prostate cancer, testis cancer, breast cancer, cervical cancer, endometrial cancer, ovarian cancer, melanoma and multiple myeloma.

Such a use comprises administering to a subject in need of such treatment a therapeutically effective amount of a compound of the disclosure or pharmaceutically acceptable salts thereof or a pharmaceutical composition thereof by targeting cereblon.

The present disclosure contemplates administration of a compound of the disclosure alone or in combination with one or more additional therapeutic agents, such as other Tyrosine kinase inhibitors: Erlotinib hydrochloride (e.g. Tarceva(R) by Genentech/Roche), Linifanib (or ABT 869, by Genentech), sunitinib malate (e.g. Sutent(R) by Pfizer), bosutinib (or SKI-606, described in US 6,780,996 ), dasatinib (e.g. Sprycel(R) by Bristol-Myers Squibb), armala (e.g. pazopanib, e.g. Votrient(R) by GlaxoSmithKline), imatinib and imatinib mesylate (e.g. Gilvec(R) and Gleevec(R) by Novartis); Vascular Endothelial Growth Factor (VEG) receptor inhibitors (Bevacizumab, or Avastin(R) by Genentech/Roche), axitinib, (or AG013736, described in WO 01/002369), Brivanib Alaninate (or BMS-582664), motesanib (or AMG-706, described in PCT WO 02/066470), pasireotide (e.g. SOM230, described in WO 02/010192), sorafenib (e.g. Nexavar(R)); HER2 receptor inhibitors: Trastuzumab (e.g. Herceptin(R) by Genentech/Roche), neratinib (or HKI-272, described WO 05/028443), lapatinib or lapatinib ditosylate (e.g. Tykerb(R) by GlaxoSmithKline); CD20 antibodies: Rituximab (e.g. Riuxan(R) and MabThera(R) by Genentech/Roche), tositumomab (e.g. Bexxar(R) by GlaxoSmithKline), ofatumumab (e.g. Arzerra(R) by GlaxoSmithKline); Bcr/Abl kinase inhibitors: nilotinib hydrochloride (e.g. Tasigna(R) by Novartis); DNA Synthesis inhibitors: Capecitabine (e.g. Xeloda(R) by Roche), gemcitabine hydrochloride (e.g. Gemzar(R) by Eli Lilly and Company), nelarabine (or Arranon(R) and Atriance(R) by GlaxoSmithKline); Antineoplastic agents: oxaliplatin (e.g. Eloxatin(R) ay Sanofi-Aventis described in US 4,169,846 ); Epidermal growth factor receptor (EGFR) inhibitors: Gefitinib (or Iressa(R)), Afatinib (orTovok(R) by Boehringer Ingelheim), cetuximab (e.g. Erbitux(R) by Bristol-Myers Squibb), panitumumab (e.g. Vectibix(R) by Amgen); HER dimerization inhibitors: Pertuzumab (e.g. Omnitarg(R), by Genentech); Human Granulocyte colony-stimulatingfactor (G-CSF) modulators: Filgrastim (e.g. Neupogen(R) by Amgen); Immunomodulators: Afutuzumab (by Roche(R)), pegfilgrastim (e.g. Neulasta(R) by Amgen), lenalidomide (e.g. CC-5013, e.g. Revlimid(R)), thalidomide (e.g. Thalomid(R)); (m) CD40 inhibitors: Dacetuzumab (e.g. SGN-40 or huS2C6, by Seattle Genetics, Inc); Proapoptotic receptor agonists (PARAs): Dulanermin (e.g. AMG-951, by Amgen/Genentech); Hedgehog antagonists: Vismodegib (or GDC-0449, described in WO 06/028958); PI3K inhibitors: Pictilisib (or GDC-0941 described in WO 09/036082 and WO 09/055730 ), Dactolisib (or BEZ 235 or NVP-BEZ 235, described in WO 06/122806); Phospholipase A2 inhibitors: Anagrelide (e.g. Agrylin(R)); BCL-2 inhibitors: Navitoclax (or ABT-263, described in WO 09/155386); Mitogen-activated protein kinase kinase (MEK) inhibitors: XL-518 (Cas No. 1029872-29-4, by ACC Corp.); Aromatase inhibitors: Exemestane (e.g. Aromasin(R) by Pfizer), letrozole (e.g. Femara(R) by Novartis), anastrozole (e.g. Arimidex(R)); Topoisomerase I inhibitors: Irinotecan (e.g. Camptosar(R) by Pfizer), topotecan hydrochloride (e.g. Hycamtin(R) by GlaxoSmithKline); Topoisomerase II inhibitors: etoposide (e.g. VP-16 and Etoposide phosphate, e.g. Toposar(R), VePesid(R) and Etopophos(R)), teniposide (e.g. VM-26, e.g. Vumon(R)); mTOR inhibitors: Temsirolimus (e.g. Torisel(R) by Pfizer), ridaforolimus (formally known as deferolimus, (or AP23573 and MK8669, described in WO 03/064383), everolimus (e.g. Afinitor(R) by Novartis); Osteoclastic bone resorption inhibitors: zoledronic acid (or Zometa(R) by Novartis); CD33 Antibody Drug Conjugates: Gemtuzumab ozogamicin (e.g. Mylotarg(R) by Pfizer/Wyeth); CD22 Antibody Drug Conjugates: Inotuzumab ozogamicin (also referred to as CMC-544 and WAY-207294, by Hangzhou Sage Chemical Co., Ltd.); CD20 Antibody Drug Conjugates: Ibritumomab tiuxetan (e.g. Zevalin(R)); Somatostain analogs: octreotide (e.g. octreotide acetate, e.g. Sandostatin(R) and Sandostatin LAR(R)); Synthetic Interleukin-11 (IL-11): oprelvekin (e.g. Neumega(R) by Pfizer/Wyeth); Synthetic erythropoietin: Darbepoetin alfa (e.g. Aranesp(R) by Amgen); Receptor Activator for Nuclear Factor kappa B (RANK) inhibitors: Denosumab (e.g. Prolia(R) by Amgen); Thrombopoietin mimetic peptibodies: Romiplostim (e.g. Nplate(R) by Amgen; Cell growth stimulators: Palifermin (e.g. Kepivance(R) by Amgen); Anti-Insulin-like Growth Factor-1 receptor (IGF-1R) antibodies: Figitumumab (e.g. CP-751,871, by ACC Corp), robatumumab (CAS No. 934235-44-6); Anti-CS1 antibodies: Elotuzumab (HuLuc63, CAS No. 915296-00-3); CD52 antibodies: Alemtuzumab (e.g. Campath(R)); CTLA-4 inhibitors: Tremelimumab (IgG2 monoclonal antibody by Pfizer, formerly known as ticilimumab, CP-675,206), ipilimumab (CTLA-4 antibody, e.g. MDX-010, CAS No. 477202-00-9); Histone deacetylase inhibitors (HDI): Voninostat (e.g. Zolinza(R) by Merck); Alkylating agents: Temozolomide (e.g. Temodar(R) and Temodal(R) by Schering-Plough/Merck), dactinomycin (e.g. actinomycin-D and e.g. Cosmegen(R)), melphalan (e.g. L-PAM, L-sarcolysin, and phenylalanine mustard, e.g. Alkeran(R)), altretamine (e.g. hexamethylmelamine (HMM), e.g. Hexalen(R)), carmustine (e.g. BiCNU(R)), bendamustine (e.g. Treanda(R)), busulfan (e.g. Busulfex(R) and Myleran(R)), carboplatin (e.g. Paraplatin(R)), lomustine (e.g. CCNU, e.g. CeeNU(R)), cisplatin (e.g. CDDP, e.g. Platinol(R) and Platinol(R)-AQ), chlorambucil (e.g. Leukeran(R)), cyclophosphamide (e.g. Cytoxan(R) and Neosar(R)), dacarbazine (e.g. DTIC, DIC and imidazole carboxamide, e.g. DTIC-Dome(R)), altretamine (e.g. hexamethylmelamine (HMM) e.g. Hexalen(R)), ifosfamide (e.g. Ifex(R)), procarbazine (e.g. Matulane(R)), mechlorethamine (e.g. nitrogen mustard, mustine and mechloroethamine hydrochloride, e.g. Mustargen(R)), streptozocin (e.g. Zanosar(R)), thiotepa (e.g. thiophosphoamide, TESPA and TSPA, e.g. Thioplex(R); Biologic response modifiers: bacillus calmette-guerin (e.g. theraCys(R) and TICE(R) BCG), denileukin diftitox (e.g. Ontak(R)); Anti-tumor antibiotics: doxorubicin (e.g. Adriamycin(R) and Rubex(R)), bleomycin (e.g. Ienoxane(R)), daunorubicin (e.g. dauorubicin hydrochloride, daunomycin, and rubidomycin hydrochloride, e.g. Cerubidine(R)), daunorubicin liposomal (daunorubicin citrate liposome, e.g. DaunoXome(R)), mitoxantrone (e.g. DHAD, e.g. Novantrone(R)), epirubicin (e.g. Ellence^{™}), idarubicin (e.g. Idamycin(R), Idamycin PFS(R)), mitomycin C (e.g. Mutamycin(R)); Anti-microtubule agents: Estramustine (e.g. Emcyl(R)); Cathepsin K inhibitors: Odanacatib (or MK-0822, by Lanzhou Chon Chemicals, ACC Corp., and ChemieTek, described in WO 03/075836); Epothilone B analogs: Ixabepilone (e.g. Lxempra(R) by Bristol-Myers Squibb); Heat Shock Protein (HSP) inhibitors: Tanespimycin (17-allylamino-17-demethoxygeldanamycin, e.g. KOS-953 and 17-AAG, by SIGMA, described in US 4,261,989); TpoR agonists: Eltrombopag (e.g. Promacta(R) and Revolade(R) by GlaxoSmithKline); Anti-mitotic agents: Docetaxel (e.g. Taxotere(R) by Sanofi-Aventis); Adrenal steroid inhibitors: aminoglutethimide (e.g. Cytadren(R)); Antiandrogens: Nilutamide (e.g. Nilandron(R) and Anandron(R)), bicalutamide (sold under tradename Casodex(R)), flutamide (e.g. Fulexin^{™}); Androgens: Fluoxymesterone (e.g. halotestin(R)); Proteasome inhibitors: Bortezomib (e.g. Velcade(R)); CDK1 inhibitors: Alvocidib (e.g. flovopirdol or HMR-1275, described in US 5,621,002); Gonadotropin-releasing hormone (GnRH) receptor agonists: Leuprolide or leuprolide acetate (e.g. Viadure(R) by Bayer AG, Eligard(R) by Sanofi-Aventis and Lupron(R) by Abbott Lab); Taxane anti-neoplastic agents: Cabazitaxel, larotaxel; 5HT1a receptor agonists: Xaliproden (or SR57746, described in US 5,266,573); HPC vaccines: Cervarix(R) sold by GlaxoSmithKline, Gardasil(R) sold by Merck; Iron Chelating agents: Deferasinox (e.g. Exjade(R) by Novartis); Anti-metabolites: Claribine (2-chlorodeoxyadenosine, e.g. leustatin(R)), 5-fluorouracil (e.g. Adrucil(R)), 6-thioguanine (e.g. Purinethol(R)), pemetrexed (e.g. Alimta(R)), cytarabine (e.g. arabinosylcytosine (Ara-C), e.g. Cytosar-U(R)), cytarabine liposomal (e.g. Liposomal Ara-C, e.g. DepoCyt^{™}), decitabine (e.g. Dacogen(R)), hydroxyurea (e.g. Hydrea(R), Droxia^{™} and Mylocel^{™}), fludarabine (e.g. Fludara(R)), floxuridine (e.g. FUDR(R)), cladribine (e.g. 2-chlorodeoxyadenosine (2-CdA) e.g. Leustatin^{™}), methotrexate (e.g. amethopterin, methotrexate sodim (MTX), e.g. Rheumatrex(R) and Trexall^{™}), pentostatin (e.g. Nipent(R)); Bisphosphonates: Pamidronate (e.g. Aredia(R)), zoledronic acid (e.g. Zometa(R)); Demethylating agents: 5-azacitidine (e.g. Vidaza(R)), decitabine (e.g. Dacogen(R)); Plant Alkaloids: Paclitaxel protein-bound (e.g. Abraxane(R)), vinblastine (e.g. vinblastine sulfate, vincaleukoblastine and VLB, e.g. Alkaban-AQ(R) and Velban(R)), vincristine (e.g. vincristine sulfate, LCR, and VCR, e.g. Oncovin(R) and Vincasar Pfs(R)), vinorelbine (e.g. Navelbine(R)), paclitaxel (e.g. Taxol and Onxal^{™}); Retinoids: Alitretinoin (e.g. Panretin(R)), tretinoin (all-trans retinoic acid, e.g. ATRA, e.g. Vesanoid(R)), Isotretinoin (13-cis-retinoic acid, e.g. Accutane(R), Amnesteem(R), Claravis(R), Clarus(R), Decutan(R), Isotane(R), Izotech(R), Oratane(R), Isotret(R), and Sotret(R)), bexarotene (e.g. Targretin(R)); Glucocorticosteroids: Hydrocortisone (e.g. cortisone, hydrocortisone sodium succinate, hydrocortisone sodium phosphate, and e.g. Ala-Cort(R), Hydrocortisone Phosphate, Solu-Cortef(R), Hydrocort Acetate(R) and Lanacort(R)), dexamethasone, prednisolone (e.g. Delta-Cortel(R), Orapred(R), Pediapred(R) and Prelone(R)), prednisone (e.g. Deltasone(R), Liquid Red(R), Meticorten(R) and Orasone(R)), methylprednisolone (e.g. 6-Methylprednisolone, Methylprednisolone Acetate, Methylprednisolone Sodium Succinate, e.g. Duralone(R), Medralone(R), Medrol(R), M-Prednisol(R) and Solu-Medrol(R)); Cytokines: interleukin-2 (e.g. aldesleukin and IL-2, e.g. Proleukin(R)), interleukin-11 (e.g. oprevelkin, e.g. Neumega(R)), alpha interferon alfa (e.g. IFN-alpha, e.g. Intron(R) A, and Roferon-A(R)); Lutinizing hormone releasing hormone (LHRH) agonists: Goserelin (e.g. Zoladex(R)); Progesterones: megestrol (e.g. megestrol acetate, e.g. Megace(R)); Miscellaneous cytotoxic agents: Arsenic trioxide (e.g. Trisenox(R)), asparaginase (e.g. L-asparaginase, Erwinia L-asparaginase, e.g. Elspar(R) and Kidrolase(R)); Anti-nausea drugs: NK-1 receptor antagonists: Casopitant (e.g. Rezonic(R) and Zunrisa(R) by GlaxoSmithKline); and Cytoprotective agents: Amifostine (e.g. Ethyol(R)), leucovorin (e.g. calcium leucovorin, citrovorum factor and folinic acid).

### Examples

### General Procedure A:

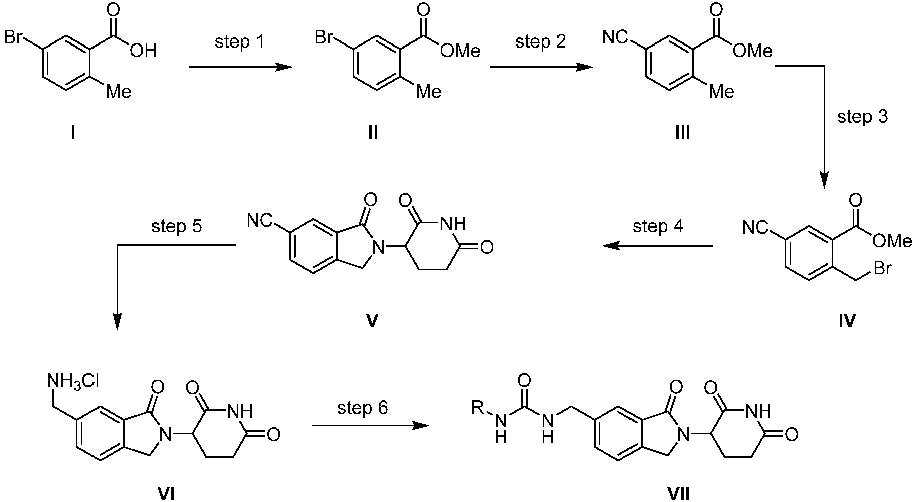

**II:** To a solution of 5-bromo-2-methyl-benzoic acid (100 g, 465 mmol, 1.00 eq) in methanol (700 mL) was added sulfuric acid (31.3 g, 313 mmol, 0.672 eq) dropwise at 0 °C. The mixture was stirred at 25 °C for 1 h and then heat to 70 °C for 12 h. The mixture was cooled to 25 °C and concentrated under reduced pressure. The residue was poured into ice-water (200 mL) and basified with solid sodium carbonate to pH = 8. The aqueous phase was extracted with ethyl acetate (3 × 300 mL). The combined organic phase was washed with brine (2 × 100 mL), dried over anhydrous sodium sulfate, filtered and concentrated in vacuum to afford methyl 5-bromo-2-methyl-benzoate II (113 g, crude) as a yellow solid. The product was taken into the next step without purification.

**III:** To a solution of methyl 5-bromo-2-methyl-benzoate **II** (113 g, 493 mmol, 1.00 eq) in N,N-dimethylformamide (300 mL) was added copper(I)cyanide (66.3 g, 1.50 eq). The mixture was stirred at 150 °C for 12 h. The mixture was quenched by ice slowly and then extracted with ethyl acetate (2 × 500 mL). The aqueous phase was extracted with ethyl acetate (2 × 300 mL). The combined organic phase was washed with brine (2 × 100 mL), dried over anhydrous sodium sulfate, filtered and concentrated in vacuum. The residue was purified by silica gel column chromatography (petroleum ether/ethyl acetate = 0/1 to 100/1) to afford methyl 5-cyano-2-methyl-benzoate **III** (61.0 g, 348 mmol, 70% yield) as a yellow solid. ¹H NMR (400 MHz, CDCl₃-d) δ = 8.22 (d, J = 1.5 Hz, 1H), 7.67 (dd, J = 1.7, 7.9 Hz, 1H), 7.38 (d, J = 7.9 Hz, 1H), 3.93 (s, 3H), 2.68 (s, 3H).

**IV:** To a solution of methyl 5-cyano-2-methyl-benzoate **III** (61.0 g, 348 mmol, 1.00 eq) and N-bromosuccinimide (75.8 g, 383 mmol, 1.10 eq) in carbon tetrachloride (650 mL) was added benzoyl peroxide (8.43 g, 34.8 mmol, 0.100 eq). The mixture was stirred at 80 °C for 12 h. The mixture was concentrated in vacuum, suspended in water (500 mL) and then extracted with ethyl acetate (2 × 500 mL). The aqueous phase was extracted with ethyl acetate (2 × 300 mL). The combined organic phase was washed with brine (2 × 100 mL), dried over anhydrous sodium sulfate, filtered and concentrated in vacuum. The residue was purified by silica gel chromatography (petroleum ether/ethyl acetate = 0/1 to 5/1) to afford methyl 2-(bromomethyl)-5-cyano- benzoate **IV** (80.0 g, 315 mmol, 90% yield) as a yellow solid. ¹H NMR (400 MHz, CDCl₃-d) δ = 8.20 (d, J = 1.6 Hz, 1H), 7.70 (dd, J = 1.8, 8.0 Hz, 1H), 7.54 (d, J = 8.0 Hz, 1H), 4.89 (s, 2H), 3.91 (s, 3H).

**V:** To a solution of methyl 2-(bromomethyl)-5-cyano-benzoate **IV** (40 g, 157 mmol, 1.00 eq) and 3-aminopiperidine-2,6-dione (25.9 g, 157 mmol, 1.00 eq, hydrochloric acid) in dimethylsulfoxide (200 mL) was added triethylamine (65.7 mL, 3.00 eq). The mixture was stirred at 100 °C for 2 h. The crude product was triturated with water (300 mL). The mixture was filtered, and the filtrate was concentrated in vacuum, and then the solid was triturated with methanol (30.0 mL). The residue was filtered, and the filtrate was concentrated in vacuum to afford 2-(2,6-dioxo-3-piperidyl)-3-oxo-isoindoline- 5-carbonitrile V (21.0 g, 78.0 mmol, 49% yield) as an off-white solid. ¹H NMR (400 MHz, DMSO-d6) δ = 11.01 (s, 1H), 8.20 (s, 1H), 8.09 (dd, J = 1.5, 7.9 Hz, 1H), 7.85 (d, J = 7.9 Hz, 1H), 5.15 (dd, J = 5.1, 13.3 Hz, 1H), 4.64 - 4.40 (m, 2H), 2.99 - 2.85 (m, 1H), 2.65 - 2.56 (m, 1H), 2.44 - 2.34 (m, 1H), 2.03 (dtd, J = 2.0, 5.2, 12.5 Hz, 1H).

VI: To a solution of 2-(2,6-dioxo-3-piperidyl)-3-oxo-isoindoline-5-carbonitrile V (5.00 g, 18.6 mmol, 1.00 eq) and hydrochloric acid (12 M, 5.00 mL, 3.23 eq) in methanol (500 mL) was added platinum(IV)oxide (1.05 g, 4.64 mmol, 0.250 eq) under nitrogen. The suspension was degassed under vacuum and purged with hydrogen several times. The mixture was stirred under hydrogen (15 psi) at 25 °C for 12 h. The reaction mixture was filtered, and the filtrate was concentrated in vacuum. The crude product was triturated with ethyl alcohol (10.0 mL), the mixture was filtered and the filtrate was dried in vacuum to afford 3-(6-(aminomethyl)-1-oxoisoindolin-2-yl)piperidine-2,6-dione hydrochloride VI (5.30 g, 17.1 mmol, 92% yield, hydrochloric acid) as a white solid. ¹H NMR (400 MHz, DMSO-d6) δ = 10.99 (s, 1H), 8.54 (br s, 3H), 7.89 (d, J = 0.7 Hz, 1H), 7.75 (dd, J = 1.6, 7.8 Hz, 1H), 7.65 (d, J = 7.8 Hz, 1H), 5.12 (dd, J = 5.1, 13.3 Hz, 1H), 4.54 - 4.30 (m, 2H), 4.14 (q, J = 5.3 Hz, 2H), 2.97 - 2.85 (m, 1H), 2.65 - 2.57 (m, 1H), 2.44 - 2.38 (m, 1H), 2.01 (dtd, J = 2.0, 5.2, 12.5 Hz, 1H).

VII:
Variant i): To a solution of 3-(6-(aminomethyl)-1-oxoisoindolin-2-yl)piperidine-2,6-dione hydrochloride VI (1.00 eq, hydrochloride) and triethylamine (3.0 eq) in N,N-dimethylformamide (0.15 M) was added the isocyanate R-NCO (1 eq) in N,N-dimethylformamide (0.5 M) at 0 °C. The mixture was stirred at rt for 45 min. Polymer supported trisamine (2.0 eq) was added and stirred for 30 min. The mixture was filtered, washed with N,N-dimethylformamide, and the filtrate concentrated in vacuum. The residue was purified by silica column chromatography (Biotage SNAP ultra, 0-15% methanol in dichloromethane) to afford afford the final urea-compounds VII as solids.

Variant ii): To a solution of 3-(6-(aminomethyl)-1-oxoisoindolin-2-yl)piperidine-2,6-dione hydrochloride VI (1.00 eq, hydrochloride) and triethylamine (1.10 eq) in N,N-dimethylformamide (0.1 M) was added the isocyanate R-NCO (1.10 eq) at 0 °C. The mixture was stirred at 25 °C for 1 h. The mixture was quenched by the addition of methanol and concentrated in vacuum. The residue was purified by preparative HPLC (column: Shim-pack C18 150*25*10µm. Mobile phase: [water(0.225%FA)-ACN];B%: 10%-40%,10min) and lyophilized to afford the final urea-compounds VII as solids.

Variant iii): To a solution of 3-(6-(aminomethyl)-1-oxoisoindolin-2-yl)piperidine-2,6-dione hydrochloride VI (0.8-1.3 eq, hydrochloride) and triethylamine (1.2-20 eq) in a polar aprotic solvent (dimethylformamide, dichloromethane, or tetrahydrofuran, 0.03-0.71 M) was added the O-phenylcarbamate R-N(CO)O-Ph (0.8-2 eq). The reaction was stirred at a temperature range of 20 to 60°C for 1 to 24 h. The reaction was extracted (the mixture was diluted with water and extracted with ethyl acetate, and the combined organic layers were washed with brine, dried over anhydrous sodium sulfate, filtered, and concentrated under reduced pressure) or it was directly concentrated under reduced pressure (with or without prior dilution with DMSO and/or filtration) to give a residue. Unless otherwise specified, the obtained residue was purified by reversed phase preparative HPLC or by trituration (ethyl acetate was added, and the mixture was cooled to 15°C for 5 min; the precipitate was filtered and dried). The purified compounds were lyophilized to afford the final urea compounds VII as solids.

Variant iv): To a solution of 3-(6-(aminomethyl)-1-oxoisoindolin-2-yl)piperidine-2,6-dione hydrochloride VI (1.00 eq, hydrochloride) and triethylamine (1.10-3.00 eq) in a polar aprotic solvent (dimethylformamide or dichloromethane, 0.09-0.33 M) was added the isocyanate R-NCO (1.00-5.00 eq, neat or as a solution in dichloromethane dropwise) at 0°C or 20°C. The reaction was stirred at a temperature range of 20-30°C for 1-12 h. Unless otherwise specified, the reaction was concentrated under reduced pressure (with or without prior dilution with DMSO and/or filtration) to give a residue. Unless otherwise specified, the obtained residue was purified by reversed phase preparative HPLC. The purified compounds were lyophilized to afford the final urea compounds VII as solids.

**Table 1: Specific examples**

| Compound | No. | Compound | No. |
|---|---|---|---|
| 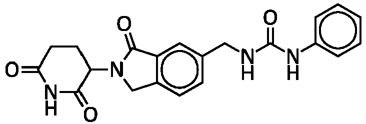 | 1 | 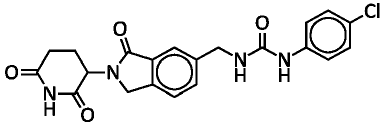 | 2 |
| 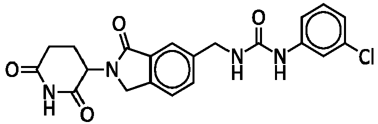 | 3 | 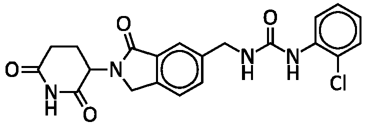 | 4 |
| 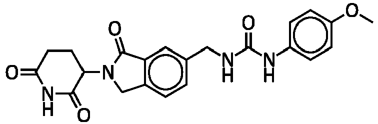 | 5 | 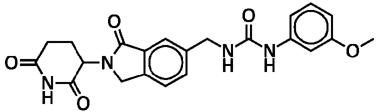 | 6 |
| 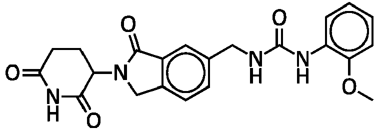 | 7 | 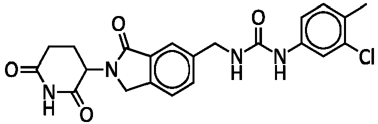 | 8 |
| 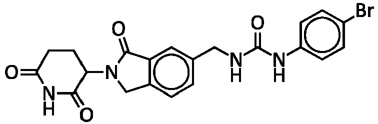 | 9 | 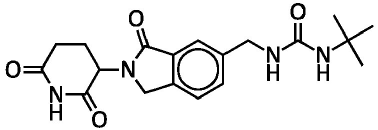 | 10 |
| 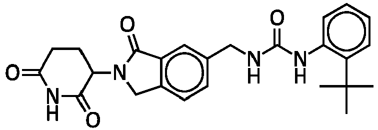 | 11 | 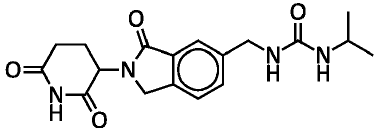 | 12 |
| 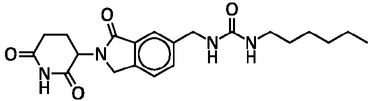 | 13 | 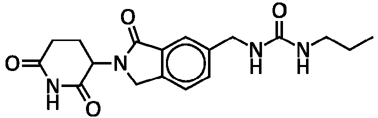 | 14 |
| 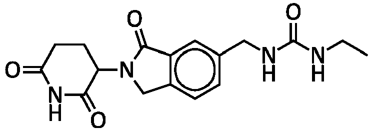 | 15 | 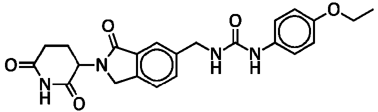 | 16 |
| 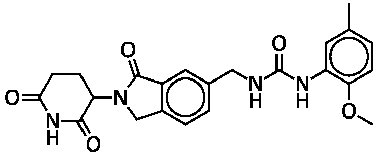 | 17 | 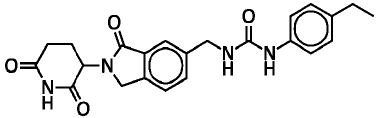 | 18 |
| 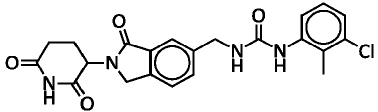 | 19 | 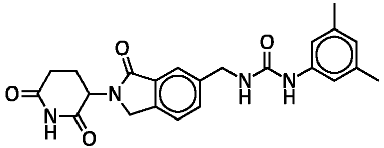 | 20 |
| 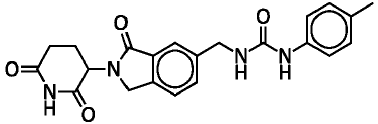 | 21 | 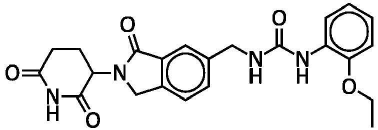 | 22 |
| 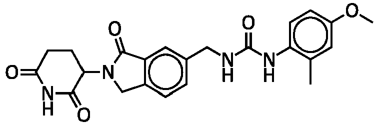 | 23 | 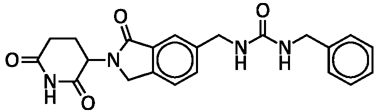 | 24 |
| 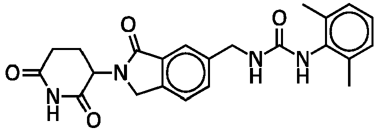 | 25 | 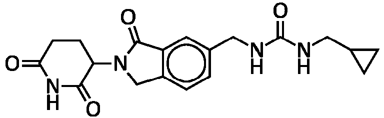 | 26 |
| 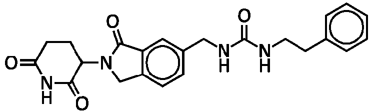 | 27 | 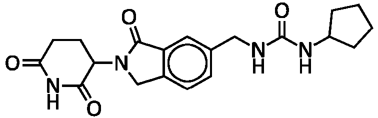 | 28 |
| 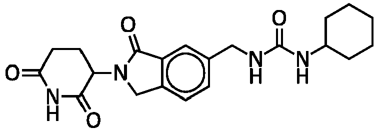 | 29 | 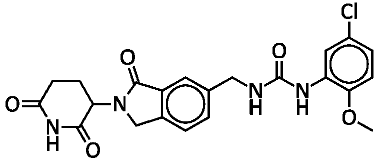 | 30 |
| 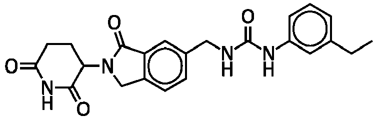 | 31 | 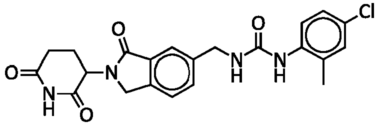 | 32 |
| 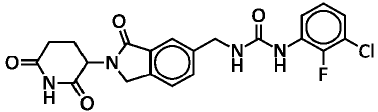 | 33 | 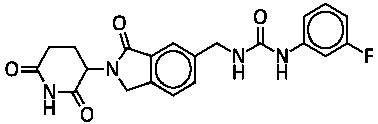 | 34 |
| 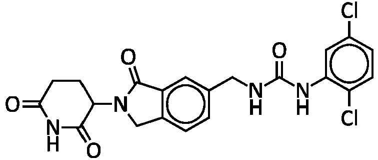 | 35 | 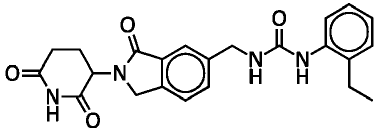 | 36 |
| 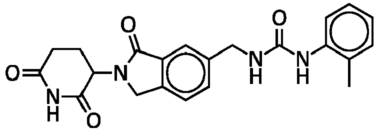 | 37 | 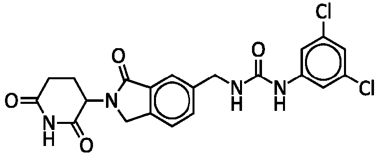 | 38 |
| 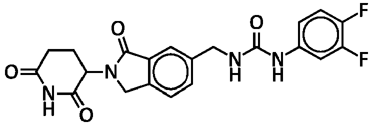 | 39 | 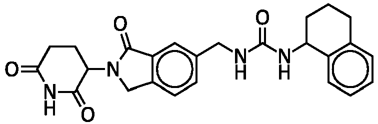 | 40 |
| 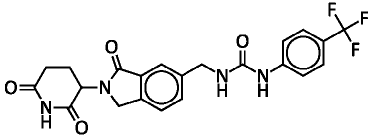 | 41 | 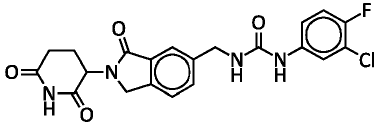 | 42 |
| 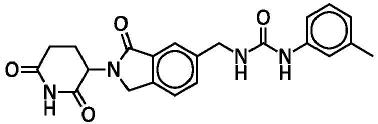 | 43 | 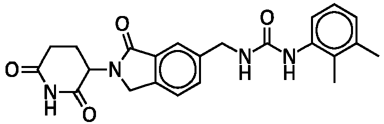 | 44 |
| 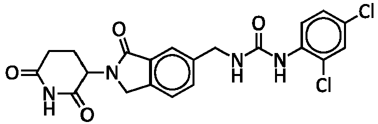 | 45 | 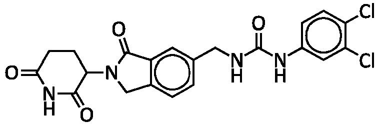 | 46 |
| 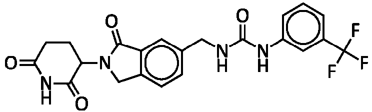 | 47 | 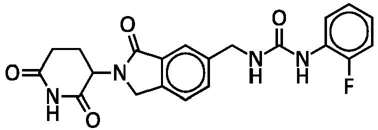 | 48 |
| 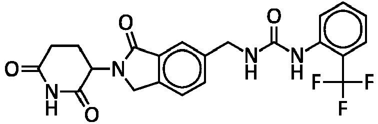 | 49 | 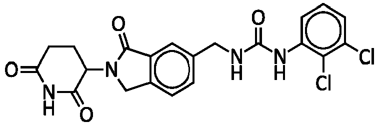 | 50 |
| 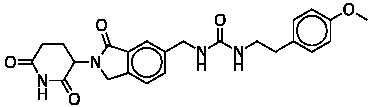 | 51 | 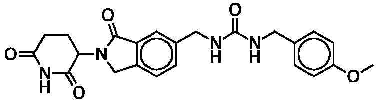 | 52 |
| 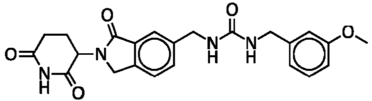 | 53 | 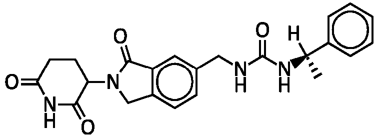 | 54 |
| 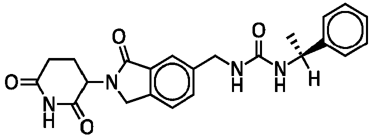 | 55 | 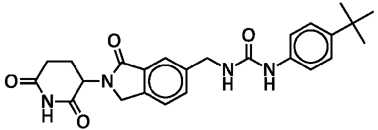 | 56 |
| 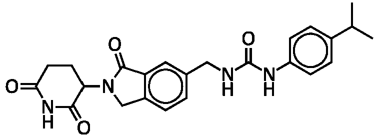 | 57 | 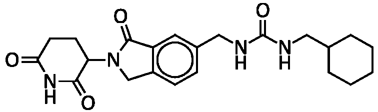 | 58 |
| 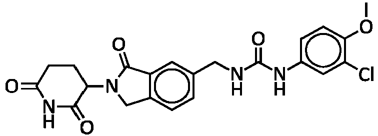 | 59 | 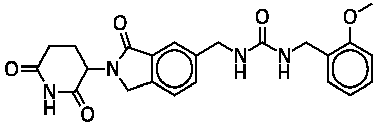 | 60 |
| 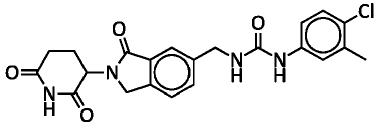 | 61 | 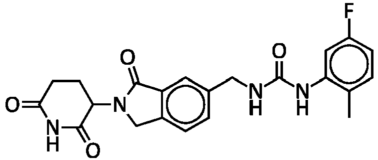 | 62 |
| 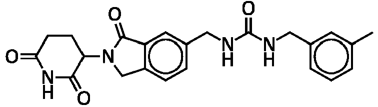 | 63 | 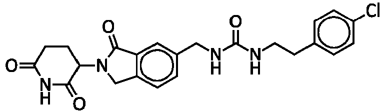 | 64 |
| 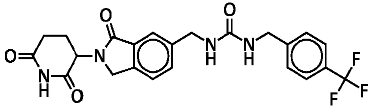 | 65 | 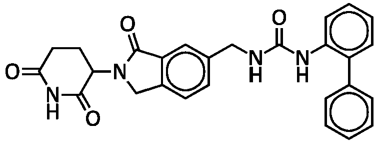 | 66 |
| 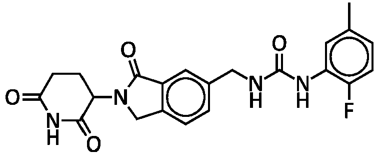 | 67 | 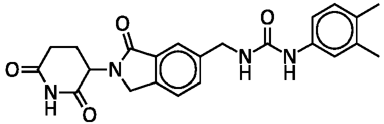 | 68 |
| 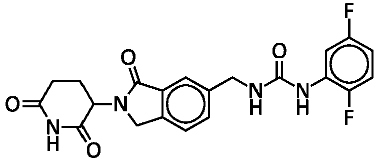 | 69 | 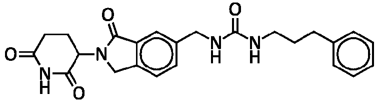 | 70 |
| 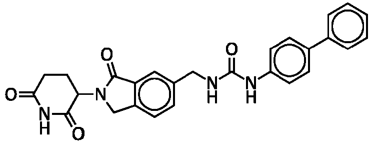 | 71 | 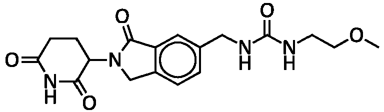 | 72 |
| 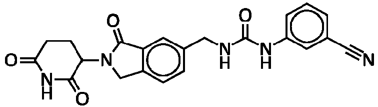 | 73 | 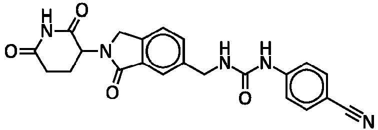 | 74 |
| 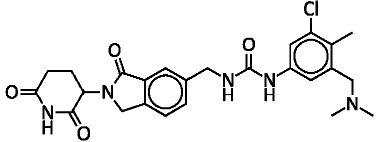 | 75 | 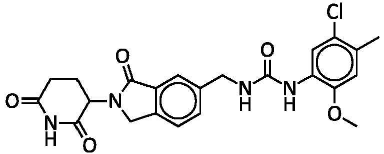 | 76 |
| 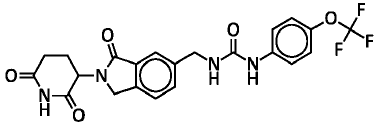 | 77 | 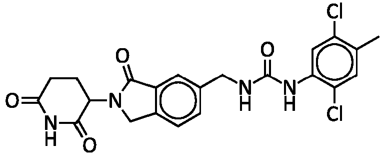 | 78 |
| 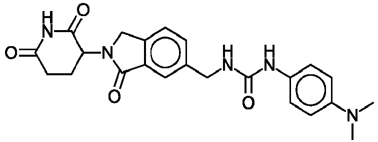 | 79 | 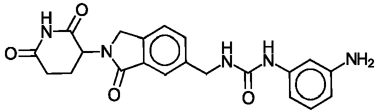 | 80 |
| 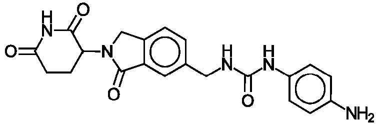 | 81 | 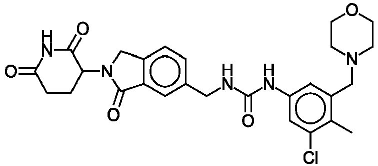 | 82 |
| 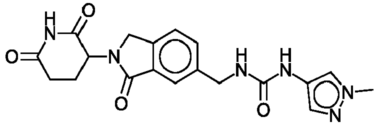 | 83 | 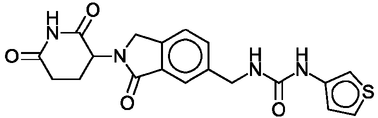 | 84 |
| 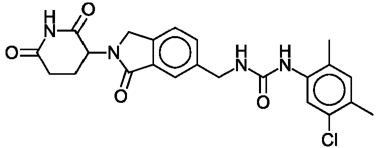 | 85 | 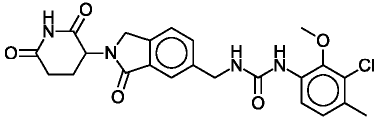 | 86 |
| 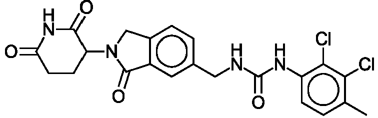 | 87 | 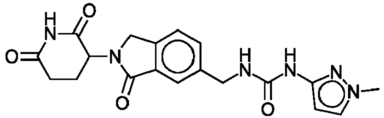 | 88 |
| 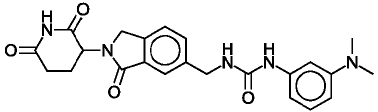 | 89 | 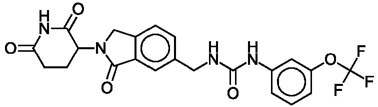 | 90 |
| 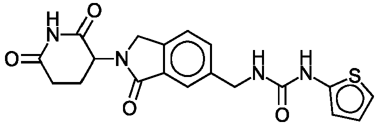 | 91 | 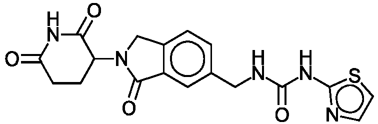 | 92 |
| 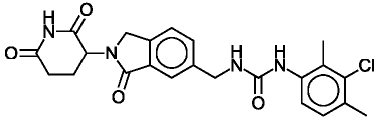 | 93 | 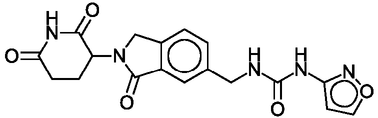 | 94 |
| 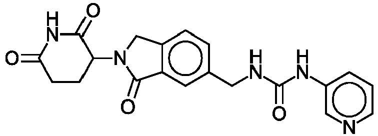 | 95 | 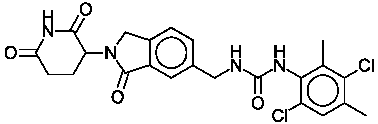 | 96 |
| 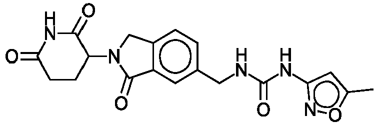 | 97 | 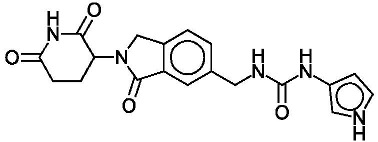 | 98 |
| 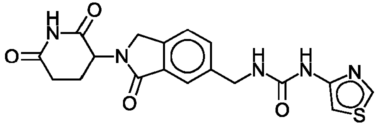 | 99 | 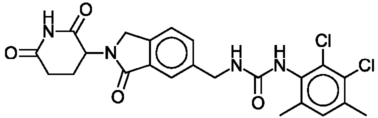 | 100 |
| 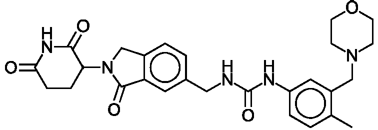 | 101 | 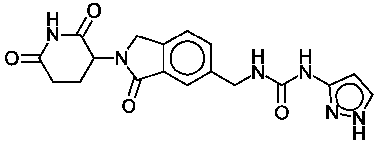 | 102 |
| 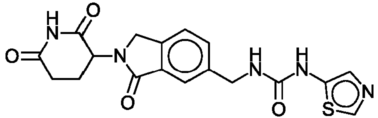 | 103 | 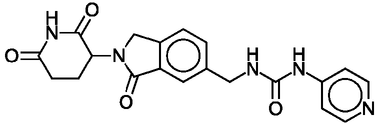 | 104 |
| 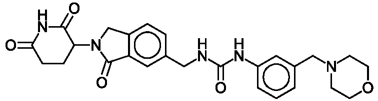 | 105 | 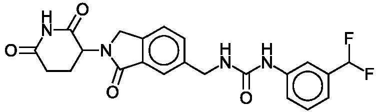 | 106 |
| 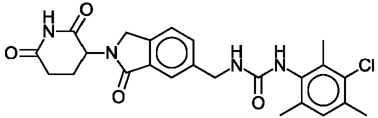 | 107 | 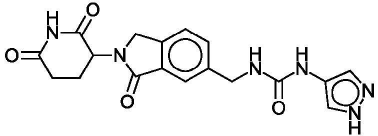 | 108 |
| 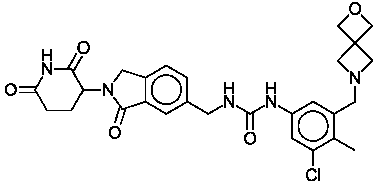 | 109 | 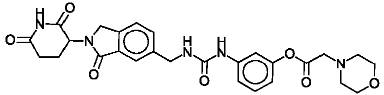 | 110 |
| 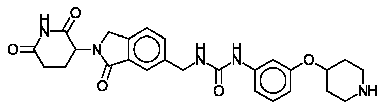 | 111 | 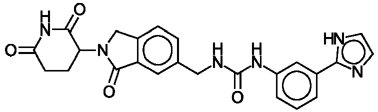 | 112 |
| 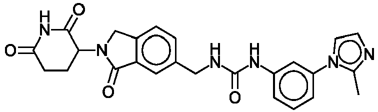 | 113 | 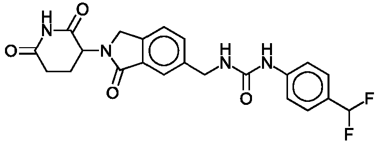 | 114 |
| 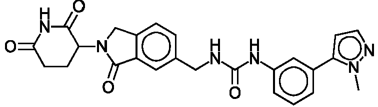 | 115 | 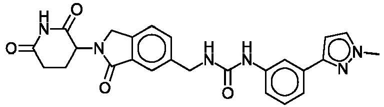 | 116 |
| 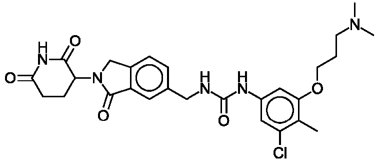 | 117 | 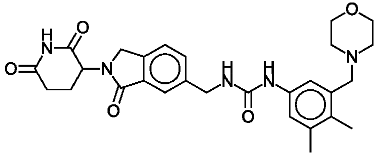 | 118 |
| 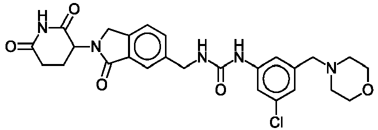 | 119 | 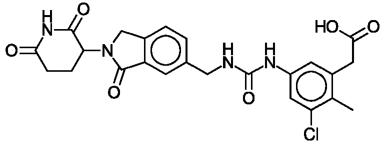 | 120 |
| 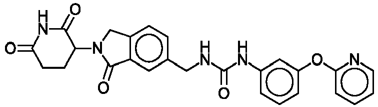 | 121 | 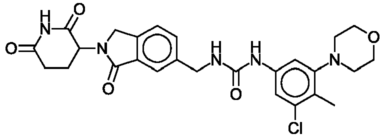 | 122 |
| 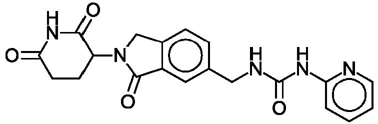 | 123 | 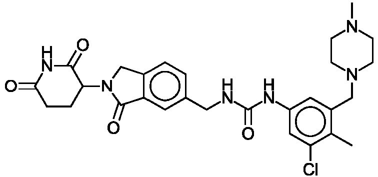 | 124 |
| 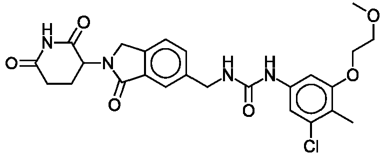 | 125 | 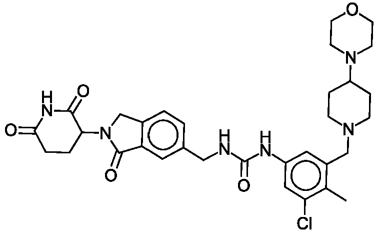 | 126 |
| 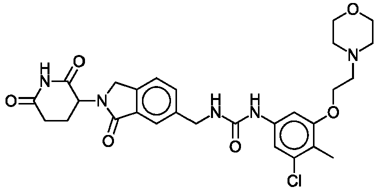 | 127 | 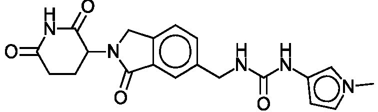 | 128 |
| 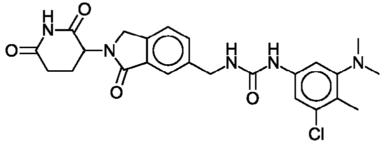 | 129 | 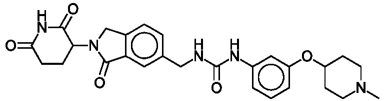 | 130 |
| 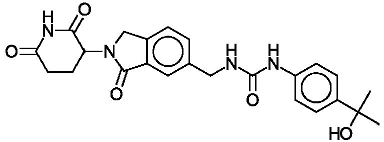 | 131 | 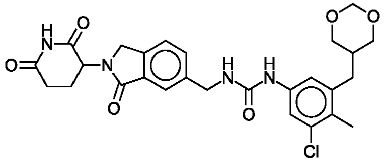 | 132 |
| 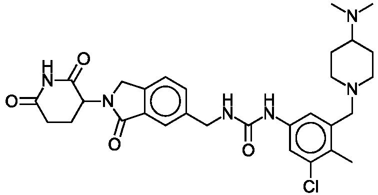 | 133 | 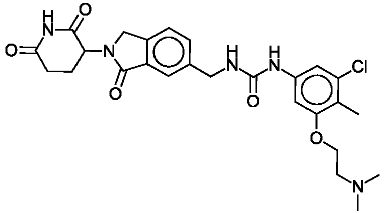 | 134 |
| 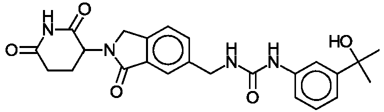 | 135 | 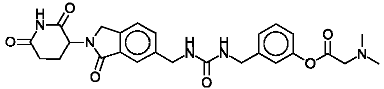 | 136 |
| 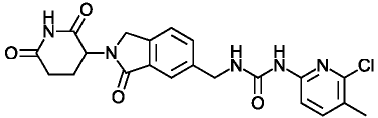 | 137 | 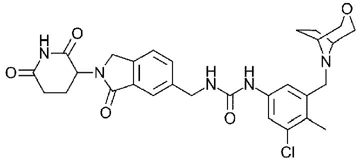 | 138 |
| 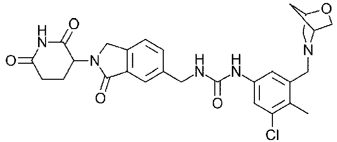 | **19** | 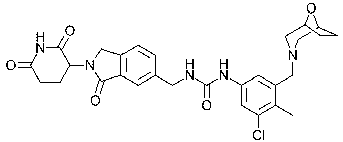 | 140 |
| 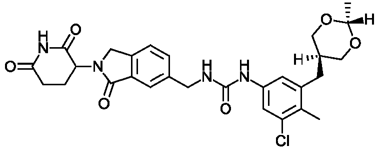 | 141 | 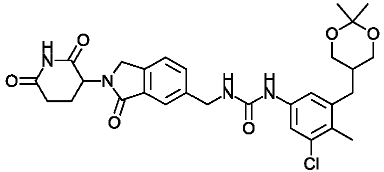 | 142 |
| 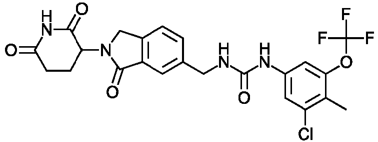 | 143 | 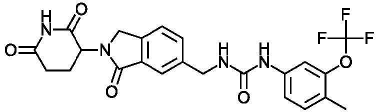 | 144 |
| 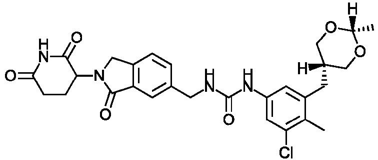 | 145 | 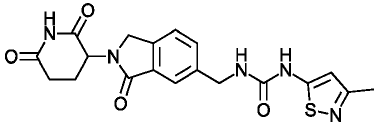 | 146 |
| 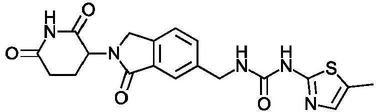 | 147 | 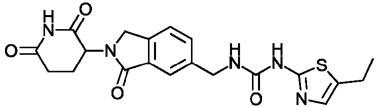 | 148 |
| 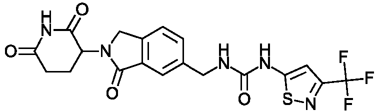 | 149 | 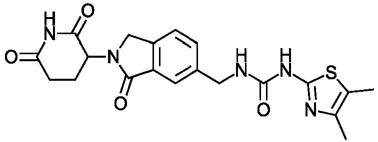 | 150 |
| 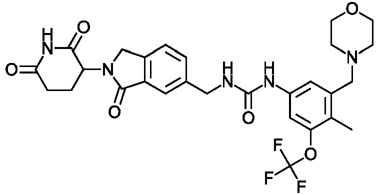 | 151 | 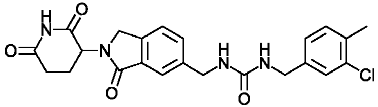 | 152 |
| 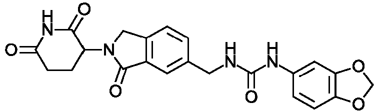 | 153 | 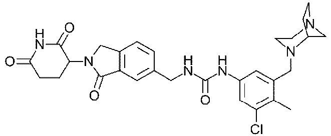 | 154 |
| 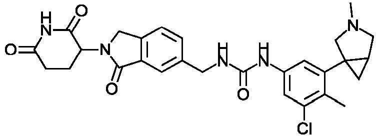 | 155 | 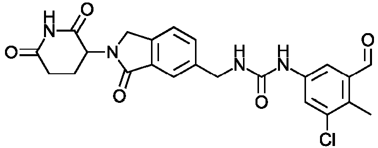 | 156 |
| 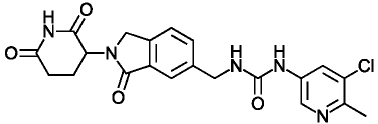 | 157 | 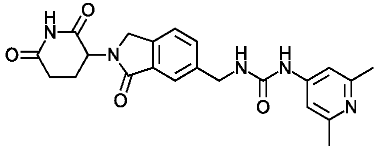 | 158 |
| 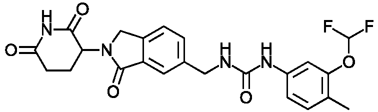 | 159 | 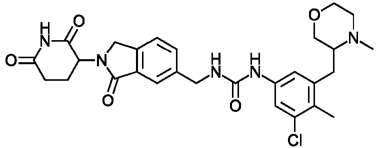 | 160 |
| 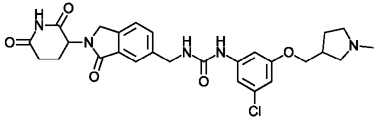 | 161 | 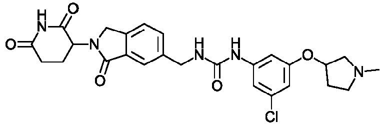 | 162 |
| 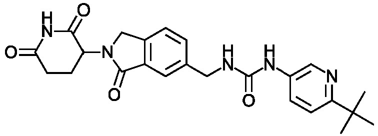 | 163 | 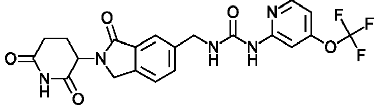 | 164 |
| 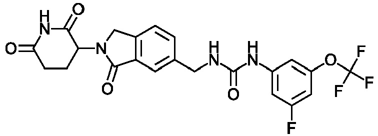 | 165 | | |

**Compound 1:** General procedure A with variant i) was used for the preparation with a yield of 75% from compound VI employing phenyl-isocyanate. ¹H NMR (600 MHz, DMSO-*d*₆) δ = 10.98 (s, 1H), 8.61 (s, 1H), 7.67 (s, 1H), 7.60 - 7.53 (m, 2H), 7.43 - 7.37 (m, 2H), 7.25 - 7.18 (m, 2H), 6.93 - 6.86 (m, 1H), 6.74 (t, *J=* 6.1 Hz, 1H), 5.11 (dd, *J* = 13.3, 5.1 Hz, 1H), 4.43 (d, J= 17.1 Hz, 1H), 4.40 (d, J= 6.0 Hz, 2H), 4.31 (d, J= 17.1 Hz, 1H), 2.96 - 2.86 (m, 1H), 2.64 - 2.56 (m, 1 H), 2.45 - 2.33 (m, 1H), 2.04 - 1.96 (m, 1H). MS (ESI) m/z 393.1 [M+H]⁺

**Compound 2:** General procedure A with variant i) was used for the preparation with a yield of 65% from compound **VI** employing 4-Chloro-phenyl-isocyanate. ¹H NMR (600 MHz, DMSO-*d*₆) δ 10.98 (s, 1H), 8.79 (s, 1H), 7.66 (s, 1H), 7.58 - 7.54 (m, 2H), 7.47 - 7.41 (m, 2H), 7.29 - 7.22 (m, 2H), 6.81 (t, *J* = 6.0 Hz, 1H), 5.11 (dd, *J* = 13.3, 5.1 Hz, 1H), 4.43 (d, *J=* 17.1 Hz, 1H), 4.40 (d, *J=* 6.0 Hz, 2H), 4.30 (d, *J= 17.1* Hz, 1 H), 2.96 - 2.85 (m, 1H), 2.64 - 2.55 (m, 1H), 2.45 - 2.33 (m, 1H), 2.04 - 1.95 (m, 1H). MS (ESI) m/z 427.1 [M+H, Cl³⁵] ⁺

**Compound 3:** General procedure A with variant i) was used for the preparation with a yield of 62% from compound **VI** employing 3-Chloro-phenyl-isocyanate. ¹H NMR (600 MHz, DMSO-*d*₆) δ = 10.98 (s, 1H), 8.85 (s, 1H), 7.68 - 7.67 (m, 1H), 7.67 - 7.66 (m, 1H), 7.58 - 7.54 (m, 2H), 7.26 - 7.19 (m, 2H), 6.95 - 6.93 (m, 1H), 6.84 (t, *J =* 6.1 Hz, 1H), 5.11 (dd, *J=* 13.3, 5.1 Hz, 1H), 4.43 (d, *J=* 17.1 Hz, 1H), 4.40 (d, *J* = 6.0 Hz, 2H), 4.31 (d, *J* = 17.1 Hz, 1H), 2.96 - 2.86 (m, 1H), 2.64 - 2.56 (m, 1H), 2.45 - 2.34 (m, 1H), 2.04 - 1.96 (m, 1H). MS (ESI) m/z 427.1 [M+H, Cl³⁵]⁺

**Compound 4:** General procedure A with variant i) was used for the preparation with a yield of 71% from compound **VI** employing 2-Chloro-phenyl-isocyanate. ¹H NMR (600 MHz, DMSO-*d*₆) δ = 10.99 (s, 1H), 8.19 - 8.13 (m, 2H), 7.69 (d, *J =* 1.2 Hz, 1H), 7.61 - 7.54 (m, 3H), 7.44 - 7.38 (m, 1H), 7.28 - 7.21 (m, 1H), 7.00 - 6.93 (m, 1H), 5.13 (dd, *J* = 13.3, 5.1 Hz, 1H), 4.48 - 4.42 (m, 3H), 4.32 (d, *J= 17.1* Hz, 1H), 2.97 - 2.87 (m, 1H), 2.65 - 2.57 (m, 1H), 2.46 - 2.35 (m, 1H), 2.05 - 1.97 (m, 1H). MS (ESI) m/z 427.1 [M+H, Cl³⁵]⁺

**Compound 5:** General procedure A with variant i) was used for the preparation with a yield of 73% from compound **VI** employing 4-Methoxy-phenyl-isocyanate. ¹H NMR (600 MHz, DMSO-*d*₆) δ = 10.98 (s, 1H), 8.41 (s, 1H), 7.68 - 7.64 (m, 1H), 7.58 - 7.54 (m, 2H), 7.35 - 7.27 (m, 2H), 6.86 - 6.78 (m, 2H), 6.64 (t, *J =* 6.1 Hz, 1H), 5.11 (dd, *J* = 13.3, 5.1 Hz, 1H), 4.43 (d, *J= 17.1* Hz, 1H), 4.38 (d, *J* = 6.0 Hz, 2H), 4.30 (d, *J=* 17.1 Hz, 1H), 3.69 (s, 3H), 2.96 - 2.86 (m, 1H), 2.64 - 2.56 (m, 1H), 2.45 - 2.34 (m, 1H), 2.04 - 1.96 (m, 1H). MS (ESI) m/z 423.1 [M+H]⁺

**Compound 6:** General procedure A with variant i) was used for the preparation with a yield of 79% from compound **VI** employing 3-Methoxy-phenyl-isocyanate. ¹H NMR (600 MHz, DMSO-*d*₆) δ = 10.98 (s, 1H), 8.64 (s, 1H), 7.68 - 7.64 (m, 1H), 7.58 - 7.54 (m, 2H), 7.18 - 7.08 (m, 2H), 6.92 - 6.86 (m, 1H), 6.74 (t, *J* = 6.0 Hz, 1H), 6.51 - 6.45 (m, 1H), 5.11 (dd, J= 13.3, 5.1 Hz, 1H), 4.43 (d, *J=* 17.1 Hz, 1H), 4.40 (d, *J* = 6.0 Hz, 2H), 4.31 (d, *J=* 17.1 Hz, 1H), 3.70 (s, 3H), 2.96 - 2.86 (m, 1H), 2.64 - 2.56 (m, 1H), 2.45 - 2.33 (m, 1H), 2.04 - 1.96 (m, 1 H). MS (ESI) m/z 423.1 [M+H]⁺

**Compound 7:** General procedure A with variant i) was used for the preparation with a yield of 82% from compound **VI** employing 2-Methoxy-phenyl-isocyanate. ¹H NMR (600 MHz, DMSO-*d*₆) δ = 10.98 (s, 1H), 8.11 - 8.07 (m, 1H), 8.05 (s, 1H), 7.67 - 7.64 (m, 1H), 7.58 - 7.54 (m, 2H), 7.41 (t, *J= 5.9* Hz, 1H), 6.98 - 6.95 (m, 1H), 6.90 - 6.86 (m, 1H), 6.85 - 6.81 (m, 1H), 5.12 (dd, *J* = 13.3, 5.1 Hz, 1H), 4.44 (d, *J* = 17.1 Hz, 1H), 4.41 (d, J= 5.9 Hz, 2H), 4.31 (d, *J=* 17.1 Hz, 1H), 3.84 (s, 3H), 2.95 - 2.85 (m, 1H), 2.64 - 2.56 (m, 1H), 2.44 - 2.35 (m, 1H), 2.03 - 1.97 (m, 1 H). MS (ESI) m/z 423.1 [M+H]⁺

**Compound 8:** General procedure A with variant ii) was used for the preparation with a yield of 44% from compound **VI** employing 4-Chloro-3-methyl-phenyl-isocyanate. ¹H NMR (400 MHz, DMSO-*d*₆) δ = 10.99 (s, 1H), 8.75 (s, 1H), 7.67 (s, 2H), 7.57 (s, 2H), 7.22 - 7.11 (m, 2H), 6.82 (br t, *J=* 5.9 Hz, 1H), 5.12 (dd, *J* = 5.1, 13.3 Hz, 1H), 4.48 - 4.36 (m, 3H), 4.36 - 4.26 (m, 1H), 2.98 - 2.85 (m, 1H), 2.60 (br dd, *J =* 2.0, 15.5 Hz, 1H), 2.46 - 2.37 (m, 1H), 2.24 (s, 3H), 2.06 - 1.95 (m, 1H). MS (ESI) m/z 441.2 [M+H]⁺

**Compound 9:** General procedure A with variant ii) was used for the preparation with a yield of 51% from compound **VI** employing 4-Bromo-3-methyl-phenyl-isocyanate. ¹H NMR (400 MHz, DMSO-*d*₆) δ = 10.98 (s, 1H), 8.52 (s, 1H), 7.66 (s, 1H), 7.56 (d, *J =* 0.9 Hz, 2H), 7.34 - 7.27 (m, 2H), 7.26 - 7.20 (m, 2H), 6.68 (t, *J=* 6.0 Hz, 1H), 5.11 (dd, *J= 5.1,* 13.3 Hz, 1H), 4.48 - 4.34 (m, 3H), 4.34 - 4.26 (m, 1H), 2.96 - 2.84 (m, 1H), 2.59 (td, J= 1.9, 15.4 Hz, 1H), 2.43 - 2.36 (m, 1H), 2.04 - 1.94 (m, 1H), 2.04 - 1.94 (m, 1H), 1.24 (s, 9H). MS (ESI) m/z 449.3 [M+H]⁺

**Compound 10:** General procedure A with variant ii) was used for the preparation with a yield of 45% from compound **VI** employing tert-Butyl-isocyanate. ¹H NMR (400 MHz, DMSO-*d*₆) δ = 10.98 (s, 1H), 7.59 (s, 1H), 7.56 - 7.52 (m, 1H), 7.51 - 7.47 (m, 1H), 6.20 (t, J = 6.1 Hz, 1H), 5.77 (s, 1H), 5.10 (dd, J = 5.0, 13.2 Hz, 1H), 4.46 - 4.30 (m, 2H), 4.27 (d, J = 6.1 Hz, 2H), 2.96 - 2.86 (m, 1H), 2.62 (br s, 1H), 2.43 - 2.34 (m, 1H), 2.04 - 1.96 (m, 1 H), 1.23 (s, 9H). MS (ESI) m/z 373.3 [M+H]⁺

**Compound 11:** General procedure A with variant ii) was used for the preparation with a yield of 46% from compound **VI** employing 2-tert-Butyl-phenyl-isocyanate. ¹H NMR (400 MHz, DMSO-*d*₆) δ = 10.99 (s, 1H), 7.69 (s, 1H), 7.56 (s, 2H), 7.38 (s, 1H), 7.33 (dd, *J =* 1.2, 7.8 Hz, 1H), 7.28 - 7.23 (m, 1H), 7.18 - 7.12 (m, 1H), 7.08 (br t, *J=* 6.7 Hz, 2H), 5.12 (dd,*J* = 5.1, 13.3 Hz, 1H), 4.52 - 4.38 (m, 3H), 4.35 - 4.27 (m, 1H), 2.98 - 2.85 (m, 1H), 2.60 (br d, *J* = 17.5 Hz, 1H), 2.40 (br dd, *J= 4.3,* 13.1 Hz, 1H), 2.05 - 1.96 (m, 1H), 1.35 (s, 9H). MS (ESI) m/z 449.1 [M+H]⁺

**Compound 12:** General procedure A with variant ii) was used for the preparation with a yield of 27% from compound **VI** employing iso-Propyl-isocyanate. ¹H NMR (400 MHz, DMSO-*d*₆) δ = 10.98 (s, 1H), 7.60 (s, 1H), 7.52 (q, *J=* 7.7 Hz, 2H), 6.29 (br t, *J* = 6.0 Hz, 1H), 5.82 (d, *J* = 7.8 Hz, 1H), 5.11 (dd, *J* = 5.1, 13.4 Hz, 1H), 4.49 - 4.37 (m, 1H), 4.34 - 4.25 (m, 3H), 3.75 - 3.63 (m, 1H), 2.98 - 2.83 (m, 1H), 2.60 (br d, *J=* 16.9 Hz, 1H), 2.39 (br dd, J = 4.5, 13.1 Hz, 1H), 2.05 - 1.96 (m, 1H), 1.04 (d, *J =* 6.5 Hz, 6H). MS (ESI) m/z 359.1 [M+H]⁺

**Compound 13:** General procedure A with variant ii) was used for the preparation with a yield of 51% from compound **VI** employing n-Hexyl-isocyanate. ¹H NMR (400 MHz, DMSO-*d*₆) δ = 10.98 (s, 1H), 7.60 (s, 1H), 7.54 - 7.48 (m, 2H), 6.38 (br t, J= 5.9 Hz, 1H), 5.95 (br s, 1H), 5.11 (dd,J= 5.1, 13.3 Hz, 1H), 4.46 - 4.38 (m, 1H), 4.33 - 4.26 (m, 3H), 3.03 - 2.96 (m, 2H), 2.96 - 2.86 (m, 1H), 2.60 (br d, *J* = 17.7 Hz, 1H), 2.39 (br dd, *J* = 4.5, 13.1 Hz, 1H), 2.04 - 1.95 (m, 1H), 1.41 - 1.32 (m, 2H), 1.24 (br s, 6H), 0.88 - 0.83 (m, 3H). MS (ESI) m/z 401.1 [M+H]⁺

**Compound 14:** General procedure A with variant ii) was used for the preparation with a yield of 51% from compound VI employing n-Propyl-isocyanate. ¹H NMR (400 MHz, DMSO-*d*₆) δ = 10.98 (s, 1H), 7.60 (s, 1H), 7.56 - 7.48 (m, 2H), 6.40 (br t, J= 5.9 Hz, 1H), 5.98 (br t, *J=* 5.6 Hz, 1H), 5.11 (dd, *J* = 5.1, 13.3 Hz, 1H), 4.47 - 4.38 (m, 1H), 4.33 - 4.26 (m, 3H), 3.00 - 2.94 (m, 2H), 2.93 - 2.89 (m, 1H), 2.60 (br d, *J= 15.7* Hz, 1H), 2.39 (br dd, J = 4.5, 13.0 Hz, 1H), 2.06 - 1.96 (m, 1H), 1.42 - 1.35 (m, 2H), 0.83 (t, *J =* 7.3 Hz, 3H). MS (ESI) m/z 359.2 [M+H]⁺

**Compound 15:** General procedure A with variant ii) was used for the preparation with a yield of 54% from compound **VI** employing Ethyl-isocyanate. ¹H NMR (400 MHz, DMSO-*d*₆) δ = 10.97 (s, 1H), 7.60 (s, 1H), 7.56 - 7.52 (m, 1H), 7.51 - 7.47 (m, 1H), 6.41 (t, *J=* 6.1 Hz, 1H), 5.93 (t, J= 5.6 Hz, 1H), 5.10 (dd, J= 5.1, 13.3 Hz, 1H), 4.46 - 4.38 (m, 1H), 4.34 - 4.25 (m, 3H), 3.03 (dq, J= 5.7, 7.1 Hz, 2H), 2.91 (ddd, J= 5.4, 13.7, 17.5 Hz, 1H), 2.60 (br dd, *J* = 2.2, 15.4 Hz, 1H), 2.45 - 2.36 (m, 1H), 2.04 - 1.95 (m, 1H), 1.00 (t, *J* = 7.2 Hz, 3H). MS (ESI) m/z 345.3 [M+H]⁺

**Compound 16:** General procedure A with variant ii) was used for the preparation with a yield of 31% from compound **VI** employing 4-Ethoxy-phenyl-isocyanate. ¹H NMR (400 MHz, DMSO-*d*₆) δ = 10.98 (br s, 1H), 8.40 (d, *J=* 2.4 Hz, 1H), 7.65 (s, 1H), 7.55 (d, J= 1.0 Hz, 2H), 7.33 - 7.26 (m, 2H), 6.82 - 6.76 (m, 2H), 6.64 (br d, *J* = 3.3 Hz, 1H), 5.11 (dd, *J* = 5.1, 13.3 Hz, 1H), 4.48 - 4.34 (m, 3H), 4.33 - 4.26 (m, 1H), 3.94 (q, J= 6.9 Hz, 2H), 2.97 - 2.85 (m, 1H), 2.59 (td, *J=* 2.0, 15.3 Hz, 1H), 2.45 - 2.36 (m, 1H), 2.05 - 1.95 (m, 1H), 1.29 (t, *J= 7.0* Hz, 3H). MS (ESI) m/z 437.3 [M+H]⁺

**Compound 17:** General procedure A with variant ii) was used for the preparation with a yield of 59% from compound **VI** employing 2-Methoxy-5-Methylphenyl-isocyanate. ¹H NMR (400 MHz, DMSO-*d*₆) δ = 10.97 (s, 1H), 7.98 (s, 1H), 7.93 (d, *J =* 2.0 Hz, 1H), 7.65 (s, 1H), 7.56 (s, 2H), 7.40 (br t, *J=* 5.9 Hz, 1H), 6.83 (d, *J= 8.2* Hz, 1H), 6.67 (dd, J= 1.5, 8.3 Hz, 1H), 5.11 (dd, *J* = 5.0, 13.3 Hz, 1H), 4.48 - 4.36 (m, 3H), 4.35 - 4.26 (m, 1H), 3.79 (s, 3H), 2.97 - 2.85 (m, 1H), 2.64 - 2.56 (m, 1H), 2.39 (br dd, J= 4.3, 13.1 Hz, 1H), 2.19 (s, 3H), 2.04 - 1.96 (m, 1H). MS (ESI) m/z 437.3 [M+H]⁺

**Compound 18:** General procedure A with variant ii) was used for the preparation with a yield of 11% from compound **VI** employing 4-Ethylphenyl-isocyanate. ¹H NMR (400 MHz, DMSO-*d*₆) δ = 10.98 (s, 1H), 8.50 (s, 1H), 7.66 (s, 1H), 7.56 (s, 2H), 7.30 (d, *J =* 8.4 Hz, 2H), 7.05 (br d, *J* = 8.4 Hz, 2H), 6.67 (br t, *J* = 5.9 Hz, 1H), 5.11 (dd,*J =* 4.9, 13.2 Hz, 1H), 4.47 - 4.36 (m, 3H), 4.34 - 4.26 (m, 1H), 2.97 - 2.83 (m, 1H), 2.64 - 2.53 (m, 3H), 2.39 (br dd, *J=* 4.4, 13.3 Hz, 1H), 2.06 - 1.94 (m, 1H), 1.13 (t, *J* = 7.6 Hz, 3H). MS (ESI) m/z 421.3 [M+H]⁺

**Compound 19:** General procedure A with variant ii) was used for the preparation with a yield of 10% from compound **VI** employing 3-Chloro-2-methylphenyl-isocyanate. ¹H NMR (400 MHz, DMSO-*d*₆) δ = 10.99 (br s, 1H), 8.02 (s, 1H), 7.74 (br d, *J* = 7.4 Hz, 1H), 7.68 (s, 1H), 7.57 (s, 2H), 7.16 - 7.05 (m, 3H), 5.12 (br dd, *J* = 4.5, 13.1 Hz, 1H), 4.50 - 4.38 (m, 3H), 4.35 - 4.27 (m, 1H), 2.99 - 2.82 (m, 1H), 2.62 (br s, 1H), 2.39 (br s, 1H), 2.24 (s, 3H), 2.05 - 1.95 (m, 1H). MS (ESI) m/z 441.2 [M+H]⁺

**Compound 20:** General procedure A with variant ii) was used for the preparation with a yield of 26% from compound **VI** employing 3,5-Dimethylphenyl-isocyanate. ¹H NMR (400 MHz, DMSO-*d*₆) δ = 10.97 (br s, 1H), 8.43 (s, 1H), 7.66 (s, 1H), 7.56 (s, 2H), 7.03 (s, 2H), 6.69 (br t, *J=* 5.9 Hz, 1H), 6.54 (s, 1H), 5.11 (dd, *J* = 5.0, 13.3 Hz, 1H), 4.47 - 4.36 (m, 3H), 4.34 - 4.27 (m, 1H), 2.97 - 2.85 (m, 1H), 2.59 (br dd, J= 2.1, 15.3 Hz, 1H), 2.45 - 2.36 (m, 1H), 2.19 (s, 6H), 2.05 - 1.95 (m, 1H). MS (ESI) m/z 421.3 [M+H]⁺

**Compound 21:** General procedure A with variant ii) was used for the preparation with a yield of 5% from compound **VI** employing 4-Methylphenyl-isocyanate. ¹H NMR (400 MHz, DMSO-*d*₆)δ = 10.97 (s, 1H), 8.47 (s, 1H), 7.66 (s, 1H), 7.56 (s, 2H), 7.28 (d, *J* = 8.4 Hz, 2H), 7.02 (d, *J* = 8.2 Hz, 2H), 6.67 (t, *J* = 6.0 Hz, 1H), 5.11 (dd, J= 5.0, 13.3 Hz, 1H), 4.46 - 4.36 (m, 3H), 4.34 - 4.27 (m, 1H), 2.97 - 2.85 (m, 1H), 2.63 - 2.56 (m, 1H), 2.46 - 2.35 (m, 1H), 2.21 (s, 3H), 2.06 - 1.95 (m, 1H). MS (ESI) m/z 407.3 [M+H]⁺

**Compound 22:** General procedure A with variant ii) was used for the preparation with a yield of 49% from compound **VI** employing 2-Ethoxyphenyl-isocyanate. ¹H NMR (400 MHz, DMSO-*d*₆) δ = 8.04 (br d, *J* = 7.3 Hz, 1H), 7.65 (s, 1H), 7.57 (s, 2H), 6.96 - 6.91 (m, 1H), 6.89 - 6.79 (m, 2H), 5.07 (br dd, *J* = 4.5, 13.4 Hz, 1H), 4.49 - 4.36 (m, 3H), 4.35 - 4.26 (m, 1H), 4.06 (q, *J* = 6.9 Hz, 2H), 2.95 - 2.82 (m, 1H), 2.65 - 2.57 (m, 1H), 2.45 - 2.35 (m, 1H), 2.06 - 1.94 (m, 1H), 1.36 (t, *J =* 7.0 Hz, 3H). MS (ESI) m/z 437.2 [M+H]⁺

**Compound 23:** General procedure A with variant ii) was used for the preparation with a yield of 47% from compound **VI** employing 4-Methoxy-2-methylphenyl-isocyanate. ¹H NMR (400 MHz, DMSO-*d*₆) δ = 10.98 (s, 1H), 7.65 (br d, *J* = 13.2 Hz, 2H), 7.56 (s, 2H), 7.47 (br d, *J* = 8.7 Hz, 1H), 6.84 (br t, *J=* 5.5 Hz, 1H), 6.74 (d, *J* = 2.4 Hz, 1H), 6.68 (dd, *J* = 2.7, 8.7 Hz, 1H), 5.11 (br dd, *J* = 5.0, 13.2 Hz, 1H), 4.49 - 4.36 (m, 3H), 4.34 - 4.27 (m, 1H), 3.69 (s, 3H), 2.98 - 2.84 (m, 1H), 2.60 (br d, *J* = 17.5 Hz, 1H), 2.46 - 2.36 (m, 1H), 2.16 (s, 3H), 2.05 - 1.92 (m, 1H). MS (ESI) m/z 437.3 [M+H]⁺

**Compound 24:** General procedure A with variant ii) was used for the preparation with a yield of 24% from compound **VI** employing Benzyl-isocyanate. ¹H NMR (400 MHz, DMSO-*d*₆) δ = ¹H NMR (400 MHz, DMSO-*d*₆)δ = 10.97 (s, 1H), 7.64 (s, 1H), 7.56 - 7.48 (m, 2H), 7.33 - 7.18 (m, 5H), 6.57 (br t, *J* = 6.1 Hz, 1H), 6.51 (br t, *J* = 5.9 Hz, 1H), 5.12 (dd, *J =* 5.1, 13.3 Hz, 1H), 4.48 - 4.39 (m, 1H), 4.36 - 4.26 (m, 3H), 4.24 (d, *J =* 6.0 Hz, 2H), 2.97 - 2.86 (m, 1H), 2.64 - 2.56 (m, 1H), 2.46 - 2.36 (m, 1H), 2.05 - 1.96 (m, 1H). MS (ESI) m/z 407.1 [M+H]⁺

**Compound 25:** General procedure A with variant ii) was used for the preparation with a yield of 51% from compound **VI** employing 2,6-Dimethylphenyl-isocyanate. ¹H NMR (400 MHz, DMSO-*d*₆) δ = 10.98 (s, 1H), 7.68 (s, 1H), 7.61 (s, 1H), 7.55 (s, 2H), 7.07 - 6.99 (m, 3H), 6.65 (br s, 1H), 5.11 (dd,J= 5.1, 13.3 Hz, 1H), 4.48 - 4.40 (m, 1H), 4.39 - 4.27 (m, 3H), 2.96 - 2.86 (m, 1H), 2.60 (br dd, *J =* 2.1, 15.3 Hz, 1H), 2.41 (dt, *J* = 4.4, 13.2 Hz, 1H), 2.17 (s, 6H), 2.06 - 1.95 (m, 1H). MS (ESI) m/z 421.3 [M+H]⁺

**Compound 26:** General procedure A with variant ii) was used for the preparation with a yield of 17% from compound VI employing Methylcyclopropyl-isocyanate. ¹H NMR (400 MHz, DMSO-*d*₆) δ = 10.98 (s, 1H), 7.61 (s, 1H), 7.56 - 7.47 (m, 2H), 6.43 (brt, *J* = 5.9 Hz, 1H), 6.04 (br t, *J* = 5.6 Hz, 1H), 5.11 (dd, *J* = 5.0, 13.2 Hz, 1H), 4.47 - 4.37 (m, 1H), 4.35 - 4.25 (m, 3H), 2.90 (br t, J= 6.2 Hz, 3H), 2.60 (br d, *J=* 17.2 Hz, 1H), 2.39 (br dd, J= 4.3, 13.0 Hz, 1H), 2.05 - 1.95 (m, 1H), 0.93 - 0.82 (m, 1H), 0.42 - 0.33 (m, 2H), 0.13 (br d, J = 4.6 Hz, 2H). MS (ESI) m/z 371.3 [M+H]⁺

**Compound 27:** General procedure A with variant ii) was used for the preparation with a yield of 8% from compound **VI** employing 2-Phenylethyl-isocyanate. ¹H NMR (400 MHz, DMSO-d₆) δ = 10. 98 (s, 1H), 7.61 (s, 1H), 7.55 - 7.47 (m, 2H), 7.33 - 7.26 (m, 2H), 7.23 - 7.16 (m, 3H), 6.50 (br t, *J= 5.9* Hz, 1H), 5.99 (br t, *J= 5.6* Hz, 1H), 5.12 (dd,*J* = 5.1, 13.3 Hz, 1H), 4.47 - 4.38 (m, 1H), 4.34 - 4.26 (m, 3H), 3.25 (q, *J =* 6.8 Hz, 2H), 2.97 - 2.86 (m, 1H), 2.69 (t, *J* = 7.3 Hz, 2H), 2.60 (br d, *J* = 17.5 Hz, 1H), 2.39 (br dd, *J* = 4.4, 13.2 Hz, 1H), 2.04 - 1.96 (m, 1H). MS (ESI) m/z 421.2 [M+H]⁺

**Compound 28:** General procedure A with variant ii) was used for the preparation with a yield of 27% from compound VI employing Cyclopentyl-isocyanate. ¹H NMR (400 MHz, DMSO-*d*₆) δ = 10.98 (s, 1H), 7.60 (s, 1H), 7.56 - 7.48 (m, 2H), 6.27 (br t, J= 6.0 Hz, 1H), 5.98 (d, J= 7.3 Hz, 1H), 5.11 (dd, J= 5.1, 13.3 Hz, 1H), 4.46 - 4.37 (m, 1H), 4.33 - 4.26 (m, 3H), 3.92 - 3.81 (m, 1 H), 2.97 - 2.85 (m, 1H), 2.60 (br d, J= 17.6 Hz, 1H), 2.39 (br dd, J = 4.4, 13.1 Hz, 1H), 2.04 - 1.96 (m, 1H), 1.84 - 1.73 (m, 2H), 1.64 - 1.55 (m, 2H), 1.52 - 1.44 (m, 2H), 1.35 - 1.25 (m, 2H). MS (ESI) m/z 385.3 [M+H]⁺

**Compound 29:** General procedure A with variant ii) was used for the preparation with a yield of 45% from compound **VI** employing Cyclohexyl-isocyanate. ¹H NMR (400 MHz, DMSO-*d*₆) δ = 10.99 (s, 1H), 7.60 (s, 1H), 7.56 - 7.47 (m, 2H), 6.30 (br t, J= 5.9 Hz, 1H), 5.89 (d, *J=* 8.1 Hz, 1H), 5.11 (dd, J= 5.1, 13.3 Hz, 1H), 4.47 - 4.37 (m, 1H), 4.33 - 4.25 (m, 3H), 3.44 - 3.35 (m, 1H), 2.98 - 2.85 (m, 1H), 2.60 (br d, *J =* 17.6 Hz, 1H), 2.45 - 2.37 (m, 1H), 2.05 - 1.94 (m, 1H), 1.81 - 1.71 (m, 2H), 1.69 - 1.59 (m, 2H), 1.57 - 1.47 (m, 1H), 1.32 - 1.19 (m, 2H), 1.18 - 1.04 (m, 3H). MS (ESI) m/z 399.3 [M+H]⁺

**Compound 30:** General procedure A with variant ii) was used for the preparation with a yield of 46% from compound **VI** employing 5-Chloro-2-methoxyphenyl-isocyanate. ¹H NMR (400 MHz, DMSO-*d*₆) δ = 10.97 (s, 1H), 8.22 (br d, *J* = 15.0 Hz, 2H), 7.66 (s, 1H), 7.61 - 7.48 (m, 3H), 7.01 - 6.95 (m, 1H), 6.94 - 6.87 (m, 1H), 5.12 (dd, J= 5.0, 13.2 Hz, 1H), 4.49 - 4.38 (m, 3H), 4.36 - 4.26 (m, 1H), 3.84 (s, 3H), 2.97 - 2.85 (m, 1H), 2.64 - 2.56 (m, 1H), 2.47 - 2.36 (m, 1H), 2.07 - 1.96 (m, 1H). MS (ESI) m/z 457.0 [M+H]⁺

**Compound 31:** General procedure A with variant ii) was used for the preparation with a yield of 43% from compound **VI** employing 3-Ethylphenyl-isocyanate. ¹H NMR (400 MHz, DMSO-*d*₆) δ = 10.97 (s, 1 H), 8.54 (s, 1H), 7.66 (s, 1H), 7.56 (s, 2H), 7.27 (s, 1H), 7.23 - 7.17 (m, 1H), 7.15 - 7.09 (m, 1H), 6.77 - 6.67 (m, 2H), 5.11 (br dd, J= 5.0, 13.2 Hz, 1H), 4.49 - 4.37 (m, 3H), 4.35 - 4.27 (m, 1H), 2.98 - 2.85 (m, 1H), 2.63 - 2.52 (m, 3H), 2.44 - 2.36 (m, 1H), 2.05 - 1.94 (m, 1H), 1.15 (t, *J=* 7.6 Hz, 3H). MS (ESI) m/z 421.0 [M+H]⁺

**Compound 32:** General procedure A with variant ii) was used for the preparation with a yield of 67% from compound **VI** employing 4-Chloro-2-Methylphenyl-isocyanate. ¹H NMR (400 MHz, DMSO-*d*₆) δ = 10.97 (br s, 1H), 7.91 - 7.83 (m, 2H), 7.68 (s, 1H), 7.57 (s, 2H), 7.21 (d, *J=* 2.3 Hz, 1H), 7.19 - 7.11 (m, 2H), 5.11 (dd, *J* = 5.1, 13.3 Hz, 1H), 4.48 - 4.38 (m, 3H), 4.35 - 4.28 (m, 1H), 2.97 - 2.86 (m, 1H), 2.64 - 2.56 (m, 1H), 2.44 - 2.37 (m, 1H), 2.19 (s, 3H), 2.05 - 1.97 (m, 1 H). MS (ESI) m/z 441.0 [M+H]⁺

**Compound 33:** General procedure A with variant ii) was used for the preparation with a yield of 21% from compound **VI** employing 3-Chloro-2-Flourophenyl-isocyanate. ¹H NMR (400 MHz, DMSO-*d*₆) δ = 10.97 (s, 1H), 8.63 (br s, 1H), 8.15 - 8.04 (m, 1H), 7.67 (s, 1H), 7.57 (s, 2H), 7.23 (br t, J= 5.7 Hz, 1H), 7.16 - 7.05 (m, 2H), 5.11 (dd, J= 5.1, 13.3 Hz, 1H), 4.50 - 4.38 (m, 3H), 4.37 - 4.26 (m, 1H), 2.97 - 2.85 (m, 1H), 2.60 (br d, *J =* 17.4 Hz, 1H), 2.40 (br dd, *J* = 4.3, 13.2 Hz, 1H), 2.05 - 1.96 (m, 1H). MS (ESI) m/z 444.9 [M+H]⁺

**Compound 34:** General procedure A with variant ii) was used for the preparation with a yield of 22% from compound **VI** employing 3-Flourophenyl-isocyanate. ¹H NMR (400 MHz, DMSO-*d*₆)δ = 10.97 (s, 1H), 8.87 (s, 1H), 7.66 (s, 1H), 7.56 (d, *J* = 0.7 Hz, 2H), 7.45 (td, *J=* 2.3, 1 2.2 Hz, 1H), 7.29 - 7.20 (m, 1H), 7.06 (dd, *J=* 1.2, 8.1 Hz, 1H), 6.83 (t, J= 5.9 Hz, 1H), 6.70 (dt, *J =* 2.0, 8.5 Hz, 1H), 5.11 (dd, *J* = 5.0, 13.2 Hz, 1H), 4.48 - 4.38 (m, 3H), 4.35 - 4.27 (m, 1H), 2.97 - 2.85 (m, 1H), 2.59 (br dd, *J=* 2.1, 15.5 Hz, 1H), 2.39 (br dd, *J=* 4.4, 13.0 Hz, 1H), 2.04 - 1.96 (m, 1H). MS (ESI) m/z 411.0 [M+H]⁺

**Compound 35:** General procedure A with variant ii) was used for the preparation with a yield of 14% from compound **VI** employing 2,5-Dichlorophenyl-isocyanate. ¹H NMR (400 MHz, DMSO-*d*₆) δ = 10.97 (br s, 1H), 8.32 (s, 2H), 7.74 - 7.64 (m, 2H), 7.58 (s, 2H), 7.44 (d, J = 8.6 Hz, 1H), 7.02 (dd, *J=* 2.4, 8.6 Hz, 1H), 5.12 (dd, *J* = 5.1, 13.3 Hz, 1H), 4.49 - 4.40 (m, 3H), 4.37 - 4.27 (m, 1H), 2.97 - 2.85 (m, 1H), 2.60 (br d, *J* = 17.5 Hz, 1H), 2.40 (br dd, *J=* 4.2, 13.1 Hz, 1H), 2.07 - 1.95 (m, 1H). MS (ESI) m/z 460.9 [M+H]⁺

**Compound 36:** General procedure A with variant ii) was used for the preparation with a yield of 46% from compound **VI** employing 2-Ethylphenyl-isocyanate. ¹H NMR (400 MHz, DMSO-*d*₆) δ = 10.97 (br s, 1H), 7.76 (br d, *J* = 6.7 Hz, 2H), 7.68 (s, 1H), 7.57 (s, 2H), 7.17 - 7.06 (m, 3H), 6.94 (dt, J= 1.1, 7.4 Hz, 1H), 5.12 (dd, J= 5.1, 13.2 Hz, 1H), 4.48 - 4.37 (m, 3H), 4.35 - 4.26 (m, 1H), 2.96 - 2.85 (m, 1H), 2.64 - 2.58 (m, 1H), 2.58 - 2.53 (m, 2H), 2.46 - 2.37 (m, 1H), 2.05 - 1.95 (m, 1H), 1.14 (t, *J =* 7.5 Hz, 3H). MS (ESI) m/z 421.3 [M+H]⁺

**Compound 37:** General procedure A with variant ii) was used for the preparation with a yield of 42% from compound **VI** employing 2-Methylphenyl-isocyanate. ¹H NMR (400 MHz, DMSO-*d*₆) δ = 10.97 (br s, 1H), 7.84 - 7.76 (m, 2H), 7.69 (s, 1H), 7.57 (s, 2H), 7.16 - 7.05 (m, 3H), 6.88 (dt, *J =* 0.9, 7.4 Hz, 1H), 5.12 (dd, *J* = 5.1, 13.3 Hz, 1H), 4.49 - 4.38 (m, 3H), 4.36 - 4.26 (m, 1H), 2.98 - 2.85 (m, 1H), 2.64 - 2.55 (m, 1H), 2.47 - 2.36 (m, 1H), 2.19 (s, 3H), 2.05 - 1.96 (m, 1H). MS (ESI) m/z 407.3 [M+H]⁺

**Compound 38:** General procedure A with variant ii) was used for the preparation with a yield of 30% from compound **VI** employing 3,5-Dichlorophenyl-isocyanate. ¹H NMR (400 MHz, DMSO-*d*₆) δ = 10.97 (br s, 1H), 9.13 (s, 1H), 7.66 (s, 1H), 7.56 (d, *J* = 0.7 Hz, 2H), 7.50 (d, *J* = 1.8 Hz, 2H), 7.07 (br d, *J =* 1.7 Hz, 2H), 5.11 (dd, J = 5.1, 13.3 Hz, 1H), 4.48 - 4.36 (m, 3H), 4.35 - 4.27 (m, 1H), 2.97 - 2.83 (m, 1H), 2.64 - 2.56 (m, 1H), 2.45 - 2.37 (m, 1H), 2.04 - 1.96 (m, 1H). MS (ESI) m/z 461.2 [M+H]⁺

**Compound 39:** General procedure A with variant ii) was used for the preparation with a yield of 29% from compound **VI** employing 3,4-Diflourophenyl-isocyanate. ¹H NMR (400 MHz, DMSO-*d*₆) δ = 10.97 (s, 1H), 8.90 (s, 1H), 7.66 (s, 1H), 7.62 (dt, *J =* 2.6, 6.8 Hz, 1H), 7.56 (d, *J* = 0.9 Hz, 2H), 7.33 - 7.22 (m, 1H), 7.09 - 7.03 (m, 1H), 6.93 - 6.82 (m, 1H), 5.11 (dd,*J* = 5.1, 13.3 Hz, 1H), 4.48 - 4.37 (m, 3H), 4.34 - 4.26 (m, 1H), 2.98 - 2.85 (m, 1H), 2.64 - 2.56 (m, 1H), 2.46 - 2.36 (m, 1H), 2.04 - 1.95 (m, 1H). MS (ESI) m/z 429.2 [M+H]⁺

**Compound 40:** General procedure A with variant ii) was used for the preparation with a yield of 69% from compound VI employing 1,2,3,4-Tetrahydronaphth-1-yl-isocyanate. ¹H NMR (400 MHz, DMSO-*d*₆) δ = 10.98 (s, 1H), 7.65 (s, 1H), 7.57 - 7.51 (m, 2H), 7.26 - 7.20 (m, 1H), 7.17 - 7.11 (m, 2H), 7.09 - 7.03 (m, 1 H), 6.41 - 6.29 (m, 2H), 5.12 (dd,J= 5.1, 13.3 Hz, 1H), 4.85 - 4.77 (m, 1H), 4.47 - 4.40 (m, 1H), 4.39 - 4.28 (m, 3H), 2.97 - 2.85 (m, 1H), 2.77 - 2.66 (m, 2H), 2.64 - 2.57 (m, 1H), 2.40 (dd, J= 4.6, 13.1 Hz, 1H), 2.06 - 1.97 (m, 1H), 1.92 - 1.64 (m, 4H). MS (ESI) m/z 447.3 [M+H]⁺

**Compound 41:** General procedure A with variant ii) was used for the preparation with a yield of 16% from compound VI employing 4-Triflouromethylphenyl-isocyanate. ¹H NMR (400 MHz, DMSO-*d*₆) δ = 10.98 (s, 1H), 9.26 - 9.05 (m, 1H), 7.70 - 7.61 (m, 3H), 7.61 - 7.54 (m, 4H), 7.00 (br d, *J= 6.1* Hz, 1H), 5.12 (dd, J= 5.1, 13.3 Hz, 1H), 4.52 - 4.38 (m, 3H), 4.37 - 4.27 (m, 1H), 2.92 (ddd, J= 5.4, 13.6, 17.4 Hz, 1H), 2.65 - 2.57 (m, 1H), 2.47 - 2.36 (m, 1H), 2.10 - 1.95 (m, 1H). MS (ESI) m/z 461.0 [M+H]⁺

**Compound 42:** General procedure A with variant ii) was used for the preparation with a yield of 26% from compound **VI** employing 3-Chloro-4-Fluorophenyl-isocyanate. ¹H NMR (400 MHz, DMSO-*d*₆) δ = 10.97 (s, 1H), 8.85 (s, 1H), 7.80 - 7.72 (m, 1H), 7.66 (s, 1H), 7.56 (d, *J= 0.9* Hz, 2H), 7.31 - 7.21 (m, 2H), 6.86 (t, *J= 5.9* Hz, 1H), 5.11 (dd, J= 5.1, 13.3 Hz, 1H), 4.49 - 4.37 (m, 3H), 4.35 - 4.26 (m, 1H), 2.98 - 2.84 (m, 1H), 2.60 (br d, *J* = 17.6 Hz, 1H), 2.45 - 2.36 (m, 1H), 2.05 - 1.95 (m, 1H). MS (ESI) m/z 445.2 [M+H]⁺

**Compound 43:** General procedure A with variant ii) was used for the preparation with a yield of 42% from compound VI employing 3-Methylphenyl-isocyanate. ¹H NMR (400 MHz, DMSO-*d*₆) δ = 10.97 (br s, 1H), 8.62 - 8.50 (m, 1H), 7.66 (s, 1H), 7.56 (s, 2H), 7.25 (s, 1H), 7.18 (br d, J= 8.3 Hz, 1H), 7.14 - 7.05 (m, 1H), 6.81 - 6.68 (m, 2H), 5.11 (dd,*J* = 5.1, 13.3 Hz, 1H), 4.49 - 4.36 (m, 3H), 4.35 - 4.26 (m, 1H), 2.97 - 2.85 (m, 1H), 2.64 - 2.56 (m, 1 H), 2.45 - 2.36 (m, 1H), 2.24 (s, 3H), 2.06 - 1.95 (m, 1H). MS (ESI) m/z 407.2 [M+H]⁺

**Compound 44:** General procedure A with variant ii) was used for the preparation with a yield of 53% from compound **VI** employing 2,3-Dimethylphenyl-isocyanate. ¹H NMR (400 MHz, DMSO-*d*₆) δ = 10.97 (s, 1H), 7.80 (s, 1H), 7.68 (s, 1H), 7.57 (s, 2H), 7.50 (d, *J* = 8.1 Hz, 1H), 7.03 - 6.94 (m, 2H), 6.84 (d, *J= 7.2* Hz, 1H), 5.11 (dd, J= 5.1, 13.3 Hz, 1H), 4.49 - 4.37 (m, 3H), 4.36 - 4.27 (m, 1H), 2.98 - 2.85 (m, 1H), 2.64 - 2.57 (m, 1H), 2.46 - 2.37 (m, 1H), 2.23 (s, 3H), 2.08 (s, 3H), 2.04 - 1.97 (m, 1H). MS (ESI) m/z 421.3 [M+H]⁺

**Compound 45:** General procedure A with variant ii) was used for the preparation with a yield of 29% from compound **VI** employing 2,4-Dichlorophenyl-isocyanate. ¹H NMR (400 MHz, DMSO-*d*₆) δ = 10.97 (s, 1H), 8.26 (s, 1H), 8.19 (d, *J* = 8.9 Hz, 1H), 7.68 (s, 1H), 7.58 (s, 3H), 7.56 (d, *J* = 2.4 Hz, 1H), 7.32 (dd, *J* = 2.5, 9.0 Hz, 1H), 5.11 (dd, *J =* 5.1, 13.4 Hz, 1H), 4.51 - 4.40 (m, 3H), 4.37 - 4.27 (m, 1H), 2.98 - 2.85 (m, 1H), 2.64 - 2.56 (m, 1 H), 2.46 - 2.37 (m, 1H), 2.06 - 1.97 (m, 1H). MS (ESI) m/z 460.9 [M+H]⁺

**Compound 46:** General procedure A with variant ii) was used for the preparation with a yield of 42% from compound **VI** employing 3,4-Dichlorophenyl-isocyanate. ¹H NMR (400 MHz, DMSO-*d*₆) δ = 10.98 (s, 1H), 9.08 (br s, 1H), 7.85 (d, *J* = 2.4 Hz, 1H), 7.66 (s, 1H), 7.56 (s, 2H), 7.45 (d, *J* = 8.8 Hz, 1H), 7.28 (dd, *J* = 2.4, 8.8 Hz, 1H), 7.02 (br d, *J =* 5.7 Hz, 1 H), 5.11 (dd,J= 5.0, 13.3 Hz, 1H), 4.48 - 4.36 (m, 3H), 4.34 - 4.27 (m, 1H), 2.97 - 2.85 (m, 1H), 2.59 (br d, *J* = 17.6 Hz, 1H), 2.42 - 2.38 (m, 1H), 2.04 - 1.96 (m, 1H). MS (ESI) m/z 460.9 [M+H]⁺

**Compound 47:** General procedure A with variant ii) was used for the preparation with a yield of 32% from compound **VI** employing 3-Trifluoromethylphenyl-isocyanate. ¹H NMR (400 MHz, DMSO-*d*₆) δ = 10.98 (s, 1H), 9.08 (s, 1H), 7.98 (s, 1H), 7.67 (s, 1H), 7.57 (s, 2H), 7.54 (br d, *J=* 8.8 Hz, 1H), 7.45 (t, *J=* 7.9 Hz, 1H), 7.23 (d, *J=* 7.5 Hz, 1H), 6.95 (br t, *J* = 5.9 Hz, 1H), 5.11 (dd, J= 5.1, 13.3 Hz, 1 H), 4.48 - 4.37 (m, 3H), 4.35 - 4.26 (m, 1H), 2.98 - 2.84 (m, 1H), 2.59 (br d, J= 17.5 Hz, 1H), 2.39 (br dd, J= 4.5, 13.1 Hz, 1H), 2.04 - 1.95 (m, 1H). MS (ESI) m/z 461.2 [M+H]⁺

**Compound 48:** General procedure A with variant ii) was used for the preparation with a yield of 20% from compound VI employing 2-Fluorophenyl-isocyanate. ¹H NMR (400 MHz, DMSO-*d*₆) δ = 10.99 (s, 1H), 8.47 (br s, 1H), 8.16 - 8.08 (m, 1H), 7.66 (s, 1H), 7.57 (s, 2H), 7.24 - 7.14 (m, 2H), 7.08 (t, *J =* 7.6 Hz, 1H), 6.97 - 6.90 (m, 1H), 5.12 (dd, *J* = 5.1, 13.3 Hz, 1H), 4.50 - 4.38 (m, 3H), 4.36 - 4.25 (m, 1H), 2.98 - 2.84 (m, 1H), 2.62 - 2.57 (m, 1H), 2.44 - 2.36 (m, 1H), 2.05 - 1.95 (m, 1H). MS (ESI) m/z 411.2 [M+H]⁺

**Compound 49:** General procedure A with variant ii) was used for the preparation with a yield of 14% from compound VI employing 2-Triflouromethylphenyl-isocyanate. ¹H NMR (400 MHz, DMSO-*d*₆) δ = 10.99 (s, 1H), 8.00 - 7.90 (m, 2H), 7.68 (s, 1H), 7.62 (d, *J* = 7.9 Hz, 1H), 7.60 - 7.55 (m, 4H), 7.20 (t, *J=* 7.6 Hz, 1H), 5.12 (dd, J= 5.1, 13.3 Hz, 1H), 4.49 - 4.39 (m, 3H), 4.35 - 4.27 (m, 1H), 2.97 - 2.85 (m, 1H), 2.64 - 2.56 (m, 1H), 2.42 - 2.37 (m, 1H), 2.05 - 1.96 (m, 1H). MS (ESI) m/z 461.2 [M+H]⁺

**Compound 50:** General procedure A with variant ii) was used for the preparation with a yield of 41% from compound VI employing 2,3-Dichlorophenyl-isocyanate. ¹H NMR (400 MHz, DMSO-*d*₆) δ = 10.97 (s, 1H), 8.33 (s, 1H), 8.16 (dd, J= 1.5, 8.2 Hz, 1H), 7.72 - 7.60 (m, 2H), 7.58 (s, 2H), 7.32 - 7.16 (m, 2H), 5.12 (dd, J= 5.0, 13.2 Hz, 1H), 4.50 - 4.37 (m, 3H), 4.36 - 4.28 (m, 1H), 2.97 - 2.85 (m, 1H), 2.60 (br dd, J= 2.1, 15.5 Hz, 1H), 2.40 (br dd, J= 4.5, 13.1 Hz, 1H), 2.05 - 1.94 (m, 1H). MS (ESI) m/z 461.1 [M+H]⁺

**Compound 51:** General procedure A with variant ii) was used for the preparation with a yield of 35% from compound VI employing 2-(4-Methoxyphenyl)-ethyl-isocyanate. ¹H NMR (400 MHz, DMSO-*d*₆) δ = 10.99 (s, 1H), 7.61 (s, 1H), 7.56 - 7.45 (m, 2H), 7.11 (br d, *J=* 8.6 Hz, 2H), 6.85 (d, *J =* 8.6 Hz, 2H), 6.49 (br t, *J* = 5.7 Hz, 1H), 5.96 (br t, *J =* 5.6 Hz, 1H), 5.11 (dd, *J =* 5.0, 13.3 Hz, 1H), 4.48 - 4.37 (m, 1H), 4.35 - 4.24 (m, 3H), 3.71 (s, 3H), 3.25 - 3.16 (m, 2H), 2.98 - 2.84 (m, 1H), 2.65 - 2.55 (m, 3H), 2.39 (br dd, *J =* 4.3, 13.0 Hz, 1H), 2.05 - 1.95 (m, 1H). MS (ESI) m/z 451.0 [M+H]⁺

**Compound 52:** General procedure A with variant ii) was used for the preparation with a yield of 35% from compound **VI** employing 4-Methoxyphenylmethyl-isocyanate. ¹H NMR (400 MHz, DMSO-*d*₆) δ = 10.99 (s, 1H), 7.63 (s, 1H), 7.57 - 7.47 (m, 2H), 7.17 (d, *J* = 8.6 Hz, 2H), 6.87 (d, *J* = 8.7 Hz, 2H), 6.52 (br t, *J* = 6.0 Hz, 1H), 6.42 (br t, *J* = 5.9 Hz, 1H), 5.12 (dd, *J* = 5.0, 13.3 Hz, 1H), 4.47 - 4.38 (m, 1H), 4.36 - 4.26 (m, 3H), 4.16 (d, *J* = 5.9 Hz, 2H), 3.72 (s, 3H), 2.98 - 2.84 (m, 1H), 2.64 - 2.56 (m, 1H), 2.44 - 2.36 (m, 1H), 2.05 - 1.94 (m, 1H). MS (ESI) m/z 437.3 [M+H]⁺

**Compound 53:** General procedure A with variant ii) was used for the preparation with a yield of 35% from compound **VI** employing 3-Methoxyphenylmethyl-isocyanate. ¹H NMR (400 MHz, DMSO-*d*₆) δ = 10.99 (s, 1H), 7.64 (s, 1H), 7.57 - 7.47 (m, 2H), 7.22 (t, *J* = 7.8 Hz, 1H), 6.86 - 6.75 (m, 3H), 6.58 (br t, J= 6.1 Hz, 1H), 6.50 (br t, *J* = 6.0 Hz, 1H), 5.12 (dd, *J* = 5.1, 13.3 Hz, 1H), 4.49 - 4.38 (m, 1H), 4.36 - 4.26 (m, 3H), 4.21 (d, *J* = 5.9 Hz, 2H), 3.71 (s, 3H), 2.98 - 2.85 (m, 1H), 2.64 - 2.56 (m, 1H), 2.40 (br dd, *J= 4.4,* 13.1 Hz, 1H), 2.05 - 1.95 (m, 1H). MS (ESI) m/z 437.3 [M+H]⁺

**Compound 54:** General procedure A with variant ii) was used for the preparation with a yield of 36% from compound VI employing (R)-1-Phenylethyl-isocyanate. ¹H NMR (400 MHz, DMSO-*d*₆) δ = 10.99 (s, 1H), 7.61 (s, 1H), 7.55 - 7.45 (m, 2H), 7.34 - 7.27 (m, 4H), 7.20 (dt, *J* = 2.6, 5.7 Hz, 1H), 6.50 (d, *J =* 8.1 Hz, 1H), 6.41 (br t, *J* = 5.9 Hz, 1H), 5.11 (dd, J = 5.1, 13.3 Hz, 1H), 4.76 (quin, *J* = 7.3 Hz, 1H), 4.47 - 4.38 (m, 1H), 4.34 - 4.25 (m, 3H), 2.98 - 2.85 (m, 1H), 2.63 - 2.56 (m, 1H), 2.39 (br dd, *J* = 4.5, 13.2 Hz, 1H), 2.05 - 1.95 (m, 1H), 1.33 (d, *J=* 7.0 Hz, 3H). MS (ESI) m/z 421.0 [M+H]⁺

**Compound 55:** General procedure A with variant ii) was used for the preparation with a yield of 34% from compound **VI** employing (S)-1-Phenylethyl-isocyanate. ¹H NMR (400 MHz, DMSO-*d*₆) *δ* = 10.99 (s, 1H), 7.61 (s, 1H), 7.55 - 7.45 (m, 2H), 7.34 - 7.26 (m, 4H), 7.20 (dt, *J* = 2.7, 5.6 Hz, 1H), 6.50 (d, *J=* 8.1 Hz, 1H), 6.40 (br t, *J* = 5.9 Hz, 1H), 5.11 (dd, *J* = 5.0, 13.3 Hz, 1H), 4.76 (quin, *J= 7.2* Hz, 1H), 4.46 - 4.38 (m, 1H), 4.35 - 4.23 (m, 3H), 2.98 - 2.85 (m, 1H), 2.60 (br d, *J* = 17.6 Hz, 1H), 2.39 (br dd, *J* = 4.4, 13.1 Hz, 1H), 2.05 - 1.95 (m, 1H), 1.33 (d, *J* = 7.0 Hz, 3H). MS (ESI) m/z 421.0 [M+H]⁺

**Compound 56:** General procedure A with variant ii) was used for the preparation with a yield of 35% from compound **VI** employing 4-tert-Butylphenyl-isocyanate. ¹H NMR (400 MHz, DMSO-*d*₆) *δ* = 10.98 (s, 1H), 8.52 (s, 1H), 7.66 (s, 1H), 7.56 (d, *J=* 0.9 Hz, 2H), 7.34 - 7.27 (m, 2H), 7.26 - 7.20 (m, 2H), 6.68 (t, *J* = 6.0 Hz, 1H), 5.11 (dd, *J* = 5.1, 13.3 Hz, 1H), 4.48 - 4.34 (m, 3H), 4.34 - 4.26 (m, 1H), 2.96 - 2.84 (m, 1H), 2.59 (td, J= 1.9, 15.4 Hz, 1H), 2.43 - 2.36 (m, 1H), 2.04 - 1.94 (m, 1H), 2.04 - 1.94 (m, 1H), 1.24 (s, 9H). MS (ESI) m/z 449.3 [M+H]⁺

**Compound 57:** General procedure A with variant ii) was used for the preparation with a yield of 74% from compound **VI** employing 4-iso-Propylphenyl-isocyanate. ¹H NMR (400 MHz, DMSO-*d*₆) *δ* = 10.98 (s, 1H), 8.50 (s, 1H), 7.66 (s, 1H), 7.56 (s, 2H), 7.31 (d, *J* = 8.6 Hz, 2H), 7.09 (d, *J* = 8.6 Hz, 2H), 6.67 (br t, *J* = 5.9 Hz, 1H), 5.11 (dd, *J* = 5.0, 13.3 Hz, 1H), 4.52 - 4.37 (m, 3H), 4.35 - 4.25 (m, 1H), 2.97 - 2.85 (m, 1H), 2.84 - 2.74 (m, 1H), 2.59 (br d, *J =* 17.5 Hz, 1H), 2.46 - 2.36 (m, 1H), 2.05 - 1.92 (m, 1H), 1.16 (d, *J =* 6.8 Hz, 6H). MS (ESI) m/z 435.3 [M+H]⁺

**Compound 58:** General procedure A with variant ii) was used for the preparation with a yield of 75% from compound **VI** employing Cyclohexylmethyl-isocyanate. ¹H NMR (400 MHz, DMSO-*d*₆) *δ* = 10.98 (s, 1H), 7.60 (s, 1H), 7.51 (q, *J* = 7.7 Hz, 2H), 6.36 (br t, *J* = 6.1 Hz, 1H), 6.00 (t, *J* = 5.7 Hz, 1H), 5.11 (dd, *J* = 5.1, 13.3 Hz, 1H), 4.48 - 4.37 (m, 1H), 4.34 - 4.25 (m, 3H), 2.98 - 2.83 (m, 3H), 2.63 - 2.56 (m, 1H), 2.44 - 2.36 (m, 1H), 2.06 - 1.92 (m, 1H), 1.74 - 1.56 (m, 5H), 1.42 - 1.25 (m, 1H), 1.24 - 1.07 (m, 3H), 0.91 - 0.78 (m, 2H). MS (ESI) m/z 413.3 [M+H]⁺

**Compound 59:** General procedure A with variant ii) was used for the preparation with a yield of 23% from compound **VI** employing 3-Chloro-4-methoxyphenyl-isocyanate. ¹H NMR (400 MHz, DMSO-*d*₆) *δ* = 10.97 (br s, 1H), 8.73 - 8.59 (m, 1H), 7.68 - 7.60 (m, 2H), 7.56 (s, 2H), 7.21 (dd, *J* = 2.3, 8.9 Hz, 1H), 7.02 (d, *J =* 8.9 Hz, 1H), 6.80 (br d, *J* = 3.4 Hz, 1H), 5.11 (dd, *J* = 5.0, 13.3 Hz, 1H), 4.49 - 4.35 (m, 3H), 4.34 - 4.26 (m, 1H), 3.78 (s, 3H), 2.97 - 2.84 (m, 1H), 2.59 (br d, *J* = 17.1 Hz, 1H), 2.39 (br dd, *J* = 4.2, 13.0 Hz, 1H), 2.05 - 1.95 (m, 1H). MS (ESI) m/z 457.2 [M+H]⁺

**Compound 60:** General procedure A with variant ii) was used for the preparation with a yield of 9% from compound **VI** employing 2-Methoxyphenylmethyl-isocyanate. ¹H NMR (400 MHz, DMSO-*d*₆) *δ* = 10.98 (br s, 1H), 7.63 (s, 1H), 7.57 - 7.48 (m, 2H), 7.25 - 7.15 (m, 2H), 6.96 (d, *J* = 8.1 Hz, 1H), 6.90 (t, *J* = 7.3 Hz, 1H), 6.60 (t, *J* = 6.0 Hz, 1H), 6.31 (t, *J* = 5.9 Hz, 1H), 5.12 (dd, *J* = 5.1, 13.4 Hz, 1 H), 4.47 - 4.38 (m, 1H), 4.36 - 4.26 (m, 3H), 4.19 (d, *J* = 6.0 Hz, 2H), 3.80 (s, 3H), 3.01 - 2.83 (m, 1H), 2.60 (br d, *J* = 17.6 Hz, 1H), 2.40 (br dd, *J* = 4.4, 13.1 Hz, 1H), 2.04 - 1.96 (m, 1H). MS (ESI) m/z 437.3 [M+H]⁺

**Compound 61:** General procedure A with variant ii) was used for the preparation with a yield of 72% from compound **VI** employing 4-Chloro-3-methylphenyl-isocyanate. ¹H NMR (400 MHz, DMSO-*d*₆) *δ* = 10.98 (s, 1H), 8.71 (s, 1H), 7.66 (s, 1H), 7.56 (s, 2H), 7.40 (d, *J* = 1.8 Hz, 1H), 7.30 - 7.25 (m, 1H), 7.24 - 7.19 (m, 1H), 6.87 - 6.75 (m, 1H), 5.11 (dd, *J =* 5.0, 13.3 Hz, 1H), 4.48 - 4.35 (m, 3H), 4.34 - 4.25 (m, 1H), 2.98 - 2.84 (m, 1H), 2.60 (br dd, J= 1.6, 17.4 Hz, 1H), 2.46 - 2.36 (m, 1H), 2.26 (s, 3H), 2.06 - 1.94 (m, 1H). MS (ESI) m/z 441.3 [M+H]⁺

**Compound 62:** General procedure A with variant ii) was used for the preparation with a yield of 64% from compound **VI** employing 5-Fluoro-2-methylphenyl-isocyanate. ¹H NMR (400 MHz, DMSO-*d*₆) *δ* = 10.98 (s, 1H), 7.96 - 7.79 (m, 2H), 7.68 (s, 1H), 7.58 (s, 2H), 7.31 (t, *J =* 5.8 Hz, 1H), 7.12 (t, *J* = 7.6 Hz, 1H), 6.66 (dt, *J* = 2.8, 8.3 Hz, 1H), 5.12 (dd, *J* = 5.1, 13.3 Hz, 1H), 4.51 - 4.36 (m, 3H), 4.36 - 4.27 (m, 1H), 3.02 - 2.80 (m, 1H), 2.65 - 2.56 (m, 1H), 2.46 - 2.36 (m, 1H), 2.16 (s, 3H), 2.05 - 1.95 (m, 1H). MS (ESI) m/z 425.3 [M+H]⁺

**Compound 63:** General procedure A with variant ii) was used for the preparation with a yield of 56% from compound **VI** employing 3-Methylphenylmethyl-isocyanate. ¹H NMR (400 MHz, DMSO-*d*₆) *δ* = 10.98 (s, 1H), 7.64 (s, 1H), 7.57 - 7.49 (m, 2H), 7.23 - 7.15 (m, 1H), 7.11 - 6.96 (m, 3H), 6.56 (br t, *J* = 5.9 Hz, 1H), 6.48 (br t, *J* = 5.9 Hz, 1H), 5.12 (dd, *J* = 5.1, 13.3 Hz, 1H), 4.48 - 4.39 (m, 1H), 4.36 - 4.27 (m, 3H), 4.20 (d, *J =* 6.0 Hz, 2H), 2.98 - 2.84 (m, 1H), 2.60 (br dd, *J* = 1.6, 17.4 Hz, 1H), 2.44 - 2.36 (m, 1H), 2.27 (s, 3H), 2.05 - 1.96 (m, 1H). MS (ESI) m/z 421.3 [M+H]⁺

**Compound 64:** General procedure A with variant ii) was used for the preparation with a yield of 67% from compound **VI** employing 2-(4-Chlorophenyl)-ethyl-isocyanate. ¹H NMR (400 MHz, DMSO-*d*₆) *δ* = 10.98 (s, 1H), 7.61 (s, 1H), 7.57 - 7.51 (m, 1H), 7.50 - 7.45 (m, 1H), 7.34 (d, *J* = 8.3 Hz, 2H), 7.22 (d, *J* = 8.4 Hz, 2H), 6.48 (br t, *J* = 6.0 Hz, 1H), 5.99 (t,*J* = 5.6 Hz, 1H), 5.12 (dd, *J* = 5.1, 13.3 Hz, 1H), 4.48 - 4.37 (m, 1H), 4.35 - 4.25 (m, 3H), 3.24 (q, *J* = 6.8 Hz, 2H), 2.98 - 2.85 (m, 1H), 2.69 (t, *J* = 7.0 Hz, 2H), 2.65 - 2.56 (m, 1H), 2.45 - 2.37 (m, 1H), 2.05 - 1.94 (m, 1H). MS (ESI) m/z 455.3 [M+H]⁺

**Compound 65:** General procedure A with variant ii) was used for the preparation with a yield of 44% from compound **VI** employing 4-Trifluoromethoylphenylmethyl -isocyanate.¹H NMR (400 MHz, DMSO-*d*₆) *δ* = 10.98 (s, 1H), 7.71 - 7.62 (m, 3H), 7.56 - 7.50 (m, 2H), 7.47 (br d, *J =* 8.1 Hz, 2H), 6.74 - 6.60 (m, 2H), 5.12 (dd, *J* = 5.1, 13.3 Hz, 1H), 4.48 - 4.39 (m, 1H), 4.37 - 4.27 (m, 5H), 2.99 - 2.85 (m, 1H), 2.65 - 2.56 (m, 1H), 2.45 - 2.36 (m, 1H), 2.05 - 1.95 (m, 1H). MS (ESI) m/z 475.3 [M+H]⁺

**Compound 66:** General procedure A with variant ii) was used for the preparation with a yield of 72% from compound **VI** employing 2-Biphenyl-isocyanate. ¹H NMR (400 MHz, DMSO-*d*6) *δ* = 10.98 (s, 1H), 7.85 (d, *J =* 7.9 Hz, 1H), 7.63 (s, 1H), 7.58 - 7.54 (m, 1H), 7.53 - 7.46 (m, 4H), 7.42 - 7.34 (m, 3H), 7.31 - 7.25 (m, 1H), 7.17 (dd, *J =* 1.4, 7.5 Hz, 1H), 7.15 - 7.06 (m, 2H), 5.12 (dd, *J* = 5.0, 13.3 Hz, 1H), 4.49 - 4.39 (m, 1H), 4.38 - 4.26 (m, 3H), 2.98 - 2.85 (m, 1H), 2.64 - 2.56 (m, 1H), 2.40 (br dd, *J* = 4.5, 13.1 Hz, 1H), 2.05 - 1.94 (m, 1H). MS (ESI) m/z 469.3 [M+H]⁺

**Compound 67:** General procedure A with variant ii) was used for the preparation with a yield of 51% from compound **VI** employing 2-Fluoro-5-methylphenyl-isocyanate. ¹H NMR (400 MHz, DMSO-*d*₆) *δ* = 10.98 (s, 1H), 8.36 (d, *J* = 2.1 Hz, 1H), 7.95 (dd, *J* = 1.6, 7.9 Hz, 1H), 7.66 (s, 1H), 7.57 (s, 2H), 7.16 (t, *J =* 5.8 Hz, 1H), 7.04 (dd, *J =* 8.3, 11.5 Hz, 1H), 6.77 - 6.67 (m, 1H), 5.11 (dd, *J* = 5.1, 13.3 Hz, 1H), 4.48 - 4.39 (m, 3H), 4.35 - 4.28 (m, 1H), 2.97 - 2.86 (m, 1H), 2.64 - 2.56 (m, 1H), 2.39 (dq, *J* = 5.0, 13.4 Hz, 1H), 2.23 (s, 3H), 2.04 - 1.96 (m, 1H). MS (ESI) m/z 425.0 [M+H]⁺

**Compound 68:** General procedure A with variant ii) was used for the preparation with a yield of 38% from compound **VI** employing 3,4-Dimethylphenyl-isocyanate. ¹H NMR (400 MHz, DMSO-*d*₆) *δ* = 10.98 (s, 1H), 8.40 (s, 1H), 7.65 (s, 1H), 7.56 (s, 2H), 7.18 (s, 1H), 7.11 (dd, *J* = 2.0, 8.0 Hz, 1H), 6.96 (d, *J* = 8.2 Hz, 1H), 6.66 (t, *J* = 5.9 Hz, 1H), 5.11 (dd, *J* = 5.0, 13.2 Hz, 1H), 4.49 - 4.26 (m, 4H), 2.96 - 2.86 (m, 1H), 2.63 - 2.56 (m, 1H), 2.45 - 2.33 (m, 1H), 2.15 (s, 3H), 2.12 (s, 3H), 2.04 - 1.96 (m, 1H). MS (ESI) m/z 421.0 [M+H]⁺

**Compound 69:** General procedure A with variant ii) was used for the preparation with a yield of 18% from compound **VI** employing 2,5-Difluorophenyl-isocyanate. ¹H NMR (400 MHz, DMSO-*d*₆) *δ* = 10.98 (s, 1H), 8.69 (br s, 1H), 8.02 (ddd, *J =* 3.2, 6.6, 11.3 Hz, 1H), 7.66 (s, 1H), 7.57 (s, 2H), 7.34 - 7.18 (m, 2H), 6.83 - 6.67 (m, 1H), 5.11 (dd, *J* = 5.1, 13.3 Hz, 1H), 4.49 - 4.39 (m, 3H), 4.36 - 4.27 (m, 1H), 2.98 - 2.85 (m, 1H), 2.64 - 2.56 (m, 1H), 2.45 - 2.34 (m, 1H), 2.05 - 1.95 (m, 1H). MS (ESI) m/z 429.0 [M+H]⁺

**Compound 70:** General procedure A with variant ii) was used for the preparation with a yield of 36% from compound **VI** employing 3-Phenyl-n-propyl-isocyanate. ¹H NMR (400 MHz, DMSO-*d*₆) *δ* = 10.98 (s, 1H), 7.62 (s, 1H), 7.56 - 7.47 (m, 2H), 7.31 - 7.24 (m, 2H), 7.22 - 7.12 (m, 3H), 6.44 (br t, *J* = 6.1 Hz, 1H), 6.07 (t, *J* = 5.6 Hz, 1H), 5.11 (dd, *J* = 5.1, 13.3 Hz, 1H), 4.46 - 4.38 (m, 1H), 4.35 - 4.25 (m, 3H), 3.02 (q, *J =* 6.6 Hz, 2H), 2.96 - 2.86 (m, 1H), 2.64 - 2.58 (m, 1H), 2.58 - 2.54 (m, 2H), 2.45 - 2.33 (m, 1H), 2.05 - 1.95 (m, 1H), 1.67 (quin, *J* = 7.3 Hz, 2H). MS (ESI) m/z 435.0 [M+H]⁺

**Compound 71:** General procedure A with variant ii) was used for the preparation with a yield of 23% from compound **VI** employing 4-Biphenyl-isocyanate. ¹H NMR (400 MHz, DMSO-*d*₆) *δ* = 10.98 (s, 1H), 8.77 (s, 1H), 7.68 (s, 1H), 7.63 - 7.49 (m, 8H), 7.42 (t, *J* = 7.7 Hz, 2H), 7.32 - 7.27 (m, 1H), 6.82 (br t, *J* = 5.9 Hz, 1H), 5.11 (dd, *J* = 5.1, 13.3 Hz, 1H), 4.50 - 4.37 (m, 3H), 4.35 - 4.27 (m, 1H), 2.97 - 2.85 (m, 1H), 2.64 - 2.57 (m, 1H), 2.45 - 2.34 (m, 1H), 2.05 - 1.95 (m, 1H). MS (ESI) m/z 469.0 [M+H]⁺

**Compound 72:** General procedure A with variant ii) was used for the preparation with a yield of 58% from compound **VI** employing 2-Methoxyethyl-isocyanate. ¹H NMR (400 MHz, DMSO-*d*₆) *δ* = 10.96 (br s, 1H), 7.60 (s, 1H), 7.56 - 7.45 (m, 2H), 6.52 (br t, *J* = 5.7 Hz, 1H), 6.05 (br t, *J* = 5.3 Hz, 1H), 5.11 (br dd, *J* = 5.0, 13.2 Hz, 1H), 4.47 - 4.38 (m, 1H), 4.35 - 4.25 (m, 3H), 3.32 - 3.31 (m, 2H), 3.25 (s, 3H), 3.18 (q, *J =* 5.5 Hz, 2H), 2.97 - 2.85 (m, 1H), 2.63 - 2.56 (m, 1H), 2.45 - 2.33 (m, 1H), 2.05 - 1.94 (m, 1H). MS (ESI) m/z 375.0 [M+H]⁺.

**Compound 73:** General procedure with variant ii) was used for the preparation with a yield of 40% from compound **VI** employing 3-isocyanatobenzonitrile. ¹H NMR (400 MHz, DMSO-*d*₆) *δ* = 10.98 (s, 1H), 9.10 (s, 1H), 7.95 (t, *J* = 1.7 Hz, 1H), 7.67 (s, 1H), 7.64 - 7.59 (m, 1H), 7.56 (s, 2H), 7.43 (t, *J =* 7.9 Hz, 1H), 7.34 (td, *J =* 1.2, 7.7 Hz, 1H), 7.04 (br t, *J =* 5.9 Hz, 1H), 5.11 (dd, *J* = 5.1, 13.3 Hz, 1H), 4.48 - 4.37 (m, 3H), 4.34 - 4.27 (m, 1H), 2.97 - 2.84 (m, 1H), 2.62 - 2.57 (m, 1H), 2.43 - 2.29 (m, 1H), 2.05 - 1.94 (m, 1H).

### MS (ESI) m/z 418.2 [M+H]⁺

**Compound 74:** General procedure with variant ii) was used for the preparation with a yield of 44% from compound **VI** employing 4-isocyanatobenzonitrile. ¹H NMR (400 MHz, DMSO-*d*₆) *δ* = 10.97 (br s, 1H), 9.23 (s, 1H), 7.70 - 7.63 (m, 3H), 7.62 - 7.54 (m, 4H), 7.02 (t, *J* = 5.9 Hz, 1H), 5.11 (dd, *J* = 5.1, 13.3 Hz, 1H), 4.49 - 4.25 (m, 4H), 2.98 - 2.82 (m, 1H), 2.64 - 2.55 (m, 1H), 2.43 - 2.31 (m, 1H), 2.05 - 1.92 (m, 1H).

### MS (ESI) m/z 418.2 [M+H]⁺

**Compound 75:** General procedure with variant ii) was used for the preparation with a yield of 5% from compound **VI** employing 1-(3-chloro-5-isocyanato-2-methylphenyl)-N,N-dimethylmethanamine. ¹H NMR (400 MHz, DMSO-*d*₆) *δ* = 10.97 (br s, 1H), 8.82 (s, 1H), 7.72 - 7.50 (m, 4H), 7.13 (d, *J =* 2.2 Hz, 1H), 6.83 (t, *J* = 5.9 Hz, 1H), 5.12 (dd, *J* = 5.1, 13.3 Hz, 1H), 4.52 - 4.25 (m, 4H), 3.30 (s, 2H), 2.98 - 2.84 (m, 1H), 2.65 - 2.56 (m, 1H), 2.43 - 2.32 (m, 1H), 2.25 (s, 3H), 2.14 (s, 6H), 2.05 - 1.95 (m, 1H).

### MS (ESI) m/z 498.4 [M+H]⁺

### Scheme for the preparation of 1-(3-chloro-5-isocyanato-2-methylphenyl)-N,N-dimethylmethanamine:

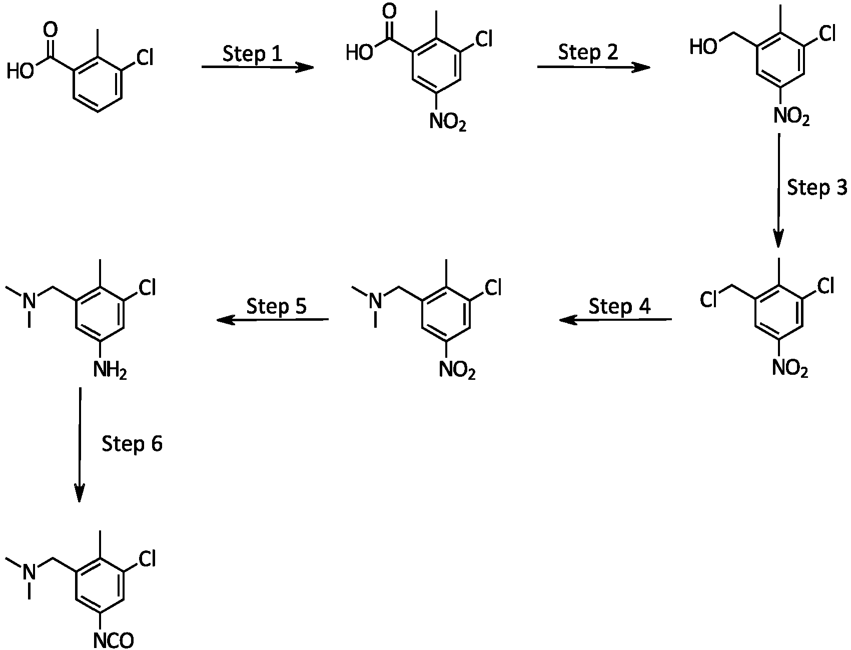

**Step 1:** 3-chloro-2-methyl-5-nitrobenzoic acid. To a solution of 3-chloro-2-methylbenzoic acid (10.0 g, 58.6 mmol, 1.00 *eq)* in sulfuric (50.0 mL) was added nitric acid (4.19 g, 64.5 mmol, 2.99 mL, 1.10 eq) dropwise at -10°C. Then the mixture was stirred at -10 °C for 1 h. The reaction mixture was poured into ice water (about 200 ml) and stirred, the precipitated solid was collected by filtration and washed with water. 3-chloro-2-methyl-5-nitrobenzoic acid (16.0 g, crude) as a white solid was used for the next step without purification.

**Step 2:** (3-chloro-2-methyl-5-nitrophenyl)methanol. To a solution of 3-chloro-2-methyl-5-nitrobenzoic acid (14.0 g, 64.9 mmol, 1.00 *eq)* in tetrahydrofuran (100 mL) was added borane dimethyl sulfide complex (10.0 M, 13.0 mL, 2.00 *eq*) at 0 °C. Then the mixture was stirred at 25 °C for 10 h. The reaction mixture was quenched by addition of methanol (15.0 mL) at 0 °C, and then filtered and concentrated under reduced pressure to give a residue. The residue was purified by column chromatography (petroleum ether/ethyl acetate = 5/1) to afford (3-chloro-2-methyl-5-nitrophenyl)methanol (12.0 g, 59.5 mmol, 92% yield) as a yellow solid.

**Step 3:** 1-chloro-3-(chloromethyl)-2-methyl-5-nitrobenzene. To a solution of (3-chloro-2-methyl-5-nitrophenyl)methanol (2.00 g, 9.92 mmol, 1.00 *eq*) in dichloromethane (20.0 mL) was added thionyl chloride (5.90 g, 49.6 mmol, 3.60 mL, 5.00 *eq*) at 0 °C. Then the mixture was stirred at 25 °C for 2 h. The reaction mixture was concentrated under reduced pressure to afford 1-chloro-3-(chloromethyl)-2-methyl-5-nitrobenzene (2.20 g, crude) as yellow oil.

**Step 4:** 1-(3-chloro-2-methyl-5-nitrophenyl) -N,N-dimethylmethanamine. To a solution of 1-chloro-3-(chloromethyl)-2-methyl-5-nitrobenzene (2.18 g, 9.92 mmol, 1.00 *eq)* and triethylamine (2.51 g, 24.8 mmol, 3.45 mL, 2.50 *eq)* in acetonitrile (20.0 mL) was added dimethylamine hydrochloride (1.01 g, 12.4 mmol, 1.25 eq). Then the mixture was stirred at 25 °C for 10 h. The reaction mixture was concentrated under reduced pressure to give a residue. The residue was purified by column chromatography (petroleum ether/ethyl acetate = 3/1) to afford 1-(3-chloro-2-methyl-5- nitrophenyl)-N,N-dimethylmethanamine (0.450 g, 1.97 mmol, 20% yield) as yellow oil.

**Step 5:** 3-chloro-5-((dimethylamino)methyl)-4-methylaniline. A mixture of 1-(3-chloro-2-methyl-5-nitrophenyl)-N,N-dimethylmethanamine (0.450 g, 1.97 mmol, 1.00 *eq),* ammonium chloride (105 mg, 1.97 mmol, 1.00 *eq)* and ferrous powder (549 mg, 9.84 mmol, 5.00 *eq)* in ethyl alcohol (6.00 mL) and water (3.00 mL) was stirred at 90 °C for 10 h. The reaction mixture was filtered, and then the filtrate was extracted with ethyl acetate (3 × 25.0 mL). The combined organic layers were washed with brine (10.0 mL), dried over sodium sulfate, filtered and concentrated under reduced pressure to give 3-chloro-5-((dimethylamino)methyl)-4-methylaniline (0.400 g, 2.01 mmol, crude) as yellow oil.

**Step 6:** 1-(3-chloro-5-isocyanato-2-methylphenyl)-N,N-dimethylmethanamine. A mixture of 3-chloro-5-((dimethylamino)methyl)-4-methylaniline (0.350 g, 1.76 mmol, 1.00 *eq)* and triphosgene (261 mg, 881 umol, 0.50 *eq)* in toluene (5.00 mL) was stirred at 110 °C for 2 h. The reaction mixture was concentrated under reduced pressure to give 1-(3-chloro-5-isocyanato-2-methylphenyl)-*N*,*N* -dimethylmethanamine (0.450 g, crude, HCl) as a yellow solid.

**Compound 76:** General procedure with variant ii) was used for the preparation with a yield of 63% from compound **VI** employing 1-chloro-5-isocyanato-4-methoxy-2-methylbenzene. ¹H NMR (400 MHz, DMSO-*d*₆) *δ* = 10.97 (s, 1H), 8.15 (s, 1H), 8.12 (s, 1H), 7.64 (s, 1H), 7.59 - 7.51 (m, 2H), 7.43 (t, J = 5.9 Hz, 1H), 6.95 (s, 1 H), 5.11 (dd, J = 5.1, 13.3 Hz, 1 H), 4.48 - 4.22 (m, 4H), 3.83 (s, 3H), 2.96 - 2.82 (m, 1 H), 2.62 - 2.54 (m, 1 H), 2.38 (br dd, J = 4.5, 12.9 Hz, 1H), 2.24 (s, 3H), 2.05 - 1.91 (m, 1H).

### MS (ESI) m/z 471.2 [M+H]⁺

### Scheme for the preparation of 1-chloro-5-isocyanato-4-methoxy-2-methylbenzene:

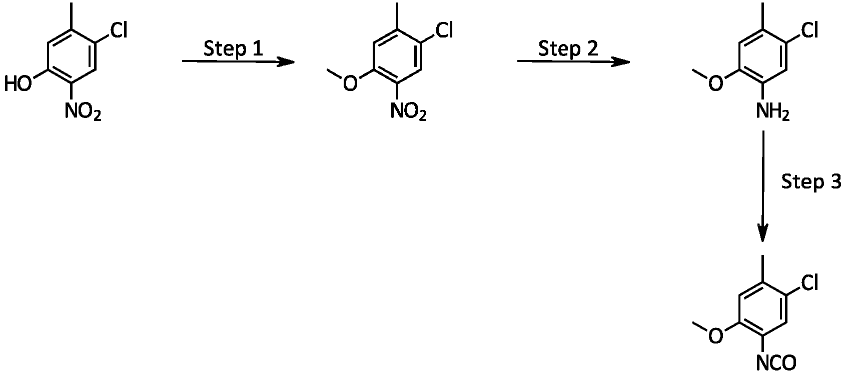

**Step 1:** 1-chloro-4-methoxy-2-methyl-5-nitrobenzene. To a solution of 4-chloro-5-methyl-2-nitrophenol (4.60 g, 24.5 mmol, 1.00 *eq*) in acetonitrile (50.0 mL) was added dimethyl sulfate (3.71 g, 29.4 mmol, 2.79 mL, 1.20 *eq*) and potassium carbonate (6.78 g, 49.1 mmol, 2.00 *eq*). The reaction was stirred at 80 °C for 12 h. The reaction mixture was quenched with water (50.0 mL), extracted with ethyl acetate (3 × 100 mL). The combined extracts were washed with water (2 × 50.0 mL), dried over sodium sulfate, filtered and concentrated in vacuo to give 1-chloro-4-methoxy-2-methyl-5-nitrobenzene (4.90 g, 24.3 mmol, 99% yield) as a white oil.

**Step 2:** 5-chloro-2-methoxy-4-methylaniline. To a solution of 1-chloro-4-methoxy-2-methyl-5-nitrobenzene (5.00 g, 24.8 mmol, 1.00 *eq*) in ethanol (30.0 mL) and water (10.0 mL) was added iron powder (4.15 g, 74.4 mmol, 3.00 *eq*) and ammonium chloride (6.63 g, 124 mmol, 5.00 *eq*). The reaction was stirred at 80 °C for 12 h. The reaction mixture was filtered and concentrated in vacuo. The residue was suspended in water (50.0 mL) and extracted with ethyl acetate (3 × 100 mL). The combined extracts washed with water (2 × 50.0 mL), dried over sodium sulfate, filtered and concentrated in vacuo to give 5-chloro-2-methoxy-4-methylaniline (3.00 g, 17.5 mmol, 70% yield) as a yellow solid.

**Step 3:** 1-chloro-5-isocyanato-4-methoxy-2-methylbenzene. To a solution of 5-chloro-2-methoxy-4-methylaniline (1.00 g, 5.83 mmol, 1.00 *eq*) in toluene (10.0 mL) was added triphosgene (1.73 g, 5.83 mmol, 1.00 eq). The reaction was stirred at 100 °C for 2 h. The reaction mixture was concentrated in vacuo to give 1-chloro-5-isocyanato-4-methoxy-2-methylbenzene (1.15 g, 5.82 mmol, 100% yield) as a yellow oil (a solution in DCM (3 mL)), which was used for the next step directly.

**Compound 77:** General procedure with variant ii) was used for the preparation with a yield of 54% from compound **VI** employing 1-isocyanato-4-(trifluoromethoxy)benzene. ¹H NMR (400 MHz, DMSO-*d*₆) *δ* = 10.97 (br s, 1H), 8.90 (s, 1H), 7.66 (s, 1 H), 7.56 (d, J = 0.8 Hz, 2H), 7.53 - 7.48 (m, 2H), 7.22 (d, J = 8.4 Hz, 2H), 6.87 (brt, J = 5.6 Hz, 1H), 5.11 (dd, J = 5.2, 13.6 Hz, 1H), 4.47 - 4.38 (m, 3H), 4.34 - 4.27 (m, 1H), 2.99 - 2.85 (m, 1H), 2.64 - 2.55 (m, 1H), 2.43 - 2.35 (m, 1H), 2.04 - 1.96 (m, 1H).

### MS (ESI) m/z 477.2 [M+H]⁺

**Compound 78:** General procedure with variant ii) was used for the preparation with a yield of 32% from compound **VI** employing 1,4-dichloro-2-isocyanato-5-methylbenzene. ¹H NMR (400 MHz, DMSO-*d*₆) *δ* = 10.99 (s, 1H), 8.28 (s, 1H), 8.23 (s, 1H), 7.68 (s, 1H), 7.64 - 7.56 (m, 3H), 7.45 (s, 1H), 5.12 (dd, J = 5.1, 13.3 Hz, 1H), 4.52 - 4.39 (m, 3H), 4.38 - 4.26 (m, 1H), 3.00 - 2.81 (m, 1 H), 2.64 - 2.58 (m, 1H), 2.39 (br dd, J = 7.9, 12.2 Hz, 1H), 2.25 (s, 3H), 2.05 - 1.95 (m, 1H).

### MS (ESI) m/z 475.1 [M+H]⁺

### Scheme for the preparation of 1,4-dichloro-2-isocyanato-5-methylbenzene:

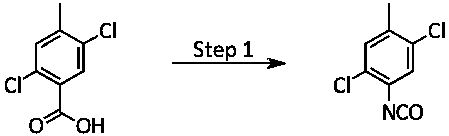

**Step 1:** To a solution of 2,5-dichloro-4-methyl-benzoic acid (1.00 g, 4.88 mmol, 1.00 eq) and triethylamine (523 mg, 5.17 mmol, 720 uL, 1.06 eq) in toluene (50.0 mL) was added diphenyl phosphorazidate (1.37 g, 4.97 mmol, 1.08 mL, 1.02 eq) at 20 °C. The reaction mixture was stirred 120 °C for 2 h. The reaction mixture was concentrated in vacuo. The residue was suspended in dichloromethane (4.00 mL) to give 1,4-dichloro-2-isocyanato- 5-methylbenzene (985 mg, 4.88 mmol, 99.96% yield) as a yellow oil, which (in dichloromethane (4.00 mL)) was used for the next step.

**Compound 79:** General procedure A with variant iv) was used for the preparation with a yield of 22% from compound **VI** employing 4-isocyanato-*N*,*N*-dimethylaniline.

¹H NMR (400 MHz, DMSO-*d*₆ + D₂O) *□* = 7.72 - 7.46 (m, 7H), 5.06 (dd, *J* = 5.1, 13.3 Hz, 1H), 4.47 - 4.25 (m, 4H), 3.10 (s, 6H), 2.93 - 2.81 (m, 1H), 2.65 - 2.55 (m, 1H), 2.37 (dq, *J* = 4.3, 13.2 Hz, 1H), 2.05 - 1.93 (m, 1H). MS (ESI) m/z 436.2 [M+H]⁺

### Scheme for the preparation of 4-isocyanato-N,N-dimethylaniline.

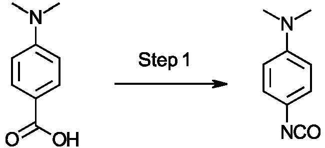

**Step 1:** To a solution of 4-(dimethylamino)benzoic acid (1.00 g, 6.05 mmol, 1.00 *eq*) in toluene (50.0 mL) was added triethylamine (0.89 mL, 6.42 mmol, 1.06 *eq*) and diphenylphosphoryl azide (1.34 mL, 6.17 mmol, 1.02 *eq*)*.* The reaction was stirred at 20°C for 0.5 h, then at 120°C for 2 h. The mixture was concentrated under reduced pressure to give a residue. Dichloromethane (4.00 mL) was added to the residue to afford the 4-isocyanato-*N*,*N*-dimethylaniline (982 mg, crude) as red oil.

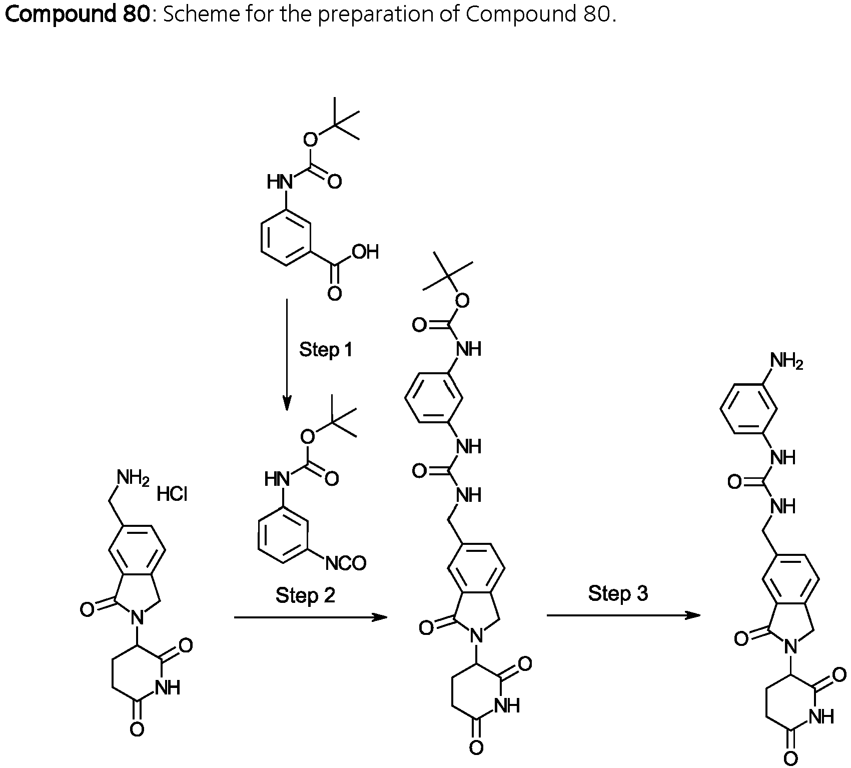

**Step 1:** To a solution of 3-((*tert*-butoxycarbonyl)amino)benzoic acid (500 mg, 2.11 mmol, 1.00 eq) in toluene (25.0 mL) was added triethylamine (0.31 mL, 2.23 mmol, 1.06 eq) and diphenyl phosphorazidate (0.47 mL, 2.15 mmol, 1.02 eq). The reaction was stirred at 20°C for 0.5 h, then at 120°C for 2 h. The mixture was concentrated under reduced pressure to afford *tert*-butyl-(3-isocyanatophenyl)carbamate (490 mg, 2.09 mmol, 99% yield) as a yellow oil.

**Step 2:** To a solution of 3-(6-(aminomethyl)-1-oxoisoindolin-2-yl)piperidine-2,6-dione hydrochloride **VI** (1.00 eq, hydrochloride) in dimethylformamide (2.00 mL) was added triethylamine (0.11 mL, 0.77 mmol, 1.20 eq) and *tert*-butyl (3-isocyanatophenyl)carbamate (182 mg, 0.77 mmol, 1.20 eq) at 0°C. The reaction was stirred at 20°C for 1 h. The mixture was concentrated under reduced pressure to give a residue, which was purified by reversed phase column chromatography and lyophilized to afford *tert*-butyl (3-(3-((2-(2,6-dioxopiperidin-3-yl)-3-oxoisoindolin-5-yl)methyl)ureido)phenyl)carbamate (170 mg, 0.34 mmol, 52% yield) as a white solid.

**Step 3:** To a solution of tert-butyl (3-(3-((2-(2,6-dioxopiperidin-3-yl)-3-oxoisoindolin-5-yl)methyl)ureido) phenyl)carbamate (150 mg, 0.30 mmol, 1.00 eq) in methanol (1.00 mL) was added 4N of hydrochloric acid in methanol (1.00 mL). The reaction was stirred at 20°C for 0.5 h. The mixture was concentrated under reduced pressure to give a residue, which was purified by preparative HPLC and lyophilized to afford **Compound 80** (77.8 mg, 0.17 mmol, 59% yield) as an off-white solid.

¹H NMR (400 MHz, DMSO-*d*₆) *□* = 7.83 - 7.71 (m, 1H), 7.68 - 7.61 (m, 1H), 7.56 (d, *J* = 0.9 Hz, 2H), 7.39 - 7.29 (m, 1H), 7.28 - 7.19 (m, 1H), 7.00 - 6.76 (m, 1H), 5.07 (dd, *J* = 5.1, 13.3 Hz, 1H), 4.45 - 4.37 (m, 3H), 4.34 - 4.20 (m, 1H), 2.93 - 2.80 (m, 1H), 2.59 (td, *J =* 2.0, 15.3 Hz, 1H), 2.40 - 2.40 (m, 1H), 2.04 - 1.94 (m, 1H). MS (ESI) m/z 408.1 [M+H]⁺

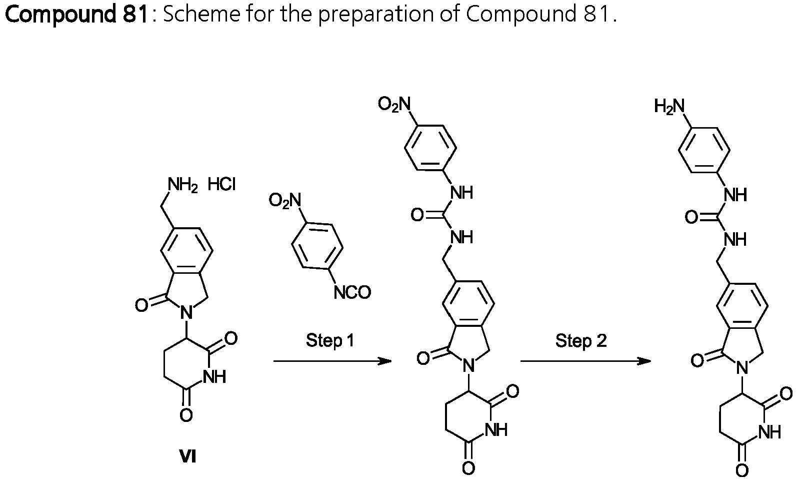

**Step 1:** To a solution of 3-(6-(aminomethyl)-1-oxoisoindolin-2-yl)piperidine-2,6-dione hydrochloride **VI** (1.00 eq, hydrochloride) in dimethylformamide (2.00 mL) was added triethylamine (0.09 ml, 645 µmol, 1.00 eq) and 1-isocyanato-4-nitro-benzene (106 mg, 0.65 mmol, 1.00 eq) at 0°C. The reaction was stirred 20°Cfor 1 h. The mixture was quenched with water (10.0 mL) and extracted with ethyl acetate (2 × 50 mL). The combined organic layers were washed with brine (30.0 mL), dried over anhydrous sodium sulfate, filtered, and the filtrate was concentrated under reduced pressure to afford 1-[[2-(2,6-dioxo-3-piperidyl)-3-oxo-isoindolin-5-yl]methyl]-3-(4-nitrophenyl)urea (200 mg, 0.56 mmol, 71% yield) as a black oil. It was used directly in the next step.

**Step 2:** To a solution of 1-[[2-(2,6-dioxo-3-piperidyl)-3-oxo-isoindolin-5-yl]methyl]-3-(4-nitrophenyl)urea (150 mg, 343 µmol, 1.00 eq) in methanol (3.00 mL) was added Pd/C 10.0% weight on C (5 mg,) and hydrochloric acid 4M (8.57 µL, 0.10 eq). The reaction was stirred at 20°C for 2 h under hydrogen atmosphere (15 psi). The mixture was filtered, and the filtrate was concentrated under reduced pressure to give a residue. The residue was purified by reversed phase preparative HPLC and lyophilized to afford **Compound 81** (20.0 mg, 43.8 µmol, 13% yield) as a black solid.

¹H NMR (400 MHz, DMSO-*d*₆) *□* = 11.0 (s, 1H), 10.52 - 9.67 (b, 3H), 9.24 (s, 1H), 7.66 (s, 1H), 7.57 (s, 2H), 7.53 (d, *J =* 8.9 Hz, 2H), 7.25 (d, *J =* 8.9 Hz, 2H), 7.08 (br s, 1H), 5.11 (dd, *J* = 5.0, 13.2 Hz, 1H), 4.48 - 4.27 (m, 4H), 2.98 - 2.81 (m, 1H), 2.60 (br d, *J=* 17.4 Hz, 1H), 2.45 - 2.36 (m, 1H), 2.05 - 1.95 (m, 1H). MS (ESI) m/z 408.4 [M+H]⁺

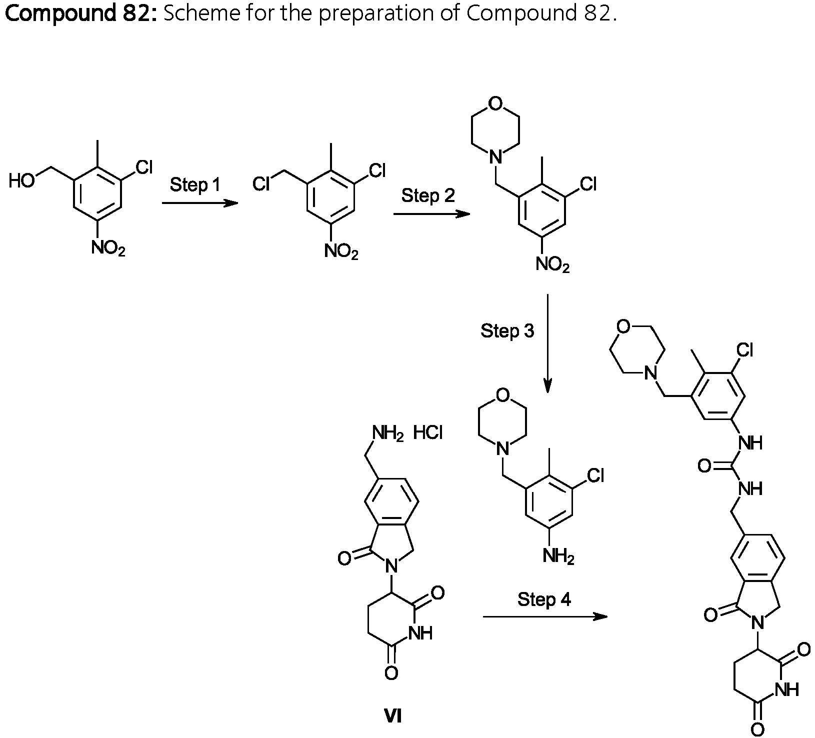

**Step 1:** To a solution of (3-chloro-2-methyl-5-nitrophenyl)methanol (2.00 g, 9.92 mmol, 1.00 eq) in dichloromethane (20.0 mL) was added thionyl chloride (3.60 mL, 49.6 mmol, 5.00 eq). The reaction was stirred at 25°C for 2 h. The mixture was concentrated under reduced pressure to afford 1-chloro-3-(chloromethyl)-2-methyl-5-nitro-benzene (2.20 g, 10.0 mmol, crude) as a yellow oil. It was used directly in the next step.

**Step 2:** To a solution of 1-chloro-3-(chloromethyl)-2-methyl-5-nitrobenzene (2.20 g, 10.0 mmol, 1.00 eq) and triethylamine (3.48 mL, 25.0 mmol, 2.50 eq) in acetonitrile (20.0 mL) was added morpholine (1.14 mL, 13.0 mmol, 1.30 eq). The reaction was stirred at 25°C for 10 h. The mixture was concentrated under reduced pressure to give a residue, which was purified by silica gel column chromatography (petroleum ether/ethyl acetate = 3/1) to afford 4-(3-chloro-2-methyl-5-nitrobenzyl) morpholine (0.510 g, 1.88 mmol, 19% yield) as a yellow solid.

**Step 3:** To a mixture of ethanol (8.00 mL) and water (4.00 mL) was added 4-(3-chloro-2-methyl-5-nitrobenzyl)morpholine (0.500 g, 1.85 mmol, 1.00 eq), ammonium chloride (98.8 mg, 1.85 mmol, 1.00 *eq*) and ferrous powder (516 mg, 9.23 mmol, 5.00 eq). The reaction was stirred at 90°C for 10 h. The mixture was filtered and washed with ethyl acetate (20.0 mL). The filtrate was extracted with ethyl acetate (3 × 25.0 mL), and the combined organic layers were washed with brine (20.0 mL), dried over sodium sulfate, filtered, and concentrated under reduced pressure to afford 3-chloro-4-methyl-5-(morpholinomethyl)aniline (0.45 g, 1.85 mmol, crude) as a yellow oil. It was used directly in the next step.

**Step 4:** To a solution of 3-chloro-4-methyl-5-(morpholinomethyl)aniline (0.100 g, 0.41 mol, 1.00 eq) in tetrahydrofuran (1.00 mL) was added 1,1'-carbonyldiimidazole (74.1 mg, 0.46 mmol, 1.10 eq). The mixture was stirred at 25°C for 2 h, then 3-(6-(aminomethyl)-1-oxoisoindolin-2-yl)piperidine-2,6-dione hydrochloride **VI** (129 mg, 0.41 mmol, 1.00 eq, hydrochloride) was added. The reaction was stirred at 25°C for 10 h. The mixture was adjusted to pH = 5 by addition of HCl (1N), then it was concentrated under reduced pressure to give a residue. The residue was purified by reversed phase preparative HPLC to afford **Compound 82** (16.26 mg, 29.8 µmol, 7% yield) as a white solid.

¹H NMR (400 MHz, DMSO-*d*₆) *□* = 11.0 (s, 1H), 8.82 (s, 1H), 8.21 (s, 1H), 7.68 - 7.61 (m, 2H), 7.56 (d, *J = 0.6* Hz, 2H), 7.12 (d, *J =* 2.0 Hz, 1H), 6.83 (br t, *J* = 5.6 Hz, 1H), 5.12 (dd, *J* = 5.0, 13.1 Hz, 1H), 4.46 - 4.27 (m, 4H), 3.54 (br s, 4H), 3.38 (br s, 2H), 2.97 - 2.85 (m, 1H), 2.63 - 2.59 (m, 1H), 2.42 - 2.38 (m, 1H), 2.35 (br s, 4H), 2.26 (s, 3H), 2.04 - 1.94 (m, 1H). MS (ESI) m/z 540.1 [M+H]⁺

**Compound 83:** General procedure A with variant iv) was used for the preparation with a yield of 6% from compound **VI** employing 4-isocyanato-1-methyl-1*H*-pyrazole.

¹H NMR (400 MHz, DMSO-*d*₆) *□*= 10.98 (br s, 1H), 8.85 (s, 1H), 7.64 (s, 1H), 7.58 - 7.52 (m, 2H), 7.47 (d, *J =* 2.4 Hz, 1H), 7.28 (br s, 1H), 6.05 (d, *J =* 2.0 Hz, 1H), 5.11 (dd, *J =* 4.8, 13.2 Hz, 1H), 4.49 - 4.26 (m, 4H), 3.68 (s, 3H), 2.98 - 2.85 (m, 1H), 2.64 - 2.55 (m, 1H), 2.46 - 2.36 (m, 1H), 2.06 - 1.94 (m, 1H). MS (ESI) m/z 397.1 [M+H]⁺

### Scheme for the preparation of 4-isocyanato-1-methyl-1H-pyrazole.

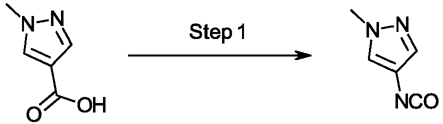

**Step 1:** To a mixture of 1-methyl-1 N-pyrazole-4-carboxylic acid (400 mg, 3.17 mmol, 1.00 eq) and triethylamine (0.55 mL, 3.96 mmol, 1.25 eq) in toluene (5.00 mL) was added diphenylphosphoryl azide (0.83 mL, 3.81 mmol, 1.20 eq) in one portion at 20°C. The mixture was stirred at 105°C under nitrogen for 3 h. The mixture was cooled to 20°C, then concentrated under reduced pressure to afford 4-isocyanato-1-methyl-1 *H*-pyrazole (1.80 g, crude) as black oil. It was used directly in the next step.

**Compound 84:** General procedure A with variant iv) was used for the preparation with a yield of 37% from compound **VI** employing 3-isocyanatothiophene.

¹H NMR (400 MHz, DMSO-*d*₆) *□* = 11.04 (s, 1H), 8.99 (s, 1H), 7.71 (s, 1H), 7.61 (s, 2H), 7.42 (dd, *J* = 3.2, 5.2 Hz, 1H), 7.22 (dd, *J =* 1.2, 3.2 Hz, 1H), 7.04 (dd, *J =* 1.2, 5.2 Hz, 1H), 6.79 (t, *J* = 6.0 Hz, 1H), 5.17 (dd, *J* = 5.0, 13.2 Hz, 1H), 4.51 - 4.33 (m, 4H), 3.02 - 2.90 (m, 1H), 2.75 - 2.60 (m, 1H), 2.51 - 2.37 (m, 1H), 2.10 - 2.00 (m, 1H). MS (ESI) m/z 399.0 [M+H]⁺

### Scheme for the preparation of 3-isocyanatothiophene.

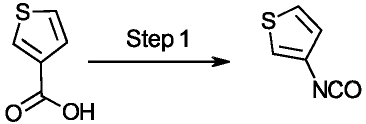

**Step 1:** To a suspension of thiophene-3-carboxylic acid (300 mg, 2.34 mmol, 1.00 eq) and triethylamine (407 µL, 2.93 mmol, 1.25 eq) in dry toluene (5.00 mL) was added diphenylphosphoryl azide (609 µL, 2.81 mmol, 1.20 eq) at 25°C under nitrogen. The reaction was stirred at 25°C for 30 min, then heated to 100°C for 2 h. The mixture was concentrated under reduced pressure to afford 3-isocyanatothiophene (300 mg, crude) as a white solid, which was used directly in the next step.

**Compound 85:** General procedure A with variant iv) was used for the preparation with a yield of 59% from compound **VI** employing 1-chloro-5-isocyanato-2,4-dimethylbenzene.

¹H NMR (400 MHz, DMSO-*d*₆) *□* = 10.98 (br s, 1H), 7.98 (s, 1H), 7.84 (br d, *J* = 3.1 Hz, 1H), 7.67 (s, 1H), 7.58 (d, *J* = 0.9 Hz, 2H), 7.25 - 7.15 (m, 1H), 7.09 (s, 1H), 5.12 (dd, J = 5.0, 13.3 Hz, 1H), 4.50 - 4.24 (m, 4H), 3.00 - 2.81 (m, 1H), 2.62 - 2.55 (m, 1H), 2.43 - 2.36 (m, 1H), 2.21 (s, 3H), 2.14 (s, 3H), 2.01 (ddd, J= 2.4, 5.3, 10.0 Hz, 1H). MS (ESI) m/z 455.2 [M+H]⁺

### Scheme for the preparation of 1-chloro-5-isocyanato-2,4-dimethylbenzene.

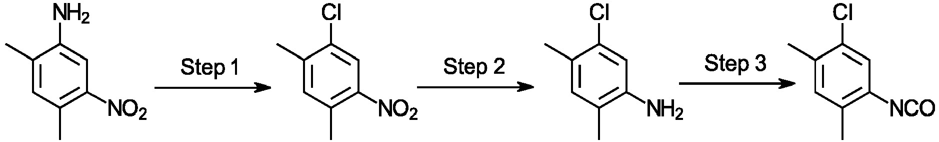

**Step 1:** To a solution of 2,4-dimethyl-5-nitroaniline (500 mg, 3.01 mmol, 1.00 eq) in concentrated hydrochloric acid (9.00 mL) was added a solution of sodium nitrite (208 mg, 3.01 mmol, 1.00 eq) in water (0.60 mL) at 0°C. The reaction was stirred at 0°C for 1 h, then cuprous chloride (477 mg, 4.81 mmol, 1.60 eq) was added at 0°C. The reaction was stirred at 20°C for 11 h. Water (50.0 mL) was added, followed by potassium carbonate until pH = 7: The mixture was extracted with ethyl acetate (3 x 100 mL). The combined organic layers were washed with brine (50.0 mL), dried over sodium sulfate, filtered, and concentrated under reduced pressure to give a residue. The residue was purified by silica gel column chromatography (petroleum ether/ethyl acetate = 1/0 to 10/1) to afford 1-chloro-2,4-dimethyl-5-nitrobenzene (400 mg, 2.16 mmol, 72% yield) as a yellow solid.

**Step 2:** To a solution of 1-chloro-2,4-dimethyl-5-nitrobenzene (350 mg, 1.89 mmol, 1.00 eq) in ethanol (12.0 mL) and water (3.00 mL) was added iron powder (315 mg, 5.66 mmol, 3 eq) and ammonium chloride (504 mg, 9.43 mmol, 5.00 eq). The reaction was stirred at 80°C for 2 h. The mixture was filtered and concentrated under reduced pressure to give a residue. The residue was purified by silica gel column chromatography (petroleum ether/ethyl acetate = 1/0 to 10/1) to afford 5-chloro-2,4-dimethylaniline (290 mg, 1.86 mmol, 99.0% yield) as a white solid.

**Step 3:** To a solution of 5-chloro-2,4-dimethylaniline (270 mg, 1.73 mmol, 1.00 eq) in toluene (10.0 mL) was added triphosgene (515 mg, 1.73 mmol, 1.00 eq). The reaction was stirred at 100°C for 2 h. The mixture was concentrated under reduced pressure to afford 1-chloro-5-isocyanato-2,4-dimethylbenzene (315 mg, 1.73 mmol, 99.0% yield) as yellow oil. **Compound 86:** General procedure A with variant iv) was used for the preparation with a yield of 41 % from compound **VI** employing 2-chloro-4-isocyanato-3-methoxy-1-methylbenzene.

¹H NMR (400 MHz, DMSO-*d*₆) □ = 10.9 (br s, 1 H), 8.24 (s, 1H), 8.02 (d, *J* = 8.4 Hz, 1H), 7.68 (s, 1 H), 7.57 (d, *J* = 0.7 Hz, 2H), 7.44 (br t, *J* = 5.8 Hz, 1 H), 6.99 (d, *J* = 8.7 Hz, 1 H), 5.12 (dd, *J* = 5.1, 13.3 Hz, 1H), 4.48 - 4.37 (m, 3H), 4.36 - 4.25 (m, 1H), 3.74 (s, 3H), 2.96 - 2.83 (m, 1H), 2.65 - 2.55 (m, 1H), 2.42 - 2.34 (m, 1H), 2.25 (s, 3H), 2.05 - 1.91 (m, 1H). MS (ESI) m/z 471.2 [M+H]⁺

### Scheme for the preparation of 2-chloro-4-isocyanato-3-methoxy-1-methylbenzene.

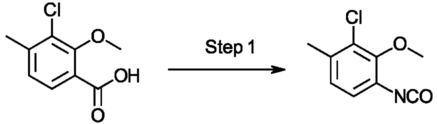

**Step 1:** To a solution of 3-chloro-2-methoxy-4-methylbenzoic acid (300 mg, 1.50 mmol, 1.00 eq) in toluene (15.0 mL) was added triethylamine (0.22 mL, 1.59 mmol, 1.06 eq) and diphenyl phosphorazidate (0.33 mL, 1.53 mmol, 1.02 eq). The reaction was stirred at 20°C for 0.5 h, then at 120°C for 2 h. The mixture was concentrated under reduced pressure to give a residue. Dichloromethane (4.00 mL) was added to afford 2-chloro-4-isocyanato-3-methoxy-1-methylbenzene (296 mg, crude) as a yellow oil.

**Compound 87:** General procedure A with variant iv) was used for the preparation with a yield of 41% from compound **VI** employing 2,3-dichloro-1-isocyanato-4-methylbenzene.

¹H NMR (400 MHz, DMSO-*d*₆) □ = 10.9 (br s, 1H), 8.24 (s, 1H), 8.07 - 7.97 (m, 1H), 7.68 (s, 1H), 7.63 - 7.50 (m, 3H), 7.24 (d, *J =* 8.7 Hz, 1H), 5.12 (dd, *J* = 5.1, 13.3 Hz, 1H), 4.49 - 4.38 (m, 3H), 4.37 - 4.27 (m, 1H), 2.98 - 2.85 (m, 1H), 2.65 - 2.56 (m, 1H), 2.40 (brdd, *J* = 4.5, 13.1 Hz, 1H), 2.32 (s, 3H), 2.05 - 1.97 (m, 1H). MS (ESI) m/z 475.2 [M+H]⁺

### Scheme for the preparation of 2,3-dichloro-1-isocyanato-4-methylbenzene.

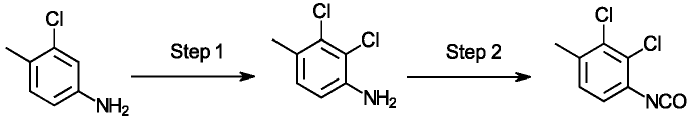

**Step 1:** To a solution of 3-chloro-4-methylaniline (5.00 g, 35.3 mmol, 1.00 eq) in N,N-dimethyformamide (50.0 mL) at 0°C was added 1-chloropyrrolidine-2, 5-dione (5.00 g, 37.4 mmol, 1.06 eq) in dimethyformamide (20.0 mL) dropwise. The reaction was stirred at 20°C for 12 h. Water (50.0 mL) was added and the mixture was extracted with ethyl acetate (2 × 100 mL). The combined organic layers were washed with brine (2 × 50.0 mL), dried over sodium sulfate, and concentrated under reduced pressure to give a residue. The residue was purified by silica gel column chromatography (petroleum ether/ethyl acetate = 1/0 to 3/1) to give 2,3-dichloro-4-methylaniline (2.50 g, 14.2 mmol, 40.0% yield) as a black oil.

**Compound 88:** General procedure A with variant iii) was used for the preparation with a yield of 41 % from compound **VI** employing phenyl (1-methyl-1 *H*-pyrazol-3-yl)carbamate.

¹H NMR (400 MHz, DMSO-*d*₆) *□* = 10.98 (br s, 1H), 8.85 (s, 1 H), 7.64 (s, 1H), 7.58 - 7.52 (m, 2H), 7.47 (d, *J* = 2.4 Hz, 1H), 7.28 (br s, 1H), 6.05 (d, *J* = 2.0 Hz, 1H), 5.11 (dd, *J* = 4.8, 13.2 Hz, 1H), 4.49 - 4.26 (m, 4H), 3.68 (s, 3H), 2.98 - 2.85 (m, 1H), 2.64 - 2.55 (m, 1H), 2.46 - 2.36 (m, 1H), 2.06 - 1.94 (m, 1H). MS (ESI) m/z 397.1 [M+H]⁺

### Scheme for the preparation of phenyl (1-methyl-1H-pyrazol-3-yl)carbamate.

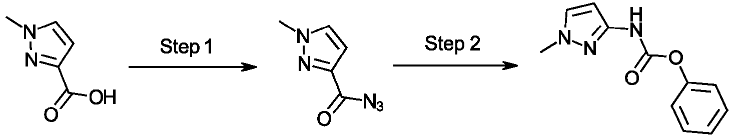

**Step 1:** To a solution of 1-methyl-1 *H*-pyrazole-3-carboxylic acid (500 mg, 3.96 mmol, 1.00 eq), triethylamine (0.55 mL, 3.96 mmol, 1.00 eq) in toluene (5.00 mL) was added diphenyl phosphorazidate (0.86 mL, 3.96 mmol, 1.00 eq) at 25°C. The reaction was stirred at 25°C for 30 min. The mixture was concentrated under reduced pressure to afford 1-methyl-1 *H-*pyrazole-3-carbonyl azide (600 mg, crude) as a black solid. It was used directly in the next step.

**Step 2:** To a solution of phenol (2.96 mL, 33.7 mmol, 11.3 eq) in toluene (22.0 mL) was added 1-methyl-1 *H*-pyrazole-3-carbonyl azide (450 mg, 2.98 mmol, 1.00 eq) at 100°C. The reaction was stirred at 100°C for 3 hr. The mixture was concentrated under reduced pressure to give a residue. The residue was purified by silica gel column chromatography (petroleum ether/ethyl acetate = 0/1 to 3/1) to give phenyl (1-methyl-1 *H*-pyrazol-3-yl)carbamate (160 mg, 0.66 mmol, 22% yield) as a red solid.

**Compound 89:** General procedure A with variant iv) was used for the preparation with a yield of 7% from compound **VI** employing 3-isocyanato-*N*,*N*-dimethylaniline.

¹H NMR (400 MHz, DMSO-*d*₆) *□* = 11.00 (br s, 1H), 8.52 (s, 1H), 8.32 (s, 1H), 7.66 (s, 1H), 7.56 (d, *J* = 0.9 Hz, 2H), 7.04 - 6.97 (m, 1H), 6.88 (t, *J* = 2.1 Hz, 1H), 6.79 - 6.65 (m, 2H), 6.30 (dd, *J =* 2.1, 8.2 Hz, 1H), 5.12 (dd, J = 5.1, 13.3 Hz, 1H), 4.46 - 4.26 (m, 4H), 3.02 - 2.89 (m, 1H), 2.85 (s, 6H), 2.60 (td, *J* = 1.9, 15.4 Hz, 1H), 2.39 (br dd, J = 4.5, 13.0 Hz, 1H), 2.05 - 1.95 (m, 1H). MS (ESI) m/z 436.2 [M+H]⁺

### Scheme for the preparation of 3-isocyanato-N,N-dimethylaniline.

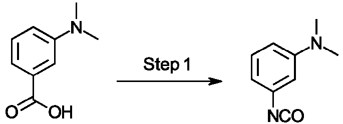

**Step 1:** To a solution of 3-(dimethylamino)benzoic acid (1.00 g, 6.05 mmol, 1.00 eq) in toluene (50.0 mL) was added triethylamine (0.89 mL, 6.42 mmol, 1.06 eq) and diphenylphosphoryl azide (1.34 mL, 6.17 mmol, 1.02 eq). The reaction was stirred at 20°C for 0.5 h, then at 120°C for 2 h. The mixture was concentrated under reduced pressure to give a residue. Dichloromethane (4.00 mL) was added to the residue to afford 4-isocyanato-*N*,*N-*dimethylaniline (982 mg, crude) as red oil.

**Compound 90:** General procedure A with variant iv) was used for the preparation with a yield of 35% from compound **VI** employing 1-isocyanato-3-(trifluoromethoxy)benzene.

¹H NMR (400 MHz, DMSO-*d*₆) *□* = 10.98 (br s, 1H), 9.02 (s, 1H), 7.67 (s, 2H), 7.57 (d, *J* = 0.7 Hz, 2H), 7.37 - 7.30 (m, 1H), 7.29 - 7.22 (m, 1H), 6.91 (br t, *J* = 5.9 Hz, 1H), 6.88 - 6.83 (m, 1H), 5.12 (dd, *J* = 5.1, 13.3 Hz, 1H), 4.49 - 4.36 (m, 3H), 4.36 - 4.26 (m, 1H), 2.98 - 2.85 (m, 1H), 2.60 (td, *J =* 2.0, 15.4 Hz, 1H), 2.40 (br dd, *J =* 4.5, 13.0 Hz, 1H), 2.05 - 1.96 (m, 1H). MS (ESI) m/z 477.2 [M+H]⁺

**Compound 91:** General procedure A with variant iv) was used for the preparation with a yield of 11% from compound **VI** employing 2-isocyanatothiophene.

¹H NMR (400 MHz, DMSO-*d*₆) *□* = 10.96 (br s, 1H), 9.64 (s, 1H), 7.65 (s, 1H), 7.56 (s, 2H), 6.86 (br t, *J* = 6.0 Hz, 1H), 6.79 - 6.72 (m, 2H), 6.44 (dd, *J* = 1.6, 3.2 Hz, 1H), 5.11 (dd, *J* = 5.2, 13.2 Hz, 1H), 4.49 - 4.24 (m, 4H), 2.97 - 2.84 (m, 1H), 2.64 - 2.55 (m, 1H), 2.44 - 2.36 (m, 1H), 2.04 - 1.95 (m, 1H). MS (ESI) m/z 399.0 [M+H]⁺

### Scheme for the preparation of 2-isocyanatothiophene:

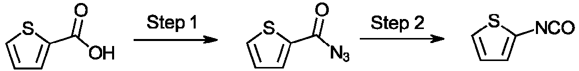

**Step 1:** To a solution of thiophene-2-carboxylic acid (300 mg, 2.34 mmol, 1.00 eq), triethylamine (0.33 mL, 2.34 mmol, 1.00 eq) in toluene (1.50 mL) was added diphenyl phosphorazidate (0.51 mL, 2.34 mmol, 1.00 eq) at 25°C. The reaction was stirred at 25°C for 30 min. Water (1.00 mL) was added, and the mixture was extracted with ethyl acetate (3 x 5.00 ml). The organic layers were combined, washed with saturated sodium bicarbonate (1.00 ml), dried over magnesium sulfate, filtered, and concentrated under reduced pressure to give thiophene-2-carbonyl azide (350 mg, crude) as an off-white solid.

**Step 2:** Thiophene-2-carbonyl azide (400 mg, 2.61 mmol, 1.00 eq) in toluene (4.00 mL) was stirred at 120°C for 30 min to afford 2-isocyanatothiophene. It was used directly in the next step.

**Compound 92:** General procedure A with variant iii) was used for the preparation with a yield of 12% from compound **VI** employing phenyl thiazol-2-ylcarbamate.

¹H NMR (400 MHz, DMSO-*d*₆) *□* = 1 1.0 (br s, 1 H), 10.8 - 10.5 (m, 1H), 8.38 (s, 1H), 7.65 (s, 1H), 7.57 (d, *J* = 0.6 Hz, 2H), 7.31 (d, *J* = 3.6 Hz, 1 H), 7.27 (br s, 1H), 7.02 (d, *J* = 3.6 Hz, 1 H), 5.11 (dd, *J* = 5.1, 13.3 Hz, 1H), 4.51 - 4.37 (m, 3H), 4.34 - 4.25 (m, 1H), 3.02 - 2.84 (m, 1H), 2.69 - 2.55 (m, 1H), 2.46 - 2.28 (m, 1H), 2.06 - 1.91 (m, 1H). MS (ESI) m/z 400.2 [M+H]⁺

### Scheme for the preparation of phenyl thiazol-2-ylcarbamate.

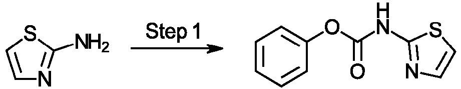

**Step 1:** To a solution of thiazol-2-amine (1.00 g, 9.99 mmol, 1.00 eq) in dichloromethane (5.00 mL) was added pyridine (4.84 mL, 60.0 mmol, 6.00 eq) and phenyl chloroformate (1.50 mL, 12.0 mmol, 1.20 eq). The reaction was stirred at 20°C for 12 h. The mixture was concentrated under reduced pressure to give a residue. The residue was purified by silica gel column chromatography (petroleum ether/ethyl acetate = 1/0 to 1/1) to give phenyl thiazol-2-ylcarbamate (1.00 g, 4.54 mmol, 45% yield) as a white solid.

**Compound 93:** General procedure A with variant iv) was used for the preparation with a yield of 34% from compound VI employing 2-chloro-4-isocyanato-1 ,3-dimethylbenzene.

¹H NMR (400 MHz, DMSO-*d*₆) □ = 11.0 (s, 1 H), 7.97 (s, 1 H), 7.67 (s, 1 H), 7.57 (d, *J* = 0.7 Hz, 2H), 7.54 (d, *J* = 8.3 Hz, 1H), 7.09 (d, *J* = 8.3 Hz, 1H), 7.03 (br t, *J* = 5.9 Hz, 1H), 5.12 (dd, *J* = 5.0, 1 3.3 Hz, 1H), 4.50 - 4.36 (m, 3H), 4.35 - 4.26 (m, 1 H), 2.98 - 2.83 (m, 1H), 2.60 (br d, *J=* 18.0 Hz, 1H), 2.40 (br dd, *J* = 4.4*,* 13.1 Hz, 1H), 2.27 (s, 3H), 2.24 (s, 3H), 2.05 - 1.95 (m, 1H). MS (ESI) m/z 455.2 [M+H]⁺

### Scheme for the preparation of 2-chloro-4-isocyanato-1,3-dimethylbenzene.

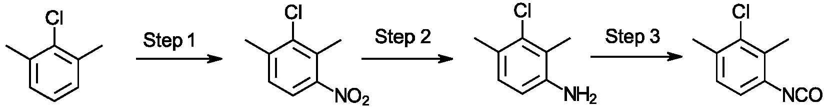

**Step 1:** Nitric acid (1.50 mL, 33.4 mmol, 1.00 eq) was added dropwise to a solution of 2-chloro-1,3-dimethylbenzene (4.43 mL, 33.4 mmol, 1.00 eq) in sulfuric acid (20.0 mL) at 0°C. The reaction was stirred at 20°C for 2 h. The mixture was poured into ice water (20.0 mL) and extracted with ethyl acetate (2 × 20.0 mL). The combined organic layers were washed with brine (30.0 mL), dried over anhydrous sodium sulfate, filtered, and the filtrate was concentrated under reduced pressure to give a residue. The residue was purified by silica gel column chromatography (petroleum ether/ethyl acetate = 1/0) to afford 2-chloro-1,3-dimethyl-4-nitrobenzene (2.10 g, 11.3 mmol, 34.0% yield) as a white solid.

**Step 2:** To a solution of 2-chloro-1,3-dimethyl-4-nitrobenzene (2.10 g, 11.3 mmol, 1.00 eq) in ethanol (24.0 mL) and water (8.00 mL) was added ammonium chloride (6.05 g, 113 mmol, 10.0 eq) and Fe (3.79 g, 67.9 mmol, 6.00 eq). The reaction was stirred at 80°C for 2 h. The mixture was filtered, and the filtrate was concentrated under reduced pressure to give a residue. The residue was purified by silica gel column chromatography (petroleum ether/ethyl acetate = 1/0 to 3/1 ) to afford 3-chloro-2,4-dimethylaniline (1.25 g, 8.03 mmol, 71.0% yield) as light-yellow oil.

**Step 3:** To a solution of 3-chloro-2,4-dimethylaniline (300 mg, 1.93 mmol, 1.00 eq) in toluene (12.0 mL) was added bis(trichloromethyl) carbonate (572 mg, 1.93 mmol, 1.00 eq). The reaction was stirred at 100°C for 3 h. The mixture was concentrated under reduced pressure to give a residue. Dichloromethane (1.00 mL) was added to the residue to afford 2-chloro-4-isocyanato-1,3-dimethylbenzene (350 mg, crude) as a yellow oil.

**Compound 94:** General procedure A with variant iii) was used for the preparation with a yield of 16% from compound VI employing phenyl isoxazol-3-ylcarbamate.

¹H NMR (400 MHz, DMSO-*d*₆) *□* = 11.0 (s, 1 H), 9.63 (s, 1H), 8.67 (d, *J =* 1.6 Hz, 1H), 7.65 (s, 1H), 7.59-7.51 (m, 2H), 7.12 (t, *J* = 6.0 Hz, 1H), 6.72 (d, *J* = 1.6 Hz, 1H), 5.12 (dd, *J =* 4.2, 13.2 Hz, 1H), 4.51-4.24 (m, 4H), 3.00-2.80 (m, 1H), 2.71 -2.58 (m, 1H), 2.39-2.25 (m, 1H), 2.06-1.91 (m, 1H). LCMS m/z = 384.0 [M+H]⁺

### Scheme for the preparation of phenyl isoxazol-3-ylcarbamate.

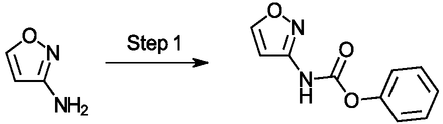

**Step** 1: To a solution of isoxazol-3-amine (500 mg, 5.95 mmol, 1.00 eq) in tetrahydrofuran (20.0 mL) were added phenyl chloroformate (1.02 g, 6.54 mmol, 1.10 eq) and triethylamine (1.20 g, 11.9 mmol, 2.00 eq) dropwise at 20°C. The reaction was stirred at 25°C for 12 h. The mixture was concentrated under reduced pressure to give a residue. The residue was purified by reversed phase column chromatography to afford phenyl isoxazol-3-ylcarbamate (130 mg, 598 µmol, 10% yield) as a yellow solid.

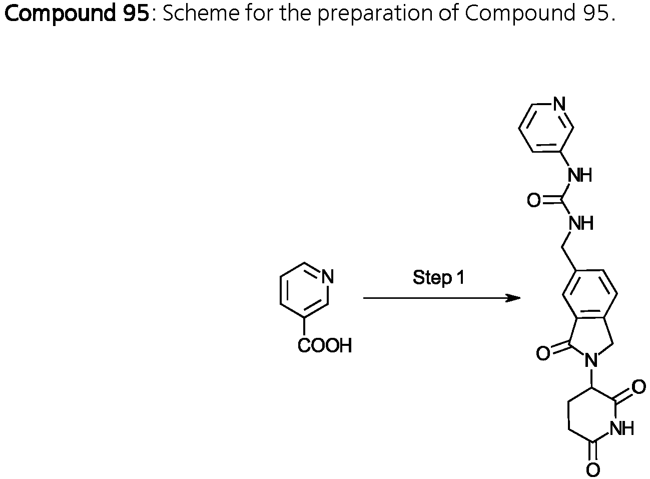

**Step 1:** To a mixture of nicotinic acid (500 mg, 4.06 mmol, 1.00 eq) and triethylamine (534 mg, 5.28 mmol, 1.30 eq) in toluene (5 mL) was added diphenylphosphoryl azide (1.68 g, 6.09 mmol, 1.50 eq) dropwise at 20°C. The reaction was stirred at 20°C for 1h. Triethylamine (1.64 g, 16.3 mmol, 4.00 eq) and 3-(6-(aminomethyl)-1-oxoisoindolin-2-yl)piperidine-2,6-dione hydrochloride **VI** (555 mg, 1.79 mmol, 0.44 eq, hydrochloride) were added in one portion. The reaction was stirred at 100°C for 2 h. The mixture was diluted with ethyl acetate (50 mL) and poured into saturated aqueous sodium bicarbonate (50 mL). The organic layer was separated, and the aqueous layer was extracted with ethyl acetate (10 × 50 mL). The combined organic layers were dried over anhydrous sodium sulfate, filtered, and the filtrate was concentrated under reduced pressure to give a residue. The residue was purified by silica gel column chromatography (dichloromethane/methanol = 100/0 to 85/15), preparative TLC (dichloromethane/methanol = 10/1), and reversed phase preparative HPLC to afford **Compound 95** (19.3 mg, 48.9 µmol, 3% yield) as a white solid.

¹H NMR (400 MHz, DMSO-*d*₆) *□* = 11.0 (s, 1H), 8.94 (s, 1H), 8.56 (d, *J =* 2.4 Hz, 1H), 8.15-8.08 (m, 1H), 7.94-7.86 (m, 1H), 7.68 (s, 1H), 7.57 (s, 2H), 7.29-7.20 (m, 1H), 7.02 (m, 1H), 5.17-5.06 (m, 1H), 4.48-4.28 (m, 4H), 2.94-2.86 (m, 1H), 2.65-2.58 (m, 1H), 2.46-2.37 (m, 1H), 2.05-1.96 (m, 1H). LCMS m/z 394.1 [M+H]⁺

**Compound 96:** General procedure A with variant iv) was used for the preparation with a yield of 56% from compound **VI** employing 1,4-dichloro-2-isocyanato-3,5-dimethylbenzene.

¹H NMR (400 MHz, DMSO-*d*₆) □ =11.0 (br s, 1H), 8.06 (s, 1H), 7.69 (s, 1H), 7.55 (s, 2H), 7.40 (s, 1H), 6.94 (br s, 1H), 5.11 (dd, *J* = 5.1, 13.3 Hz, 1H), 4.49 - 4.27 (m, 4H), 2.97 - 2.83 (m, 1H), 2.60 (td, *J* = 2.0, 15.3 Hz, 1H), 2.40 (br dd, *J* = 4.5, 13.0 Hz, 1H), 2.32 (s, 3H), 2.28 - 2.21 (m, 3H), 2.04 - 1.96 (m, 1H). MS (ESI) m/z 489.2 [M+H]⁺

### Scheme for the preparation of 1,4-dichloro-2-isocyanato-3,5-dimethylbenzene.

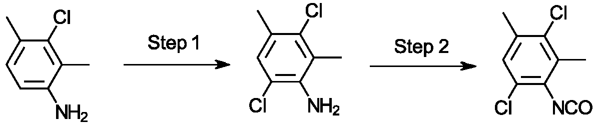

**Step** 1: To a solution of 3-chloro-2,4-dimethylaniline (780 mg, 5.01 mmol, 1.00 eq) in dimethylformamide (10.5 mL) was added a solution of 1-chloropyrrolidine-2,5-dione (709 mg, 5.31 mmol, 1.06 eq) in dimethylformamide (7.00 mL). The reaction was stirred at 20°C for 12 h, then it was concentrated under reduced pressure to give a residue. The residue was purified by silica gel column chromatography (petroleum ether/ethyl acetate = 10/1) to give 3,6-dichloro-2,4-dimethylaniline (290 mg, 1.53 mmol, 30% yield) as red oil.

**Step** 2: To a solution of 3,6-dichloro-2,4-dimethylaniline (290 mg, 1.53 mmol, 1.00 eq) in toluene (12.0 mL) was added bis(trichloromethyl) carbonate (453 mg, 1.53 mmol, 1.00 eq). The reaction was stirred at 100°C for 3 h. The mixture was concentrated under reduced pressure to give a residue. Dichloromethane (1.00 mL) was added to the residue to afford 1,4-dichloro-2-isocyanato-3,5-dimethylbenzene (329 mg, crude) as a red oil.

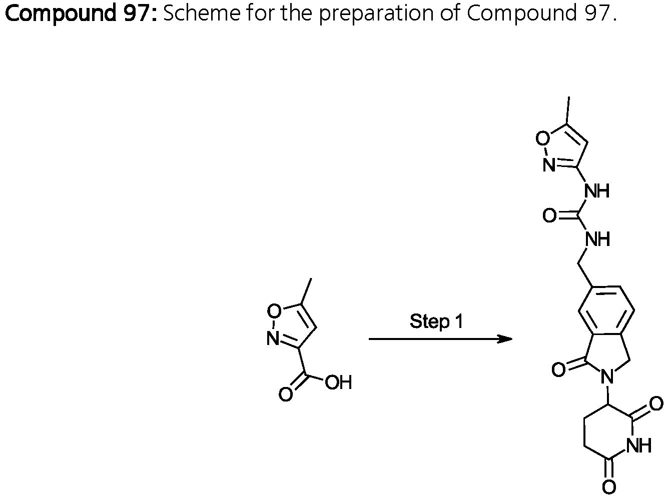

**Step** 1: To a mixture of 5-methylisoxazole-3-carboxylic acid (700 mg, 5.51 mmol, 1.00 eq) and triethylamine (725 mg, 7.16 mmol, 1.30 eq) in toluene (5 mL) was added diphenylphosphoryl azide (2.58 g, 9.36 mmol, 1.70 eq) dropwise at 20°C. The mixture was stirred at 20°C for 1 h. Triethylamine (836 mg, 8.26 mmol, 1.5 eq) and 3-(6-(aminomethyl)-1-oxo-isoindolin-2-yl)piperidine-2,6-dione hydrochloride **VI** (500 mg, 1.61 mmol, 0.293 eq, hydrochloride) were added, and the reaction was stirred at 100°C for 2 h. The mixture was cooled to 20°C and diluted with ethyl acetate (50.0 mL). The mixture was poured into saturated aqueous sodium bicarbonate (100 mL), the organic layer was separated, and the aqueous layer was extracted with ethyl acetate (5 × 50 mL). The combined organic layers were dried over anhydrous sodium sulfate, filtered, and the filtrate was concentrated under reduced pressure to give a residue. The residue was purified by silica gel column chromatography (dichloromethane/methanol = 100/0 to 90/10), preparative TLC (dichloromethane/methanol = 10/1), and reversed phase preparative HPLC to afford **Compound 97** (51.5 mg, 129 µmol, 2% yield) as a white solid.

¹H NMR (400 MHz, DMSO-*d*₆) *□* = 10.9 (br s, 1H), 9.57-9.43 (m, 1H), 7.64 (s, 1H), 7.56 (s, 2H), 7.13 (br s, 1H), 6.40 (s, 1H), 5.19-5.04 (m, 1H), 4.49-4.37 (m, 3H), 4.35-4.24 (m, 1H), 2.98-2.83 (m, 1H), 2.64-2.55 (m, 1H), 2.43-2.36 (m, 1H), 2.32 (s, 3H), 2.05-1.93 (m, 1H). LCMS m/z 398.1 [M+H]⁺

**Compound 98:** General procedure A with variant iii) was used for the preparation with a yield of 20% from compound **VI** employing phenyl 1*H*-pyrrol-3-ylcarbamate.

¹H NMR (400 MHz, DMSO-*d*₆) *□* = 11.0 (br s, 1H), 10.3 (br s, 1H), 7.98 (s, 1H), 7.63 (s, 1H), 7.56 - 7.51 (m, 2H), 6.73 (s, 1H), 6.52 (d, *J =* 2.4 Hz, 1H), 6.43 (t, *J =* 6.0 Hz, 1H), 5.85 (d, *J =* 1.6 Hz, 1H), 5.11 (dd, *J* = 7.6, 13.2 Hz, 1H), 4.49 - 4.23 (m, 4H), 2.97 - 2.84 (m, 1H), 2.64 - 2.55 (m, 1H), 2.47 - 2.35 (m, 1H), 2.03 - 1.95 (m, 1H). MS (ESI) m/z 382.1 [M+H]⁺

### Scheme for the preparation of phenyl 1H-pyrrol-3-ylcarbamate.

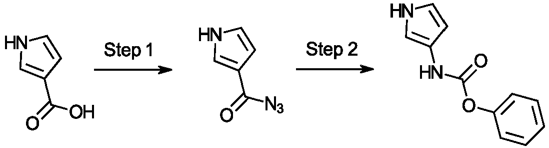

**Step** 1: To a solution of 1*H*-pyrrole-3-carboxylic acid (500 mg, 4.50 mmol, 1.00 eq), triethylamine (0.63 mL, 4.50 mmol, 1.00 eq) in toluene (1.00 mL) was added diphenyl phosphorazidate (0.97 mL, 4.50 mmol, 1.00 eq) at 25°C. The reaction was stirred at 25°C for 1 hr. The mixture was used in the next step directly.

**Step 2:** To a solution of phenol (4.39 mL, 49.9 mmol, 11.3 eq) in toluene (15.0 mL) was added 1*H*-pyrrole-3-carbonyl azide (600 mg, 4.41 mmol, 1.00 eq) at 100°C. The mixture was stirred at 100°C for 1 hr. The mixture was concentrated under reduced pressure to give a residue. The residue was purified by silica gel column chromatography (petroleum ether/ethyl acetate = 0/1 to 4/1) to give phenyl 1*H*-pyrrol-3-ylcarbamate (0.50 g, 2.32 mmol, 53% yield) as a red solid.

**Compound 99:** General procedure A with variant iii) was used for the preparation with a yield of 20% from compound VI employing phenyl thiazol-4-ylcarbamate.

¹H NMR (400 MHz, DMSO-*d*₆) *□* = 11.0 (s, 1 H), 9.46 (s, 1 H), 8.89 (d, *J =* 2.0 Hz, 1 H), 7.66 (s, 1H), 7.57 (s, 2H), 7.21 (d, *J =* 2.4 Hz, 1 H), 6.94 (br t, *J =* 5.6 Hz, 1H), 5.12 (dd, *J =* 5.2, 1 3.2 Hz, 1H), 4.49 - 4.26 (m, 4H), 2.98 - 2.85 (m, 1H), 2.65 - 2.56 (m, 1H), 2.45 - 2.37 (m, 1H), 2.06 - 1.94 (m, 1H). MS (ESI) m/z 400.2 [M+H]⁺

### Scheme for the preparation of phenyl thiazol-4-ylcarbamate.

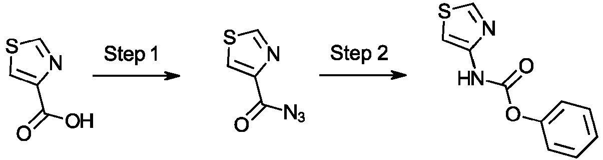

**Step 1:** To a solution of thiazole-4-carboxylic acid (0.50 g, 3.87 mmol, 1.00 eq), triethylamine (0.54 mL, 3.87 mmol, 1.00 eq) in toluene (4.00 mL) was added diphenyl phosphorazidate (0.84 mL, 1.07 g, 3.87 mmol, 1.00 eq) at 25°C. The mixture was stirred at 25°C for 30 min. The mixture was used in the next step directly.

**Step 2:** To a solution of phenol (4.39 mL, 49.9 mmol, 11.3 eq) in toluene (15.0 mL) was added thiazole-4-carbonyl azide (0.60 g, 4.41 mmol, 1.00 eq) at 100°C. The reaction was stirred at 100°C for 1 hr. The mixture was concentrated under reduced pressure to give a residue. The residue was purified by silica gel column chromatography (petroleum ether/ethyl acetate = 0/1 to 5/1 ) to give phenyl thiazol-4-ylcarbamate (200 mg, 0.91 mmol, 23% yield) as a white solid.

**Compound 100:** General procedure A with variant iv) was used for the preparation with a yield of 20% from compound **VI** employing 2,3-dichloro-4-isocyanato-1,5-dimethylbenzene.

¹H NMR (400 MHz, DMSO-*d*₆) *□* = 11.0 (s, 1H), 8.01 (s, 1H), 7.69 (s, 1H), 7.55 (s, 2H), 7.22 (s, 1H), 6.91 (br t, *J =* 5.4 Hz, 1H), 5.11 (dd, *J* = 5.1, 13.3 Hz, 1H), 4.52 - 4.24 (m, 4H), 2.91 (ddd, *J* = 5.4, 13.7, 17.3 Hz, 1H), 2.63 - 2.57 (m, 1H), 2.44 - 2.38 (m, 1H), 2.34 (s, 3H), 2.17 (s, 3H), 2.05 - 1.93 (m, 1H). MS (ESI) m/z 489.2 [M+H]⁺

### Scheme for the preparation of 2,3-dichloro-4-isocyanato-1,5-dimethylbenzene.

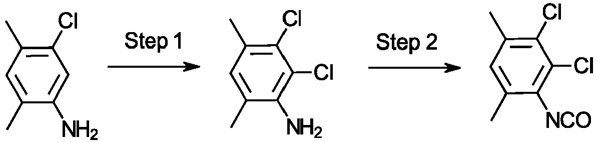

**Step 1:** To a solution of 5-chloro-2,4-dimethylaniline (1.50 g, 9.64 mmol, 1.00 eq) in dimethyformamide (15.00 mL) was added a solution of 1-chloropyrrolidine-2,5-dione(1.36 g, 10.2 mmol, 1.06 eq) in dimethyformamide (6.00 mL). The reaction was stirred at 20°C for 12 h. Water (20.0 mL) was added to the mixture and it was extracted with ethyl acetate (2 × 100 mL). The organic layer was washed with brine (2 × 50.0 mL), dried over with anhydrous sodium sulfate, filtered, and concentrated under reduced pressure. The residue was purified by silica gel column chromatography (petroleum ether/ethyl acetate = 1/0 to 10/1) to give 2,3-dichloro-4,6-dimethylaniline (1.00 g, 5.26 mmol, 55% yield) as a yellow oil.

**Step 2:** To a solution of 2,3-dichloro-4,6-dimethylaniline (500 mg, 2.63 mmol, 1.00 eq) in toluene (10.0 mL) was added triphosgene (781 mg, 2.63 mmol, 1.00 eq). The reaction was stirred at 100°C for 2 h. The mixture was concentrated under reduced pressure to give 2,3-dichloro-4-isocyanato-1,5-dimethylbenzene (560 mg, 2.59 mmol, 99% yield) as a yellow oil.

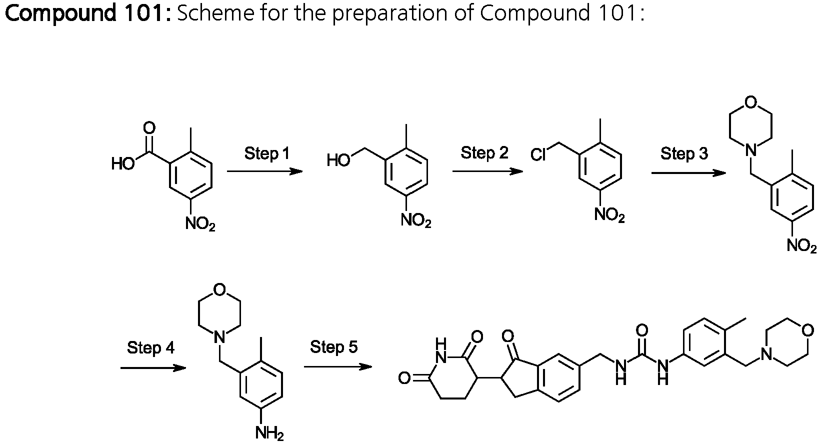

**Step 1:** To a mixture of 2-methyl-5-nitrobenzoic acid (5.00 g, 27.6 mmol, 1.00 eq) in tetrahydrofuran (138 mL) was added borane dimethyl sulfide complex (10.0 M, 5.52 mL, 2.00 eq) dropwise at 20°C. The reaction was stirred at 75°C under nitrogen atmosphere for 4 h. The mixture was cooled to 5°C and methanol/water (25.0 mL, v/v = 1:1) was added, followed by 5N hydrochloric acid (50.0 mL). The mixture was concentrated under reduced pressure to give a slurry, which was poured into water (50.0 ml) and extracted with ethyl acetate (4 × 50.0 mL). The combined organic layers were washed with brine (50.0 mL), dried over sodium sulfate, filtered, and the filtrate was concentrated under reduced pressure to give a residue. The residue was purified by silica gel column chromatography (petroleum ether/ethyl acetate = 100/1 to 80/20) to afford (2-methyl-5-nitrophenyl)methanol (4.75 g, 27.6 mmol, 100% yield) as a white solid.

**Step 2:** To a mixture of (2-methyl-5-nitrophenyl)methanol (1.00 g, 5.98 mmol, 1.00 eq) in dichloromethane (10.0 mL) were added thionyl chloride (4.34 mL, 59.8 mmol, 10.0 eq) and N-methyl pyrrolidone (0.58 mL, 5.98 mmol, 1.00 eq) dropwise at 20°C. The reaction was stirred at 25°C for 4 h. The reaction was carefully quenched with water (50.0 ml) and extracted with ethyl acetate (4 × 20.0 mL). The combined organic layers were washed with brine (20.0 mL), dried over sodium sulfate, filtered, and the filtrate was concentrated under reduced pressure to give a residue. The residue was purified by silica gel column chromatography (petroleum ether/ethyl acetate = 70/30 to 60/40) to give 2-(chloromethyl)-1-methyl-4-nitrobenzene (1.10 g, 5.93 mmol, 99% yield) as a white solid.

**Step 3:** To a mixture of 2-(chloromethyl)-1-methyl-4-nitrobenzene (500 mg, 2.69 mmol, 1.00 eq) in dimethylformamide (1.50 mL) and acetonitrile (1.50 mL) were added N,N-diisopropylethylamine (1.04 g, 8.08 mmol, 3.00 eq) and morpholine (258 mg, 2.96 mmol, 1.10 eq) dropwise at 20°C. The reaction was stirred at 60°C for 10 h. The mixture was cooled to 20°C and concentrated under reduced pressure to give a slurry, which was poured into water (50.0 mL) and the mixture was extracted with ethyl acetate (4 × 50.0 mL). The combined organic layers were washed with brine (50.0 mL), dried over sodium sulfate, filtered, and concentrated under reduced pressure to give a residue. The residue was purified by silica gel column chromatography (petroleum ether/ethyl acetate = 10/90 to 0/100) to afford 4-(2-methyl-5-nitrobenzyl)morpholine (584 mg, 2.47 mmol, 91% yield) as a light yellow solid.

**Step 4:** To a mixture of 4-(2-methyl-5-nitrobenzyl)morpholine (584 mg, 2.47 mmol, 1.00 eq) in ethanol (5.00 mL) and water (5.00 mL) were added ammonium chloride (132 mg, 2.47 mmol, 1.00 eq) and ferrous powder (690 mg, 12.4 mmol, 5.00 eq). The reaction was stirred at 90°C for 10 h. The mixture was cooled to 20°C and filtered. The filter cake was washed with methanol (2 × 20.0 mL). The filtrate was concentrated under reduced pressure to give a slurry. The slurry was poured into saturated aqueous sodium bicarbonate (50.0 mL) and extracted with ethyl acetate (4 × 50.0 mL). The combined organic layers were washed with brine (50.0 mL), dried over sodium sulfate, filtered, and concentrated under reduced pressure to give a residue. The residue was purified by silica gel column chromatography (petroleum ether/ethyl acetate = 10/90 to 0/100) to afford 4-methyl-3-(morpholinomethyl)aniline (482 mg, 2.34 mmol, 95% yield) as a light yellow solid.

**Step 5:** To a mixture of 4-methyl-3-(morpholinomethyl)aniline (200 mg, 0.969 mmol, 1.50 eq) and triethylamine (98.0 mg, 0.969 mmol, 1.50 eq) in tetrahydrofuran (10.0 mL) was added 1,1'-carbonyldiimidazole (157 mg, 0.969 mmol, 1.50 eq). The reaction was stirred at 20°C for 1 h. A mixture of 3-(6-(aminomethyl)-1-oxoisoindolin-2-yl)piperidine-2,6-dione hydrochloride **IV** (200 mg, 0.646 mmol, 1.00 eq, hydrochloride) and triethylamine (196 mg, 1.94 mmol, 3.00 eq) in tetrahydrofuran (2.00 mL) was added. The reaction was stirred at 25°C for 14 h. The mixture was quenched with water (50.0 ml) and extracted with ethyl acetate (4 × 50.0 mL). The combined organic layers were washed with brine (20.0 ml), dried over sodium sulfate, filtered, and concentrated under reduced pressure to give a residue. The residue was purified by reversed phase preparative HPLC to afford 1-((2-(2,6-dioxopiperidin-3-yl)-3-oxoisoindolin-5-yl)methyl)-3-(4-methyl-3-(morpholinomethyl)phenyl)urea (68.4 mg, 0.135 mmol, 20% yield) as a white solid.

¹H NMR (400 MHz, DMSO-*d*₆) *□* = 10.98 (br s, 1H), 8.56 (s, 1H), 7.66 (s, 1H), 7.56 (d, *J* = 0.8 Hz, 2H), 7.31 - 7.20 (m, 2H), 6.99 (d, *J =* 8.0 Hz, 1H), 6.71 (t, *J* = 6.0 Hz, 1H), 5.11 (dd, *J* = 4.8, 13.2 Hz, 1H), 4.38 (d, *J* = 5.6 Hz, 2H), 4.37 (dd, *J =* 17.2, 51.6 Hz, 2H), 3.55 (t, *J =* 4.4 Hz, 4H), 3.34 (s, 2H), 2.91 (dddd, *J* = 5.6, 9.6, 17.6, 31.2 Hz, 1H), 2.64-2.56 (m, 1H), 2.45 - 2.38 (m, 1H), 2.37-2.30 (m, 4H), 2.22 (s, 3H), 2.04-1.95 (m, 1H). LCMS m/z 506.5 [M+H]⁺

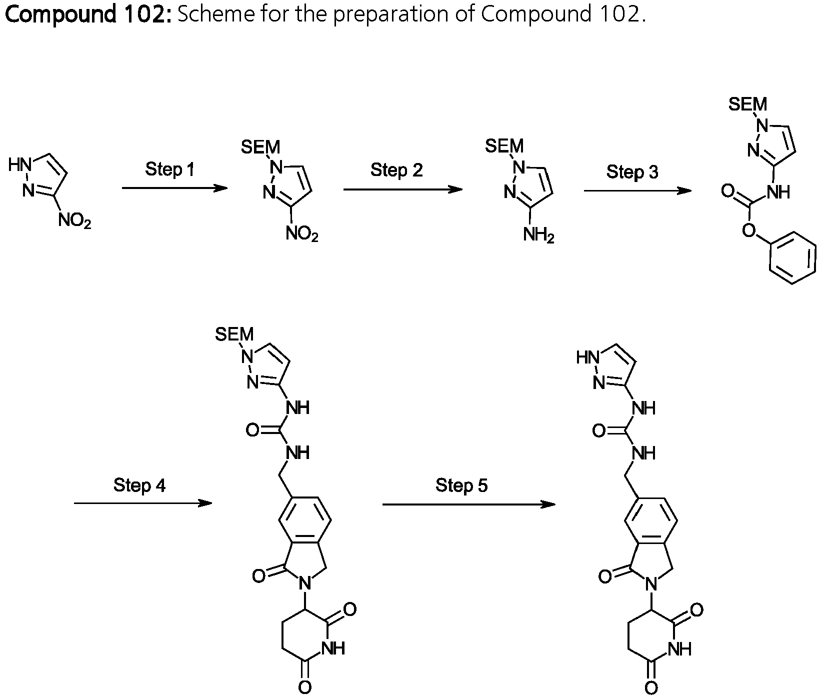

**Step 1**: To a solution of 3-nitro-1 H-pyrazole (5.00 g, 44.2 mmol, 1.00 eq) in tetrahydrofuran (100 mL) was added sodium hydride 60% purity (2.13 g, 53.3 mmol, 1.20 eq) at 0°C. The reaction was stirred for 10 min, and 2-(trimethylsilyl)ethoxymethyl chloride (8.60 mL, 48.6 mmol, 1.10 eq) was added dropwise. The reaction was stirred at 20°C for 1 h. The mixture was quenched with ice water (150 mL) and extracted with ethyl acetate (2 × 200 mL). The combined organic layers were washed with brine (200 mL), dried over sodium sulfate, filtered, and concentrated under reduced pressure to give a residue. The residue was purified by silica gel column chromatography (petroleum ether/ethyl acetate = 10/1 to 1/1) to afford trimethyl-[2-[(3-nitropyrazol-1-yl)methoxy]ethyl]silane (6.20 g, 25.4 mmol, 57% yield) as a transparent oil.

**Step 2:** To a solution of trimethyl-[2-[(3-nitropyrazol-1-yl)methoxy]ethyl]silane (3.00 g, 12.3 mmol, 1.00 eq) in methanol (50.0 mL) was added Pd/C 10% weight on C (0.50 g). The reaction was stirred at 30°C for 4 h under hydrogen atmosphere (15 psi). The mixture was filtered, and the filtrate was concentrated under reduced pressure to afford 1-(2-trimethylsilylethoxymethyl)pyrazol-3-amine (2.40 g, 11.2 mmol, 91% yield) as a white solid.

**Step 3:** To a solution of 1-(2-trimethylsilylethoxymethyl)pyrazol-3-amine (1.00 g, 4.69 mmol, 1.00 eq) and pyridine (0.76 mL, 9.37 mmol, 2.00 eq) in acetonitrile (8.00 mL) was added phenyl chloroformate (0.70 mL, 5.62 mmol, 1.20 eq) in acetonitrile (2.00 mL) at 0°C. The reaction was stirred at 25°C for 3 h. The mixture was diluted with water (20.0 mL) and extracted with ethyl acetate (2 × 20.0 mL). The combined organic layers were dried over sodium sulfate, filtered, and concentrated under reduced pressure to give a residue. The residue was purified by silica gel column chromatography (petroleum ether/ethyl acetate = 6/1) and concentrated under reduced pressure to afford phenyl (1-((2-(trimethylsilyl)ethoxy)methyl)-1H-pyrazol-3-yl)carbamate (1.40 g, 4.20 mmol, 89% yield) as a yellow solid.

**Step 4:** To a solution of 3-[6-(aminomethyl)-1-oxo-isoindolin-2-yl]piperidine-2,6-dione hydrochloride **VI** (200 mg, 645 µmol, 1.00 eq, hydrochloride) and triethylamine (196 mg, 1.94 mmol, 267 µL, 3.00 eq) in dimethylformamide (3.00 mL) was added phenyl (1-((2-(trimethylsilyl)ethoxy)methyl)-1H-pyrazol-3-yl)carbamate (258 mg, 775 µmol, 1.20 eq). The reaction was stirred at 25°C for 12 h. The mixture was diluted with water (20.0 mL) and extracted with dichloromethane/isopropyl alcohol = 3/1 (3 × 15.0 mL). The combined organic layers were washed with brine (2 × 20.0 mL), dried over sodium sulfate, filtered, and concentrated under reduced pressure to afford 1-((2-(2,6-dioxopiperidin-3-yl)-3-oxoisoindolin-5-yl)methyl)-3-(1-((2-(trimethylsilyl)ethoxy)methyl)-1H-pyrazol-3-yl)urea (360 mg, crude) as a yellow solid. It was used directly in the next reaction.

**Step 5:** A mixture of 1-((2-(2,6-dioxopiperidin-3-yl)-3-oxoisoindolin-5-yl)methyl)-3-(1-((2-(trimethylsilyl)ethoxy) methyl)-1H-pyrazol-3-yl)urea (360 mg, 702 µmol, 1.00 eq) in trifluoroacetic acid (1.00 mL) and dichloromethane (1.00 mL) was stirred at 20°C for 12 h. The mixture was concentrated under reduced pressure to give a residue. The residue was purified by reversed phase preparative HPLC and lyophilized to afford **Compound 102** (88 mg, 205 µmol, 29% yield) as a white solid.

¹H NMR (400 MHz, DMSO-*d*₆) *□* = 12.12 (br s, 1H), 10.99 (br s, 1H), 8.91 (s, 1H), 8.30 (br s, 1H), 7.64 (s, 1H), 7.56 (s, 2H), 7.53 (d, *J =* 2.0 Hz, 1H), 7.44 (br s, 1H), 6.06 (s, 1H), 5.11 (dd, *J* = 5.2, 13.2 Hz, 1H), 4.46 - 4.40 (m, 3H), 4.33 - 4.27 (m, 1H), 2.97 - 2.85 (m, 1H), 2.64 - 2.55 (m, 1H), 2.45 - 2.32 (m, 1H), 2.05 - 1.94 (m, 1H). MS (ESI) m/z 383.3 [M+H]⁺

**Compound 103:** General procedure A with variant iii) was used for the preparation with a yield of 61 % from compound **VI** employing phenyl *N*-thiazol-5-ylcarbamate.

¹H NMR (400 MHz, DMSO-*d*₆) *□* = 1.96 - 2.03 (m, 1 H) 2.39 (br dd, *J =* 13.39, 4.71 Hz, 1 H) 2.55 - 2.64 (m, 1 H) 2.83 - 2.97 (m, 1 H) 4.26 - 4.48 (m, 4 H) 5.11 (dd, *J =* 13.2, 5.2 Hz, 1 H) 7.09 - 7.16 (m, 1 H) 7.39 (d, *J =* 0.8 Hz, 1 H) 7.56 (s, 2 H) 7.65 (s, 1 H) 8.40 (s, 1 H) 9.92 (br s, 1 H) 10.91 - 11.05 (m, 1 H). LCMS m/z 400.0 [M+H]⁺

### Scheme for the preparation of phenyl N-thiazol-5-ylcarbamate.

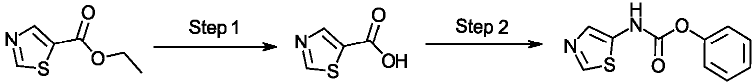

**Step** 1: To a vigorously stirred suspension of ethyl thiazole-5-carboxylate (1.84 g, 11.7 mmol, 1.00 eq) in methanol (11.0 mL) was added sodium hydroxide (1.40 g, 35.1 mmol, 3.00 eq). The reaction was stirred at 20°C for 12 h. The mixture was concentrated under reduced pressure, and the remaining aqueous suspension was acidified with 6 M aqueous solution of hydrogen chloride to pH = 1-2: The solid was filtered to afford thiazole-5-carboxylic acid (1.31 g, 10.2 mmol, 86% yield) as a white solid.

**Step 2:** To a solution of thiazole-5-carboxylic acid (1.31 g, 10.2 mmol, 1.00 eq) in 1,4-dioxane (33.0 mL) was added triethylamine (1.61 mL, 11.59 mmol, 1.14 eq) and diphenylphosphoryl azide (2.51 mL, 11.6 mmol, 1.14 eq). The reaction was stirred at 20°C for 3 h. Phenol (10.2 mL, 116.21 mmol, 11.43 eq) was added dropwise, and the reaction was stirred at 100°C for 3 h. After cooling to 20°C, the mixture was concentrated under reduced pressure to give a residue. The residue was purified by silica gel column chromatography (petroleum ether/ethyl acetate = 1/0 to 1/1) to afford phenyl N-thiazol-5-ylcarbamate (813 mg, 3.69 mmol, 36% yield) as a brown solid.

**Compound 104:** General procedure A with variant iii) was used for the preparation with a yield of 61% from compound **VI** employing phenyl pyridin-4-ylcarbamate.

¹H NMR (400 MHz, DMSO-*d*₆) *□* = 10.98 (s, 1H), 9.14 (s, 1H), 8.29 (d, *J =* 6.4 Hz, 2H), 8.14 (s, 1H), 7.67 (s, 1H), 7.57 (d, *J =* 0.8 Hz, 2H), 7.43-7.37 (m, 2H), 7.02 (t, *J* = 6.0 Hz, 1H), 5.11 (dd, *J* = 4.8, 13.2 Hz, 1H), 4.415 (d, *J* = 5.6 Hz, 2H), 4.37 (dd, *J* = 17.2, 52.4 Hz, 2H), 2.96-2.84 (m, 1H), 2.65-2.56 (m, 1H), 2.43-2.34 (m, 1H), 2.05-1.95 (m, 1H). LCMS m/z 394.1 [M+H]⁺

### Scheme for the preparation of phenyl pyridin-4-ylcarbamate.

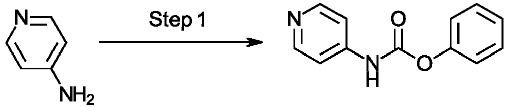

A mixture of pyridin-4-amine (3.57 mL, 21.3 mmol, 1.00 eq), phenyl chloroformate (2.93 mL, 23.4 mmol, 1.10 eq) and triethylamine (5.92 mL, 42.5 mmol, 2.00 eq) in tetrahydrofuran (30.0 mL) was stirred at 20°C for 0.5 h. The mixture was concentrated under reduced pressure to give a residue. The residue was purified by reversed phase column chromatography and lyophilized to afford phenyl pyridin-4-ylcarbamate(1.13 g, 5.26 mmol, 56% yield) as a white solid.

### Compound 105: Scheme for the preparation of Compound 105.

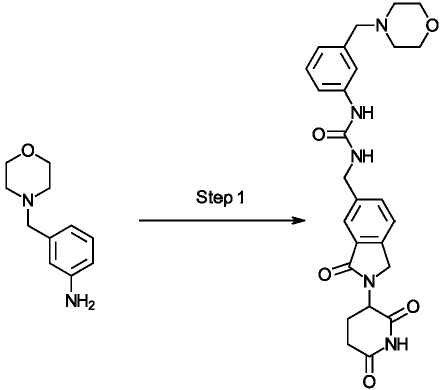

**Step 1:** To a solution of 3-(morpholinomethyl)aniline (279 mg, 1.45 mmol, 1.50 eq) and triethylamine (196 mg, 1.94 mmol, 2.00 eq) in dimethylformamide (5.00 mL) was added di(1H-imidazol-1-yl)methanone (236 mg, 1.45 mmol, 1.50 eq). The reaction was stirred at 20°C for 2 h, then a solution of 3-(6-(aminomethyl)-1-oxo-isoindolin-2-yl)piperidine-2,6-dione hydrochloride **VI** (300 mg, 969 µmol, 1.00 eq, hydrochloride) and triethylamine (294 mg, 2.91 mmol, 3.00 eq) in dimethylformamide (5.00 mL) was added. The reaction was stirred at 20°C for 12 h. The mixture was concentrated under reduced pressure to give a residue. The residue was purified by silica gel column chromatography (ethyl acetate/methanol = 100/0 to 80/20) followed by reversed phase preparative HPLC, then lyophilized to afford **Compound 105** (29.6 mg, 53.5 µmol, 5 % yield) as a white solid.

¹H NMR (400 MHz, DMSO-*d*₆) *□* = 10.98 (s, 1H), 8.69 (s, 1H), 8.19 (s, 1H), 7.66 (s, 1H), 7.56 (d, *J* = 0.8 Hz, 2H), 7.37 (s, 1H), 7.33-7.28 (m, 1H), 7.15 (t, *J=* 7.6 Hz, 1H), 6.83 (d, *J* = 7.6 Hz, 1H), 6.77 (t, *J* = 6.0 Hz, 1H), 5.11 (dd, *J* = 5.2, 13.2 Hz, 1H), 4.39 (d,*J* = 5.6 Hz, 2H), 4.37 (dd, *J* = 16.8, 52.0 Hz, 2H), 3.58-3.54 (m, 4H), 3.38 (s, 2H), 2.91 (ddd, *J* = 5.2, 13.6, 17.2 Hz, 1H), 2.65-2.55 (m, 1H), 2.43-2.36 (m, 1H), 2.36-2.30 (m, 4H), 2.04-1.96 (m, 1H). LCMS m/z 492.2 [M+H]⁺

### Compound 106: Scheme for the synthesis of Compound 106.

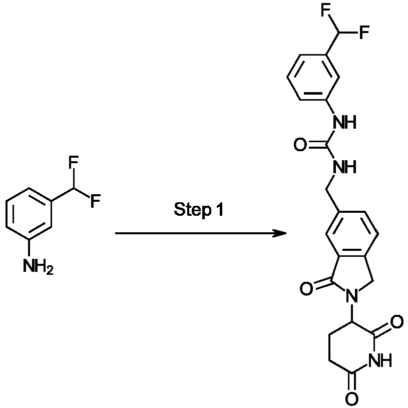

**Step 1:** To a solution of 3-(difluoromethyl)aniline (416 mg, 2.91 mmol, 3.00 eq) and triethylamine (392 mg, 3.87 mmol, 4.00 eq) in dimethylformamide (10.0 mL) was added di(1H-imidazol-1-yl)methanone (518 mg, 3.20 mmol, 3.30 eq) in one portion at 20°C. The reaction was stirred at 20°C for 1 h, then a solution of 3-[6-(aminomethyl)-1-oxo-isoindolin-2-yl]piperidine-2,6-dione hydrochloride **VI** (300 mg, 969 µmol, 1.00 eq, hydrochloride) and triethylamine (196 mg, 1.94 mmol, 2.00 eq) in dimethylformamide (1.00 mL) was added. The reaction was stirred at 20°C for 12 h. The mixture was poured into saturated aqueous sodium bicarbonate (100 mL) and extracted with ethyl acetate (5 × 100 mL). The combined organic layers were dried over anhydrous sodium sulfate, filtered, and the filtrate was concentrated under reduced pressure to give a residue. The residue was purified by reversed phase column chromatography and reversed phase preparative HPLC (twice), then lyophilized to afford **Compound 106** (118 mg, 264 µmol, 27% yield) as a white solid.

¹H NMR (400 MHz, DMSO-*d*₆) *□* = 11.0 (s, 1H), 8.94 (s, 1H), 7.76 (s, 1H), 7.67 (s, 1H), 7.60-7.53 (m, 2H), 7.46 (dd, *J* = 0.8, 8.0 Hz, 1H), 7.35 (t, *J* = 8.0 Hz, 1H), 7.10-7.06 (m, 1H), 6.96 (s, 1H), 6.88 (t, *J =* 6.0 Hz, 1H), 5.11 (dd, *J =* 4.8, 13.2 Hz, 1H), 4.41 (s, 2H), 4.36 (dd, *J* = 17.2, 52.0 Hz, 2H), 2.97-2.85 (m, 1H), 2.64-2.55 (m, 1H), 2.45-2.36 (m, 1H), 2.05-1.95 (m, 1H). LCMS m/z 443.1 [M+H]⁺

**Compound 107:** General procedure A with variant iv) was used for the preparation with a yield of 63% from compound **VI** employing 2-chloro-4-isocyanato-1,3,5-trimethylbenzene.

¹H NMR (400 MHz, DMSO-*d*₆) □ = 11.0 (br s, 1H), 7.79 (s, 1H), 7.66 (s, 1H), 7.58 - 7.45 (m, 2H), 7.05 (s, 1H), 6.86 - 6.60 (m, 1H), 5.10 (dd, *J* = 5.1*,* 13.3 Hz, 1H), 4.50 - 4.20 (m, 4H), 2.99 - 2.82 (m, 1H), 2.65 - 2.56 (m, 1H), 2.39 (dq, *J* = 4.3, 13.2 Hz, 1H), 2.27 (s, 3H), 2.20 (s, 3H), 2.12 (s, 3H), 2.05 - 1.93 (m, 1H). MS (ESI) m/z 469.2 [M+H]⁺

### Scheme for the preparation of 2-chloro-4-isocyanato-1,3,5-trimethylbenzene.

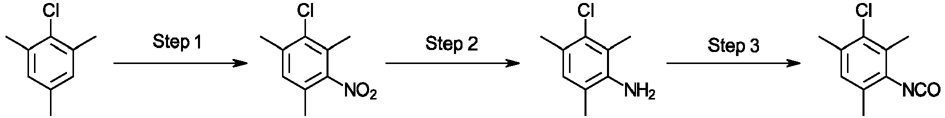

**Step** 1: To a solution of 2-chloro-1,3,5-trimethylbenzene (2.9 g, 18.8 mmol, 1.00 eq) in acetic anhydride (21.0 mL) was added a solution of nitric acid (2.53 mL, 56.3 mmol, 3.00 eq) in acetic anhydride (10.5 mL) at -78°C. The reaction was stirred at -78°C for 2 h. Water (50.0 mL) was added to quench the reaction, and the pH was adjusted to pH = 7 with an aqueous solution of sodium bicarbonate. The mixture was extracted with ethyl acetate (2 × 100 mL). The combined organic layers were washed with brine (2 × 50.0 mL), dried over sodium sulfate, filtered, and concentrated under reduced pressure to give a residue. The residue was purified by silica gel column chromatography (petroleum ether/ethyl acetate = 1/0 to 10/1) to give 2-chloro-1,3,5-trimethyl-4-nitrobenzene (950 mg, 4.76 mmol, 25% yield) as a white solid.

**Step 2:** To a solution of 2-chloro-1,3,5-trimethyl-4-nitrobenzene (950 mg, 4.76 mmol, 1.00 eq) in ethanol (10.0 mL) and water (3.00 mL) was added iron powder (797 mg, 14.3 mmol, 3.00 eq) and ammonium chloride (1.27 g, 23.8 mmol, 5.00 eq). The reaction was stirred at 80°C for 12 h. The mixture was filtered and concentrated under reduced pressure to give a residue. Water (50.0 mL) was added, and the pH was adjusted to pH = 7 with an aqueous solution of sodium bicarbonate. The mixture was extracted with ethyl acetate (2 × 100 mL). The combined organic layers were washed with brine (2 × 50.0 mL), dried over sodium sulfate, filtered, and concentrated under reduced pressure to afford 3-chloro-2,4,6-trimethylaniline (600 mg, 3.54 mmol, 74% yield) as a yellow oil.

**Step 3:** To a solution of 3-chloro-2,4,6-trimethylaniline (300 mg, 1.77 mmol, 1.00 eq) in toluene (10.0 mL) was added triphosgene (525 mg, 1.77 mmol, 1.00 eq). The reaction was stirred at 100°C for 2 h. The mixture was concentrated under reduced pressure to afford 2-chloro-4-isocyanato-1,3,5-trimethylbenzene (345 mg, 1.76 mmol, 99% yield) as a yellow oil.

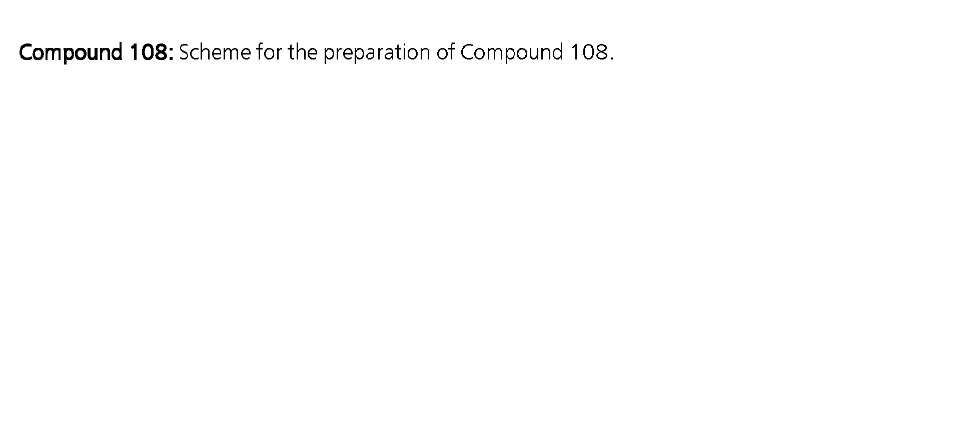

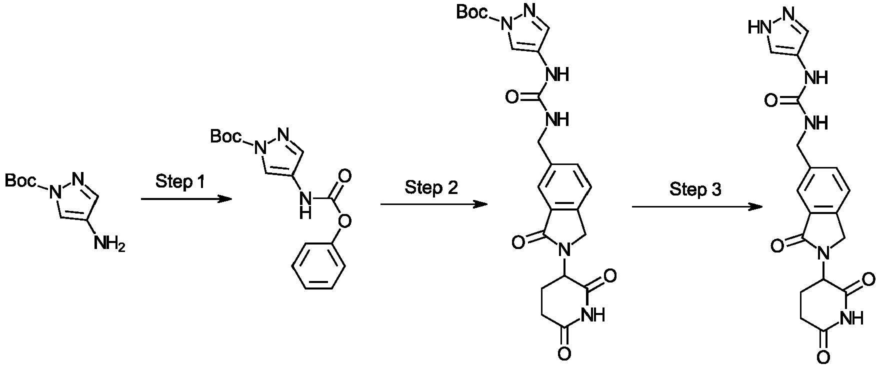

**Step 1:** To a solution of tert-butyl 4-aminopyrazole-1-carboxylate (500 mg, 2.73 mmol, 1.00 eq), pyridine (0.44 mL, 5.46 mmol, 2.00 eq) in acetonitrile (5.00 mL) was added phenyl chloroformate (0.41 mL, 3.27 mmol, 1.20 eq) at 0°C. The reaction was stirred at 30°C for 2 h. The mixture was diluted with water (15.0 mL) and extracted with ethyl acetate (2 × 20.0 mL). The combined organic layers were washed with brine (20 mL), dried over sodium sulfate, filtered, and concentrated under reduced pressure to give a residue. The residue was purified by silica gel column chromatography (petroleum ether/ethyl acetate = 10/1 to 5/1) and concentrated under reduced pressure to afford tert-butyl 4-(phenoxycarbonylamino)pyrazole-1-carboxylate (0.60 g, 1.98 mmol, 72% yield) as a white solid.

**Step 2:** To a mixture of 3-[6-(aminomethyl)-1-oxo-isoindolin-2-yl]piperidine-2,6-dione hydrochloride **VI** (500 mg, 1.61 mmol, 1.00 eq, hydrochloride) and triethylamine (674 µL, 4.84 mmol, 3.00 eq) in dimethylformamide (5.00 mL) was added *tert*-butyl 4-(phenoxycarbonylamino)pyrazole-1-carboxylate (587 mg, 1.94 mmol, 1.20 eq). The reaction was stirred at 30°C for 6 h. The mixture was diluted with water (20.0 mL) and extracted with dichloromethane/isopropyl alcohol = 3/1 (3 × 30.0 mL). The combined organic layers were washed with brine (20.0 mL), dried over sodium sulfate, filtered, and concentrated under reduced pressure to give a residue. The residue was purified by silica gel column chromatography (petroleum ether/ethyl acetate = 3/1 to dichloromethane/methanol = 10/1) and concentrated under reduced pressure to afford *tert*-butyl 4-[[2-(2,6-dioxo-3-piperidyl)-3-oxo-isoindolin-5-yl]methylcarbamoylamino]pyrazole-1-carboxylate (550 mg, 1.14 mmol, 70% yield) as a white solid.

**Step 3:** To a solution of *tert*-butyl 4-[[2-(2,6-dioxo-3-piperidyl)-3-oxo-isoindolin-5-yl] methylcarbamoylamino]pyrazole-1-carboxylate (500 mg, 1.04 mmol, 1.00 eq) in dioxane (5.00 mL) was added hydrochloric acid/dioxane (4 M, 2.5 mL, 9.65 eq). The reaction was stirred at 25°C for 1 h. The mixture was concentrated under reduced pressure to give a residue. The residue was purified by reversed phase preparative HPLC and lyophilized to afford **Compound 108** (192 mg, 478 µmol, 46% yield) as a white solid.

¹H NMR (400 MHz, DMSO-*d₆*) *□* = 12.37 (br s, 1H), 10.99 (br s, 1H), 8.28 (s, 1H), 7.64 (s, 1H), 7.54 (s, 2H), 7.50 (br s, 1H), 6.68 (br t, *J* = 5.6 Hz, 1H), 5.11 (dd, *J* = 5.2, 13.3 Hz, 1H), 4.47 - 4.26 (m, 4H), 2.97 - 2.85 (m, 1H), 2.64 - 2.55 (m, 1H), 2.45 - 2.33 (m, 1H), 2.04 - 1.94 (m, 1H). MS (ESI) m/z 383.0 [M+H]⁺

**Compound 109:** General procedure A with variant iii) was used for the preparation with a yield of 17% from compound **VI** employing phenyl (3-(2-oxa-6-azaspiro[3.3]heptan-6-ylmethyl)-5-chloro-4-methylphenyl)carbamate.

¹H NMR (400 MHz, DMSO-*d*₆) *□* = 11.0 (br s, 1H), 8.91 - 8.62 (m, 1H), 7.66 (s, 1H), 7.62 (d, *J =* 2.1 Hz, 1H), 7.56 (s, 2H), 7.06 (d, *J* = 2.0 Hz, 1H), 6.88 - 6.69 (m, 1H), 5.11 (dd, *J =* 5.0, 13.2 Hz, 1H), 4.60 (s, 4H), 4.48 - 4.27 (m, 4H), 3.44 (s, 2H), 3.30 (s, 4H), 2.97 - 2.84 (m, 1H), 2.59 (td, *J =* 2.0, 15.2 Hz, 1H), 2.41 (br dd, *J* = 8.9, 13.3 Hz, 1H), 2.17 (s, 3H), 2.04 - 1.93 (m, 1H). MS (ESI) m/z 552.2 [M+H]⁺

### Scheme for the preparation of phenyl (3-(2-oxa-6-azaspiro[3.3]heptan-6-ylmethyl)-5-chloro-4-methylphenyl)carbamate.

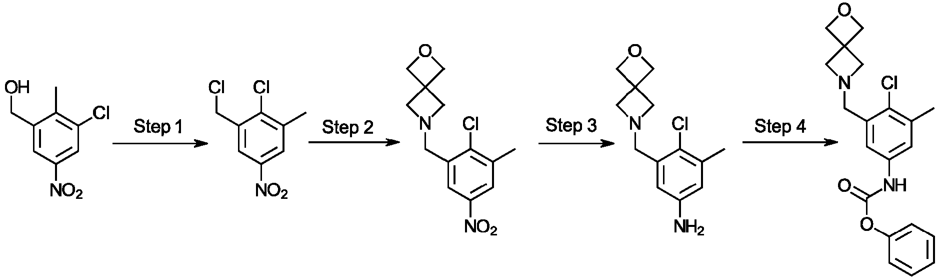

**Step 1:** To a solution of (3-chloro-2-methyl-5-nitrophenyl)methanol (1.50 g, 7.44 mmol, 1.00 eq) in dichloromethane (20.0 mL) was added thionyl chloride (2.70 mL, 37.2 mmol, 5.00 eq) at 0°C. The reaction was stirred at 25°C for 12 h. The mixture was concentrated under reduced pressure to give a residue. The residue was purified by silica gel column chromatography (petroleum ether/ethyl acetate = 1/0 to 10/1) to afford 1-chloro-3-(chloromethyl)-2-methyl-5-nitrobenzene (1.30 g, 5.91 mmol, 79% yield) as a yellow oil.

**Step 2:** To a solution of 1-chloro-3-(chloromethyl)-2-methyl-5-nitrobenzene (900 mg, 4.09 mmol, 1.00 eq) in dimethylformamide (2.00 mL) was added potassium carbonate (1.13 g, 8.18 mmol, 2.00 eq), potassium iodide (67.9 mg, 0.41 mmol, 0.100 eq) and 2-oxa-6-azaspiro[3.3]heptane (811 mg, 8.18 mmol, 2.00 eq). The reaction was stirred at 20°C for 12 h. The mixture was filtered and concentrated under reduced pressure to give a residue. The residue was purified by silica gel column chromatography (petroleum ether/ethyl acetate = 1/0 to 3/1) to afford 6-(3-chloro-2-methyl-5-nitrobenzyl)-2-oxa-6-azaspiro[3.3]heptane (600 mg, 2.12 mmol, 52% yield) as a white solid.

**Step 3:** To a solution of 6-(3-chloro-2-methyl-5-nitrobenzyl)-2-oxa-6-azaspiro[3.3]heptane (600 mg, 2.12 mmol, 1.00 eq) in ethanol (6.00 mL) and water (2.00 mL) was added iron powder (356 mg, 6.37 mmol, 3.00 eq) and ammonium chloride (568 mg, 10.6 mmol, 5.00 eq). The reaction was stirred at 80°C for 2 h. The mixture was filtered and concentrated under reduced pressure to give a residue. Ethyl acetate (50.0 mL) was added to the residue, and the mixture was washed with a solution of saturated sodium bicarbonate (2 × 30.0 mL) and water (2 × 30.0 mL). The organic layer was dried over sodium sulfate, filtered, and concentrated under reduced pressure to afford 3-(2-oxa-6-azaspiro[3.3]heptan-6-ylmethyl)-5-chloro-4-methylaniline (300 mg, 1.19 mmol, 56% yield) as a yellow solid.

**Step 4:** To a solution of 3-(2-oxa-6-azaspiro[3.3]heptan-6-ylmethyl)-5-chloro-4-methylaniline (140 mg, 554 µmol, 1.00 eq) in dichloromethane (2.00 mL) was added pyridine (0.27 mL, 3.32 mmol, 6.00 eq) and a solution of phenyl chloroformate (69.4 µL, 554 µmol, 1.00 eq) in dichloromethane (2.00 mL) dropwise at 0°C. The reaction was stirred at 0°C for 1 h. The mixture was concentrated under reduced pressure to give a residue. The residue was purified by silica gel column chromatography (dichloromethane/methanol = 1/0 to 10/1) to afford phenyl (3-(2-oxa-6-azaspiro[3.3]heptan-6-ylmethyl)-5-chloro-4-methylphenyl)carbamate (180 mg, 483 µmol, 87% yield) as a yellow solid.

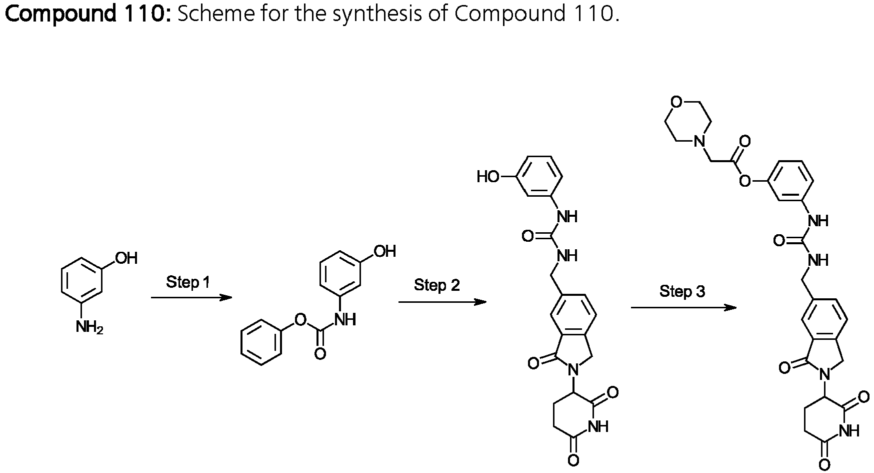

**Step 1:** To a mixture of 3-aminophenol (1.00 g, 9.16 mmol, 1.00 eq) and sodium bicarbonate (0.43 mL, 11.0 mmol, 1.20 eq) in tetrahydrofuran (10.0 mL) and water (1.00 mL) was added phenyl chloroformate (1.21 mL, 9.62 mmol, 1.05 eq) at 0°C. The reaction was stirred at 0°C for 2 h. The mixture was quenched with water (10.0 mL) and extracted with ethyl acetate (10.0 mL). The organic phase was dried over sodium sulfate, filtered, and concentrated under reduced pressure to afford phenyl N-(3-hydroxyphenyl)carbamate (2.00 g, 8.72 mmol, 95% yield) as white solid.

**Step 2:** A mixture of 3-[6-(aminomethyl)-1-oxo-isoindolin-2-yl]piperidine-2,6-dione hydrochloride **VI** (0.800 g, 2.58 mmol, 1.00 eq, hydrochloride), phenyl N-(3-hydroxyphenyl)carbamate (651 mg, 2.84 mmol, 1.10 eq) and triethylamine (1.08 mL, 7.75 mmol, 3.00 eq) in dimethyl formamide (10.0 mL) was heated to 50°C for 2 h. The mixture was added dropwise to ethyl acetate (50.0 mL) at 0°C, and the resulting solid was filtered, washed with ethyl acetate (2 x 3 mL) and dried to afford 1-[[2-(2,6-dioxo-3-piperidyl)-3-oxo-isoindolin-5-yl] methyl]-3-(3-hydroxyphenyl)urea (1.02 g, 2.50 mmol, 96% yield) as an off-white solid.

**Step 3:** To a mixture of 2-morpholinoacetic acid (89 mg, 612 µmol, 1.00 eq) and N,N-dimethylpyridin-4-amine (7.5 mg, 61.2 µmol, 0.10 eq) in dimethyl formamide (3.00 mL) was added N,N'-methanediylidenedicyclohexanamine (136 µL, 673 µmol, 1.10 eq) at 0°C. The reaction was stirred at 0°C for 30 min, then 1-[[2-(2,6-dioxo-3-piperidyl)-3-oxo-isoindolin-5-yl]methyl]-3-(3-hydroxyphenyl) urea (250 mg, 612 µmol, 1.00 eq) was added. The reaction was stirred at 20°C for 16 h. The mixture was filtered, and the filtrate was concentrated under reduced pressure to give a residue. The residue was purified by reversed phase preparative HPLC to afford **Compound 110** (77.4 mg, 144 µmol, 38% yield) as white solid.

1H NMR (400 MHz, DMSO-*d₆*) *□* = 10.99 (s, 1 H), 8.83 (s, 1 H), 7.67 (s, 1 H), 7.57 (s, 2 H), 7.40 (s, 1 H), 7.29 - 7.21 (m, 1 H), 7.14 (br d, *J* = 8.1 Hz, 1 H), 6.81 (br t, *J* = 5.6 Hz, 1 H), 6.66 (br d, *J* = 7.9 Hz, 1 H), 5.12 (br dd, *J* = 13.3, 5.0 Hz, 1 H), 4.49 - 4.36 (m, 3 H), 4.35 - 4.26 (m, 1 H), 3.64 - 3.56 (m, 4 H), 3.51 (s, 2 H), 2.97 - 2.85 (m, 1 H), 2.62 (br s, 1 H), 2.58 (br d, *J =* 4.0 Hz, 4 H), 2.46 - 2.35 (m, 1 H), 2.05 - 1.95 (m, 1 H). MS (ESI) m/z 536.2 [M+H]⁺

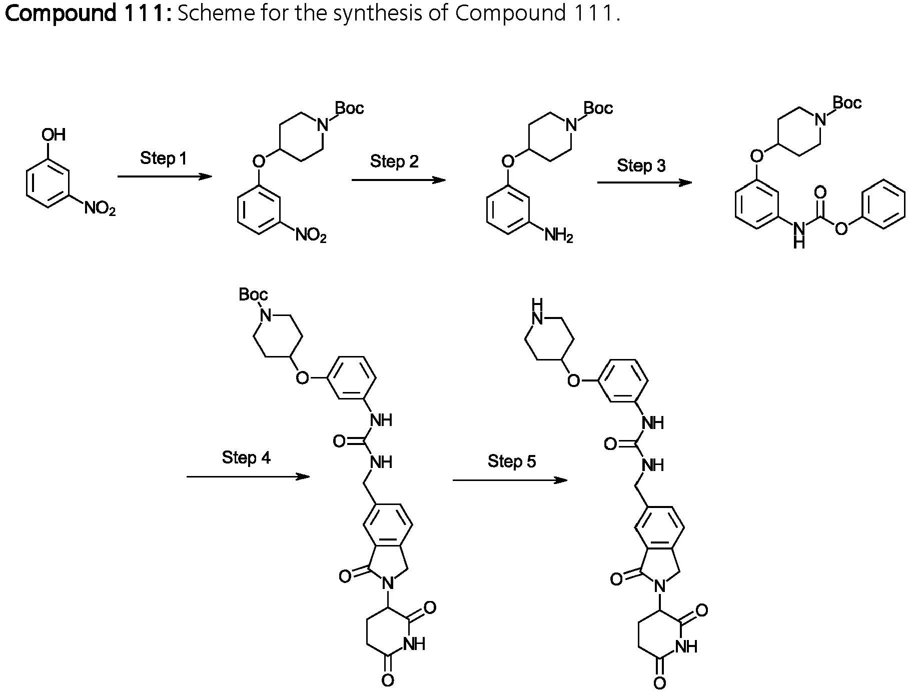

**Step 1:** A mixture of tert-butyl 4-((methylsulfonyl)oxy)piperidine-1-carboxylate (2.41 g, 8.63 mmol, 1.20 eq), 3-nitrophenol (1.43 mL, 7.19 mmol, 1.00 eq), potassium carbonate (1.29 g, 9.35 mmol, 1.30 eq) in anhydrous dimethylformamide (20.0 mL) was stirred at 80°C for 8 h under nitrogen. The mixture was diluted with water (20.0 mL) and extracted with ethyl acetate (3 × 10.0 mL). The combined organic layers were washed with brine (10.0 mL), dried over anhydrous sodium sulfate, filtered, and concentrated under reduced pressure to give a residue. The residue was purified by silica gel column chromatography (petroleum ether/ethyl acetate = 10/1 to 7/1) to afford *tert*-butyl 4-(3-nitrophenoxy)piperidine -1-carboxylate (1.90 g, 5.89 mmol, 81% yield) as yellow oil.

**Step 2:** To a solution of *tert*-butyl 4-(3-nitrophenoxy)piperidine-1-carboxylate (500 mg, 1.55 mmol, 1.00 eq) in ethanol (10.0 mL) was added Pd/C 10% weight on C (400 mg, 1.00 eq) under hydrogen atmosphere (15 psi). The reaction was stirred at 25°C for 6 h. The mixture was filtered, and the filtrate was concentrated under reduced pressure to afford *tert-*butyl 4-(3-aminophenoxy)piperidine-1-carboxylate (428 mg, 1.46 mmol, 94% yield) as yellow oil.

**Step 3:** To a solution of *tert*-butyl 4-(3-aminophenoxy)piperidine-1-carboxylate (428 mg, 1.46 mmol, 1.00 eq) in dichloromethane (5.00 mL) was added pyridine (0.15 mL, 1.90 mmol, 1.30 eq) and phenyl chloroformate (0.20 mL, 1.61 mmol, 1.10 eq) at 0°C. The reaction was stirred at 25°C for 3 h. The mixture was diluted with water (5.00 mL) and extracted with ethyl acetate (3 × 5.00 mL). The combined organic layers were washed with brine (5.00 mL), dried over anhydrous sodium sulfate, filtered, and concentrated under reduced pressure to afford tert-butyl 4-(3-((phenoxycarbonyl)amino)phenoxy) piperidine-1-carboxylate (538 mg, 1.30 mmol, 89% yield) as yellow oil.

**Step 4:** To a solution of tert-butyl 4-(3-((phenoxycarbonyl)amino)phenoxy)piperidine-1-carboxylate (399 mg, 968 µmol, 1.20 eq) in dimethyl formamide (4.00 mL) was added 3-[6-(aminomethyl)-1-oxo-isoindolin-2-yl]piperidine-2,6-dione hydrochloride **VI** (250 mg, 807 µmol, 1.00 eq, hydrochloride) and triethylamine (727 mg, 7.18 mmol, 1.00 mL, 8.90 eq). The reaction was stirred at 40°C for 2 h under nitrogen. The mixture was diluted with water (10.0 mL) and extracted with ethyl acetate (3 × 15.0 mL). The combined organic layers were washed with brine (10.0 mL), dried over anhydrous sodium sulfate, filtered, and concentrated under reduced pressure to give a residue. The residue was purified by silica gel column chromatography (dichloromethane/methanol = 25/1 to 20/1) to give tert-butyl 4-(3-(3-((2-(2,6-dioxopiperidin-3-yl)-3-oxoisoindolin-5-yl)methyl)ureido)phenoxy)piperidine-1-carboxylate (327 mg, 552 µmol, 68% yield) as yellow oil.

**Step 5:** A solution of *tert*-butyl 4-(3-(3-((2-(2,6-dioxopiperidin-3-yl)-3-oxoisoindolin-5-yl)methyl)ureido)phenoxy)piperidine-1-carboxylate (210 mg, 354 µmol, 1.00 eq) in hydrochloric acid/ethyl acetate (4.00 mL) was stirred at 20°C for 0.5 h. The mixture was concentrated under reduced pressure to give a residue. The residue was triturated with acetonitrile at 20°C for 20 min to afford **Compound 111** (75.0 mg, 152 µmol, 43% yield) as a white solid.

¹H NMR (400 MHz, DMSO-*d*₆) *□* = 10.98 (s, 1H), 8.82 (br s, 1H), 8.68 (br s, 2H), 7.64 (s, 1H), 7.55 (s, 2H), 7.27 (s, 1H), 7.12 (t, *J* = 8.1 Hz, 1H), 6.89 (br s, 1H), 6.84 (br d, *J* = 7.9 Hz, 1H), 6.54 (dd, *J =* 2.1, 8.2 Hz, 1H), 5.11 (dd, *J* = 5.0, 13.3 Hz, 1H), 4.56 (br d, *J* = 3.3 Hz, 1H), 4.46 - 4.36 (m, 3H), 4.33 - 4.26 (m, 1H), 3.24 - 3.15 (m, 2H), 3.11 - 3.01 (m, 2H), 2.96 - 2.84 (m, 1H), 2.59 (br d, *J =* 18.3 Hz, 1H), 2.42 - 2.30 (m, 1H), 2.10 - 1.95 (m, 3H), 1.83 (br d, *J* = 3.5 Hz, 2H). MS (ESI) m/z 492.2 [M+H]⁺

**Compound 112:** General procedure A with variant iii) was used for the preparation with a yield of 29% from compound **VI** employing phenyl (3-(1H-imidazol-2-yl)phenyl)carbamate.

¹H NMR (400MHz, DMSO-*d*₆) *□* = 11.00 (br s, 1H), 8.80 (s, 1H), 8.18 (s, 1H), 7.98 (s, 1H), 7.68 (s, 1H), 7.61 - 7.52 (m, 2H), 7.46 - 7.36 (m, 2H), 7.31 - 7.23 (m, 1H), 7.09 (br s, 2H), 6.86 (t, *J =* 6.0 Hz, 1 H), 5.12 (dd, *J* = 5.1*,* 13.3 Hz, 1H), 4.48 - 4.37 (m, 3H), 4.34 - 4.25 (m, 1H), 2.97 - 2.83 (m, 1H), 2.58 (br dd, *J* = 2.2, 15.3 Hz, 1H), 2.42 - 2.33 (m, 1H), 2.03 - 1.94 (m, 1H). MS (ESI) m/z 459.1 [M+H]⁺

### Scheme for the preparation of phenyl (3-(1H-imidazol-2-yl)phenyl)carbamate.

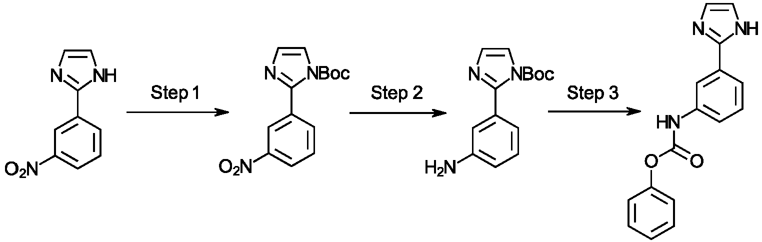

**Step 1:** To a mixture of 2-(3-nitrophenyl)-1H-imidazole (900 mg, 4.76 mmol, 1.00 eq), di-tert-butyl dicarbonate (1.64 mL, 7.14 mmol, 1.50 eq), and 4-dimethylaminopyridine (58.1 mg, 475 µmol, 0.10 eq) in dichloromethane (9.00 mL) was added triethylamine (993 µL, 7.14 mmol, 1.50 eq) and the reaction was stirred for 16 h at 25°C. The mixture was diluted with water (10.0 mL) and extracted with dichloromethane (3 × 10.0 mL). The combined organic layers were washed with brine (10.0 mL), dried over anhydrous sodium sulfate, filtered, and concentrated under reduced pressure to give a residue. The residue was purified by silica gel column chromatography (petroleum ether/ethyl acetate = 10/1 to 1/1) to give tert-butyl 2-(3-nitrophenyl)-1H-imidazole-1-carboxylate (1.12 g, 3.87 mmol, 81% yield) as yellow oil.

**Step 2:** To a solution of tert-butyl 2-(3-nitrophenyl)-1*H*-imidazole-1-carboxylate (500 mg, 1.73 mmol, 1.00 eq) in ethyl alcohol (10.0 mL) was added Pd/C 10% weight on C (400 mg, 1.73 mmol, 1.00 eq), and the mixture was stirred at 25°C for 3 h under hydrogen atmosphere (15 psi). The mixture was filtered, and the filtrate was concentrated under reduced pressure to give *tert*-butyl 2-(3-aminophenyl)-1H-imidazole-1-carboxylate (842 mg, 3.25 mmol, 93% yield) as a yellow solid.

**Step 3:** To a solution of *tert*-butyl 2-(3-aminophenyl)-1*H*-imidazole-1-carboxylate (790 mg, 3.05 mmol, 1.00 eq) in dichloromethane (10.0 mL) was added pyridine (0.32 mL, 3.96 mmol, 1.30 eq) and phenyl chloroformate (524 mg, 3.35 mmol, 419 µL, 1.10 *eq)* at 0°C. The reaction was stirred for 3 h at 25°C. The mixture was diluted with water (50.0 mL) and extracted with dichloromethane (3 × 50.0 mL). The combined organic layers were washed with brine (20.0 mL), dried over anhydrous sodium sulfate, filtered, and concentrated under reduced pressure to give phenyl (3-(1H-imidazol-2-yl)phenyl)carbamate (740 mg, 2.65 mmol, 86% yield) as a yellow solid.

**Compound 113:** General procedure A with variant iii) was used for the preparation with a yield of 68% from compound **VI** employing phenyl (3-(2-methyl-1H-imidazol-1-yl)phenyl)carbamate.

¹H NMR (400 MHz, DMSO-*d*₆) *□* = 10.99 (s, 1H), 8.96 (s, 1H), 8.15 (s, 1H), 7.67 (s, 1H), 7.60 (s, 1H), 7.59 - 7.54 (m, 2H), 7.37 (d, *J =* 4.6 Hz, 2H), 7.26 (s, 1H), 6.99 - 6.88 (m, 3H), 5.11 (dd, *J* = 5.1, 13.5 Hz, 1H), 4.46 - 4.38 (m, 3H), 4.33 - 4.28 (m, 1H), 2.97 - 2.85 (m, 1H), 2.60 (br d, *J =* 17.2 Hz, 1H), 2.39 (dq, *J* = 4.3, 13.3 Hz, 1H), 2.28 (s, 3H), 2.05 - 1.94 (m, 1H). MS (ESI) m/z 473.1 [M+H]⁺

### Scheme for the preparation of phenyl (3-(2-methyl-1H-imidazol-1-yl)phenyl)carbamate.

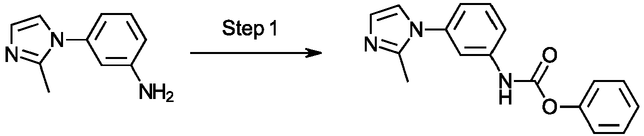

**Step 1:** To a solution of 3-(2-methylimidazol-1-yl)aniline (90.0 mg, 520 µmol, 1.00 eq) in dichloromethane (3.00 mL) was added pyridine (84 µL, 1.04 mmol, 2.00 eq). Phenyl chloroformate (72 µL, 572 µmol, 1.10 eq) was added to the mixture portion-wise. The reaction was stirred at 20°C for 2 h. The mixture was poured into water (5.00 mL) and extracted with dichloromethane (2 x 5.00 m). The combined organic layers were concentrated under reduced pressure to give a residue. The residue was purified by preparative TLC (ethyl acetate/methanol = 10/1, R_{f} = 0.55) to afford phenyl (3-(2-methyl-1H-imidazol-1-yl)phenyl)carbamate (140 mg, crude product) as a white solid. It was used as such in the next reaction.

**Compound 114:** General procedure A with variant iii) was used for the preparation with a yield of 62% from compound **VI** employing phenyl (4-(difluoromethyl)phenyl)carbamate.

¹H NMR (400 MHz, DMSO-*d*₆) □ = 10.98 (s, 1H), 8.93 (s, 1H), 7.67 (s, 1H), 7.58 - 7.48 (m, 4H), 7.42 (d, *J* = 8.4 Hz, 2H), 7.07 - 6.73 (m, 2H), 5.11 (dd, *J =* 5.1, 13.3 Hz, 1H), 4.48 - 4.37 (m, 3H), 4.36 - 4.25 (m, 1H), 2.91 (ddd, *J* = 5.4, 13.5, 17.5 Hz, 1H), 2.62 - 2.57 (m, 1H), 2.39 (br dd, *J* = 4.5, 13.1 Hz, 1H), 2.04 - 1.95 (m, 1H). MS (ESI) m/z 423.3 [M-20]⁺

### Scheme for the preparation of phenyl (4-(difluoromethyl)phenyl)carbamate.

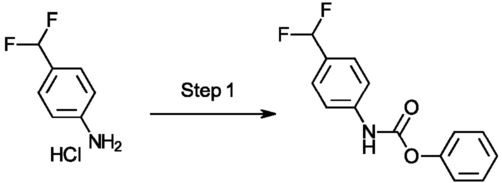

**Step 1:** To a solution of 4-(difluoromethyl)aniline hydrochloride (400 mg, 2.23 mmol, 1.00 eq) in tetrahydrofuran (8.00 mL) was added triethylamine (0.62 mL, 4.45 mmol, 2.00 eq) and phenyl chloroformate (0.31 mL, 2.45 mmol, 1.10 eq). The reaction was stirred at 20°C for 1 h. The mixture was filtered and concentrated under reduced pressure to give a residue. The residue was purified by silica gel column chromatography (petroleum ether/ethyl acetate = 1/0 to 5/1) to give phenyl (4-(difluoromethyl)phenyl)carbamate (440 mg, 1.67 mmol, 75% yield) as a white solid.

**Compound 115:** General procedure A with variant iii) was used for the preparation with a yield of 37% from compound VI employing phenyl (3-(1-methyl-1H-pyrazol-5-yl)phenyl)carbamate.

¹H NMR (400 MHz, DMSO-*d*₆) *□* = 10.98 (s, 1H), 8.81 (s, 1H), 7.67 (s, 1H), 7.61 (s, 1H), 7.59 - 7.55 (m, 2H), 7.46 - 7.41 (m, 2H), 7.37 - 7.31 (m, 1H), 7.05 (d, *J=* 7.9 Hz, 1H), 6.84 (t, *J* = 6.1 Hz, 1H), 6.35 (d, *J* = 1.8 Hz, 1H), 5.11 (dd, *J* = 5.1*,* 13.2 Hz, 1H), 4.47 - 4.39 (m, 3H), 4.34 - 4.27 (m, 1H), 3.83 (s, 3H), 2.97 - 2.86 (m, 1H), 2.60 (br d, *J =* 15.4 Hz, 1H), 2.44 - 2.31 (m, 1H), 2.04 - 1.96 (m, 1H). MS (ESI) m/z 473.1 [M+H]⁺

### Scheme for the preparation of phenyl (3-(1-methyl-1H-pyrazol-5-yl)phenyl)carbamate.

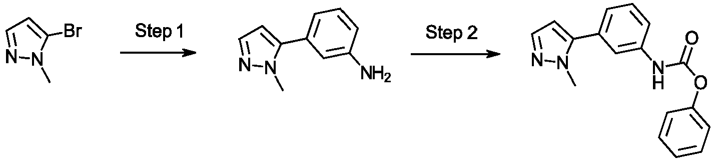

**Step 1:** To a mixture of 5-bromo-1-methyl-pyrazole (0.500 g, 3.11 mmol, 1.00 eq) and (3-aminophenyl)boronic acid (510 mg, 3.73 mmol, 1.20 eq) in dioxane (10.0 mL) and water (1.00 mL) was added tetrakis(triphenylphosphine)palladium (359 mg, 311 µmol, 0.10 eq) and potassium phosphate (1.98 g, 9.32 mmol, 3.0 eq). The reaction was stirred at 110°C for 16 h. The mixture was poured into water (20.0 mL), and the product was extracted with ethyl acetate (2 x 20.0 mL). The combined organic layers were washed with brine (10.0 mL) and concentrated under reduced pressure to give a residue. The residue was purified by silica gel column chromatography (petroleum ether/ethyl acetate = 5/1 to 1/1) to afford 3-(2-methylpyrazol-3-yl)aniline (400 mg, 2.15 mmol, 69% yield) as a yellow oil.

**Step 2:** To a solution of 3-(2-methylpyrazol-3-yl)aniline (0.400 g, 2.31 mmol, 1.00 eq) in dichloromethane (5.00 mL) was added pyridine (0.34 mL, 4.62 mmol, 2.00 eq). The mixture was cooled to 0°C, and phenyl chloroformate (0.32 mL, 2.54 mmol, 1.10 eq) was added dropwise. The reaction was stirred at 20°C for 1 h. Water (5.00 mL) was added to the mixture. The organic layer was separated and washed with brine (5.00 mL), dried over anhydrous sodium sulfate, filtered, and concentrated under reduced pressure to give phenyl (3-(1-methyl-1H-pyrazol-5-yl)phenyl)carbamate (650 mg, crude) as a yellow oil. It was used directly in the next reaction.

**Compound 116:** General procedure A with variant iii) was used for the preparation with a yield of 33% from compound **VI** employing phenyl *N*-[3-(1-methylpyrazol-3-yl)phenyl]carbamate.

¹H NMR (400 MHz, DMSO-*d*₆) □ ppm 10.98 (br s, 1 H), 8.70 (br s, 1 H), 7.89 (br s, 1 H), 7.76 - 7.65 (m, 2 H), 7.58 (br s, 2 H), 7.39 - 7.18 (m, 3 H), 6.74 (br s, 1 H), 6.58 (br d, J = 2.1 Hz, 1 H), 5.12 (br dd, *J =* 13.1, 4.9 Hz, 1 H), 4.49 - 4.26 (m, 4 H), 3.88 (s, 3 H), 2.99 - 2.83 (m, 1 H), 2.60 (br d, *J =* 17.4 Hz, 1 H), 2.45 - 2.36 (m, 1 H), 2.07 - 1.94 (m, 1 H). MS (ESI) m/z 473.2 [M+H]⁺

### Scheme for the preparation of phenyl N-[3-(1-methylpyrazol-3-yl)phenyl]carbamate.

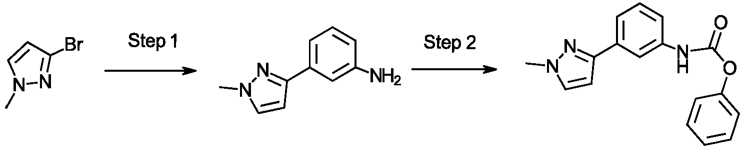

**Step 1:** A mixture of 3-bromo-1-methyl-pyrazole (0.550 g, 3.42 mmol, 1.00 eq), (3-aminophenyl)boronic acid (561 mg, 4.10 mmol, 1.20 eq), potassium phosphate (2.18 g, 10.3 mmol, 3.00 eq) and tetrakis(triphenylphosphine)palladium (197 mg, 171 µmol, 0.05 eq) in dioxane (10.0 mL) and water (1.00 mL) was heated to 110°C for 16 h under nitrogen. Water (10.0 mL) was added to the mixture, and it was extracted with ethyl acetate (2 x 10.0 mL). The combined organic layers were washed with brine (10.0 mL), dried over sodium sulfate, filtered, and concentrated under reduced pressure to give a residue. The residue was purified by reversed phase preparative HPLC to give 3-(1-methylpyrazol-3-yl)aniline (0.410 g, 2.37 mmol, 69% yield) as yellow solid.

**Step 2:** To a solution of 3-(1-methylpyrazol-3-yl)aniline (0.380 g, 2.19 mmol, 1.00 eq) and pyridine (0.53 mL, 6.58 mmol, 3.00 eq) in dichloromethane (10.0 mL) at 0°C was added phenyl chloroformate (0.30 mL, 2.41 mmol, 1.10 eq). The mixture was allowed to warm to 20°C and stirred for 2 h. Water (10.0 mL) was added, and the organic layer was separated, dried over sodium sulfate, filtered and concentrated under reduced pressure to give phenyl N-[3-(1-methylpyrazol-3-yl)phenyl]carbamate (0.600 g, 2.05 mmol, 93% yield) as a yellow solid. It was used directly in the next step.

**Compound 117:** General procedure A with variant iii) was used for the preparation with a yield of 37% from compound **VI** employing (3-chloro-5-(3-(dimethylamino)propoxy)-4-methylphenyl)carbamate.

¹H NMR (400 MHz, DMSO-*d*₆) *□* = 10.99 (s, 1H), 8.91 (s, 1H), 8.23 (s, 1H), 7.66 (s, 1H), 7.56 (d, *J =* 1.0 Hz, 2H), 7.15 (d, *J* = 1.9 Hz, 1H), 7.05 (d, *J =* 1.8 Hz, 1H), 7.03 - 6.89 (m, 1H), 5.12 (dd, *J* = 5.1, 13.3 Hz, 1H), 4.42 - 4.30 (m, 4H), 3.98 - 3.94(m, 2H), 2.95 - 2.88 (m, 1H), 2.63 - 2.58 (m, 1H), 2.47 - 2.44 (m, 2H), 2.42 - 2.36 (m, 1H), 2.21 (s, 6H), 2.11 (s, 3H), 2.03 - 1.97 (m, 1H), 1.89 (t, *J* = 6.8 Hz, 2H). MS (ESI) m/z 542.2 [M+H]⁺

### Scheme for the preparation of (3-chloro-5-(3-(dimethylamino)propoxy)-4-methylphenyl)carbamate.

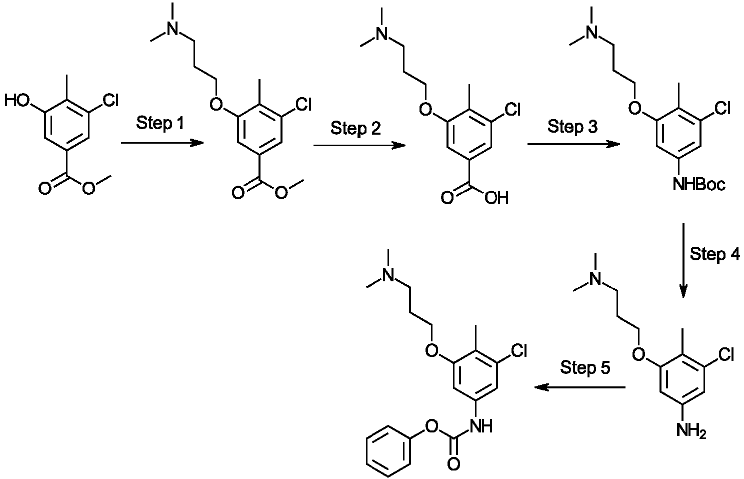

**Step 1:** A mixture of methyl 3-chloro-5-hydroxy-4-methyl-benzoate (1.00 g, 4.98 mmol, 1.00 eq), 3-chloro-N,N-dimethyl-propan-1-amine hydrochloride (709 mg, 4.49 mmol, 0.90 eq, hydrochloride) and potassium carbonate (2.07 g, 14.9 mmol, 3.00 eq) in acetonitrile (20.0 mL) was stirred at 85°C for 12 h. The mixture was diluted with water (50.0 mL) and extracted with ethyl acetate (3 x 20.0 mL). The combined organic layers were washed with brine (3 x 10.0 mL), dried over sodium sulfate, filtered, and concentrated under reduced pressure to give a residue. The residue was purified by reversed phase preparative HPLC to give methyl 3-chloro-5-(3-(dimethylamino)propoxy)-4-methylbenzoate (1.00 g, 3.50 mmol, 70% yield) as a yellow oil.

**Step 2:** To a solution of methyl 3-chloro-5-(3-(dimethylamino)propoxy)-4-methylbenzoate (900 mg, 3.15 mmol, 1.00 eq) in water (10.0 mL) and methanol (20.0 mL) was added sodium hydroxide (252 mg, 6.30 mmol, 2.00 eq). The reaction was stirred at 60°C for 2 h. The mixture was concentrated under reduced pressure to give a residue. The residue was purified by reversed phase preparative HPLC to afford 3-chloro-5-(3-(dimethylamino)propoxy)-4-methylbenzoic acid (750 mg, 2.76 mmol, 87% yield) as a yellow solid.

**Step 3:** To a mixture of 3-chloro-5-(3-(dimethylamino)propoxy)-4-methylbenzoic acid (600 mg, 2.21 mmol, 1.00 eq) and triethylamine (614 µL, 4.42 mmol, 2.00 eq) in 2-methylpropan-2-ol (10.0 mL) was added diphenyl phosphoryl azide (0.96 mL, 4.42 mmol, 2.00 eq). The reaction was stirred at 100°C for 12 h. The mixture was concentrated under reduced pressure to give a residue. The residue was purified by reversed phase preparative HPLC to afford tert-butyl (3-chloro-5-(3-(dimethylamino)propoxy)-4-methylphenyl)carbamate (360 mg, 1.05 mmol, 47% yield) as a yellow oil.

**Step 4:** A solution of tert-butyl (3-chloro-5-(3-(dimethylamino)propoxy)-4-methylphenyl)carbamate (350 mg, 1.02 mmol, 1.00 eq) in hydrogen chloride/ethyl acetate (4.00 M, 10.0 mL, 39.2 eq) was stirred at 25°C for 0.5 h. The mixture was concentrated under reduced pressure to give 3-chloro-5-(3-(dimethylamino)propoxy)-4-methylaniline (360 mg, crude, hydrogen chloride) as a yellow solid. It was used directly in the next step.

**Step 5:** To a solution of 3-chloro-5-(3-(dimethylamino)propoxy)-4-methylaniline (300 mg, 1.07 mmol, 1.00 eq, hydrogen chloride) and triethylamine (326 mg, 3.22 mmol, 3.00 *eq)* in dichloromethane (5.00 mL) was added phenyl chloroformate (252 mg, 1.61 mmol, 1.50 eq) at 25°C. The mixture was stirred at 25°C for 0.5 h. The mixture was concentrated under reduced pressure to give a residue. The residue was purified by reversed phase preparative HPLC and lyophilized to give phenyl (3-chloro-5-(3-(dimethylamino)propoxy)-4-methylphenyl)carbamate (150 mg, 0.41 mmol, 38% yield) as a yellow oil.

**Compound 118:** General procedure A with variant iii) was used for the preparation with a yield of 60% from compound **VI** employing phenyl N-[3,4-dimethyl-5-(morpholinomethyl) phenyl]carbamate.

¹H NMR (400 MHz, DMSO-*d₆*) *□* = 10.98 (s, 1H), 8.43 (s, 1H), 8.15 (s, 1H), 7.65 (s, 1H), 7.55 (d, *J =* 0.8 Hz, 2H), 7.17 (d, *J=* 2.0 Hz, 1H), 7.10 (d, *J=* 2.0 Hz, 1H), 6.65 (t, *J* = 6.0 Hz, 1H), 5.11 (dd, *J* = 5.2, 13.2 Hz, 1H), 4.46 - 4.28 (m, 4H), 3.54 (br t, *J* = 4.0 Hz, 4H), 3.34 (s, 2H), 2.97 - 2.85 (m, 1H), 2.59 (br d, *J* = 17.4 Hz, 1H), 2.45 - 2.38 (m, 1H), 2.34 (br s, 4H), 2.17 (s, 3H), 2.12 (s, 3H), 2.04 - 1.95 (m, 1H). MS (ESI) m/z 520.2 [M+H]⁺

### Scheme for the preparation of phenyl N-[3,4-dimethyl-5-(morpholinomethyl) phenyl]carbamate.

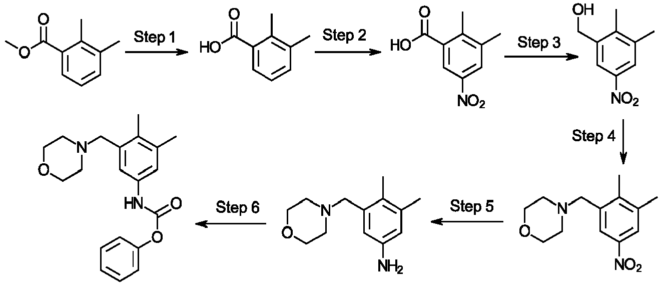

**Step 1:** To a solution of sodium hydroxide (2.19 g, 54.8 mmol, 3.00 eq) in water (10.0 mL) and ethanol (10.0 mL) was added methyl 2,3-dimethylbenzoate (3.00 g, 18.3 mmol, 1.00 eq) in one portion. The reaction was stirred at 20°C for 12 h. The mixture was concentrated under reduced pressure and poured into water (50.0 mL). The pH was adjusted to pH = 1-2 with 36% aqueous hydrochloric acid, and the mixture was filtered. The filter cake was washed with water (2 x 20.0 mL) and dried under reduced pressure to afford 2,3-dimethylbenzoic acid (2.50 g, 16.6 mmol, 91 % yield) as a white solid.

**Step 2:** To a solution of 2,3-dimethylbenzoic acid (2.50 g, 16.6 mmol, 1.00 eq) in sulfuric acid (25.0 mL) was added potassium nitrate (2.02 g, 19.98 mmol, 1.20 eq) in portions at 0°C. The reaction was warmed to 15°C and stirred for 12 h. The mixture was poured into ice-water (200 mL), filtered, and the filter cake was washed with water (2 x 50.0 mL). The filter cake was dried under reduced pressure to afford 2,3-dimethyl-5-nitro-benzoic acid (3.00 g, 15.4 mmol, 92% yield) as a white solid.

**Step 3:** To a solution of 2,3-dimethyl-5-nitro-benzoic acid (4.50 g, 23.1 mmol, 1.00 eq) in tetrahydrofuran (100 mL) was added dimethyl sulfide borane (10.0 M, 4.61 mL, 2.00 eq) dropwise at 20°C. The reaction was heated to 60°C and stirred for 5 h. The mixture was cooled to 0°C, then quenched with methanol (5.00 mL) and water (5.00 mL), and stirred at 20°C for 0.5 h. The mixture was concentrated under reduced pressure and poured into saturated aqueous sodium bicarbonate (50.0 mL). The mixture was extracted with ethyl acetate (4 x 50.0 mL), and the combined organic layers were dried over anhydrous sodium sulfate, filtered, and concentrated under reduced pressure to give a residue. The residue was purified by silica gel column chromatography (petroleum ether/ethyl acetate = 1/0 to 7/3) to afford (2,3-dimethyl-5-nitro-phenyl)methanol (2.50 g, 13.80 mmol, 59% yield) as a yellow solid.

**Step 4:** To a solution of (2,3-dimethyl-5-nitro-phenyl)methanol (2.50 g, 13.8 mmol, 1.00 eq) in dichloromethane (20.0 mL) was added thionyl chloride (10.0 mL, 138 mmol, 10.0 eq) and N-methyl pyrrolidone (1.34 mL, 13.8 mmol, 1.00 eq) dropwise at 0°C. The reaction was stirred at 20°C for 12 h. The mixture was concentrated under reduced pressure to give a residue. Acetonitrile (20.0 mL) was added, followed by triethylamine (5.76 mL, 41.4 mmol, 3.00 eq) and morpholine (1.46 mL, 16.5 mmol, 1.20 eq). The reaction was stirred at 20°C for 12 h. The mixture was concentrated under reduced pressure to give a residue. The residue was purified by silica gel column chromatography (petroleum ether/ethyl acetate = 1/0 to 0/1) to afford 4-[(2,3-dimethyl-5-nitro-phenyl)methyl]morpholine (2.50 g, 9.79 mmol, 70% yield) as yellow solid.

**Step 5:** To a solution of 4-[(2,3-dimethyl-5-nitro-phenyl)methyl]morpholine (2.50 g, 10.0 mmol, 1.00 eq) in ethanol (30.0 mL) and water (15.0mL) were added ammonium chloride (534 mg, 10.0 mmol, 1 eq) and iron powder (2.79 g, 50.0 mmol, 5.00 eq) in portions at 20°C. The reaction was stirred at 90°C for 12 h. The mixture was filtered, and the filter cake was washed with methanol (50.0 mL). The filtrate was concentrated under reduced pressure to give a residue. The residue was poured into saturated aqueous sodium bicarbonate (50.0 mL) and extracted with ethyl acetate (4 x 50.0 mL). The combined organic layers were dried over sodium sulfate, filtered, and the filtrate was concentrated under reduced pressure to afford 3,4-dimethyl-5-(morpholinomethyl)aniline (2.10 g, 9.53 mmol, 95% yield) as a yellow solid.

**Step 6:** To a solution of 3,4-dimethyl-5-(morpholinomethyl)aniline (1.00 g, 4.54 mmol, 1.00 eq) and triethylamine (1.26 mL, 9.08 mmol, 2.00 eq) in tetrahydrofuran (10.0 mL) was added phenyl chloroformate (0.68 mL, 5.45 mmol, 1.20 eq) dropwise at 20°C. The reaction was stirred at 20°C for 12 h. The mixture was concentrated under reduced pressure to give a residue. The residue was purified by silica gel column chromatography (ethyl acetate/methanol = 1/0 to 10/1) to afford phenyl N-[3,4-dimethyl-5-(morpholinomethyl)phenyl]carbamate (600 mg, 1.67 mmol, 36% yield) as a yellow solid.

**Compound 119:** General procedure A with variant iii) was used for the preparation with a yield of 24% from compound **VI** employing phenyl N-[3-chloro-5-(morpholinomethyl)phenyl]carbamate.

¹H NMR (400 MHz, DMSO-*d₆*) *□* = 10.98 (s, 1H), 8.92 (s, 1H), 8.15 (s, 1H), 7.69 - 7.53 (m, 4H), 7.19 (s, 1H), 6.92 - 6.81 (m, 2H), 5.11 (dd, *J* = 5.2, 13.2 Hz, 1H), 4.48 - 4.27 (m, 4H), 3.60 - 3.53 (m, 4H), 3.39 (s, 2H), 2.98 - 2.82 (m, 1H), 2.64 - 2.56 (m, 1H), 2.45 - 2.37 (m, 1H), 2.34 (br s, 4H), 2.05 - 1.95 (m, 1H). MS (ESI) m/z 526.1 [M+H]⁺

### Scheme for the preparation of phenyl N-[3-chloro-5-(morpholinomethyl)phenyl]carbamate.

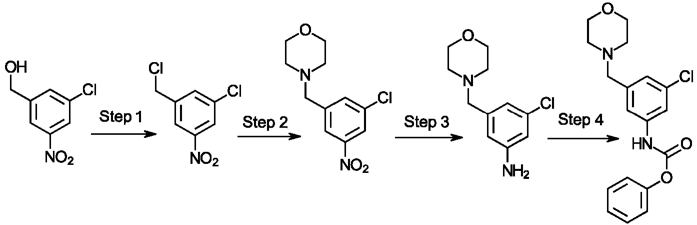

**Step 1:** To a solution of (3-chloro-5-nitro-phenyl)methanol (880 mg, 4.69 mmol, 1.00 eq) and N-methyl pyrrolidone (1.50 mL) in dichloromethane (10.0 mL) was added thionyl chloride (3.4 mL, 46.9 mmol, 10.0 eq) at 0°C. The reaction was stirred at 25° C for 2 h. The mixture was concentrated under reduced pressure to give a residue. The residue was diluted with saturated aqueous sodium bicarbonate (60.0 mL) and extracted with ethyl acetate (4 x 50.0 mL). The combined organic layers were dried over sodium sulfate, filtered, and concentrated under reduced pressure to give a residue. The residue was purified by silica gel column chromatography (petroleum ether/ethyl acetate = 3/1) to afford 1-chloro-3-(chloromethyl)-5-nitro-benzene (1.49 g, crude) as a yellow liquid.

**Step 2:** To a solution of 1-chloro-3-(chloromethyl)-5-nitro-benzene (1.40 g, 6.80 mmol, 1.00 eq) in acetonitrile (17.0 mL) were added triethylamine (2.36 mL, 17.0 mmol, 2.50 eq) and morpholine (0.78 mL, 8.83 mmol, 1.30 eq). The reaction was stirred at 25°C for 12 h. The mixture was concentrated under reduced pressure to give a residue. The residue was purified by silica gel column chromatography (petroleum ether/ethyl acetate = 7/1) to afford 4-[(3-chloro-5-nitro-phenyl)methyl]morpholine (1.0 g, 3.90 mmol, 57% yield) as a yellow liquid.

**Step 3:** To a solution of 4-[(3-chloro-5-nitro-phenyl)methyl]morpholine (980 mg, 3.82 mmol, 1.00 eq) in ethanol (16.0 mL) and water (8.00 mL) were added ammonium chloride (204 mg, 3.82 mmol, 1.00 eq) and iron powder (1.07 g, 19.1 mmol, 5.00 eq). The reaction was stirred at 90°C for 12 h. The mixture was filtered and washed with ethyl acetate (20.0 mL), and the filtrate was extracted with ethyl acetate (3 × 20.0 mL). The combined organic layers were washed with brine (25.0 mL), dried over anhydrous sodium sulfate, filtered, and concentrated under reduced pressure to afford 3-chloro-5-(morpholinomethyl)aniline (730 mg, 3.22 mmol, 84% yield) as a yellow liquid.

**Step 4:** To a solution of phenyl chloroformate (0.21 mL, 1.70 mmol, 1.10 eq) in tetrahydrofuran (20.0 mL) was added triethylamine (0.43 mL, 3.09 mmol, 2.00 eq) and 3-chloro-5-(morpholinomethyl)aniline (350 mg, 1.54 mmol, 1.00 eq). The mixture was stirred at 25°C for 0.5 h. The mixture was filtered and concentrated under reduced pressure to give a residue. The residue was purified by silica gel column chromatography (petroleum ether/ethyl acetate = 3/1) to afford phenyl N-[3-chloro-5-(morpholinomethyl)phenyl]carbamate (460 mg, 1.33 mmol, 85% yield) as a yellow solid.

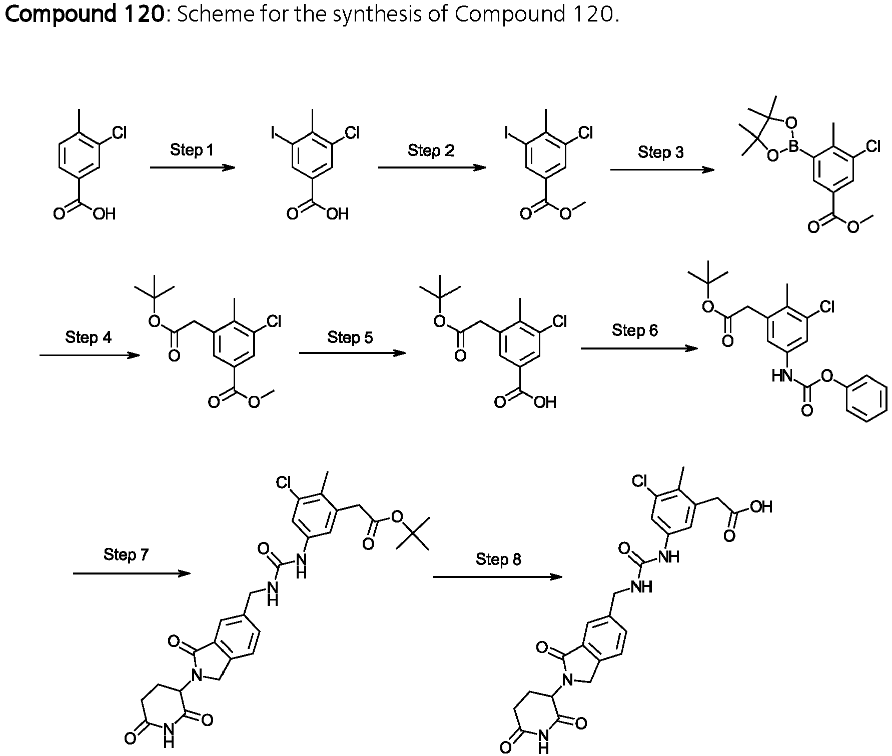

**Step 1:** To a solution of 3-chloro-4-methyl-benzoic acid (20.0 g, 117 mmol, 1.00 eq) in sulfuric acid (80.0 mL) was added 1-iodopyrrolidine-2,5-dione (29.0 g, 129 mmol, 1.10 eq). The reaction was stirred at 25°C for 1 h. The mixture was poured slowly into stirred ice water (300 mL). The resulting suspension was filtered, and the filter cake was washed with water (100 mL) and dried under reduced pressure. Methanol (200 mL) was added, and the mixture was concentrated under reduced pressure to afford 3-chloro-5-iodo-4-methylbenzoic acid (36.0 g, crude) as a white solid. It was used directly in the next step.

**Step 2:** To a solution of 3-chloro-5-iodo-4-methyl-benzoic acid (34.8 g, 117 mmol, 1.00 eq) in methanol (500 mL) was added thionyl chloride (27.9 g, 234 mmol, 17.0 mL, 2.00 eq) dropwise at 0°C. The reaction was stirred at 60°C for 12 h. The mixture was concentrated to 100 mL under reduced pressure, and the resulting suspension was filtered. The filter cake was washed with methanol (30.0 mL) and dried under reduced pressure to afford methyl 3-chloro-5-iodo-4-methyl-benzoate (31.0 g, 99.8 mmol, 85% yield) as a white solid.

**Step 3:** A solution of methyl 3-chloro-5-iodo-4-methyl-benzoate (10.0 g, 32.2 mmol, 1.00 eq), 4,4,5,5-tetramethyl-2-(4,4,5,5-tetramethyl-1,3,2-dioxaborolan-2-yl)-1,3,2-dioxaborolane (16.4 g, 64.4 mmol, 2.00 eq), potassium acetate (9.48 g, 96.6 mmol, 3.00 eq) and (1,1-bis(diphenylphosphino)ferrocene) dichloropalladium(II) (2.36 g, 3.22 mmol, 0.10 eq) in dioxane (200 mL) was stirred at 110°C for 12 h. The mixture was concentrated under reduced pressure to give a residue. The residue was diluted with water (200 mL) and extracted with ethyl acetate (2 × 200 mL). The combined organic layers were washed with water (100 mL), dried over anhydrous sodium sulfate, filtered, and the filtrate was concentrated to afford a residue. The residue was purified by silica gel column chromatography (petroleum ether/ethyl acetate = 1/0 to 100/5) to afford a residue. The residue was triturated with petroleum ether (100 mL), filtered, and the filter cake was washed with petroleum ether (50.0 mL) and dried under reduced pressure to afford methyl 3-chloro-4-methyl-5-(4,4,5,5-tetramethyl-1,3,2-dioxaborolan-2-yl)benzoate (6.00 g, 19.3 mmol, 60% yield) as a white solid.

**Step 4:** To a solution of tert-butyl 2-bromoacetate (0.79 mL, 5.37 mmol, 1.00 eq), palladium acetate (36.1 mg, 161 µmol, 0.03 eq), potassium phosphate (5.70 g, 26.8 mmol, 5.00 eq) and tris-o-tolylphosphane (147 mg, 483 µmol, 0.09 eq) in tetrahydrofuran (40.0 mL) was added methyl 3-chloro-4-methyl-5-(4,4,5,5-tetramethyl-1,3,2-dioxaborolan-2-yl)benzoate (2.00 g, 6.44 mmol, 1.20 eq). The reaction was stirred at 25°C for 12 h under nitrogen. The mixture was filtered, and the filtrate was concentrated under reduced pressure to afford a residue. The residue was purified by silica gel column chromatography (petroleum ether/ethyl acetate = 1/0 to 5/1) to afford a residue. The residue was triturated with petroleum ether (10.0 mL), filtered, and the filtrate was concentrated to afford methyl 3-(2-tert-butoxy-2-oxo-ethyl)-5-chloro-4-methyl-benzoate (400 mg, crude) as a transparent oil. It was used directly in the next step.

**Step 5:** To a solution of methyl 3-(2-tert-butoxy-2-oxo-ethyl)-5-chloro-4-methyl-benzoate (400 mg, 1.34 mmol, 1.00 eq) in methanol (3.00 mL) was added a solution of sodium hydroxide (107 mg, 2.68 mmol, 2.00 eq) in water (3.00 mL). The reaction was stirred at 25°C for 12 h. The mixture was concentrated under reduced pressure to give a residue. The residue was diluted with 1 M hydrochloric acid (3.00 mL) and extracted with ethyl acetate (50.0 mL). The organic layer was dried over anhydrous sodium sulfate, filtered, and the filtrate was concentrated to afford 3-(2-(*tert*-butoxy)-2-oxoethyl)-5-chloro-4-methylbenzoic acid (350 mg, crude) as a white solid. It was used directly in the next step.

**Step 6:** To a solution of 3-(2-*tert*-butoxy-2-oxo-ethyl)-5-chloro-4-methyl-benzoic acid (300 mg, 1.05 mmol, 1.00 eq) and triethylamine (0.16 mL, 1.16 mmol, 1.10 eq) in toluene (3.00 mL) was added diphenylphosphoryl azide (0.25 mL, 1.16 mmol, 1.10 eq). The reaction was stirred at 20°C for 10 min, then phenol (0.46 mL, 5.27 mmol, 5.00 eq) was added. The reaction was stirred at 100°C for 30 min. The mixture was concentrated under reduced pressure to give a residue. The residue was purified by silica gel column chromatography (petroleum ether/ethyl acetate = 1/0 to 5/1) to afford *tert*-butyl 2-(3-chloro-2-methyl- 5-((phenoxycarbonyl)amino)phenyl)acetate (150 mg, 399 µmol, 38% yield) as a white solid. **Step 7:** To a solution of 3-(6-(aminomethyl)-1-oxoisoindolin-2-yl)piperidine-2,6-dione hydrochloride **VI** (150 mg, 484 µmol, 1.00 eq, hydrochloride) and triethylamine (135 µL, 969 µmol, 2.00 eq) in dimethylformamide (2.00 mL) was added *tert*-butyl 2-(3-chloro-2-methyl-5-((phenoxycarbonyl)amino)phenyl)acetate (182 mg, 484 µmol, 1.00 eq). The reaction was stirred at 25°C for 12 h. The mixture was purified by reversed phase column chromatography and lyophilized to afford *tert*-butyl 2-(3-chloro-5-(3-((2-(2,6-dioxopiperidin- 3-yl)-3-oxoisoindolin-5-yl)methyl)ureido)-2-methylphenyl)acetate (250 mg, 450 µmol, 93% yield) as a white solid.

**Step 8:** To a solution of *tert*-butyl 2-(3-chloro-5-(3-((2-(2,6-dioxopiperidin-3-yl)-3-oxoisoindolin-5-yl)methyl)ureido)-2-methylphenyl)acetate (240 mg, 432 µmol, 1.00 eq) in dichloromethane (5.00 mL) was added trifluoroacetic acid (5.00 mL). The reaction was stirred at 20°C for 1 h. The mixture was concentrated under reduced pressure to give a residue. The residue was purified by reversed phase preparative HPLC and lyophilized to afford **Compound 120** (81.1 mg, 161 µmol, 37% yield) as a white solid.

¹H NMR (400 MHz, CDCl₃) *□* = 12.42 (br s, 1H), 10.98 (s, 1H), 8.71 (s, 1H), 7.66 (s, 1H), 7.59 (d, *J =* 2.0 Hz, 1H), 7.56 (s, 2H), 7.08 (d, *J =* 1.8 Hz, 1H), 6.79 (br t, *J* = 5.7 Hz, 1H), 5.11 (dd, *J* = 5.3, 13.4 Hz, 1H), 4.48 - 4.27 (m, 4H), 3.60 (s, 2H), 2.97 - 2.84 (m, 1H), 2.62 - 2.58 (m, 1H), 2.41 - 2.37 (m, 1H), 2.17 (s, 3H), 2.04 - 1.93 (m, 1H). MS (ESI) m/z 499.1 [M+H]⁺

**Compound 121:** General procedure A with variant iii) was used for the preparation with a yield of 43% from compound **VI** employing phenyl (3-(pyridin-2-yloxy)phenyl)carbamate.

¹H NMR (400 MHz, DMSO-*d*₆) *□* = 11.00 (s, 1 H), 8.80 (s, 1 H), 8.17 (dd, *J* = 4.9, 1.8 Hz, 1 H), 7.79 - 7.89 (m, 1 H), 7.66 (s, 1 H), 7.56 (s, 2 H), 7.34 (t, *J* = 2.0 Hz, 1 H), 7.20 - 7.29 (m, 1 H), 7.10 - 7.16 (m, 2 H), 7.00 (d, *J* = 8.3 Hz, 1 H), 6.75 - 6.82 (m, 1 H), 6.65 (dd, *J =* 8.0, 1.7 Hz, 1 H), 5.12 (dd, *J =* 13.3, 5.1 Hz, 1 H), 4.28 - 4.47 (m, 4 H), 2.85 - 2.98 (m, 1 H), 2.60 (br d, *J* = 16.5 Hz, 1 H), 2.35 - 2.47 (m, 1 H), 1.94 - 2.05 (m, 1 H). MS (ESI) m/z 486.1 [M+H]⁺

### Scheme for the preparation of phenyl (3-(pyridin-2-yloxy)phenyl)carbamate.

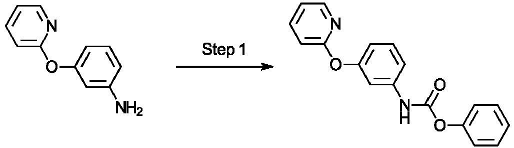

**Step 1:** To a solution of 3-(pyridin-2-yloxy)aniline (0.250 g, 1.34 mmol, 1.00 eq) and triethylamine (0.56 mL, 4.03 mmol, 3.00 eq) in dichloromethane (5.00 mL) was added phenyl chloroformate (185 µL, 1.48 mmol, 1.10 eq) dropwise at 0°C. The reaction was stirred at 20°C for 3 h. Water (5.00 mL) was added, and the organic layer was separated, dried over sodium sulfate, filtered, and concentrated under reduced pressure to give a residue. The residue was purified by silica gel column chromatography (petroleum ether/ethyl acetate = 10/1) to give phenyl (3-(pyridin-2-yloxy)phenyl)carbamate (0.200 g, 653 µmol, 49% yield) as a white solid.

**Compound 122:** General procedure A with variant iii) was used for the preparation with a yield of 68% from compound **VI** employing phenyl (3-chloro-4-methyl-5-morpholinophenyl)carbamate.

¹H NMR (400 MHz, DMSO-*d*₆) *□* = 10.98 (br s, 1H), 8.76 (s, 1H), 7.65 (s, 1H), 7.56 (s, 2H), 7.37 (d, *J =* 2.0 Hz, 1H), 7.00 (d, *J =* 1.8 Hz, 1H), 6.80 (t, *J* = 6.1 Hz, 1H), 5.11 (dd, *J =* 5.1, 13.3 Hz, 1H), 4.48 - 4.35 (m, 3H), 4.34 - 4.25 (m, 1H), 3.76 - 3.66 (m, 4H), 2.98 - 2.84 (m, 1H), 2.82 - 2.71 (m, 4H), 2.68 - 2.55 (m, 1H), 2.39 - 2.31 (m, 1H), 2.19 (s, 3H), 2.06 - 1.91 (m, 1H). MS (ESI) m/z 526.3 [M+H]⁺

### Scheme for the preparation of phenyl (3-chloro-4-methyl-5-morpholinophenyl)carbamate.

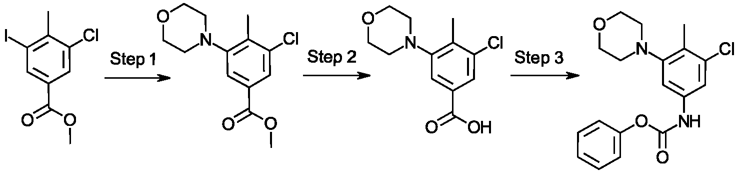

**Step 1:** To a solution of methyl 3-chloro-5-iodo-4-methylbenzoate (4.00 g, 12.9 mmol, 1.00 eq) in toluene (40.0 mL) under nitrogen was added morpholine (1.36 mL, 15.5 mmol, 1.20 eq) and cesium carbonate (21.0 g, 64.4 mmol, 5.00 eq). A separate solution of palladium acetate (289 mg, 1.29 mmol, 0.10 eq) and 2,2'-bis(diphenylphosphino)-1,1'-binaphthalene (802 mg, 1.29 mmol, 0.10 eq) in toluene (20.0 mL) was added. The reaction was stirred at 120°C for 12 hours. The mixture was filtered and concentrated under reduced pressure to give a residue. The residue was purified by silica gel column chromatography (petroleum ether/ethyl acetate = 1/0 to 10/1) to afford methyl 3-chloro-4-methyl-5-morpholinobenzoate (1.35 g, 5.01 mmol, 39% yield) as an orange oil.

**Step 2:** To a solution of methyl 3-chloro-4-methyl-5-morpholinobenzoate (1.55 g, 5.75 mmol, 1.00 eq) in tetrahydrofuran (15.0 mL) and water (5.00 mL) was added lithium hydroxide (275 mg, 11.5 mmol, 2.00 eq). The reaction was stirred at 20°C for 12 h. Water (20.0 mL) was added, and the mixture was extracted with ethyl acetate (2 x 25.0 mL). The pH of the aqueous layer was adjusted to pH = 7 by addition of 1M hydrochloric acid, and it was extracted with ethyl acetate (2 x 30.0 mL). The combined organic layers were dried over anhydrous sodium sulfate, filtered, and concentrated under reduced pressure to give 3-chloro-4-methyl-5-morpholinobenzoic acid (1.30 g, 5.08 mmol, 88 %yield) as a white solid.

**Step 3:** To a solution of 3-chloro-4-methyl-5-morpholinobenzoic acid (600 mg, 2.35 mmol, 1.00 eq) and triethylamine (0.36 mL, 2.58 mmol, 1.10 eq) in toluene (6.00 mL) was added diphenylphosphoryl azide (0.56 mL, 2.58 mmol, 1.10 eq). The reaction was stirred at 20°C for 10 min. Then phenol (1.03 mL, 11.7 mmol, 5.00 eq) was added, and the reaction was stirred at 100°C for 30 min. The mixture was concentrated under reduced pressure to give a residue. The residue was purified by silica gel column chromatography (petroleum ether/ethyl acetate = 1/0 to 3/1) to give phenyl (3-chloro-4-methyl-5-morpholinophenyl)carbamate (695 mg, 2.00 mmol, 85% yield) as a white solid.

**Compound 123:** General procedure A with variant iii) was used for the preparation with a yield of 3 % from compound **VI** employing phenyl pyridin-2-ylcarbamate.

¹H NMR (400 MHz, DMSO-*d₆*) *□* = 11.20 (s, 1H), 10.97 (s, 1H), 8.30 (s, 1H), 8.23 (dd, *J* = 1.2, 5.6 Hz, 1H), 8.09 (t, *J* = 8.0 Hz, 1H), 7.69 (s, 1H), 7.62-7.55 (m, 2H), 7.41 (d, *J* = 8.8 Hz, 1H), 7.22 (t, *J* = 6.4 Hz, 1H), 5.10 (dd, *J* = 4.8 Hz, 13.2 Hz, 1H), 4.50 (m, *J* = 5.6 Hz, 2H), 4.37 (dd, *J =* 17.2, 54.0 Hz, 2H), 2.90 (ddd, *J* = 5.2, 13.6, 17.6 Hz, 1H), 2.63-2.55 (m, 1H), 2.45-2.33 (m, 1H), 2.03-1.94 (m, 1H). LCMS m/z 394.0 [M+H]⁺

### Scheme for the preparation of phenyl pyridin-2-ylcarbamate.

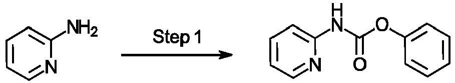

To a solution of pyridin-2-amine (2.00 g, 21.3 mmol, 1.00 eq) in tetrahydrofuran (50.0 mL) was added phenyl chloroformate (3.66 g, 23.4 mmol, 1.10 eq) and triethylamine (4.30 g, 42.5 mmol, 2.00 eq). The reaction was stirred at 25°C for 0.5 h. The mixture was concentrated under reduced pressure to give a residue. The residue was purified by reversed phase column chromatography to afford phenyl pyridin-2-ylcarbamate (220 mg, 975 µmol, 4% yield) as a white solid.

**Compound 124:** General procedure A with variant iii) was used for the preparation with a yield of 80% from compound **VI** employing phenyl (3-chloro-4-methyl-5-((4-methylpiperazin-1-yl)methyl)phenyl)carbamate.

¹H NMR (400 MHz, DMSO-*d*₆) *□* = 10.99 (s, 1H), 8.80 (br s, 1H), 8.25 - 8.14 (m, 1 H), 7.74 - 7.62 (m, 2H), 7.56 (s, 2H), 7.11 (d, *J* = 1.8 Hz, 1H), 6.83 (br s, 1H), 5.12 (dd, *J =* 5.0, 13.2 Hz, 1H), 4.55 - 4.23 (m, 4H), 3.38 (s, 2H), 3.00 - 2.85 (m, 1H), 2.69 - 2.56 (m, 2H), 2.47 - 2.29 (m, 8H), 2.25 (s, 3H), 2.19 (s, 3H), 2.07 - 1.94 (m, 1H). MS (ESI) m/z 553.3 [M+H]⁺

### Scheme for the preparation of phenyl (3-chloro-4-methyl-5-((4-methylpiperazin-1-yl)methyl)phenyl)carbamate.

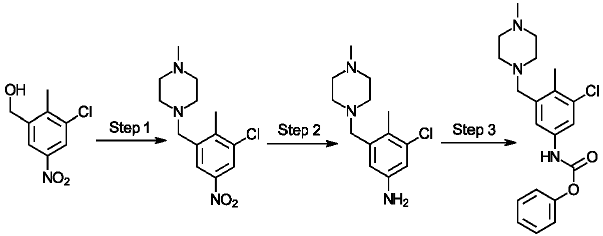

**Step 1:** To a solution of (3-chloro-2-methyl-5-nitrophenyl)methanol (2.00 g, 9.92 mmol, 1.00 eq) in dichloromethane (20.0 mL) was added thionyl chloride (5.90 g, 49.6 mmol, 3.60 mL, 5.00 eq) dropwise. The reaction was stirred at 25°C for 2 h. The mixture was concentrated under reduced pressure to afford 1-chloro-3-(chloromethyl)-2-methyl-5-nitrobenzene (2.20 g, 10.0 mmol, crude) as a gray solid. To a solution of 1-chloro-3-(chloromethyl)-2-methyl-5-nitrobenzene (2.20 g, 10.00 mmol, 1.00 eq) and triethylamine (3.48 mL, 25.0 mmol, 2.50 eq) in acetonitrile (20.0 mL) was added 1-methylpiperazine (1.44 mL, 13.0 mmol, 1.30 eq). The reaction was stirred at 25°C for 10 h. The mixture was diluted with water (6.00 mL) and extracted with ethyl acetate (3 x 25.0 mL). The combined organic layers were washed with brine (10.0 mL), dried over sodium sulfate, filtered, and concentrated under reduced pressure to give a residue. The residue was purified by silica gel column chromatography (petroleum ether/ethyl acetate = 1/0 to 1/1) to afford 1-(3-chloro-2-methyl-5-nitrobenzyl)-4-methylpiperazine (0.70 g, 2.47 mmol, 25% yield) as a yellow solid.

**Step 2:** A mixture of 1-(3-chloro-2-methyl-5-nitrobenzyl)-4-methylpiperazine (0.70 g, 2.47 mmol, 1.00 eq), ammonium chloride (132 mg, 2.47 mmol, 1.00 eq) and ferrous powder (689 mg, 12.3 mmol, 5.00 eq) in ethanol (10.0 mL) and water (5.00 mL) was stirred at 90°C for 10 h. The reaction was filtered and concentrated under reduced pressure to give a residue. The residue was purified was by reversed phase column chromatography and lyophilized to afford 3-chloro-4-methyl-5-((4-methylpiperazin-1-yl)methyl)aniline (0.60 g, 2.36 mmol, 96% yield) as a gray solid.

**Step 3:** To a solution of 3-chloro-4-methyl-5-((4-methylpiperazin-1-yl)methyl)aniline (0.30 g, 1.18 mmol, 1.00 *eq*) and potassium carbonate (326 mg, 2.36 mmol, 2.00 *eq*) in acetone (5.00 mL) was added phenyl chloroformate (0.22 mL, 1.77 mmol, 1.50 eq) dropwise at 0°C. The reaction was stirred at 25°C for 3 h. The mixture was concentrated under reduced pressure to give a residue. The residue was purified was by reversed phase column chromatography and lyophilized to afford phenyl (3-chloro-4-methyl-5-((4-methylpiperazin-1-yl)methyl)phenyl) carbamate (0.25 g, 669 µmol, 57% yield) as a gray solid.

**Compound 125:** General procedure A with variant iii) was used for the preparation with a yield of 58% from compound **VI** employing phenyl (3-chloro-5-(2-methoxyethoxy)-4-methylphenyl)carbamate.

¹H NMR (400 MHz, DMSO-*d*₆) *□* = 10.98 (br s, 1H), 8.80 (s, 1H), 7.66 (s, 1H), 7.57 (s, 2H), 7.19 (d, *J* = 1.7 Hz, 1H), 7.01 (d, *J* = 1.6 Hz, 1H), 6.88 (br t, *J* = 6.0 Hz, 1H), 5.12 (dd, *J* = 5.0, 13.3 Hz, 1H), 4.50 - 4.26 (m, 4H), 4.12 - 3.98 (m, 2H), 3.75 - 3.61 (m, 2H), 3.33 (br s, 3H), 3.01 - 2.83 (m, 1H), 2.68 - 2.59 (m, 1H), 2.44 - 2.38 (m, 1H), 2.11 (s, 3H), 2.06 - 1.96 (m, 1H). MS (ESI) m/z 515.1 [M+H]⁺

### Scheme for the preparation of phenyl (3-chloro-5-(2-methoxyethoxy)-4-methylphenyl)carbamate.

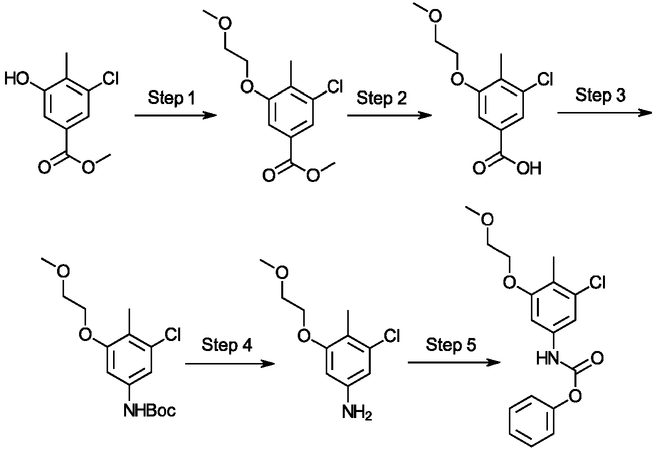

**Step 1:** A mixture of methyl 3-chloro-5-hydroxy-4-methyl-benzoate (1.00 g, 4.98 mmol, 1.00 eq), 1-bromo-2-methoxy-ethane (0.94 mL, 9.97 mmol, 2.00 eq) and potassium carbonate (2.76 g, 19.9 mmol, 4.00 eq) in acetonitrile (20.0 mL) was stirred at 85°C for 12 h. The mixture was filtered, and the filtrate was concentrated under reduced pressure to give a residue. The residue was purified by silica gel column chromatography (petroleum ether/ethyl acetate = 20/1 to 5/1) to give methyl 3-chloro-5-(2-methoxyethoxy)-4-methylbenzoate (1.10 g, 4.25 mmol, 85% yield) as a yellow solid.

**Step 2:** A mixture of methyl 3-chloro-5-(2-methoxyethoxy)-4-methyl-benzoate (1.00 g, 3.87 mmol, 1.00 eq) and sodium hydroxide (309 mg, 7.73 mmol, 2.00 eq) in methanol (10.0 mL) and water (10.0 mL) was stirred at 70°C for 12 h. The mixture was concentrated under reduced pressure to give a residue. The residue was diluted with water (10.0 mL), and the pH was adjusted to pH = 3 with hydrogen chloride (1N). The mixture was extracted with ethyl acetate (3 x 10.0 mL). The combined organic layers were washed with brine (3 x 10.0 mL), dried with anhydrous sodium sulfate, filtered, and concentrated under reduced pressure to afford 3-chloro-5-(2-methoxyethoxy)-4-methyl-benzoic acid (850 mg, 3.47 mmol, 89% yield) as a yellow solid.

**Step 3:** A solution of 3-chloro-5-(2-methoxyethoxy)-4-methyl-benzoic acid (650 mg, 2.66 mmol, 1.00 eq), diphenyl phosphoryl azide (0.86 mL, 3.98 mmol, 1.50 eq), and triethylamine (0.74 mL, 5.31 mmol, 2.00 eq) in 2-methylpropan-2-ol (10.0 mL) was stirred at 100°C for 12 h. The mixture was diluted with water (50.0 mL) and extracted with ethyl acetate (3 x 20.0 mL). The combined organic layers were washed with brine (3 x 10.0 mL), dried with anhydrous sodium sulfate, filtered, and concentrated under reduced pressure to give a residue. The residue was purified by silica gel column chromatography (petroleum ether/ethyl acetate = 10/1 to 3/1) to give tert-butyl (3-chloro-5-(2-methoxyethoxy)-4-methylphenyl)carbamate (700 mg, 2.22 mmol, 83% yield) as a yellow oil.

**Step 4:** To a solution of tert-butyl (3-chloro-5-(2-methoxyethoxy)-4-methylphenyl)carbamate (650 mg, 2.06 mmol, 1.00 eq) in hydrogen chloride/ethyl acetate (2.00 mL) was stirred at 25°C for 1 h. The mixture was concentrated under reduced pressure to afford 3-chloro-5-(2-methoxyethoxy)-4-methyl-aniline hydrochloride (500 mg, 1.98 mmol, 96% yield, hydrochloride) as a yellow solid.

**Step 5:** To a solution of 3-chloro-5-(2-methoxyethoxy)-4-methyl-aniline hydrochloride (430 mg, 1.99 mmol, 1.00 eq, hydrochloride) and triethylamine (555 µL, 3.99 mmol, 2.00 eq) in dichloromethane (10.0 mL) was added phenyl chloroformate (0.30 mL, 2.39 mmol, 1.20 eq) dropwise at 0°C. The reaction was stirred at 25°C for 0.5 h. The mixture was concentrated under reduced pressure to give a residue. The residue was purified by silica gel column chromatography (petroleum ether/ethyl acetate = 10/1 to 1/1) to afford phenyl (3-chloro-5-(2-methoxyethoxy)-4-methylphenyl)carbamate (300 mg, 893 µmol, 44% yield) as a yellow solid.

**Compound 126:** General procedure A with variant iii) was used for the preparation with a yield of 31% from compound **VI** employing phenyl (3-chloro-4-methyl-5-((4-morpholinopiperidin-1-yl)methyl)phenyl)carbamate.

¹H NMR (400 MHz, DMSO-*d*₆) *□* = 10.99 (s, 1H), 8.83 (s, 1H), 8.18 (s, 1H), 7.69 - 7.61 (m, 2H), 7.56 (d, *J* = 0.8 Hz, 2H), 7.12 (d, *J =* 2.0 Hz, 1H), 6.88 - 6.80 (m, 1H), 5.12 (dd, *J =* 5.2, 13.2 Hz, 1H), 4.46 - 4.25 (m, 4H), 3.64 - 3.48 (m, 4H), 3.37 (s, 2H), 2.98 - 2.77 (m, 3H), 2.62 - 2.57 (m, 1H), 2.46 (br d, *J* = 3.8 Hz, 4H), 2.40 (br dd, *J =* 4.4, 13.2 Hz, 1H), 2.24 (s, 3H), 2.15 (br t, *J* = 10.8 Hz, 1H), 2.05 - 1.89 (m, 3H), 1.74 (br d, *J* = 11.2 Hz, 2H), 1.45 - 1.29 (m, 2H). MS (ESI) m/z 623.1 [M+H]⁺

### Scheme for the preparation of phenyl (3-chloro-4-methyl-5-((4-morpholinopiperidin-1-yl)methyl)phenyl)carbamate.

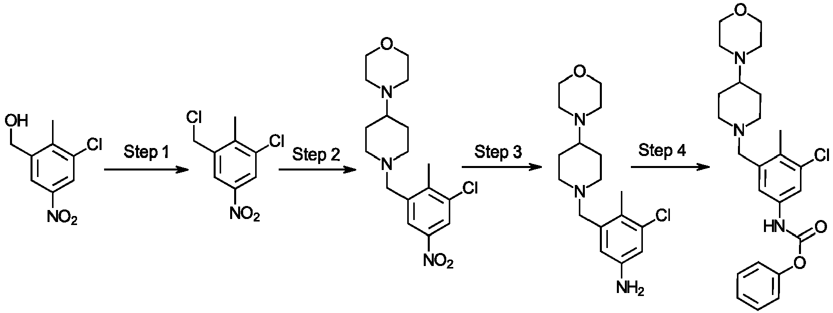

**Step 1:** To a solution of (3-chloro-2-methyl-5-nitrophenyl)methanol (1.00 g, 4.96 mmol, 1.00 eq) in dichloromethane (15.0 mL) was added thionyl chloride (2.95 g, 24.8 mmol, 1.80 mL, 5.00 eq) at 0°C. Then the reaction was stirred at 25°C for 2 h. The mixture was concentrated under reduced pressure to give 1-chloro-3-(chloromethyl)-2-methyl-5-nitrobenzene (1.10 g, 4.96 mmol, crude) as yellow oil. It was used directly in the next step.

**Step 2:** To a solution of 1-chloro-3-(chloromethyl)-2-methyl-5-nitrobenzene (1.10 g, 5.00 mmol, 1.00 eq) and triethylamine (1.26 g, 12.5 mmol, 1.74 mL, 2.50 eq) in acetonitrile (10.0 mL) was added 4-(piperidin-4-yl)morpholine (1.06 g, 6.25 mmol, 1.25 eq). The reaction was stirred at 25°C for 10 h. The mixture was concentrated under reduced pressure to give a residue. The residue was purified was by reversed phase column chromatography and lyophilized to afford 4-(1-(3-chloro-2-methyl-5-nitrobenzyl)piperidin-4-yl)morpholine (1.00 g, 2.83 mmol, 57% yield) as a yellow solid.

**Step 3:** A mixture of 4-(1-(3-chloro-2-methyl-5-nitrobenzyl)piperidin-4-yl)morpholine (1.00 g, 2.83 mmol, 1.00 eq), ammonium chloride (151 mg, 2.83 mmol, 1.00 eq) and ferrous powder (789 mg, 14.1 mmol, 5.00 eq) in ethanol (20.0 mL) and water (10.0 mL) was stirred at 90°C for 10 h. The mixture was filtered and concentrated under reduced pressure to afford 3-chloro-4-methyl-5-((4-morpholinopiperidin-1-yl)methyl)aniline (0.950 g, 2.83 mmol, crude) as yellow oil. It was used directly in the next step.

**Step 4:** To a solution of 3-chloro-4-methyl-5-((4-morpholinopiperidin-1-yl)methyl)aniline (0.92 g, 2.84 mmol, 1.00 eq) and potassium carbonate (785 mg, 5.68 mmol, 2.00 eq) in acetone (10.0 mL) was added phenyl chloroformate (0.53 mL, 4.26 mmol, 1.50 eq) dropwise. The reaction was stirred at 25°C for 10 h. The mixture was diluted with water (10.0 mL) and extracted with ethyl acetate (3 x 35.0 mL). The combined organic layers were washed with brine (15.0 mL), dried over sodium sulfate, filtered, and concentrated under reduced pressure to afford phenyl (3-chloro-4-methyl-5-((4-morpholinopiperidin-1-yl) methyl)phenyl)carbamate (1.50 g, 2.84 mmol, crude) as yellow oil. It was used directly in the next step.

**Compound 127:** General procedure A with variant iii) was used for the preparation with a yield of 34% from compound **VI** employing phenyl (3-chloro-4-methyl-5-(2-morpholinoethoxy) phenyl)carbamate.

¹H NMR (400 MHz, DMSO-*d*₆) □ = 11.0 (s, 1H), 10.8 - 10.6 (m, 1H), 9.03 (s, 1H), 7.66 (s, 1H), 7.57 (s, 2H), 7.20 - 7.11 (m, 2H), 6.99 (br t, *J =* 6.0 Hz, 1H), 5.11 (dd, *J* = 5.1, 13.3 Hz, 1H), 4.49 - 4.22 (m, 6H), 3.99 (br d, *J* = 12.5 Hz, 2H), 3.76 (br t, *J =* 11.9 Hz, 2H), 3.60 (br d, *J* = 1.1 Hz, 2H), 3.50 (br d, *J* = 12.6 Hz, 2H), 3.27 - 3.15 (m, 2H), 3.00 - 2.85 (m, 1H), 2.71 - 2.58 (m, 1H), 2.40 - 2.30 (m, 1H), 2.16 (s, 3H), 2.03 - 1.94 (m, 1H). MS (ESI) m/z 570.3 [M+H]⁺

### Scheme for the preparation of phenyl (3-chloro-4-methyl-5-(2-morpholinoethoxy)phenyl)carbamate.

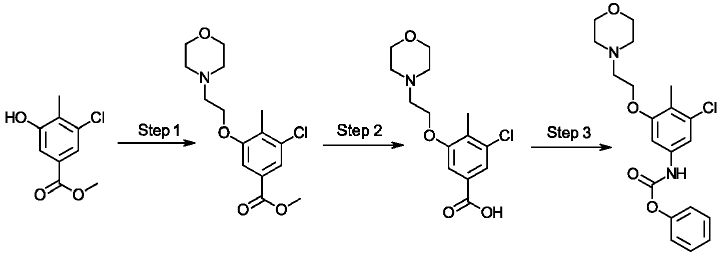

**Step** 1: To a solution of methyl 3-chloro-5-hydroxy-4-methylbenzoate (600 mg, 3.00 mmol, 1.00 eq) in acetonitrile (6.00 mL) was added potassium iodide (49.7 mg, 0.30 mmol, 0.10 eq), potassium carbonate (1.65 g, 12.0 mmol, 4.00 eq) and 4-(2-chloroethyl)morpholine hydrochloric acid (1.11 g, 5.98 mmol, 2.00 eq, hydrochloric acid). The reaction was stirred at 80°C for 12 h. The mixture was filtered and concentrated under reduced pressure to give a residue. The residue was purified by silica gel chromatography (dichloromethane/methanol = 1/0 to 10/1) to afford methyl 3-chloro-4-methyl-5-(2-morpholinoethoxy)benzoate (820 mg, 2.61 mmol, 87% yield) as a white solid.

**Step 2:** To a solution of methyl 3-chloro-4-methyl-5-(2-morpholinoethoxy)benzoate (770 mg, 2.45 mmol, 1.00 eq) in tetrahydrofuran (3.00 mL) and water (1.00 mL) was added lithium hydroxide monohydrate (309 mg, 7.36 mmol, 3.00 eq). The reaction was stirred at 20°C for 12 h. The mixture was concentrated under reduced pressure to give a residue. The residue was purified by reversed phase column chromatography and lyophilized to give a residue. Water (20 mL) and hydrochloric acid (0.20 mL) were added to the residue, and it was lyophilized to give 3-chloro-4-methyl-5-(2-morpholinoethoxy)benzoic acid (650 mg, 2.17 mmol, 88% yield) as a white solid.

**Step 3:** To a solution of 3-chloro-4-methyl-5-(2-morpholinoethoxy)benzoic acid (600 mg, 2.00 mmol, 1.00 eq) in toluene (6.00 mL) was added triethylamine (446 mg, 4.40 mmol, 2.20 eq) and diphenylphosphoryl azide (606 mg, 2.20 mmol, 1.10 eq). The reaction was stirred at 20°C for 10 min, then phenol (942 mg, 10.0 mmol, 5.00 eq) was added. The reaction was stirred at 100°C for 30 min. The mixture was concentrated under reduced pressure to give a residue. The residue was purified by silica gel column chromatography (petroleum ether/ethyl acetate = 1/0 to 0/1) to give phenyl (3-chloro-4-methyl-5-(2-morpholinoethoxy)phenyl)carbamate (80.0 mg, 205 µmol, 10% yield) as a yellow oil.

**Compound 128:** General procedure A with variant iii) was used for the preparation with a yield of 46% from compound **VI** employing phenyl (1-methyl-1H-pyrrol-3-yl)carbamate.

¹H NMR (400 MHz, DMSO-*d*₆) □ = 10.97 (s, 1H), 7.99 (s, 1H), 7.63 (s, 1H), 7.53 (s, 2H), 6.71 (t, *J =* 2.0 Hz, 1H), 6.46 (t, *J* = 2.5 Hz, 1H), 6.42 (br t, *J* = 5.9 Hz, 1H), 5.80 - 5.75 (m, 1H), 5.11 (dd, *J* = 5.1*,* 13.3 Hz, 1H), 4.47 - 4.25 (m, 4H), 3.51 (s, 3H), 2.97 - 2.84 (m, 1H), 2.63 - 2.55 (m, 1H), 2.39 (dd, *J* = 4.4, 13.1 Hz, 1H), 2.03 - 1.95 (m, 1H). MS (ESI) m/z 396.1 [M+H]⁺

### Scheme for the preparation of phenyl (1-methyl-1H-pyrrol-3-yl)carbamate.

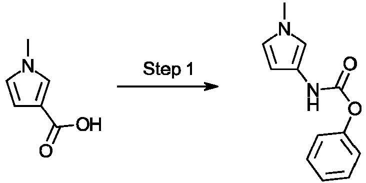

**Step 1:** To a solution of 1-methyl-1H-pyrrole-3-carboxylic acid (700 mg, 5.59 mmol, 1.00 eq) in toluene (7.00 mL) was added diphenylphosphoryl azide (1.33 mL, 6.15 mmol, 1.10 eq) and triethylamine (0.86 mL, 6.15 mmol, 1.10 eq). The reaction was stirred at 20°C for 10 min, then phenol (2.46 mL, 28.0 mmol, 5.00 eq) was added and the reaction was stirred at 100°C for 30 min. The mixture was concentrated under reduced pressure to give a residue. The residue was purified by silica gel column chromatography (petroleum ether/ethyl acetate = 1/0 to 5/1) to give phenyl (1-methyl-1H-pyrrol-3-yl)carbamate (350 mg, 1.62 mmol, 29% yield) as a pink solid.

**Compound 129:** General procedure A with variant iii) was used for the preparation with a yield of 46% from compound **VI** employing phenyl (3-chloro-5-(dimethylamino)-4-methylphenyl)carbamate.

¹H NMR (400 MHz, DMSO-*d*₆) *□* = 10.97 (br s, 1H), 8.78 (br s, 1H), 7.65 (s, 1H), 7.56 (s, 2H), 7.32 (d, *J =* 2.0 Hz, 1H), 7.00 (d, *J =* 2.0 Hz, 1H), 6.82 (br d, *J* = 2.9 Hz, 1H), 5.11 (dd, *J* = 5.1, 13.3 Hz, 1H), 4.47 - 4.27 (m, 4H), 2.97 - 2.85 (m, 1H), 2.64 - 2.60 (m, 1H), 2.59 (s, 6H), 2.39 (dd, *J* = 4.4, 13.0 Hz, 1H), 2.19 (s, 3H), 2.04 - 1.96 (m, 1H). MS (ESI) m/z 484.2 [M+H]⁺

### Scheme for the preparation of phenyl (3-chloro-5-(dimethylamino)-4-methylphenyl)carbamate.

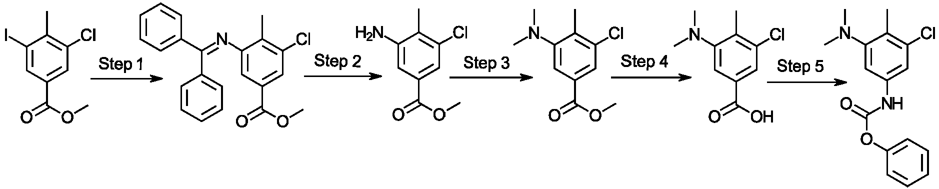

**Step 1:** To a solution of methyl 3-chloro-5-iodo-4-methylbenzoate (9.00 g, 29.0 mmol, 1.00 eq) in toluene (180 mL) was added 2,2'-bis(diphenylphosphino)-1,1'-binaphthalene (5.41 g, 8.70 mmol, 0.30 eq), diphenylmethanimine (5.84 mL, 34.8 mmol, 1.20 eq), sodium tert-butoxide (3.90 g, 40.6 mmol, 1.40 eq) and tris(dibenzylideneacetone)dipalladium (2.65 g, 2.90 mmol, 0.10 eq). The reaction was stirred at 80°C for 12 h under nitrogen. The mixture was filtered and concentrated under reduced pressure to give a residue. The residue was purified by silica gel column chromatography (petroleum ether/ethyl acetate = 1/0 to 10/1) to give methyl 3-chloro-5-((diphenylmethylene)amino)-4-methylbenzoate (6.50 g, 17.9 mmol, 62% yield) as yellow oil.

**Step 2:** To a solution of methyl 3-chloro-5-((diphenylmethylene)amino)-4-methylbenzoate (6.50 g, 17.9 mmol, 1.00 eq) in tetrahydrofuran (65.0 mL) was added hydrochloric acid (1.00 M, 17.9 mL, 1.00 eq). The reaction was stirred at 20°C for 2 h. Water (60.0 mL) was added, followed by saturated sodium bicarbonate until pH = 8: The mixture was extracted with ethyl acetate (3 × 60.0 mL). The organic phases were gathered, dried over sodium sulfate, filtered, and concentrated under reduced pressure to give a residue. The residue was purified by silica gel column chromatography (petroleum ether/ ethyl acetate = 1/0 to 5/1) to give methyl 3-amino-5-chloro-4-methylbenzoate (2.86 g, 14.3 mmol, 80% yield) as a yellow solid.

**Step 3:** To a solution of methyl 3-amino-5-chloro-4-methylbenzoate (2.86 g, 14.3 mmol, 1.00 eq) in dimethylformamide (30.0 mL) was added iodomethane (2.68 mL, 43.0 mmol, 3.00 eq) and potassium carbonate (7.92 g, 57.3 mmol, 4.00 eq). The reaction was stirred at 80°C for 5 h. Water (40.0 mL) was added, and the mixture was extracted with ethyl acetate (3 × 50.0 mL). The organic layers were gathered, dried over sodium sulfate, filtered, and concentrated under reduced pressure to give a residue. The residue was purified by silica gel column chromatography (petroleum ether/ethyl acetate = 1/0 to 5/1) to give methyl 3-chloro-5-(dimethylamino)-4-methylbenzoate (2.45 g, 10.8 mmol, 75% yield) as transparent oil.

**Step 4:** To a solution of methyl 3-chloro-5-(dimethylamino)-4-methylbenzoate (2.30 g, 10.1 mmol, 1.00 eq) in tetrahydrofuran (18.0 mL) and water (6.00 mL) was added lithium hydroxide (484 mg, 20.2 mmol, 2.00 eq). The reaction was stirred at 20°C for 12 h. Water (20.0 mL) was added, and the mixture was extracted with ethyl acetate (2 × 40.0 mL). 1M hydrochloric acid was added to the aqueous layer until pH = 7, and it was extracted with ethyl acetate (2 × 40.0 mL). The organic layers were gathered, dried over sodium sulfate, filtered, and concentrated under reduced pressure to afford 3-chloro-5-(dimethylamino)-4-methylbenzoic acid (1.90 g, 8.89 mmol, 88% yield) as a white solid.

**Step 5:** To a solution of 3-chloro-5-(dimethylamino)-4-methylbenzoic acid (700 mg, 3.28 mmol, 1.00 eq) and triethylamine (0.50 mL, 3.60 mmol, 1.10 eq) in toluene (7.00 mL) was added diphenylphosphoryl azide (0.78 mL, 3.60 mmol, 1.10 eq). The reaction was stirred at 20°C for 10 min, then phenol (1.44 mL, 16.4 mmol, 5.00 eq) was added. The reaction was stirred at 100°C for 30 min. The mixture was concentrated under reduced pressure to give a residue. The residue was purified by silica gel column chromatography (petroleum ether/ethyl acetate = 1/0 to 5/1) to give phenyl (3-chloro-5-(dimethylamino)-4-methylphenyl)carbamate (1 g, crude) as transparent oil. It was used directly in the next reaction.

**Compound 130:** General procedure A with variant iii) was used for the preparation with a yield of 50% from compound **VI** employing phenyl N-[3-[(1-methyl-4-piperidyl)oxy]phenyl]carbamate.

¹H NMR (DMSO-*d*₆) □ = 10.96 (s, 1H), 8.61 (br s, 1H), 8.16 (s, 1H), 7.65 (s, 1H), 7.55 (s, 2H), 7.17 (s, 1H), 7.12 - 7.05 (m, 1H), 6.84 (d, *J* = 8.4 Hz, 1H), 6.75 (br s, 1H), 6.48 (dd, *J =* 8.2, 2.2 Hz, 1H), 5.11 (dd, *J* = 13.3, 5.2 Hz, 1H), 4.46 - 4.37 (m, 3H), 4.30 (br d, *J* = 17.0 Hz, 2H), 2.96 - 2.85 (m, 1H), 2.67 - 2.55 (m, 3H), 2.39 (br dd, *J* = 13.1, 4.5 Hz, 1H), 2.30 - 2.23 (m, 2H), 2.27 - 2.23 (m, 2H), 2.21 (br s, 3H), 2.03 - 1.95 (m, 1H), 1.90 (br s, 2H), 1.63 (br d, *J =* 8.2 Hz, 2H). MS (ESI) m/z 506.4[M+H]⁺

### Scheme for the preparation of phenyl N-[3-[(1-methyl-4-piperidyl)oxy]phenyl]carbamate.

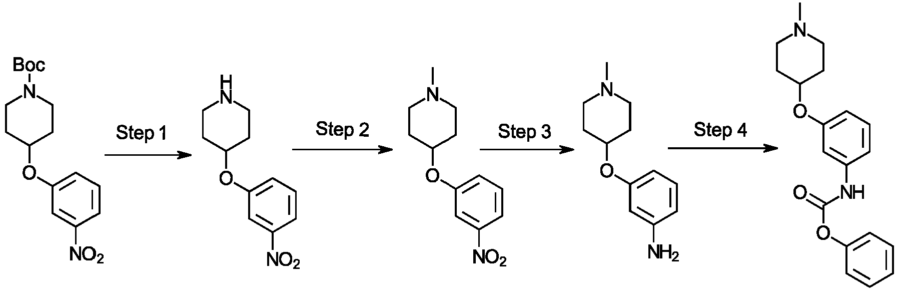

**Step 1:** A solution of tert-butyl 4-(3-nitrophenoxy)piperidine-1-carboxylate (800 mg, 2.48 mmol, 1.00 eq) in hydrochloric acid/ethyl acetate (10.0 mL) was stirred at 25°C for 0.5 h. The mixture was concentrated under reduced pressure to afford 4-(3-nitrophenoxy)piperidine (630 mg, crude) as a white solid. It was used directly in the next reaction.

**Step 2:** To a solution of 4-(3-nitrophenoxy)piperidine (500 mg, 2.25 mmol, 1.00 eq) in methanol (20.0 mL) was added formaldehyde (37% purity, 0.50 mL, 6.75 mmol, 3.00 eq) and sodium cyanoborohydride (424 mg, 6.75 mmol, 3.00 eq). The reaction was stirred at 25°C for 4 h. The mixture was concentrated under reduced pressure to give a residue. Water (10.0 mL) was added and the solution was extracted with dichloromethane (3 × 20.0 mL). The combined organic layers were washed with brine (3 × 10.0 mL), dried over anhydrous sodium sulfate, filtered, and concentrated under reduced pressure to give a residue. The residue was purified by preparative TLC (dichloromethane/methanol = 10/1) to afford 1-methyl-4- (3-nitrophenoxy) piperidine (330 mg, 1.40 mmol, 62% yield) as a yellow oil.

**Step 3:** To a solution of 1-methyl-4-(3-nitrophenoxy)piperidine (320 mg, 1.35 mmol, 1.00 eq) in tetrahydrofuran (5.00 mL) was added Pd/C 10% weight on C (100 mg, 1.00 eq). The reaction was stirred under hydrogen atmosphere (15 psi) at 20°C for 4 h. The mixture was filtered, and the filtrate was concentrated under reduced pressure to afford 3-[(1 -methyl-4-piperidyl)oxy]aniline (270 mg, 1.31 mmol, 97% yield) as a yellow solid.

**Step 4:** To a solution of 3-[(1-methyl-4-piperidyl)oxy]aniline (270 mg, 1.31 mmol, 1.00 eq) in dichloromethane (10.0 mL) was added pyridine (0.32 mL, 3.93 mmol, 3.00 eq) at 0°C, then phenyl chloroformate (0.18 mL, 1.44 mmol, 1.10 eq) was added. The reaction was stirred at 20°C for 6 h. The mixture was concentrated under reduced pressure to give a residue. The residue was diluted with water (15.0 mL) and extracted with dichloromethane (3 × 20.0 mL). The combined organic layers were washed with brine (3 × 10.0 mL), dried over anhydrous sodium sulfate, filtered, and concentrated under reduced pressure to give a residue. The residue was purified by preparative TLC (dichloromethane/methanol = 10/1) to afford phenyl N-[3-[(1-methyl-4-piperidyl)oxy] phenyl]carbamate (230 mg, 704 µmol, 54% yield) as a yellow solid.

**Compound 131:** General procedure A with variant iii) was used for the preparation with a yield of 50% from compound **VI** employing phenyl (4-(2-hydroxypropan-2-yl)phenyl)carbamate.

¹H NMR (400 MHz, DMSO-*d*₆) *□* = 10.97 (br s, 1H), 8.56 (s, 1H), 7.67 (s, 1H), 7.57 (s, 2H), 7.31 (s, 4H), 6.73 (br t, *J* = 6.0 Hz, 1H), 5.12 (dd, *J* = 5.0, 13.2 Hz, 1H), 4.85 (s, 1H), 4.50 - 4.23 (m, 4H), 3.02 - 2.84 (m, 1H), 2.66 - 2.56 (m, 1H), 2.44 - 2.34 (m, 1H), 2.05 - 1.96 (m, 1H), 1.39 (s, 6H). MS (ESI) m/z 449.5 [M-H]⁻

### Scheme for the preparation of phenyl (4-(2-hydroxypropan-2-yl)phenyl)carbamate.

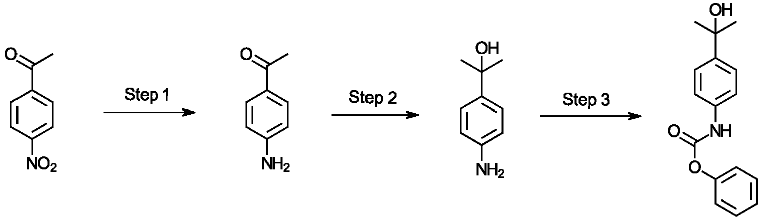

**Step 1:** To a mixture of 1-(4-nitrophenyl)ethanone (2.00 g, 12.1 mmol, 1.00 eq) in ethanol (18.0 mL) and water (9.00 mL) was added ammonium chloride (648 mg, 12.1 mmol, 1.00 eq) and ferrous powder (3.38 g, 60.6 mmol, 5.00 eq). The reaction was stirred at 90°C for 10 h. The mixture was filtered, and the filtrate was extracted with ethyl acetate (3 × 30.0 mL). The combined organic layers were washed with brine (10.0 mL), dried over sodium sulfate, filtered, and concentrated under reduced pressure to afford 1-(4-aminophenyl)ethanone (1.60 g, 11.8 mmol, 98% yield) as a yellow solid.

**Step 2:** To a solution of 1-(4-aminophenyl)ethanone (1.60 g, 11.8 mmol, 1.00 eq) in tetrahydrofuran (20.0 mL) was added methylmagnesium bromide (3.00 M, 11.8 mL, 3.00 eq) dropwise at 0°C. Then the reaction was stirred at 25°C for 10 h. The mixture was quenched by addition saturated ammonium chloride (15.0 mL), diluted with water (10.0 mL), and extracted with ethyl acetate (3 × 55.0 mL). The combined organic layers were washed with brine (25.0 mL), dried over sodium sulfate, filtered, and concentrated under reduced pressure to give a residue. The residue was purified by preparative reversed phase preparative HPLC to afford 2-(4-aminophenyl)propan-2-ol (0.45 g, 2.98 mmol, 25% yield) as a yellow solid.

**Step 3:** To a solution of 2-(4-aminophenyl)propan-2-ol (0.45 g, 2.98 mmol, 1.00 eq) and 2,6-dimethylpyridine (0.38 mL, 3.27 mmol, 1.10 eq) in tetrahydrofuran (3.00 mL) and trichloromethane (3.00 mL) was added phenyl chloroformate (0.37 mL, 2.98 mmol, 1.00 eq) at 0°C. The reaction was stirred at 0°C for 1 h. The mixture was diluted with water (10.0 mL) and extracted with dichloromethane (3 × 35.0 mL). The combined organic layers were washed with brine (10.0 mL), dried over sodium sulfate, filtered and concentrated under reduced pressure to afford phenyl (4-(2-hydroxypropan-2-yl)phenyl)carbamate (0.80 g, 2.98 mmol, crude) as a white solid. It was used directly in the next reaction.

**Compound 132:** General procedure A with variant iii) was used for the preparation with a yield of 46% from compound **VI** employing phenyl (3-((1,3-dioxan-5-yl)methyl)-5-chloro-4-methylphenyl)carbamate.

¹H NMR (400 MHz, DMSO-*d*₆) *□* = 10.98 (s, 1H), 8.77 (s, 1H), 7.66 (s, 1H), 7.57 (s, 3H), 7.02 (d, *J =* 2.0 Hz, 1H), 6.86 (t, *J =* 6.0 Hz, 1H), 5.12 (dd, *J* = 5.2, 13.2 Hz, 1H), 4.83 (d, *J =* 6.0 Hz, 1H), 4.69 (d, *J* = 6.0 Hz, 1H), 4.49 - 4.26 (m, 4H), 3.86 (dd, *J* = 3.8, 11.2 Hz, 2H), 3.51 (dd, *J* = 7.6, 11.2 Hz, 2H), 2.99 - 2.83 (m, 1H), 2.66 - 2.54 (m, 3H), 2.40 (dq, *J =* 4.4, 13.2 Hz, 1H), 2.22 (s, 3H), 2.05 - 1.97 (m, 1H), 1.93 (dt, *J* = 3.6, 7.4 Hz, 1H). MS (ESI) m/z 541.3 [M+H]⁺

### Scheme for the preparation of phenyl (3-((1,3-dioxan-5-yl)methyl)-5-chloro-4-methylphenyl)carbamate.

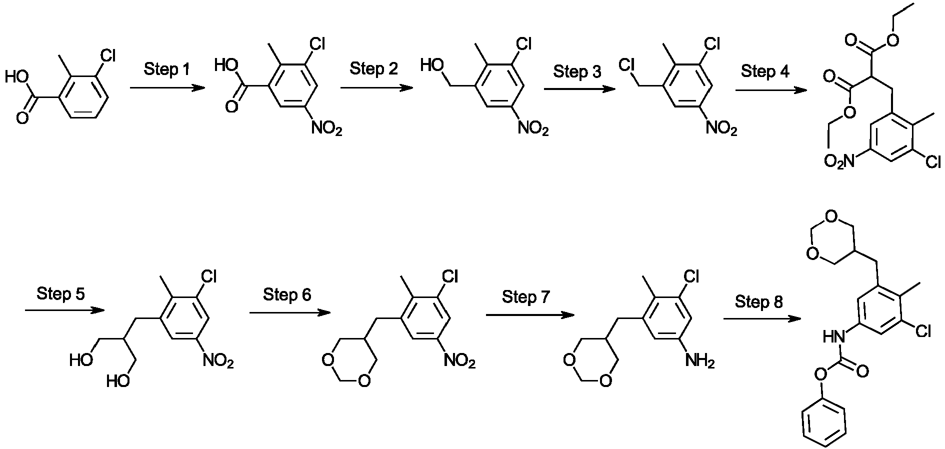

**Step 1:** To a solution of 3-chloro-2-methylbenzoic acid (41.0 g, 240 mmol, 1.00 eq) in sulfuric acid (200 mL) was added nitric acid (12.3 mL, 264 mmol, 1.10 eq) dropwise at - 10°C.The reaction was stirred at -10°C for 1 h. The mixture was poured into stirred ice water (200 mL). The resulting precipitate was collected by filtration and washed with water to afford 3-chloro-2-methyl-5-nitrobenzoic acid (52.0 g, 241 mmol, crude) as a white solid. It was used directly in the next step.

**Step 2:** To a solution of 3-chloro-2-methyl-5-nitrobenzoic acid (52.0 g, 67.5 mmol, 1.00 eq) in tetrahydrofuran (400 mL) was added borane dimethyl sulfide complex (10.0 M, 13.5 mL, 2.00 eq) at 0°C. The reaction was stirred at 25°C for 10 h. Water (25.0 mL) was added at 0°C, and the pH was adjusted to pH = 10.0 by addition of 15% sodium hydroxide solution. The mixture was extracted with ethyl acetate (3 × 300 mL). The combined organic layers were washed with brine (2 × 100 mL), dried over sodium sulfate, filtered, and concentrated under reduced pressure to give a residue. The residue was purified by silica gel column chromatography (petroleum ether/ethyl acetate= 3/1) to afford (3-chloro-2-methyl-5-nitrophenyl)methanol (3.00 g, 14.9 mmol, 22% yield) as a yellow solid.

**Step 3:** To a solution of (3-chloro-2-methyl-5-nitrophenyl)methanol (1.10 g, 5.46 mmol, 1.00 eq) in dichloromethane (10.0 mL) was added thionyl chloride (3.25 g, 27.3 mmol, 1.98 mL, 5.00 eq) at 0°C. The reaction was stirred at 25°C for 2 h. The mixture was concentrated under reduced pressure to give a residue. The residue was diluted with water (10.0 mL) and extracted with ethyl acetate (3 × 35.0 mL). The combined organic layers were washed with brine (2 × 10.0 mL), dried over sodium sulfate, filtered, and concentrated under reduced pressure to afford 1-chloro-3-(chloromethyl)-2-methyl-5- nitrobenzene (1.30 g, 5.91 mmol, crude) as yellow oil. It was used directly in the next step.

**Step 4:** To a solution of sodium hydride 60% purity (473 mg, 11.8 mmol, 2.00 eq) in tetrahydrofuran (15.0 mL) was added diethyl malonate (1.79 mL, 11.8 mmol, 2.00 eq) slowly at 0°C. After 1 h, 1-chloro-3-(chloromethyl)-2-methyl-5-nitrobenzene (1.30 g, 5.91 mmol, 1.00 eq) was added, and the reaction was stirred at 25°C for 10 h. Water (10.0 mL) was added, and the mixture was extracted with ethyl acetate (3 × 35.0 mL). The combined organic layers were washed with brine (2 × 10.0 mL), dried over sodium sulfate, filtered, and concentrated under reduced pressure to give a residue. The residue was purified by silica gel column chromatography (petroleum ether/ethyl acetate = 10/1) to afford diethyl 2-(3-chloro-2-methyl-5-nitrobenzyl)malonate (2.00 g, 5.82 mmol, 98% yield) as yellow oil.

**Step 5:** To a solution of diethyl 2-(3-chloro-2-methyl-5-nitrobenzyl)malonate (2.00 g, 5.82 mmol, 1.00 eq) in tetrahydrofuran (16.0 mL) was added sodium borohydride (1.10 g, 29.1 mmol, 5.00 eq) in portions at 0°C. Methanol (4.00 mL) was added, and the reaction was stirred at 25°C for 10 h. Water (10.0 mL) was added, and the organic solvents were removed under reduced pressure. The aqueous layer was adjusted to pH = 2.00 by addition of 1N hydrochloric acid, and it was extracted with ethyl acetate (3 × 50.0 mL). The combined organic layers were washed with brine (15.0 mL), dried over magnesium sulfate, filtered, and concentrated under reduced pressure to afford 2-(3-chloro-2-methyl-5-nitrobenzyl)propane-1,3-diol (1.40 g, 5.39 mmol, 93% yield) as a yellow solid.

**Step 6:** To a solution of 2-(3-chloro-2-methyl-5-nitrobenzyl)propane-1,3-diol (1.40 g, 5.39 mmol, 1.00 eq) in dichloromethane (10.0 mL) were added dimethoxymethane (715 µL, 8.09 mmol, 1.50 eq) and boron trifluoride diethyl etherate (1 mL, 8.09 mmol, 1.50 eq). The reaction was stirred at 25°C for 1.5 h. The mixture was diluted with water (10.0 mL) and extracted with dichloromethane (3 × 35.0 mL). The combined organic layers were washed with brine (10.0 mL), dried over sodium sulfate, filtered, and concentrated under reduced pressure to give a residue. The residue was purified by silica gel column chromatography (petroleum ether/ethyl acetate = 3/1) to afford 5-(3-chloro-2-methyl-5-nitrobenzyl)-1,3-dioxane (1.00 g, 3.68 mmol, 68% yield) as a white solid.

**Step 7:** A mixture of 5-(3-chloro-2-methyl-5-nitrobenzyl)-1,3-dioxane (1.00 g, 3.68 mmol, 1.00 eq), ammonium chloride (197 mg, 3.68 mmol, 1.00 eq) and ferrous powder (1.03 g, 18.4 mmol, 5.00 eq) in ethanol (15.0 mL) and water (7.00 mL) was stirred at 90°C for 10 h. The mixture was filtered and concentrated under reduced pressure to give a residue. Water (10.0 mL) was added, and the mixture was extracted with ethyl acetate (3 × 35.0 mL). The combined organic layers were washed with brine (10.0 mL), dried over sodium sulfate, filtered, and concentrated under reduced pressure to afford 3-((1,3-dioxan-5-yl)methyl)-5-chloro-4-methylaniline (0.88 g, 3.64 mmol, 99% yield) as a yellow solid.

**Step 8:** To a solution of 3-((1,3-dioxan-5-yl)methyl)-5-chloro-4-methylaniline (0.20 g, 827 µmol, 1.00 eq) and potassium carbonate (229 mg, 1.65 mmol, 2.00 eq) in acetone (5.00 mL) was added phenyl chloroformate (124 µL, 993 µmol, 1.20 eq). The reaction was stirred at 25°C for 10 h. The mixture was diluted with water (10.0 mL) and extracted with ethyl acetate (3 × 30.0 mL). The combined organic layers were washed with brine (10.0 mL), dried over sodium sulfate, filtered, and concentrated under reduced pressure to afford phenyl (3-((1,3-dioxan-5-yl)methyl)-5-chloro-4-methylphenyl)carbamate (0.30 g, 829 µmol, crude) as a yellow solid. It was used directly in the next step.

**Compound 133:** General procedure A with variant iii) was used for the preparation with a yield of 65% from compound **VI** employing phenyl (3-chloro-5-((4-(dimethylamino)piperidin-1-yl)methyl)-4-methylphenyl) carbamate.

¹H NMR (400 MHz, DMSO-*d₆*) *□* = 10.98 (s, 1H), 8.98 (s, 1H), 8.24 (s, 2H), 7.69 - 7.61 (m, 2H), 7.57 (d, *J =* 0.8 Hz, 2H), 7.14 (d, *J* = 2.0 Hz, 1H), 7.08 - 6.96 (m, 1H), 5.12 (dd, *J* = 5.2, 13.2 Hz, 1H), 4.47 - 4.28 (m, 4H), 3.37 (s, 2H), 2.99 - 2.57 (m, 3H), 2.47 - 2.39 (m, 2H), 2.37 (s, 6H), 2.34 - 2.26 (m, 1H), 2.25 (s, 3H), 2.05 - 1.92 (m, 3H), 1.80 (br d, *J =* 11.4 Hz, 2H), 1.44 (br dd, *J =* 3.2, 11.8 Hz, 2H). MS (ESI) m/z 581.4 [M+H]⁺

### Scheme for the preparation of phenyl (3-chloro-5-((4-(dimethylamino)piperidin-1-yl)methyl)-4-methylphenyl) carbamate.

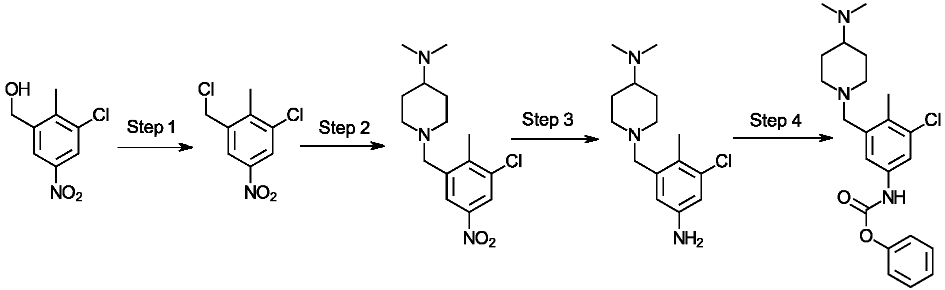

**Step** 1: To a solution of (3-chloro-2-methyl-5-nitrophenyl)methanol (0.80 g, 3.97 mmol, 1.00 eq) in dichloromethane (10.0 mL) was added thionyl chloride (1.44 mL, 19.8 mmol, 5.00 eq) at 0°C. The reaction was stirred at 25°C for 2 h. The mixture was concentrated under reduced pressure to afford 1-chloro-3-(chloromethyl)-2-methyl-5-nitrobenzene (0.90 g, 4.09 mmol, crude) as yellow oil. It was used directly in the next step.

**Step 2:** To a solution of 1-chloro-3-(chloromethyl)-2-methyl-5-nitrobenzene (0.90 g, 4.09 mmol, 1.00 eq) and triethylamine (1.42 mL, 10.2 mmol, 2.50 eq) in acetonitrile (10.0 mL) was added N,N-dimethylpiperidin-4-amine (655 mg, 5.11 mmol, 1.25 eq). The reaction was stirred at 25°C for 10 h. The mixture was filtered and concentrated under reduced pressure to afford 1-(3-chloro-2-methyl-5-nitrobenzyl)-N,N-dimethylpiperidin-4-amine (1.3 g, 4.09 mmol, crude) as a grey solid. It was used directly in the next step.

**Step 3:** A mixture of 1-(3-chloro-2-methyl-5-nitrobenzyl)-N,N-dimethylpiperidin-4-amine (1.30 g, 4.17 mmol, 1.00 eq), ammonium chloride (223 mg, 4.17 mmol, 1.00 eq) and ferrous powder (1.16 g, 20.9 mmol, 5.00 eq) in ethanol (20.0 mL) and water (10.0 mL) was stirred at 90°C for 10 h. The mixture was filtered and concentrated under reduced pressure to give a residue. The residue was purified was by reversed phase column chromatography and lyophilized to afford 1-(5-amino-3-chloro-2-methylbenzyl)-N,N-dimethylpiperidin-4-amine (0.6 g, 4.17 mmol, crude) as a brown solid. It was used directly in the next step.

**Step 4:** To a solution of 1-(5-amino-3-chloro-2-methylbenzyl)-N,N-dimethylpiperidin-4-amine (0.60 g, 2.13 mmol, 1.00 eq) and potassium carbonate (588 mg, 4.26 mmol, 2.00 eq) in acetone (10.0 mL) was added phenyl chloroformate (400 mg, 2.55 mmol, 320 µL, 1.20 eq). The reaction was stirred at 25°C for 1 h. The mixture was filtered and concentrated under reduced pressure to give a residue. The residue was purified by reversed phase preparative HPLC to afford phenyl (3-chloro-5-((4-(dimethylamino)piperidin-1-yl)methyl)-4-methylphenyl)carbamate (50.0 mg, 124 µmol, 5.84% yield) as a yellow solid.

**Compound 134:** General procedure A with variant iii) was used for the preparation with a yield of 25% from compound **VI** employing phenyl (3-chloro-5-(2-(dimethylamino)ethoxy)-4-methylphenyl)carbamate.

¹H NMR (400 MHz, DMSO-*d₆*) □ = 11.0 (s, 1H), 10.6 (br s, 1H), 9.28 (s, 1H), 7.65 (s, 1H), 7.57 (s, 2H), 7.25 - 7.07 (m, 3H), 5.12 (dd, *J =* 5.1, 13.2 Hz, 1H), 4.49 - 4.37 (m, 3H), 4.35 - 4.26 (m, 3H), 3.57 - 3.49 (m, 2H), 2.97 - 2.88 (m, 1H), 2.86 (s, 3H), 2.85 (s, 3H), 2.60 (br d, *J* = 16.7 Hz, 1H), 2.46 - 2.33 (m, 1H), 2.16 (s, 3H), 2.05 - 1.95 (m, 1H). MS (ESI) m/z 528.4 [M+H]⁺

### Scheme for the preparation of phenyl (3-chloro-5-(2-(dimethylamino)ethoxy)-4-methylphenyl)carbamate.

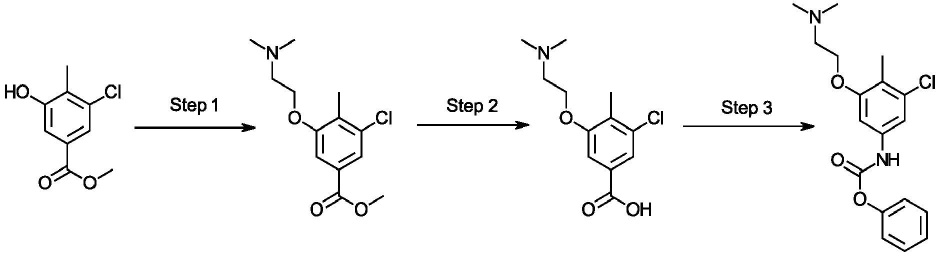

**Step 1:** To a solution of methyl 3-chloro-5-hydroxy-4-methylbenzoate (600 mg, 2.99 mmol, 1.00 eq) in acetonitrile (6.00 mL) was added potassium iodide (49.7 mg, 299 µmol, 0.10 eq), potassium carbonate (1.65 g, 12.0 mmol, 4.00 eq) and 2-chloro-N,N-dimethylethanamine (862 mg, 5.98 mmol, 2.00 eq, hydrochloric acid). The reaction was stirred at 80°C for 12 h. The mixture was filtered and concentrated under reduced pressure to give a residue. The residue was purified by silica gel column chromatography (dichloromethane/methanol = 1/0 to 10/1) to afford methyl 3-chloro-5-(2-(dimethylamino)ethoxy)-4-methylbenzoate (550 mg, 2.02 mmol, 68% yield) as a yellow oil.

**Step 2:** To a solution of methyl 3-chloro-5-(2-(dimethylamino)ethoxy)-4-methylbenzoate (500 mg, 1.84 mmol, 1.00 eq) in tetrahydrofuran (1.50 mL) and water (0.50 mL) was added lithium hydroxide monohydrate (232 mg, 5.52 mmol, 3.00 eq). The reaction was stirred at 20°C for 12 h. The mixture was concentrated under reduced pressure to give a residue. The residue was purified by reversed phase column chromatography and lyophilized to give solid. Water (20.0 mL) was added followed by hydrochloric acid (0.20 mL), and the mixture was lyophilized to afford 3-chloro-5-(2-(dimethylamino)ethoxy)-4 -methylbenzoic acid (350 mg, 1.36 mmol, 74% yield) as a white solid.

**Step 3:** To a solution of 3-chloro-5-(2-(dimethylamino)ethoxy)-4-methylbenzoic acid (300 mg, 1.16 mmol, 1.00 eq) in toluene (1.00 mL) was added triethylamine (259 mg, 2.56 mmol, 2.20 eq) and diphenylphosphoryl azide (352 mg, 1.28 mmol, 1.10 eq). The reaction was stirred at 20°C for 10 min, then phenol (548 mg, 5.82 mmol, 5.00 eq) was added. The reaction was stirred at 100°C for 30 min. The mixture was concentrated under reduced pressure to give a residue. The residue was purified by reversed phase column chromatography and lyophilized to give residue. The residue was purified by reversed phase preparative HPLC and lyophilized to afford phenyl (3-chloro-5-(2-(dimethylamino)ethoxy)-4-methylphenyl)carbamate (400 mg, 1.15 mmol, 99% yield) as a yellow solid.

**Compound 135:** General procedure A with variant iii) was used for the preparation with a yield of 13% from compound **VI** employing phenyl (3-(2-hydroxypropan-2-yl)phenyl)carbamate.

¹H NMR (400 MHz, DMSO-*d₆*) *□* = 11.00 (s, 1H), 8.65 (s, 1H), 7.67 (s, 1H), 7.57 (s, 2H), 7.46 (s, 1H), 7.33 (br d, *J =* 7.8 Hz, 1H), 7.14 (t, *J* = 7.8 Hz, 1H), 6.99 (d, *J =* 7.8 Hz, 1H), 6.73 (t, *J* = 5.8 Hz, 1H), 5.12 (dd,*J* = 5.0, 13.2 Hz, 1H), 4.94 (s, 1H), 4.52 - 4.24 (m, 4H), 2.99 - 2.85 (m, 1H), 2.62 (br d, *J =* 2.4 Hz, 1H), 2.43 - 2.31 (m, 1H), 2.07 - 1.94 (m, 1H), 1.39 (s, 6H). MS (ESI) m/z 449.4 [M-H]⁻

### Scheme for the preparation of phenyl (3-(2-hydroxypropan-2-yl)phenyl)carbamate.

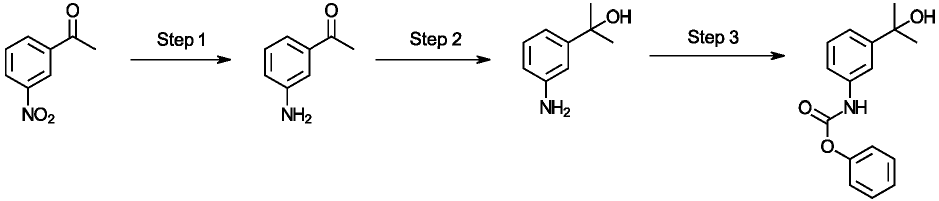

**Step 1:** A mixture of 1-(3-nitrophenyl)ethanone (2.00 g, 12.1 mmol, 1.00 eq), ammonium chloride (648 mg, 12.1 mmol, 1.00 eq) and ferrous powder (3.38 g, 60.6 mmol, 5.00 eq) in ethanol (20.0 mL) and water (10.0 mL) was stirred at 90°C for 10 h. The mixture was filtered, and the filtrate was extracted with ethyl acetate (3 × 50.0 mL). The combined organic layers were washed with brine (20.0 mL), dried over sodium sulfate, filtered, and concentrated under reduced pressure to afford 1-(3-aminophenyl)ethanone (1.60 g, 11.8 mmol, crude) as a white solid. It was used directly in the next reaction.

**Step 2:** To a solution of 1-(3-aminophenyl)ethanone (1.50 g, 11.1 mmol, 1.00 eq) in tetrahydrofuran (15.0 mL) was added methylmagnesium bromide (3.00 M, 11.1 mL, 3.00 eq) dropwise at 0°C. The reaction was stirred at 25°C for 2 h. The mixture was quenched by addition saturated ammonium chloride (8.00 mL), and then diluted with water (5.00 mL) and extracted with ethyl acetate (3 × 30.0 mL). The combined organic layers were washed with brine (10.0 mL), dried over sodium sulfate, filtered, and concentrated under reduced pressure to give a residue. The residue was purified by reversed phase preparative HPLC to afford 2-(3-aminophenyl)propan-2-ol (0.10 g, 661 µmol, 6% yield) as a white solid.

**Step 3:** To a solution of 2-(3-aminophenyl)propan-2-ol (0.10 g, 661 µmol, 1.00 eq) and 2,6-dimethylpyridine (84.7 µL, 727 µmol, 1.10 eq) in tetrahydrofuran (0.50 mL) and trichloromethane (0.50 mL) was added phenyl chloroformate (82.8 µL, 661 µmol, 1.00 eq) slowly at 0°C. The reaction was stirred at 25°C for 2 h. The mixture was diluted with water (5.00 mL) and extracted with dichloromethane (3 × 25.0 mL). The combined organic layers were washed with brine (8.00 mL), dried over sodium sulfate, filtered, and concentrated under reduced pressure to afford phenyl (3-(2-hydroxypropan-2-yl)phenyl)carbamate (0.18 g, 663 µmol, crude) as yellow oil. It was used directly in the next reaction.

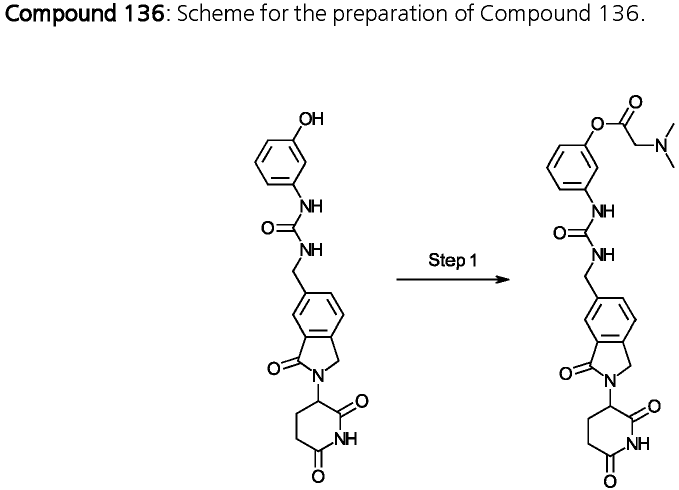

**Step 1:** A mixture of 2-(dimethylamino)acetic acid (75.7 mg, 734 µmol, 1.50 eq), 4-dimethylaminopyridine (6.0 mg, 48.9 µmol, 0.10 eq) and N,N'-methanediylidenedicyclohexanamine (148 µL, 734 µmol, 1.50 eq) in dimethylformamide (5.00 mL) was stirred at 20°C for 30 min, then 1-((2-(2,6-dioxopiperidin-3-yl)-3-oxoisoindolin-5-yl)methyl)-3-(3-hydroxyphenyl)urea (described in example **110**) (200 mg, 489 µmol, 1.00 eq) was added. The reaction was stirred at 20°C for 12h, then at 40°C for 4h. The mixture was filtered, and the filtrate was purified by reversed phase preparative HPLC to afford **Compound 136** (70.2 mg, 125.8 µmol, 41% yield, hydrochloride) as a white solid.

¹H NMR (400 MHz, DMSO-*d₆*) *□* = 10.98 (s, 1 H), 10.44 (br s, 1 H), 9.38 - 9.32 (m, 1 H), 7.66 (s, 1 H), 7.58 - 7.57 (m, 3 H), 7.32 - 7.28 (m, 1 H), 7.17 - 7.1 2 (m, 2 H), 6.75 - 6.73 (m, 1 H), 5.13 - 5.09 (m, 1 H), 4.49 - 4.33 (m, 6 H), 2.95 - 2.87 (m, 7 H), 2.62 - 2.58 (m, 1 H), 2.42 - 2.38 (m, 1 H), 2.02 - 2.00 (m, 1 H). MS (ESI) m/z 494.2 [M+H]⁺

**Compound 137:** General procedure A with variant iii) was used for the preparation with a yield of 58% from compound **VI** employing phenyl (6-chloro-5-methylpyridin-2-yl)carbamate.

¹H NMR (400 MHz, DMSO-*d₆*) *□* = 10.98 (br s, 1H), 9.44 (s, 1H), 7.69 - 7.64 (m, 2H), 7.62 - 7.59 (m, 1H), 7.57 (s, 2H), 7.48 (br t, *J* = 5.8 Hz, 1H), 5.11 (dd, *J* = 5.1, 13.3 Hz, 1H), 4.48 - 4.45 (m, 2H), 4.41 (s, 1H), 4.35 - 4.27 (m, 1H), 2.96 - 2.86 (m, 1H), 2.63 - 2.56 (m, 1 H), 2.45 - 2.36 (m, 1H), 2.22 (s, 3H), 2.04 - 1.96 (m, 1 H). MS (ESI) m/z 442.1 [M+H]⁺

### Scheme for the preparation of phenyl (6-chloro-5-methylpyridin-2-yl)carbamate.

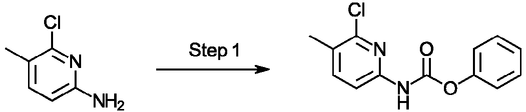

**Step** 1: To a solution of 6-chloro-5-methyl-pyridin-2-amine (300 mg, 2.10 mmol, 1.00 eq) in dichloromethane (15.0 mL) was added pyridine (0.25 mL, 3.16 mmol, 1.50 eq) and phenyl chloroformate (0.26 mL, 2.10 mmol, 1.00 eq) at 0°C. The reaction was stirred at 20°C for 2 h. The mixture was diluted with water (20.0 mL) and extracted with ethyl acetate (3 × 30.0 mL). The combined organic layers were washed with brine (3 × 20.0 mL), dried over anhydrous sodium sulfate, filtered, and concentrated under reduced pressure to afford phenyl (6-chloro-5-methylpyridin-2-yl)carbamate (400 mg, 1.52 mmol, 72% yield) as a white solid.

**Compound 138:** General procedure A with variant iii) was used for the preparation with a yield of 55% from compound **VI** employing phenyl (3-(3-oxa-8-azabicyclo[3.2.1]octan-8-ylmethyl)-5-chloro-4-methylphenyl)carbamate.

¹H NMR (400 MHz, DMSO-*d₆*) *□* = 10.98 (s, 1H), 8.74 (s, 1H), 7.66 (s, 1H), 7.64 (d, *J =* 2.3 Hz, 1H), 7.56 (d, *J =* 1.0 Hz, 2H), 7.20 (d, *J =* 2.1 Hz, 1H), 6.76 (t, *J =* 6.1 Hz, 1H), 5.11 (dd,*J* = 5.1, 13.3 Hz, 1H), 4.48 - 4.25 (m, 4H), 3.55 - 3.49 (m, 2H), 3.43 - 3.39 (m, 2H), 3.35 - 3.35 (m, 2H), 2.97 (br s, 2H), 2.95 - 2.84 (m, 1H), 2.63 - 2.56 (m, 1H), 2.45 - 2.34 (m, 1H), 2.28 (s, 3H), 2.04 - 1.90 (m, 3H), 1.82 - 1.68 (m, 2H). MS (ESI) m/z 566.2 [M+H]⁺

### Scheme for the preparation of phenyl (3-(3-oxa-8-azabicyclo[3.2.1]octan-8-ylmethyl)-5-chloro-4-methylphenyl)carbamate.

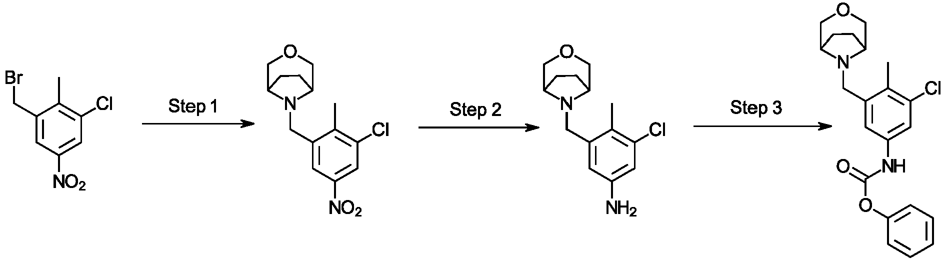

**Step** 1: To a solution of 1-(bromomethyl)-3-chloro-2-methyl-5-nitrobenzene (500 mg, 1.89 mmol, 1.00 eq) in acetonitrile (10.0 mL) was added 3-oxa-8-azabicyclo[3.2.1]octane hydrochloride (283 mg, 1.89 mmol, 1.00 eq, hydrochloride), potassium carbonate (523 mg, 3.78 mmol, 2.00 eq) and potassium iodide (31.4 mg, 189 µmol, 0.10 eq). The reaction was stirred at 80°C for 12 h. The mixture was concentrated under reduced pressure to afford a residue. The residue was diluted with water (10.0 mL) and extracted with ethyl acetate (20.0 mL). The organic layer was washed with water (10.0 mL), dried over sodium sulfate, filtered, and the filtrate was concentrated to afford a residue. The residue was purified by silica gel column chromatography (petroleum ether/ethyl acetate = 1/0 to 0/1 then dichloromethane/methanol = 10/1) to afford 8-(3-chloro-2-methyl-5-nitrobenzyl)-3-oxa-8- azabicyclo[3.2.1]octane (390 mg, 1.31 mmol, 69% yield) as a brown solid.

**Step 2:** To a solution of 8-(3-chloro-2-methyl-5-nitrobenzyl)-3-oxa-8-azabicyclo[3.2.1]octane (390 mg, 1.31 mmol, 100 eq) and ammonium chloride (492 mg, 9.20 mmol, 7.00 eq) in methanol (5.00 mL) and water (5.00 mL) was added iron powder (514 mg, 9.20 mmol, 7.00 eq). The reaction was stirred at 80°C for 1 h. The mixture was concentrated under reduced pressure to give a residue. The residue was diluted with water (20mL) and basified to pH = 10 with sodium hydroxide. The mixture was extracted with ethyl acetate (50.0 mL). The organic layer was washed with water (20.0 mL), dried over sodium sulfate, filtered, and the filtrate was concentrated under reduced pressure to afford 3-(3-oxa-8-azabicyclo[3.2.1]octan-8-ylmethyl)-5-chloro-4-methylaniline (290 mg, 1.09 mmol, 82% yield) as a brown oil.

**Step 3:** To a solution of 3-(3-oxa-8-azabicyclo[3.2.1]octan-8-ylmethyl)-5-chloro-4-methylaniline (230 mg, 862 µmol, 1.00 eq) and potassium carbonate (119 mg, 862 µmol, 1.00 eq) in acetone (3.00 mL) was added phenyl chloroformate (0.11 mL, 862 µmol, 1.00 eq) dropwise. The reaction was stirred at 20°C for 1 h. The mixture was filtered, and the filtrate was concentrated under reduced pressure to give a residue. The residue was purified by silica gel column chromatography (petroleum ether/ethyl acetate = 10/1 to 2/1) to afford phenyl (3-(3-oxa-8-azabicyclo[3.2.1]octan-8-ylmethyl)-5-chloro-4-methylphenyl)carbamate (300 mg, 775 µmol, 89% yield) as yellow oil.

**Compound 139:** General procedure A with variant iii) was used for the preparation with a yield of 19% from compound **VI** employing phenyl (3-(2-oxa-5-azabicyclo[2.2.1]heptan-5-ylmethyl)-5-chloro-4-methylphenyl) carbamate.

¹H NMR (400 MHz, DMSO-*d*₆ + D₂O, T = 80°C) *□* = 7.68 - 7.62 (m, 2H), 7.58 - 7.51 (m, 3H), 5.00 (dd, *J =* 5.3, 13.1 Hz, 1H), 4.67 (s, 1H), 4.50 - 4.32 (m, 7H), 4.26 - 4.12 (m, 1 H), 3.73 (br d, *J* = 9.8 Hz, 1H), 3.37 - 3.28 (m, 2H), 2.94 - 2.77 (m, 1H), 2.71 - 2.59 (m, 1H), 2.46 - 2.34 (m, 2H), 2.34 (s, 3H), 2.12 - 2.01 (m, 2H). MS (ESI) m/z 552.3 [M+H]⁺

### Scheme for the preparation of phenyl (3-(2-oxa-5-azabicyclo[2.2.1]heptan-5-ylmethyl)-5-chloro-4-methylphenyl) carbamate.

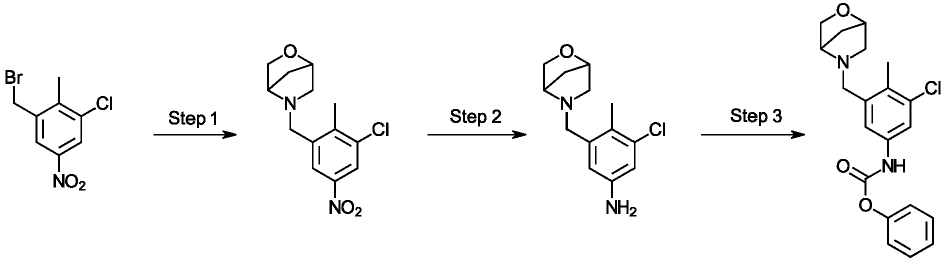

**Step** 1: To a solution of 2-oxa-5-azabicyclo[2.2.1]heptane hydrochloride (308 mg, 2.27 mmol, 1.00 eq, hydrochloride) in acetonitrile (10.0 mL) was added 1-(bromomethyl)-3-chloro-2-methyl-5-nitrobenzene (600 mg, 2.27 mmol, 1.00 eq), potassium carbonate (627 mg, 4.54 mmol, 2.00 eq) and potassium iodide (37.6 mg, 227 µmol, 0.10 eq). The reaction was stirred at 80°C for 12 h. The mixture was diluted with water (50.0 mL) and extracted with ethyl acetate (3 × 40.0 mL). The combined organic layers were washed with brine (20.0 mL), dried over sodium sulfate, filtered, and concentrated under reduced pressure to give a residue. The residue was triturated with petroleum ether (2.00 mL) and filtered. The filter cake was dissolved in ethyl acetate (50.0 mL) and silica gel was added. The mixture was filtered, and the filtrate was concentrated under reduced pressure to afford 5-(3-chloro-2-methyl-5-nitrobenzyl)-2- oxa-5-azabicyclo[2.2.1]heptane (550 mg, crude) as an orange solid.

**Step 2:** To a solution of 5-(3-chloro-2-methyl-5-nitrobenzyl)-2-oxa-5-azabicyclo[2.2.1]heptane (500 mg, 1.77 mmol, 1.00 eq), ammonium chloride (662 mg, 12.4 mmol, 7.00 eq) in methanol (8.00 mL) and water (2.00 mL) was added iron powder (691 mg, 12.4 mmol, 7.00 eq) in portions. The reaction was stirred at 70°C for 2 h. The mixture was filtered, and the filtrate was concentrated under reduced pressure to give a residue. The residue was diluted with water (60.0 mL) and extracted with ethyl acetate (3 × 40.0 mL). The combined organic layers were washed with brine (30.0 mL), dried over sodium sulfate, filtered, and concentrated under reduced pressure to give a residue. The residue was purified by silica gel column chromatography (petroleum ether/ethyl acetate = 2/1 to 0/1 ) to give 3-(2-oxa-5-azabicyclo[2.2.1]heptan-5-ylmethyl)-5-chloro-4-methylaniline (400 mg, crude) as a yellow solid. It was used directly in the next step.

**Step 3:** To a solution of 3-(2-oxa-5-azabicyclo[2.2.1]heptan-5-ylmethyl)-5-chloro-4-methylaniline (300 mg, 1.19 mmol, 1.00 eq) and phenyl chloroformate (0.15 mL, 1.19 mmol, 1.00 eq) in acetone (3.00 mL) was added potassium carbonate (492 mg, 3.56 mmol, 3.00 eq) in portions. The reaction was stirred at 15°C for 2 h. Water (30.0 mL) was added, and the mixture was extracted with ethyl acetate (3 × 30.0 mL). The combined organic layers were washed with brine (20.0 mL), dried over anhydrous sodium sulfate, filtered, and concentrated under reduced pressure to give a residue. The residue was purified by silica gel column chromatography (petroleum ether/ethyl acetate = 5/1 to 2/1) to afford phenyl (3-(2-oxa-5-azabicyclo[2.2.1]heptan-5-ylmethyl)-5-chloro-4-methylphenyl)carbamate (250 mg, 671 µmol, 56% yield) as a yellow solid.

**Compound 140:** General procedure A with variant iii) was used for the preparation with a yield of 46% from compound **VI** employing phenyl (3-(8-oxa-3-azabicyclo[3.2.1]octan-3-ylmethyl)-5-chloro-4-methylphenyl)carbamate.

¹H NMR (400 MHz, DMSO-*d₆*) *□* = 10.98 (br s, 1H), 8.77 (s, 1 H), 8.30 (s, 1H), 7.69 - 7.60 (m, 2H), 7.56 (s, 2H), 7.11 (d, *J =* 1.9 Hz, 1H), 6.85 (br t, *J* = 5.9 Hz, 1H), 5.11 (dd, *J =* 5.0, 13.3 Hz, 1H), 4.48 - 4.36 (m, 3H), 4.35 - 4.26 (m, 1H), 4.19 (br s, 2H), 2.98 - 2.85 (m, 1H), 2.68 - 2.52 (m, 3H), 2.47 - 2.33 (m, 3H), 2.26 (s, 3H), 2.19 (br d, *J =* 10.6 Hz, 2H), 2.06 - 1.92 (m, 1H), 1.84 - 1.75 (m, 2H), 1.75 - 1.63 (m, 2H). MS (ESI) m/z 566.1 [M+H]⁺

### Scheme for the preparation of phenyl (3-(8-oxa-3-azabicyclo[3.2.1]octan-3-ylmethyl)-5-chloro-4-methylphenyl)carbamate.

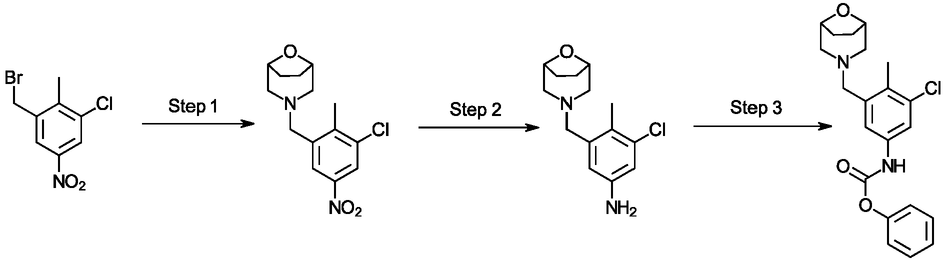

**Step 1:** To a solution of 8-oxa-3-azabicyclo[3.2.1]octane (400 mg, 3.53 mmol, 1.00 eq) and triethylamine (0.74 mL, 5.30 mmol, 1.50 eq) in tetrahydrofuran (15.0 mL) was added 1-(bromomethyl)-3-chloro-2-methyl-5-nitrobenzene (842 mg, 3.18 mmol, 0.90 eq). The reaction was stirred at 20°C for 6 h. The mixture was concentrated under reduced pressure to afford a residue. The residue was purified by preparative TLC (petroleum ether/ethyl acetate = 5/1) to afford 3-(3-chloro-2-methyl-5-nitrobenzyl)-8-oxa-3-azabicyclo[3.2.1] octane as a white solid.

**Step 2:** A mixture of 3-(3-chloro-2-methyl-5-nitrobenzyl)-8-oxa-3-azabicyclo[3.2.1]octane (750 mg, 2.53 mmol, 1.00 eq), ammonium chloride (135 mg, 2.53 mmol, 1.00 eq) and iron powder (706 mg, 12.6 mmol, 5.00 eq) in ethanol (20.0 mL) and water (10.0 mL) was stirred at 90°C for 3 h. The mixture was filtered and concentrated under reduced pressure to give a residue. The residue was purified by preparative TLC (petroleum ether/ethyl acetate = 5/1) to afford 3-(8-oxa-3-azabicyclo[3.2.1]octan-3-ylmethyl)-5-chloro-4-methylaniline (650 mg, 2.31 mmol, 91 % yield) as a yellow solid.

**Step 3:** To a solution of 3-(8-oxa-3-azabicyclo[3.2.1]octan-3-ylmethyl)-5-chloro-4-methylaniline (300 mg, 1.12 mmol, 1.00 eq) and potassium carbonate (466 mg, 3.37 mmol, 3.00 eq) in acetone (3.00 mL) was added phenyl chloroformate (0.14 mL, 1.12 mmol, 1.00 eq) dropwise. The reaction was stirred at 15°C for 2 h. Water (50.0 mL) was added, and the mixture was extracted with ethyl acetate (3 × 40.0 mL). The combined organic layers were washed with brine (30.0 mL), dried over anhydrous sodium sulfate, filtered, and concentrated under reduced pressure to afford phenyl (3-(8-oxa-3-azabicyclo[3.2.1]octan-3-ylmethyl)-5-chloro-4-methylphenyl)carbamate (400 mg, crude) as a yellow solid.

**Compound 141:** General procedure A with variant iii) was used for the preparation with a yield of 14% from compound **VI** employing phenyl (3-chloro-4-methyl-5-((2-methyl-1,3-dioxan-5-yl)methyl)phenyl)carbamate.

¹H NMR (400 MHz, DMSO-*d₆*) *□* = 10.98 (s, 1H), 8.73 (s, 1H), 7.66 (s, 1H), 7.60 - 7.49 (m, 3H), 7.01 (d, *J =* 2.0 Hz, 1H), 6.85 (br s, 1H), 5.12 (dd, *J =* 5.2, 13.3 Hz, 1H), 4.61 (q, *J =* 5.0 Hz, 1H), 4.49 - 4.27 (m, 4H), 3.86 (dd, *J* = 4.4*,* 11.6 Hz, 2H), 3.42 (t, *J =* 11.2 Hz, 2H), 2.99 - 2.84 (m, 1H), 2.60 (td, *J =* 2.0, 15.2 Hz, 1H), 2.40 (dd, *J =* 4.4, 13.0 Hz, 1H), 2.34 (d, *J* = 7.2 Hz, 2H), 2.20 (s, 3H), 2.06 - 1.95 (m, 2H), 1.16 (d, *J =* 5.0 Hz, 3H). MS (ESI) m/z 555.3 [M+H]⁺

### Scheme for the preparation of phenyl (3-chloro-4-methyl-5-((2-methyl-1,3-dioxan-5-yl)methyl)phenyl)carbamate.

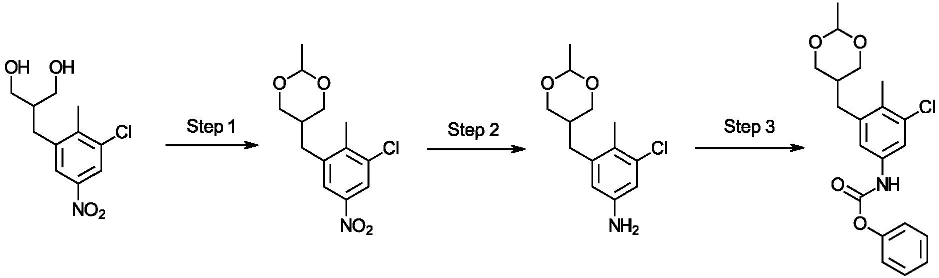

**Step** 1: To a solution of 2-(3-chloro-2-methyl-5-nitrobenzyl)propane-1,3-diol (1.00 g, 3.85 mmol, 1.00 eq) (described in example **132**) and 1,1-dimethoxyethane (611 µL, 5.78 mmol, 1.50 eq) in dichloromethane (10.0 mL) was added boron trifluoride diethyl etherate (713 µL, 5.78 mmol, 1.50 eq). The reaction was stirred at 25°C for 1.5 h. The mixture was diluted with water (10.0 mL) and extracted with ethyl acetate (3 × 35.0 mL). The combined organic layers were washed with brine (10.0 mL), dried over magnesium sulfate, filtered, and concentrated under reduced pressure to afford 5-(3-chloro-2-methyl-5-nitrobenzyl)-2-methyl-1,3-dioxane (1.10 g, 3.85 mmol, trans and cis, crude) as a yellow oil.

**Step 2:** A mixture of 5-(3-chloro-2-methyl-5-nitrobenzyl)-2-methyl-1,3-dioxane (1.10 g, 3.85 mmol, 1.00 eq), ferrous powder (1.07 g, 19.3 mmol, 5.00 eq) and ammonium chloride (206 mg, 3.85 mmol, 1.00 eq) in ethanol (10.0 mL) and water (5.00 mL) was stirred at 90°C for 10 h. The mixture was filtered and concentrated under reduced pressure to give a residue. The residue was purified by reversed phase column chromatography and lyophilized to afford 3-chloro-4-methyl-5-((2-methyl-1,3-dioxan-5-yl)methyl)aniline (0.60 g, 2.35 mmol, 61 % yield, trans and cis) as a grey solid.

**Step 3:** To a solution of 3-chloro-4-methyl-5-((2-methyl-1,3-dioxan-5-yl)methyl)aniline (0.60 g, 2.35 mmol, 1.00 eq) and potassium carbonate (649 mg, 4.69 mmol, 2.00 eq) in acetone (10.0 mL) was added phenyl chloroformate (0.35 mL, 2.82 mmol, 1.20 eq). The reaction was stirred at 25°C for 2 h. The mixture was diluted with water (10.0 mL) and extracted with ethyl acetate (3 × 35.0 mL). The combined organic layers were washed with brine (10.0 mL), dried over sodium sulfate, filtered, and concentrated under reduced pressure to afford phenyl (3-chloro-4-methyl-5-((2-methyl-1,3-dioxan-5-yl)methyl) phenyl)carbamate (0.9 g, 2.35 mmol, trans and cis, crude) as yellow oil. It was used directly in the next step.

**Compound 142:** General procedure A with variant iii) was used for the preparation with a yield of 29% from compound **VI** employing phenyl (3-chloro-5-((2,2-dimethyl-1,3-dioxan-5-yl)methyl)-4-methylphenyl)carbamate.

¹H NMR (400 MHz, DMSO-*d₆*) *□* = 11.24 - 10.65 (m, 1H), 8.73 (s, 1H), 7.66 (s, 1H), 7.60 - 7.53 (m, 3H), 7.01 (d, *J* = 2.0 Hz, 1H), 6.80 (t, *J* = 6.2 Hz, 1H), 5.12 (dd, *J =* 5.2, 13.2 Hz, 1H), 4.50 - 4.26 (m, 4H), 3.75 (dd, *J* = 4.2*,* 11.6 Hz, 2H), 3.57 (dd, *J* = 7.6, 11.6 Hz, 2H), 2.97 - 2.85 (m, 1H), 2.65 - 2.56 (m, 3H), 2.40 (dd, *J =* 4.4, 12.8 Hz, 1H), 2.22 (s, 3H), 2.06 - 1.95 (m, 1H), 1.80 (td, *J =* 3.8, 7.4 Hz, 1H), 1.37 (s, 3H), 1.30 (s, 3H). MS (ESI) m/z 569.2 [M+H]⁺

### Scheme for the preparation of phenyl (3-chloro-5-((2,2-dimethyl-1,3-dioxan-5-yl)methyl)-4-methylphenyl)carbamate.

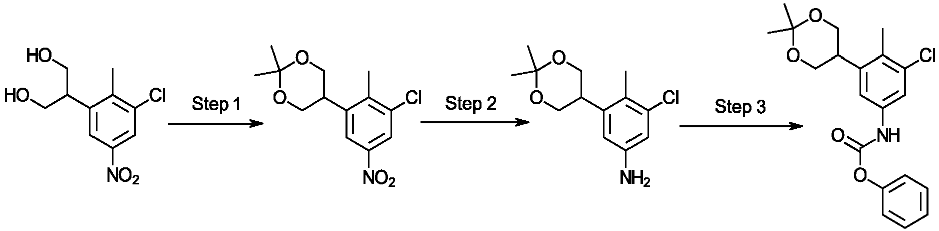

**Step 1:** A mixture of 2-(3-chloro-2-methyl-5-nitrobenzyl)propane-1,3-diol (0.82 g, 3.16 mmol, 1.00 eq) (described in example **132**), 2,2-dimethoxypropane (0.43 mL, 3.47 mmol, 1.10 eq) and indium(III) trifluoromethanesulfonate (177 mg, 316 µmol, 0.10 eq) was stirred at 25°C for 0.5 h. The mixture was diluted with water (20.0 mL) and extracted with ethyl acetate (3 × 55.0 mL). The combined organic layers were washed with brine (15.0 mL), dried over magnesium sulfate, filtered, and concentrated under reduced pressure to afford 5-(3-chloro-2-methyl-5-nitrobenzyl)-2,2-dimethyl-1,3-dioxane (0.99 g, 3.16 mmol, crude) as yellow oil. It was used directly in the next step.

**Step 2:** A mixture of 5-(3-chloro-2-methyl-5-nitrobenzyl)-2,2-dimethyl-1,3-dioxane (1.00 g, 3.34 mmol, 1.00 eq), ferrous powder (932 mg, 16.7 mmol, 5.00 eq) and ammonium chloride (178 mg, 3.34 mmol, 1.00 eq) in ethanol (10.0 mL) and water (5.00 mL) was stirred at 50°C for 10 h. The mixture was concentrated under reduced pressure to give a residue. The residue was diluted with water (15.0 mL) and extracted with ethyl acetate (3 × 35.0 mL). The combined organic layers were washed with brine (10.0 mL), dried over sodium sulfate, filtered, and concentrated under reduced pressure to give a residue. The residue was purified by silica gel column chromatography (petroleum ether/ethyl acetate = 3/1) to afford 3-chloro-5-((2,2-dimethyl-1,3-dioxan-5-yl)methyl)-4-methylaniline (0.40 g, 1.48 mmol, 44% yield) as yellow oil.

**Step 3:** To a solution of 3-chloro-5-((2,2-dimethyl-1,3-dioxan-5-yl)methyl)-4-methylaniline (0.40 g, 1.48 mmol, 1.00 eq) and potassium carbonate (410 mg, 2.97 mmol, 2.00 eq) in acetone (5.00 mL) was added phenyl chloroformate (0.22 mL, 1.78 mmol, 1.20 eq). The reaction was stirred at 25°C for 2 h. The mixture was diluted with water (15.0 mL) and extracted with ethyl acetate (3 × 35.0 mL). The combined organic layers were washed with brine (10.0 mL), dried over sodium sulfate, filtered, and concentrated under reduced pressure to afford phenyl (3-chloro-5-((2,2-dimethyl-1,3-dioxan-5-yl)methyl)-4-methylphenyl)carbamate (0.55 g, 1.48 mmol, crude) as yellow oil. It was used directly in the next step.

**Compound 143:** General procedure A with variant iii) was used for the preparation with a yield of 44% from compound **VI** employing phenyl(3-chloro-4-methyl-5-(trifluoromethoxy)phenyl)carbamate.

¹H NMR (400MHz, DMSO-*d₆*) *□* = 10.98 (s, 1H), 9.10 (s, 1H), 7.67 (s, 1H), 7.58 - 7.55 (m, 3H), 7.54 (d, *J =* 1.2 Hz, 1H), 7.00 (t, *J* = 6.0 Hz, 1H), 5.12 (dd, *J* = 5.2, 13.6 Hz, 1H), 4.47 - 4.39 (m, 3H), 4.34 - 4.28 (m, 1H), 2.96 - 2.87 (m, 1H), 2.64 - 2.57 (m, 1H), 2.47 - 2.36 (m, 1H), 2.20 (s, 3H), 2.00 (dtd, *J =* 2.0, 5.2, 12.4 Hz, 1H). MS (ESI) m/z 525.0 [M+H]⁺

### Scheme for the preparation of phenyl(3-chloro-4-methyl-5-(trifluoromethoxy)phenyl)carbamate.

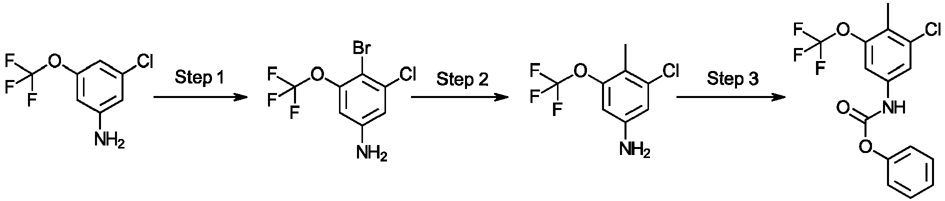

**Step** 1: To a solution of 3-chloro-5-(trifluoromethoxy)aniline (1.60 g, 7.56 mmol, 1.00 eq) in dimethyl formamide (20.0 mL) was added N-bromosuccinimide (1.35 g, 7.56 mmol, 1.00 eq). The reaction was stirred at 25°C for 12 h. The mixture was poured into water (120 mL) and stirred for 10 min. The aqueous phase was extracted with ethyl acetate (3 × 60.0 mL). The combined organic layers were washed with brine (100 mL), dried over anhydrous sodium sulfate, filtered, and concentrated under reduced pressure to afford 4-bromo-3-chloro-5-(trifluoromethoxy) aniline (2.10 g, 7.23 mmol, 96% yield) as yellow oil.

**Step 2:** To a mixture of 4-bromo-3-chloro-5-(trifluoromethoxy)aniline (1.00 g, 3.44 mmol, 1.00 eq), methylboronic acid (824 mg, 13.8 mmol, 4.00 eq) and caesium carbonate (3.37 g, 10.3 mmol, 3.00 eq) in dioxane (10.0 mL) was added [1,1'-bis(diphenylphosphino)ferrocene]dichloropalladium(II) (252 mg, 344 µmol, 0.100 eq) under nitrogen. The reaction was stirred at 100°C for 3 h. The mixture was concentrated under reduced pressure to give a residue. The residue was purified by silica gel column chromatography (petroleum ether/ethyl acetate = 20/1 to 2/1 ) to afford 3-chloro-4-methyl-5-(trifluoromethoxy)aniline (700 mg, 3.10 mmol, 90% yield) as brown oil.

**Step 3:** To a solution of 3-chloro-4-methyl-5-(trifluoromethoxy)aniline (600 mg, 2.66 mmol, 1.00 eq) and pyridine (0.64 mL, 7.98 mmol, 3.00 eq) in acetonitrile (10.0 mL) was added phenyl chloroformate (0.50 mL, 3.99 mmol, 1.50 eq). The reaction was stirred at 20°C for 3 h. The mixture was concentrated under reduced pressure to give a residue. The residue was purified by silica gel column chromatography (petroleum ether/ethyl acetate = 1/0 to 40/1) to afford phenyl (3-chloro-4-methyl-5-(trifluoromethoxy) phenyl)carbamate (800 mg, 2.31 mmol, 87% yield) as a white solid.

**Compound 144:** General procedure A with variant iii) was used for the preparation with a yield of 59% from compound **VI** employing phenyl(4-methyl-3-(trifluoromethoxy)phenyl)carbamate.

¹H NMR (400 MHz, DMSO-*d₆*) *□* = 10.98 (s, 1H), 8.88 (s, 1H), 7.69 - 7.63 (m, 2H), 7.60 - 7.53 (m, 2H), 7.25 - 7.20 (m, 1H), 7.20 - 7.14 (m, 1H), 6.83 (t, *J =* 6.0 Hz, 1H), 5.12 (dd, *J* = 5.1, 13.3 Hz, 1H), 4.46 - 4.28 (m, 4H), 2.97 - 2.86 (m, 1H), 2.63 - 2.57 (m, 1H), 2.40 (dq, *J* = 4.5, 13.2 Hz, 1H), 2.18 (s, 3H), 2.00 (dtd, *J =* 2.1, 5.2, 12.6 Hz, 1H). MS (ESI) m/z 491.2 [M+H]⁺

### Scheme for the preparation of phenyl(4-methyl-3-(trifluoromethoxy)phenyl)carbamate.

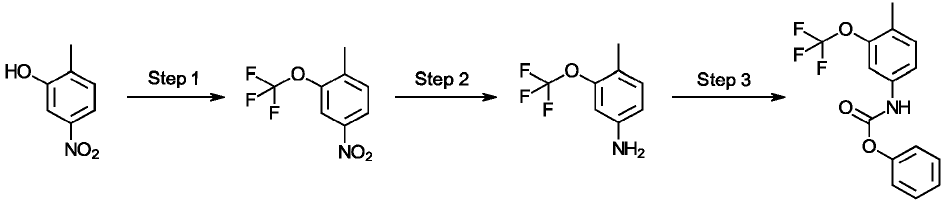

**Step** 1: To a solution of 2-methyl-5-nitro-phenol (2.00 g, 13.1 mmol, 1.00 eq), silver trifluoromethanesulfonate (16.8 g, 65.3 mmol, 5.00 eq), 1-(chloromethyl)-4-fluoro-1,4-diazoniabicyclo[2.2.2]octane ditetrafluoroborate (9.25 g, 26.1 mmol, 2.00 eq), N-fluorobenzenesulfonimide (8.24 g, 26.1 mmol, 2.00 eq), and caesium fluoride (11.9 g, 78.4 mmol, 6.00 eq) in toluene (100 mL) was added trimethyl(trifluoromethyl)silane (9.29 g, 65.3 mmol, 5.00 eq) and 2-fluoropyridine (5.61 mL, 65.3 mmol, 5.00 eq) under nitrogen atmosphere. The reaction was stirred at 20°C for 12 h under nitrogen. The mixture was filtered, and the filtrate was concentrated under reduced pressure to afford a residue. The residue was diluted with water (100 mL) and extracted with petroleum ether/ethyl acetate (10/1, 100 mL). The organic layer was washed with water (20.0 mL), dried over anhydrous sodium sulfate, filtered, and the filtrate was concentrated under reduced pressure to give a residue. The residue was purified by silica gel column chromatography (petroleum ether/ethyl acetate = 1/0 to 20/1) to afford 1-methyl-4-nitro-2-(trifluoromethoxy)benzene (1.60 g, crude) as yellow oil.

**Step 2:** To a solution of 1-methyl-4-nitro-2-(trifluoromethoxy)benzene (1.60 g, 7.24 mmol, 1.00 eq) in methanol (20.0 mL) and water (20.0 mL) was added iron powder (2.83 g, 50.7 mmol, 7.00 eq) and ammonium chloride (2.71 g, 50.7 mmol, 7.00 eq). The reaction was stirred at 80°C for 2 h. The mixture was concentrated under reduced pressure to give a residue. Ethyl acetate (50.0 mL) was added to the residue, and the mixture was washed with water (20.0 mL). The organic layer was dried over anhydrous sodium sulfate, filtered, and the filtrate was concentrated under reduced pressure to give a residue. The residue was purified by silica gel column chromatography (petroleum ether/ethyl acetate = 1/0 to 5/1 ) to afford 4-methyl-3-(trifluoromethoxy)aniline (460 mg, 2.41 mmol, 33% yield) as yellow oil.

**Step 3:** To a mixture of 4-methyl-3-(trifluoromethoxy)aniline (200 mg, 1.05 mmol, 1.00 eq) and potassium carbonate (174 mg, 1.26 mmol, 1.20 eq) in acetone (4.00 mL) was added phenyl chloroformate (180 mg, 1.15 mmol, 1.10 eq). The reaction was stirred at 20°C for 1 h. The mixture was diluted with water (3.00 mL), extracted with ethyl acetate (5.00 mL), dried over anhydrous sodium sulfate, filtered, and the filtrate was concentrated under reduced pressure to afford phenyl(4-methyl-3-(trifluoromethoxy)phenyl)carbamate (300 mg, 964 µmol, 92 % yield) as transparent oil.

**Compound 145:** General procedure A with variant iii) was used for the preparation with a yield of 2% from compound **VI** employing phenyl (3-chloro-4-methyl-5-((2-methyl-1,3-dioxan-5-yl)methyl)phenyl)carbamate.

¹H NMR (400 MHz, DMSO-*d₆*) *□* = 10.99 (br s, 1H), 8.86 (s, 1H), 8.44 (s, 1H), 7.69 - 7.59 (m, 2H), 7.56 (d, *J =* 0.8 Hz, 2H), 6.98 (d, *J =* 2.0 Hz, 1H), 6.85 (t, *J =* 6.0 Hz, 1H), 5.12 (dd, *J* = 5.2, 13.4 Hz, 1H), 4.69 (q, *J =* 5.0 Hz, 1H), 4.50 - 4.26 (m, 4H), 3.87 - 3.77 (m, 2H), 3.70 (d, *J =* 11.0 Hz, 2H), 2.99 - 2.82 (m, 3H), 2.66 - 2.56 (m, 1H), 2.46 - 2.32 (m, 1H), 2.23 (s, 3H), 2.06 - 1.96 (m, 1H), 1.54 (br t, *J =* 7.2 Hz, 1H), 1.22 (d*, J* = 5.0 Hz, 3H). MS (ESI) m/z 555.1 [M+H]⁺

**Compound 146:** General procedure A with variant iii) was used for the preparation with a yield of 74% from compound **VI** employing phenyl (3-methylisothiazol-5-yl)carbamate.

¹H NMR (400 MHz, DMSO-*d₆*) *□* = 10.98 (s, 1H), 10.38 (s, 1H), 7.65 (s, 1H), 7.59 - 7.53 (m, 2H), 7.30 (br t, *J* = 5.7 Hz, 1H), 6.51 (s, 1H), 5.11 (dd, *J* = 5.1*,* 13.3 Hz, 1H), 4.47 - 4.43 (m, 1H), 4.41 (s, 2H), 4.34 - 4.27 (m, 1H), 2.97 - 2.84 (m, 1H), 2.59 (br d, *J* = 16.8 Hz, 1H), 2.46 - 2.34 (m, 1H), 2.24 (s, 3H), 2.05 - 1.95 (m, 1H). MS (ESI) m/z 414.1 [M+H]⁺

### Scheme for the preparation of phenyl (3-methylisothiazol-5-yl)carbamate.

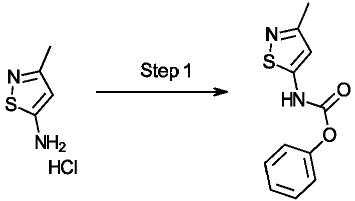

**Step 1:** To a solution of 3-methylisothiazol-5-amine hydrochloride (0.260 g, 1.73 mmol, 1.30 eq, hydrochloride) in pyridine (2.00 mL) was added phenyl chloroformate (166 µL, 1.33 mmol, 1.00 eq). The reaction was stirred for 2 h at 0°C. The mixture was concentrated under reduced pressure to give a residue. The residue was washed with water to afford phenyl (3-methylisothiazol-5-yl)carbamate (250 mg, 1.07 mmol, 80% yield) as a yellow solid.

**Compound 147:** General procedure A with variant iii) was used for the preparation with a yield of 60% from compound **VI** employing phenyl(5-methylthiazol-2-yl)carbamate.

¹H NMR (400 MHz, DMSO-*d₆*) *□* = 11.00 (s, 1H), 10.38 (br s, 1H), 7.64 (s, 1H), 7.59 - 7.52 (m, 2H), 7.11 (br s, 1H), 6.96 (d, *J* = 1.2 Hz, 1H), 5.12 (dd, *J =* 5.1, 13.4 Hz, 1H), 4.48 - 4.24 (m, 4H), 2.97 - 2.84 (m, 1H), 2.69 - 2.55 (m, 1H), 2.45 - 2.31 (m, 1H), 2.27 (d, *J* = 1.1 Hz, 3H), 2.05 - 1.94 (m, 1H). MS (ESI) m/z 414.1 [M+H]⁺

### Scheme for the preparation of phenyl(5-methylthiazol-2-yl)carbamate.

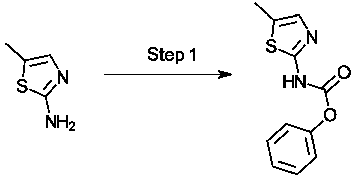

**Step 1** : To a mixture of 5-methylthiazol-2-amine (500 mg, 4.38 mmol, 1.00 eq) and pyridine (1.06 mL, 13.1 mmol, 3.00 eq) in dichloromethane (5.00 mL) was added phenyl chloroformate (575 µL, 4.60 mmol, 1.05 eq) dropwise at 0°C over 20 min under nitrogen. The reaction was stirred at 0°C for 2 h. The mixture was filtered, and the filtrate was concentrated under reduced pressure to afford phenyl (5-methylthiazol-2-yl)carbamate (300 mg, 1.28 mmol, 29% yield) as a white solid.

**Compound 148:** General procedure A with variant iii) was used for the preparation with a yield of 52% from compound **VI** employing phenyl (5-ethylthiazol-2-yl)carbamate.

¹H NMR (400 MHz, DMSO-*d₆*) *□* = 10.98 (s, 1 H), 10.39 (br s, 1 H), 7.65 (s, 1 H), 7.56 (s, 2 H), 7.13 (br s, 1 H), 6.99 (s, 1 H), 5.11 (dd, *J =* 13, 5.2 Hz, 1 H), 4.48 - 4.43 (m, 1 H), 4.42 (d, *J =* 5.8 Hz, 2 H), 4.34 - 4.27 (m, 1 H), 2.98 - 2.84 (m, 1 H), 2.67 (q, *J =* 7.4 Hz, 2 H), 2.59 (br d, *J =* 18 Hz, 1 H), 2.40 (br d, *J =* 13 Hz, 1 H), 2.05 - 1.96 (m, 1 H), 1.18 (t, *J* = 7.6 Hz, 3 H). MS (ESI) m/z 428.2 [M+H]⁺

### Scheme for the preparation of phenyl (5-ethylthiazol-2-yl)carbamate.

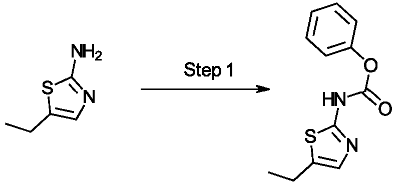

**Step** 1: To a solution of 5-ethylthiazol-2-amine (200 mg, 1.56 mmol, 1.00 eq) and pyridine (5.00 mL) in dichloromethane (10.0 mL) was added phenyl chloroformate (235 µL, 1.87 mmol, 1.20 eq) under nitrogen at 0°C. The reaction was stirred at 25°C for 12 h. The mixture was extracted with ethyl acetate (3 × 10.0 mL). The combined organic layers were washed with brine (10.0 mL), dried over anhydrous sodium sulfate, filtered, and concentrated under reduced pressure to afford phenyl (5-ethylthiazol-2-yl)carbamate (309 mg, 1.24 mmol, 80% yield) as a white solid.

**Compound 149:** General procedure A with variant iii) was used for the preparation with a yield of 50% from compound **VI** employing phenyl (3-(trifluoromethyl)isothiazol-5-yl)carbamate.

¹H NMR (400 MHz, DMSO-*d₆*) *□* = 10.88 - 10.60 (m, 2H), 7.69 (s, 1H), 7.63 - 7.47 (m, 3H), 7.06 (s, 1H), 5.08 (dd, *J* = 5.2*,* 13.1 Hz, 1H), 4.55 - 4.27 (m, 4H), 2.99 - 2.80 (m, 1H), 2.70 - 2.58 (m, 1H), 2.44 - 2.33 (m, 1H), 2.12 - 1.92 (m, 1H). MS (ESI) m/z 468.0 [M+H]⁺

### Scheme for the preparation of phenyl (3-(trifluoromethyl)isothiazol-5-yl)carbamate.

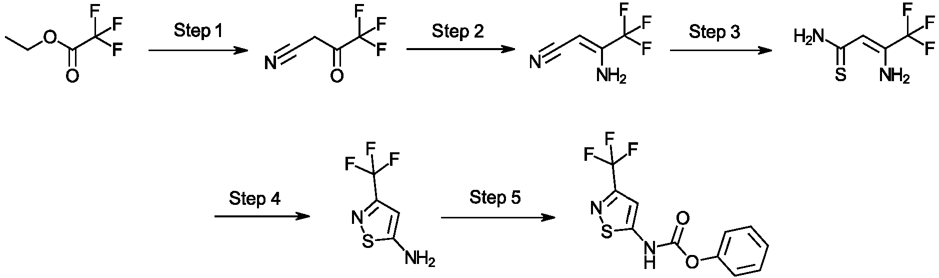

**Step 1:** To a solution of potassium tert-butoxide (1 M, 84.3 mL, 1.60 eq) at 0°C was added a solution of ethyl 2,2,2-trifluoroacetate (7.27 mL, 52.7 mmol, 1.00 eq) in acetonitrile (3.19 mL, 60.6 mmol, 1.15 eq) dropwise at 0°C. The reaction was stirred at 20°C for 24 hours. The reaction was quenched with hydrochloric acid (50.0 mL, 1 M) and extracted with ethyl acetate (3 × 50.0 mL). The organic phases were gathered, washed with brine (50.0 mL), dried over sodium sulfate, filtered, and concentrated under reduced pressure to afford 4,4,4-trifluoro-3-oxobutanenitrile (10.0 g, crude) as a yellow oil. It was used directly in the next step.

**Step 2:** A mixture of 4,4,4-trifluoro-3-oxobutanenitrile (6.00 g, 43.8 mmol, 1.00 eq), ammonium formate (8.28 g, 131 mmol, 3.00 eq) and acetic acid (0.25 mL, 4.38 mmol, 0.100 eq) in toluene (10.0 mL) was heated to reflux in a Dean-Stark apparatus for 18 hours. The mixture was concentrated under reduced pressure to afford (Z)-3-amino-4,4,4-trifluorobut-2-enenitrile (6.00 g, crude) as a yellow oil. It was used directly in the next step.

**Step 3:** To a solution of (Z)-3-amino-4,4,4-trifluorobut-2-enenitrile (6.00 g, 44.1 mmol, 1.00 eq) in dimethylformamide (30.0 mL) was added magnesium chloride (1.81 mL, 44.1 mmol, 1.00 eq) and sodium hydrogen sulfide (4.94 g, 88.2 mmol, 2.00 eq) in portions. The reaction was stirred at 25°C for 18 hours. The mixture was diluted with water (10.0 mL) and extracted with ethyl acetate (5 × 20.0 mL). The organic phases were gathered, washed with brine (20.0 mL), dried over sodium sulfate, filtered, and concentrated under reduced pressure to afford (Z)-3-amino-4,4,4-trifluoro-but-2-enethioamide (3.00 g, crude) as black oil. It was used directly in the next step.

**Step 4:** To an ice-cold mixture of (Z)-3-amino-4,4,4-trifluoro-but-2-enethioamide (1.50 g, 8.82 mmol, 1.00 eq) in pyridine (10.0 mL) was added hydrogen peroxide 30% purity (1.69 mL, 17.6 mmol, 2.00 eq) at 0°C. The mixture was stirred at 25°C for 2 hours. The mixture was dried under nitrogen to give a residue. The residue was diluted with aqueous sodium sulfite solution (20.0 mL) and extracted with ethyl acetate (3 × 20.0 mL). The combined organic layers were washed with saturated aqueous citric acid solution (2 × 20.0 mL) and brine (20.0 mL), dried over sodium sulfate, filtered, and concentrated under reduced pressure to give a residue. The residue was purified by silica gel column chromatography (petroleum ether/ethyl acetate = 5/1 to 1/1) to afford 3-(trifluoromethyl)isothiazol-5-amine (150 mg, 892 µmol, 10% yield) as yellow oil.

**Step 5:** To a solution of 3-(trifluoromethyl)isothiazol-5-amine (250 mg, 1.49 mmol, 1.00 eq) in pyridine (2.00 mL) was added phenyl chloroformate (0.28 mL, 2.23 mmol, 1.50 eq) dropwise at 0°C. The reaction was stirred for 4 hours at 25°C. The mixture was diluted with water (5.00 mL) and extracted with ethyl acetate (2 × 10.0 mL). The organic phases were gathered, washed with brine (10.0 mL), dried over sodium sulfate, filtered, and concentrated under reduced pressure to give a residue. The residue was purified by silica gel column chromatography (petroleum ether/ethyl acetate = 30/1 to 10/1) to afford phenyl (3-(trifluoromethyl)isothiazol-5-yl)carbamate (400 mg, 1.39 mmol, 93% yield) as a white solid.

**Compound 150:** General procedure A with variant iii) was used for the preparation with a yield of 54% from compound **VI** employing phenyl (4,5-dimethylthiazol-2-yl)carbamate.

¹H NMR (400 MHz, DMSO-*d₆*) *□* = 11.00 (s, 1H), 10.29 (br s, 1H), 7.64 (s, 1H), 7.60 - 7.52 (m, 2H), 7.13 (br s, 1H), 5.12 (dd, *J =* 5.0, 13.2 Hz, 1H), 4.48 - 4.43 (m, 1H), 4.41 (s, 2H), 4.35 - 4.26 (m, 1H), 2.98 - 2.85 (m, 1H), 2.59 (br d, *J* = 17.3 Hz, 1H), 2.43 - 2.35 (m, 1H), 2.17 (s, 3H), 2.08 (s, 3H), 2.04 - 1.94 (m, 1H). MS (ESI) m/z 428.1 [M+H]⁺

### Scheme for the preparation of phenyl (4,5-dimethylthiazol-2-yl)carbamate.

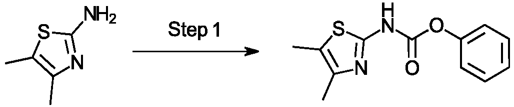

**Step** 1: Phenyl chloroformate (0.20 mL, 1.64 mmol, 1.05 eq) was added dropwise to solution of 4,5-dimethylthiazol-2-amine (0.200 g, 1.56 mmol, 1.00 eq) and pyridine (0.38 mL, 4.68 mmol, 3.00 eq) in dichloromethane (2.00 mL) at 0°C. The reaction was stirred at 0°C for 2 h. Additional phenyl chloroformate (49.0 µL, 390 µmol, 0.25 eq) was added, and the reaction was stirred at 0°C for 1 h. Water (2.00 mL) was added slowly over 30 min, and the mixture was diluted with dichloromethane (10 mL). The organic layer was washed with saturated aqueous sodium carbonate (3.00 mL) and brine (2.00 mL), dried over sodium sulfate, filtered, and concentrated under reduced pressure to give a residue. The residue was triturated with ethyl acetate/petroleum ether (1:5) at 25°C for 30 min to afford phenyl (4,5-dimethylthiazol-2-yl)carbamate (80.0 mg, 322 µmol, 21% yield) as a white solid.

**Compound 151:** General procedure A with variant iii) was used for the preparation with a yield of 60% from compound **VI** employing phenyl (4-methyl-3-(morpholinomethyl)-5-(trifluoromethoxy)phenyl)carbamate.

¹H NMR (400 MHz, DMSO-*d₆*) *□* = 10.99 (s, 1H), 8.91 (s, 1H), 8.16 (s, 1H), 7.70 - 7.64 (m, 1H), 7.64 - 7.60 (m, 1H), 7.60 - 7.52 (m, 2H), 7.17 (d, *J =* 2.2 Hz, 1H), 6.79 (t, *J =* 6.0 Hz, 1H), 5.11 (dd, *J =* 5.0, 13.3 Hz, 1H), 4.49 - 4.25 (m, 4H), 3.56 (br t, *J =* 4.3 Hz, 4H), 3.40 (s, 2H), 2.96 - 2.85 (m, 1H), 2.63 - 2.56 (m, 1H), 2.46 - 2.33 (m, 5H), 2.16 (s, 3H), 2.03 - 1.96 (m, 1H). MS (ESI) m/z 590.3 [M+H]⁺

### Scheme for the preparation of phenyl (4-methyl-3-(morpholinomethyl)-5-(trifluoromethoxy)phenyl)carbamate.

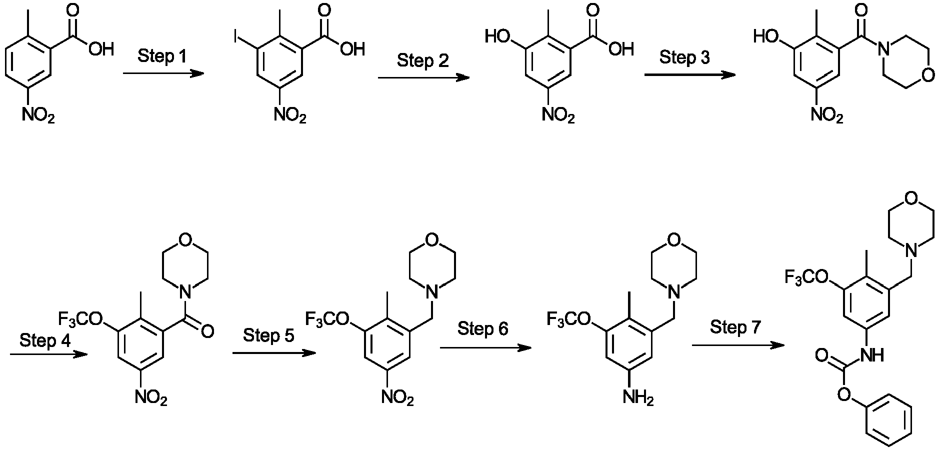

**Step 1:** To a solution of 2-methyl-5-nitro-benzoic acid (10.0 g, 55.2 mmol, 1.00 eq) in sulfuric acid (20.0 mL) was added N-lodosuccinimide (14.9 g, 66.3 mmol, 1.20 eq). The reaction was stirred at 60°C for 2 h. The mixture was diluted with ice water (200 mL) and filtered. The filter cake was washed with water (100 mL) and dried under vacuum to afford 3-iodo-2-methyl-5-nitro-benzoic acid (16.0 g, 52.1 mmol, 94% yield) as a white solid.

**Step 2:** To a solution of 3-iodo-2-methyl-5-nitro-benzoic acid (5.00 g, 16.3 mmol, 1.00 eq), copper iodide (310 mg, 1.63 mmol, 0.10 eq) and quinolin-8-ol (563 µL, 3.26 mmol, 0.20 eq) in water (3.00 mL) and dimethylsulfoxide (3.00 mL) was added a solution of potassium hydroxide (3.65 g, 65.1 mmol, 4.00 eq). The reaction was stirred at 100°C for 12 h. The mixture was diluted with water (50.0 mL) and extracted with ethyl acetate (2 × 50.0 mL). The combined organic layers were washed with water (50.0 mL), dried over anhydrous sodium sulfate, filtered, and concentrated under reduced pressure to afford 3-hydroxy-2-methyl-5-nitro-benzoic acid (3.20 g, crude) as a brown solid. It was used directly in the next step.

**Step 3:** To a solution of 3-hydroxy-2-methyl-5-nitro-benzoic acid (3.20 g, 16.2 mmol, 1.00 eq) and morpholine (1.71 mL, 19.5 mmol, 1.20 eq) in dichloromethane (100 mL) was added triethylamine (2.26 mL, 16.2 mmol, 1.00 eq) and O-(7-azabenzotriazol-1-yl)-N,N,N,N-tetramethyluroniumhexafluorophosphate (7.41 g, 19.5 mmol, 1.20 eq) at 20°C. The reaction was stirred at 20°C for 12 h. The mixture was concentrated under reduced pressure to give a residue. The residue was diluted with water (100 mL) and extracted with ethyl acetate (100 mL). The organic layer was washed with water (100 mL), dried over anhydrous sodium sulfate, filtered, and the filtrate was concentrated under reduced pressure to give a residue. The residue was purified by silica gel column chromatography (petroleum ether/ethyl acetate = 1/1 to 0/1) to afford (3-hydroxy-2-methyl-5-nitro-phenyl)-morpholino-methanone (1.40 g, crude) as a yellow solid.

**Step 4:** To a solution of (3-hydroxy-2-methyl-5-nitro-phenyl)-morpholino-methanone (1.30 g, 4.88 mmol, 1.00 eq), silver trifluoromethanesulfonate (6.27 g, 24.4 mmol, 5.00 eq), 1-(chloromethyl)-4-fluoro-1,4-diazoniabicyclo[2.2.2]octane bis(tetrafluoroborate) (3.46 g, 9.77 mmol, 2.00 eq), N-fluorobenzenesulfonimide (3.08 g, 9.77 mmol, 2.00 eq) and caesium fluoride (4.45 g, 29.3 mmol, 1.08 mL, 6.00 eq) in toluene (130 mL) was added trimethyl(trifluoromethyl)silane (3.47 g, 24.4 mmol, 5.00 eq) and 2-fluoropyridine (2.10 mL, 24.4 mmol, 5.00 eq) under nitrogen. The reaction was stirred at 20°C for 12 h under nitrogen. The mixture was filtered, and the filtrate was concentrated under reduced pressure to afford a residue. The residue was diluted with water (100 mL) and extracted with ethyl acetate (100 mL). The organic layer was washed with water (20.0 mL), dried over anhydrous sodium sulfate, filtered, and the filtrate was concentrated under reduced pressure to give a residue. The residue was purified by silica gel column chromatography (petroleum ether/ethyl acetate = 1/0 to 2/1) to afford (2-methyl-5-nitro-3-(trifluoromethoxy)phenyl)(morpholino)methanone (1.00 g, crude) as a yellow solid.

**Step 5:** To a solution of (2-methyl-5-nitro-3-(trifluoromethoxy)phenyl)-morpholino-methanone (900 mg, 2.69 mmol, 1.00 eq) in tetrahydrofuran (15.0 mL) was added borane dimethyl sulfide complex (10.0 M, 539 µL, 2.00 eq) at 0°C. The reaction was stirred at 60°C for 30 min. The mixture was quenched with methanol (2.00 mL) and concentrated under reduced pressure to afford a residue. The residue was purified by reversed phase column chromatography to afford 4-(2-methyl-5-nitro-3-(trifluoromethoxy) benzyl)morpholine (410 mg, 1.28 mmol, 48% yield) as a yellow oil.

**Step 6:** To a solution of 4-(2-methyl-5-nitro-3-(trifluoromethoxy)benzyl)morpholine (400 mg, 1.25 mmol, 1.00 eq) in methanol (5.00 mL) and water (5.00 mL) was added iron powder (488 mg, 8.74 mmol, 7.00 eq) and ammonium chloride (468 mg, 8.74 mmol, 7.00 eq). The reaction was stirred at 80°C for 2 h. The mixture was diluted with saturated sodium carbonate (1.00 mL) and extracted with ethyl acetate (2 × 10.0 mL). The combined organic layers were washed with water (5.00 mL), dried over anhydrous sodium sulfate, filtered, and the filtrate was concentrated under reduced pressure to afford a residue. The residue was purified by silica gel column chromatography (petroleum ether/ethyl acetate = 3/1 to 1/1) to afford 4-methyl-3-(morpholinomethyl)- 5-(trifluoromethoxy)aniline (270 mg, 930 µmol, 74% yield) as yellow oil.

**Step 7:** To a solution of 4-methyl-3-(morpholinomethyl)-5-(trifluoromethoxy)aniline (100 mg, 344 µmol, 1.00 eq) and potassium carbonate (57.1 mg, 413 µmol, 1.20 eq) in acetone (1.00 mL) was added phenyl chloroformate (47 µL, 379 µ, 1.10 eq) at 25°C. The reaction was stirred at 25°C for 1 h. The mixture was diluted with water (6.00 mL) and extracted with ethyl acetate (10.0 mL). The organic layer was dried over anhydrous sodium sulfate, filtered, and the filtrate was concentrated under reduced pressure to afford phenyl (4-methyl-3-(morpholinomethyl)-5-(trifluoromethoxy)phenyl)carbamate (141 mg, crude) as transparent oil. It was used directly in the next step.

**Compound 152:** General procedure A with variant iii) was used for the preparation with a yield of 78% from compound VI employing phenyl 3-chloro-4-methylbenzylcarbamate.

¹H NMR (400 MHz, DMSO-*d₆*) *□* = 10.98 (br s, 1H), 8.49 (s, 1 H), 7.63 (s, 1H), 7.56 - 7.48 (m, 2H), 7.29 - 7.23 (m, 2H), 7.13 - 7.08 (m, 1H), 6.68 - 6.53 (m, 2H), 5.15 - 5.07 (m, 1H), 4.46 - 4.38 (m, 1H), 4.35 - 4.26 (m, 3H), 4.19 (d, *J =* 6.0 Hz, 2H), 2.98 - 2.84 (m, 1 H), 2.63 - 2.58 (m, 1H), 2.43 - 2.31 (m, 1H), 2.28 (s, 3H), 2.05 - 1.93 (m, 1H). MS (ESI) m/z 455.2 [M+H]⁺

### Scheme for the preparation of phenyl 3-chloro-4-methylbenzylcarbamate.

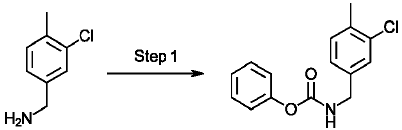

**Step** 1: To a solution of (3-chloro-4-methyl-phenyl)methanamine (500 mg, 3.21 mmol, 1.00 eq) and pyridine (0.78 mL, 9.64 mmol, 3.00 eq) in acetonitrile (5.00 mL) was added phenyl chloroformate (0.60 mL, 4.82 mmol, 1.50 eq). The reaction was stirred at 25°C for 12 h. The mixture was concentrated under reduced pressure to give a residue. The residue was diluted with ethyl acetate (30.0 mL) and water (50.0 mL). The organic layer was separated, and the aqueous phase was extracted with ethyl acetate (3 × 30.0 mL). The organic layers were gathered, dried over sodium sulfate, filtered, and concentrated under reduced pressure to give a residue. The residue was purified by silica gel column chromatography (petroleum ether/ethyl acetate = 3/1) to afford phenyl 3-chloro-4-methylbenzylcarbamate (632 mg, 2.29 mmol, 71 % yield) as a white solid.

**Compound 153:** General procedure A with variant iii) was used for the preparation with a yield of 19% from compound **VI** employing phenyl benzo[d][1,3]dioxol-5-ylcarbamate.

¹H NMR (400 MHz, DMSO-*d₆*) *□* = 10.97 (s, 1H), 8.49 (s, 1H), 7.65 (s, 1H), 7.55 (d, *J =* 1.0 Hz, 2H), 7.17 (d, *J =* 2.0 Hz, 1H), 6.80 - 6.74 (m, 1H), 6.72 - 6.68 (m, 1H), 6.65 (t, *J* = 6.0 Hz, 1H), 5.93 (s, 2H), 5.14 - 5.08 (m, 1H), 4.47 - 4.27 (m, 4H), 2.97 - 2.84 (m, 1H), 2.64 - 2.56 (m, 1H), 2.43 - 2.31 (m, 1H), 2.05 - 1.95 (m, 1H). MS (ESI) m/z 437.3[M+H]⁺

### Scheme for the preparation of phenyl benzo[d][1,3]dioxol-5-ylcarbamate.

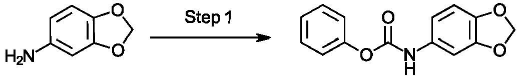

**Step 1:** To a solution of benzo[d][1,3]dioxol-5-amine (1.00 g, 7.29 mmol, 1.00 eq) and pyridine (1.77 mL, 21.9 mmol, 3.00 eq) in acetonitrile (10 mL) was added phenyl chloroformate (1.37 mL, 11.0 mmol, 1.50 eq). The reaction was stirred at 25°C for 12 h. The mixture was concentrated under reduced pressure to give a residue. The residue was diluted with ethyl acetate (30 mL) and water (50 mL). The organic layer was separated, and the aqueous layer was extracted with ethyl acetate (3 × 30 mL). The combined organic layers were dried over sodium sulfate, filtered, and concentrated under reduced pressure to give a residue. The residue was purified by silica gel column chromatography (petroleum ether/ethyl acetate = 3/1) to afford phenyl benzo[d][1,3]dioxol-5-ylcarbamate (1.80 g, crude) as a light yellow solid.

**Compound 154:** General procedure A with variant iii) was used for the preparation with a yield of 21% from compound **VI** employing phenyl (3-(1,4-diazabicyclo[3.2.1]octan-4-ylmethyl)-5-chloro-4-methylphenyl) carbamate.

¹H NMR (400 MHz, DMSO-*d₆*) *□* = 10.99 (br s, 1H), 8.95 (s, 1H), 8.28 (s, 1H), 7.66 (s, 1H), 7.62 (d, *J* = 2.2 Hz, 1H), 7.56 (d, *J =* 0.7 Hz, 2H), 7.15 (s, 1H), 7.08 - 6.96 (m, 1H), 5.12 (dd, *J* = 5.1, 13.3 Hz, 1H), 4.47 - 4.27 (m, 4H), 3.40 - 3.35 (m, 1 H), 3.30 - 3.27 (m, 1H), 3.18 - 3.14 (m, 1H), 2.96 - 2.81 (m, 5H), 2.67 - 2.53 (m, 4H), 2.46 - 2.35 (m, 2H), 2.26 (s, 3H), 2.11 - 1.95 (m, 2H), 1.60 - 1.46 (m, 1H). MS (ESI) m/z 565.3 [M+H]⁺

### Scheme for the preparation of phenyl (3-(1,4-diazabicyclo[3.2.1]octan-4-ylmethyl)-5-chloro-4-methylphenyl) carbamate.

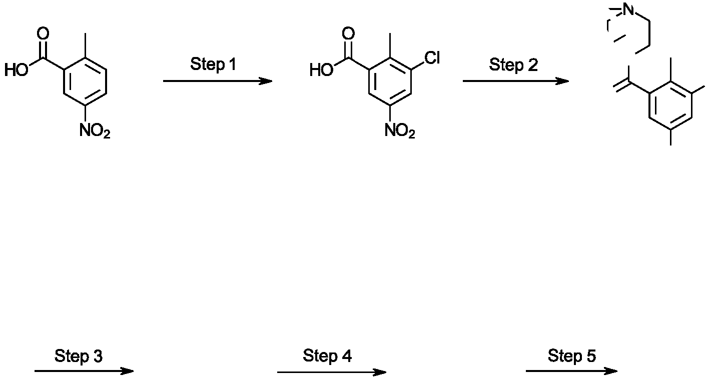

**Step 1:** To a solution of 2-methyl-5-nitro-benzoic acid (5.00 g, 27.6 mmol, 1.00 eq) in sulfuric acid (10.0 mL) was added 1,3-dichloro-5,5-dimethyl-imidazolidine-2,4-dione (6.53 g, 33.1 mmol, 1.20 eq) dropwise at 80°C. The reaction was stirred at 80°C for 12 h. The mixture was poured into ice water (100 mL) and filtered. The filter cake was dissolved into ethyl acetate (200 mL), and the organic layer was washed with water (100 mL), dried over anhydrous sodium sulfate, filtered, and concentrated under reduced pressure to afford 3-chloro-2- methyl-5-nitro-benzoic acid (5.00 g, 23.2 mmol, 84% yield) as a white solid.

**Step 2:** To a solution of 3-chloro-2-methyl-5-nitro-benzoic acid (388 mg, 1.80 mmol, 1.00 eq) and 1,4-diazabicyclo[3.2.1]octane dihydrochloride (300 mg, 1.62 mmol, 0.90 eq, dihydrochloride) in dichloromethane (5.00 mL) was added triethylamine (0.75 mL, 5.40 mmol, 3.00 eq) and O-(7-azabenzotriazol-1-yl)- N,N,N,N-tetramethyluroniumhexafluorophosphate (822 mg, 2.16 mmol, 1.20 eq) at 20°C. The reaction was stirred at 20°C for 2 h. The mixture was concentrated under reduced pressure to afford a residue. The residue was diluted with water (20.0 mL) and saturated aqueous sodium carbonate (3.00 mL), then extracted with ethyl acetate (50.0 mL). The organic layer was washed with water (10.0 mL), dried over anhydrous sodium sulfate, filtered, and the filtrate was concentrated under reduced pressure to afford (3-chloro-2-methyl-5-nitro-phenyl)-(1,4-diazabicyclo[3.2.1]octan-4-yl)methanone (600 mg, crude) as a yellow solid. It was used directly in the next step.

**Step 3:** To a solution of (3-chloro-2-methyl-5-nitro-phenyl)-(1 ,4-diazabicyclo[3.2.1]octan-4-yl)methanone (700 mg, 2.26 mmol, 1.00 eq) in tetrahydrofuran (20.0 mL) was added borane dimethyl sulfide complex (10.0 M, 0.45 mL, 2.00 eq) at 0°C. The reaction was stirred at 60°C for 30 min. The mixture was quenched with methanol (0.500 mL) and concentrated under reduced pressure to give a residue. The residue was diluted with water (10.0 mL) and saturated aqueous sodium carbonate (10.0 mL), then extracted with ethyl acetate (30.0 mL). The organic layer was washed with water (10.0 mL), dried over anhydrous sodium sulfate, filtered, and the filtrate was concentrated under reduced pressure to give a residue. The residue was purified by silica gel column chromatography (petroleum ether/ethyl acetate = 1/0 to 2/1) to afford 4-(3-chloro-2-methyl-5-nitrobenzyl)-1,4-diazabicyclo [3.2.1]octane (150 mg, 507 µmol, 22% yield) as a white solid.

**Step 4:** To a solution of 4-(3-chloro-2-methyl-5-nitrobenzyl)-1 ,4-diazabicyclo[3.2.1]octane (150 mg, 507 µmol, 1.00 eq) in methanol (1.50 mL) and water (1.50 mL) was added iron powder (198 mg, 3.55 mmol, 7.00 eq) and ammonium chloride (190 mg, 3.55 mmol, 7.00 eq). The reaction was stirred at 80°C for 2 h. The mixture was diluted with saturated aqueous sodium carbonate (1.00 mL) and extracted with ethyl acetate (2 × 10.0 mL). The organic layers were washed with water (5.00 mL), dried over anhydrous sodium sulfate, filtered, and the filtrate was concentrated under reduced pressure to afford 3-(1,4-diazabicyclo[3.2.1]octan-4-ylmethyl)-5-chloro-4-methylaniline (120 mg, crude) as yellow oil. It was used directly in the next step.

**Step 5:** To a solution of 3-(1,4-diazabicyclo[3.2.1]octan-4-ylmethyl)-5-chloro-4-methylaniline (100 mg, 376 µmol, 1.00 eq) and potassium carbonate (62.4 mg, 452 µmol, 1.20 eq) in acetone (2.00 mL) was added phenyl chloroformate (51.8 µL, 414 µmol, 1.10 eq) at 25°C. The reaction was stirred at 25°C for 1 h. The mixture was diluted with water (3.00 mL) and extracted with ethyl acetate (5.00 mL). The organic layer was dried over anhydrous sodium sulfate, filtered, and the filtrate was concentrated under reduced pressure to afford phenyl (3-(1,4-diazabicyclo[3.2.1] octan-4-ylmethyl)-5-chloro-4-methylphenyl)carbamate (145 mg, crude) as transparent oil. It was used directly in the next step.

**Compound 155:** General procedure A with variant iii) was used for the preparation with a yield of 11% from compound **VI** employing phenyl (3-chloro-4-methyl-5-(3-methyl-3-azabicyclo[3.1.0]hexan-1-yl)phenyl)carbamate.

¹H NMR (400 MHz, DMSO-*d₆*) *□* = 10.99 (br s, 1H), 9.02 (s, 1H), 8.31 (s, 1H), 7.71 - 7.50 (m, 4H), 7.19 (d, *J =* 2.0 Hz, 1H), 7.06 (br s, 1H), 5.12 (dd, *J =* 5.2, 13.2 Hz, 1H), 4.52 - 4.25 (m, 4H), 3.12 - 2.85 (m, 4H), 2.63 - 2.57 (m, 1H), 2.40 (br dd, *J =* 4.2, 13.2 Hz, 1H), 2.31 (s, 3H), 2.26 (s, 3H), 2.19 (d, *J* = 8.6 Hz, 1H), 2.06 - 1.94 (m, 1H), 1.63 (td, *J* = 3.6, 7.6 Hz, 1H), 1.32 - 1.13 (m, 1H), 0.59 (dd, *J* = 3.6, 8.0 Hz, 1H). MS (ESI) m/z 536.3 [M+H]⁺

### Scheme for the preparation of phenyl (3-chloro-4-methyl-5-(3-methyl-3-azabicyclo[3.1.0]hexan-1-yl)phenyl)carbamate.

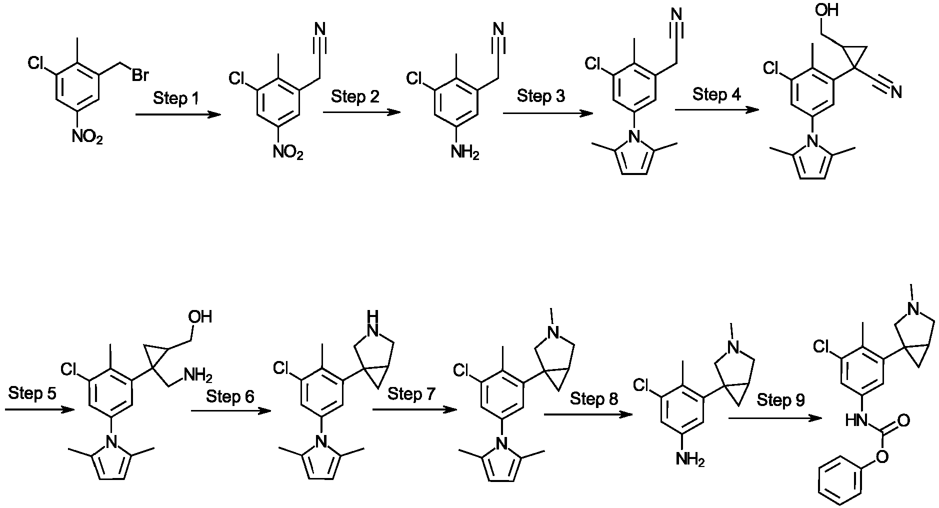

**Step** 1: A solution of 1-(bromomethyl)-3-chloro-2-methyl-5-nitrobenzene (25.0 g, 94.5 mmol, 1.00 eq) and sodium cyanide (6.02 g, 123 mmol, 1.30 eq) in acetonitrile (270 mL) and water (36.0 mL) was stirred at 80°C for 10 h under nitrogen. The reaction was quenched by addition of saturated aqueous sodium carbonate (60.0 mL), and the mixture was extracted with ethyl acetate (3 × 200 mL). The combined organic layers were washed with brine (3 × 100 mL), dried over sodium sulfate, filtered, and concentrated under reduced pressure to give a residue. The residue was purified by silica gel column chromatography (petroleum ether/ethyl acetate = 0/1 to 1/4) to afford 2-(3-chloro-2-methyl-5-nitrophenyl)acetonitrile (17.9 g, 85.0 mmol, 90% yield) as a white solid.

**Step 2:** A mixture of 2-(3-chloro-2-methyl-5-nitrophenyl)acetonitrile (17.9 g, 85.0 mmol, 1.00 eq), ferrous powder (23.7 g, 425 mmol, 5.00 eq) and ammonium chloride (4.55 g, 85.0 mmol, 1.00 eq) in ethanol (160 mL) and water (80.0 mL) was stirred at 60°C for 10 h. The mixture was concentrated under reduced pressure to give a residue. The residue was diluted with water (80.0 mL) and extracted with ethyl acetate (3 × 150 mL). The combined organic layers were washed with brine (3 × 50.0 mL), dried over sodium sulfate, filtered, and concentrated under reduced pressure to give a residue. The residue was purified by silica gel column chromatography (petroleum ether/ethyl acetate = 3/1) to afford 2-(5-amino-3-chloro-2-methylphenyl)acetonitrile (14.7 g, 81.4 mmol, 96% yield) as yellow oil.

**Step 3:** A mixture of 2-(5-amino-3-chloro-2-methylphenyl)acetonitrile (14.7 g, 81.4 mmol, 1.00 eq), hexane-2,5-dione (9.29 g, 81.4 mmol, 9.55 mL, 1.00 eq) and *p*-toluenesulfonic acid monohydrate (0.16 g, 841 µmol, 0.002 eq) in toluene (150 mL) was stirred at 110°C for 10 h. The mixture was concentrated under reduced pressure to give a residue. The residue was purified by silica gel column chromatography (petroleum ether/ethyl acetate = 5/1) to afford 2-(3-chloro-5-(2,5-dimethyl-1H-pyrrol-1-yl) -2-methylphenyl)acetonitrile (15.0 g, 58.0 mmol, 71% yield) as a yellow solid.

**Step 4:** To a solution of sodium hexamethyldisilazane (1.00 M, 34.8 mL, 1.80 eq) in tetrahydrofuran (50.0 mL) was added 2-(3-chloro-5-(2,5-dimethyl-1H-pyrrol-1-yl)-2-methylphenyl)acetonitrile (5.00 g, 19.3 mmol, 1.00 eq) at -10°C. After 12 mins, 2-(chloromethyl)oxirane (2.23 g, 24.2 mmol, 1.89 mL, 1.25 eq) was added, and the reaction was stirred at 25°C for 12 h. Water (20.0 mL) was added to quench the reaction, and the mixture was extracted with ethyl acetate (3 × 50.0 mL). The combined organic layers were washed with brine (20.0 mL), dried over sodium sulfate, filtered, and concentrated under reduced pressure to give a residue. The residue was purified by silica gel column chromatography (petroleum ether/ethyl acetate = 1/1) to afford 1-(3-chloro-5-(2,5-dimethyl-1H-pyrrol-1-yl)-2-methylphenyl)-2-(hydroxymethyl)cyclopropanecarbonitrile (3.00 g, 9.53 mmol, 49% yield) as a yellow solid.

**Step 5:** To a solution of 1-(3-chloro-5-(2,5-dimethyl-1H-pyrrol-1-yl)-2-methylphenyl)-2-(hydroxymethyl) cyclopropanecarbonitrile (3.50 g, 11.1 mmol, 1.00 eq) in tetrahydrofuran (40.0 mL) was added borane dimethyl sulfide complex (10.0 M, 3.34 mL, 3.00 eq) at 0°C. The reaction was stirred at 50°C for 0.5 h. The reaction was quenched by addition methanol (35.0 mL) and concentrated under reduced pressure to afford (2-(aminomethyl)-2-(3-chloro-5-(2,5-dimethyl-1H-pyrrol-1-yl)-2-methylphenyl) cyclopropyl) methanol (3.50 g, 11.1 mmol, crude) as a yellow solid. It was used directly in the next step.

**Step 6:** To a solution of (2-(aminomethyl)-2-(3-chloro-5-(2,5-dimethyl-1H-pyrrol-1-yl)-2-methylphenyl)cyclopropyl)methanol (3.50 g, 11.0 mmol, 1.00 eq) and triphenylphosphine (3.46 g, 13.2 mmol, 1.20 eq) in tetrahydrofuran (35.0 mL) was added diisopropyl azodicarboxylate (2.56 mL, 13.2 mmol, 1.20 eq) at 0°C. The reaction was stirred at 25°C for 10 h. The mixture was concentrated under reduced pressure to give a residue. The residue was purified twice by reversed phase column chromatography and lyophilized to afford 1-(3-chloro-5-(2,5-dimethyl-1H-pyrrol-1-yl)-2-methylphenyl)-3-azabicyclo[3.1.0]hexane (0.20 g, 565 µmol, 5.15% yield) as yellow oil.

**Step 7:** To a solution of 1-(3-chloro-5-(2,5-dimethyl-1H-pyrrol-1-yl)-2-methylphenyl)-3-azabicyclo[3.1.0]hexane (0.20 g, 577 µmol, 1.00 eq), sodium cyanoborohydride (109 mg, 1.73 mmol, 3.00 eq) and formaldehyde 37% purity (1.67 mL, 22.4 mmol, 38.8 eq) in methanol (5.00 mL) was added acetic acid (33.0 µL, 577 µmol, 1.00 eq). The reaction was stirred at 25°C for 2 h. The mixture was diluted with water (10.0 mL) and extracted with dichloromethane (3 × 30.0 mL). The combined organic layers were washed with brine (10.0 mL), dried over sodium sulfate, filtered, and concentrated under reduced pressure to afford 1-(3-chloro-5-(2,5-dimethyl-1H-pyrrol-1-yl)-2-methylphenyl)-3-methyl-3-azabicyclo[3.1.0] hexane (0.10 g, 577 €mol, crude) as yellow oil. It was used directly in the next step.

**Step 8:** A solution of 1-(3-chloro-5-(2,5-dimethyl-1H-pyrrol-1-yl)-2-methylphenyl)-3-methyl-3-azabicyclo[3.1.0] hexane (0.10 g, 318 µmol, 1.00 eq) and hydroxylamine hydrochloride (221 mg, 3.18 mmol, 10.0 eq) in ethanol (1.00 mL) and water (0.50 mL) was stirred at 100°C for 12 h. The mixture was concentrated under reduced pressure to give a residue. The residue was purified was by reversed phase column chromatography and lyophilized to afford 3-chloro-4-methyl-5-(3-methyl-3-azabicyclo[3.1.0]hexan-1-yl)aniline (50.0 mg, 211 µmol, 67% yield) as yellow oil.

**Step 9:** To a solution of 3-chloro-4-methyl-5-(3-methyl-3-azabicyclo[3.1.0]hexan-1-yl)aniline (50.0 mg, 211 µmol, 1.00 eq) and potassium carbonate (58.4 mg, 422 µmol, 2.00 eq) in acetone (1.00 mL) was added phenyl chloroformate (31.7 µL, 253 µmol, 1.20 eq). The reaction was stirred at 25°C for 10 h. The mixture was diluted with water (8.00 mL) and extracted with ethyl acetate (3 × 30.0 mL). The combined organic layers were washed with brine (10.0 mL), dried over sodium sulfate, filtered, and concentrated under reduced pressure to afford phenyl (3-chloro-4-methyl-5-(3-methyl-3-azabicyclo[3.1.0] hexan-1-yl)phenyl)carbamate (0.80 g, 253 µmol, crude) as yellow oil. It was used directly in the next step.

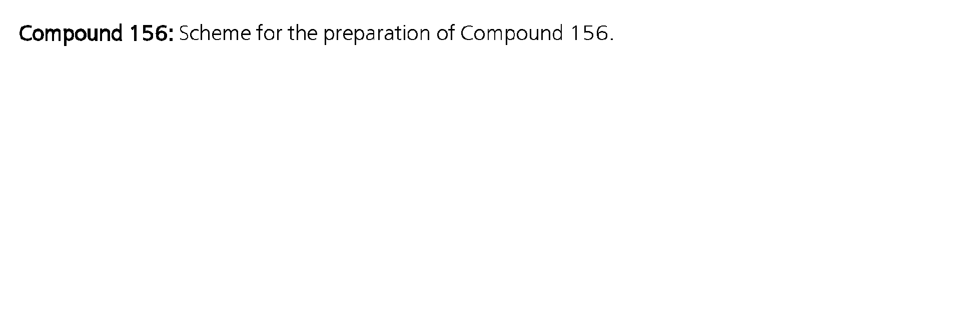

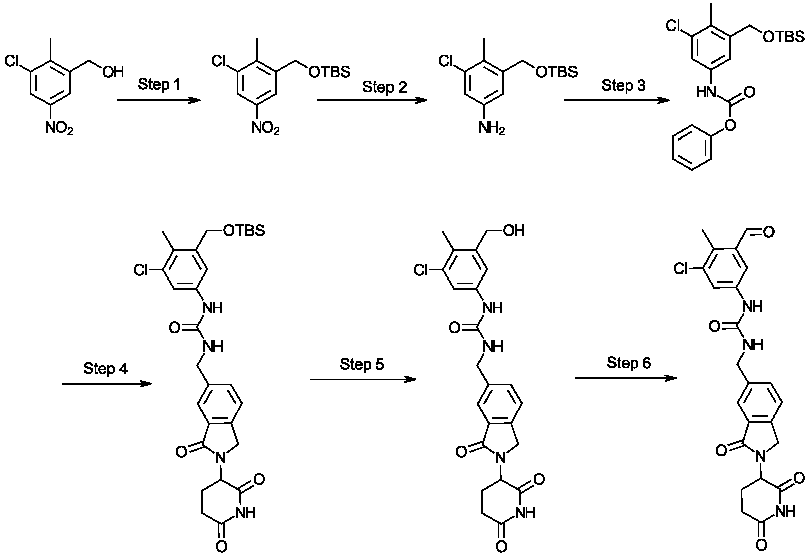

**Step** 1: To a solution of (3-chloro-2-methyl-5-nitro-phenyl)methanol (1.00 g, 4.96 mmol, 1.00 eq) and imidazole (675 mg, 9.92 mmol, 2.00 eq) in dichloromethane (10.0 mL) was added tert-butyldimethylsilyl chloride (0.73 mL, 5.95 mmol, 1.20 eq) at 0°C. The reaction was stirred at 25°C for 1 h. The mixture was concentrated under reduced pressure to give a residue. The residue was purified by silica gel column chromatography (petroleum ether/ethyl acetate = 1/0 to 50/1) to afford tert-butyl((3-chloro-2-methyl-5-nitrobenzyl)oxy)dimethylsilane (1.20 g, 3.80 mmol, 76% yield) as light yellow oil.

**Step 2:** A mixture of tert-butyl((3-chloro-2-methyl-5-nitrobenzyl)oxy)dimethylsilane (1.50 g, 4.75 mmol, 1.00 eq), iron powder (795 mg, 14.2 mmol, 3.00 eq) and ammonium chloride (1.27 g, 23.7 mmol, 5.00 eq) in methanol (10.0 mL) and water (5.00 mL) was stirred at 80°C for 1 h. The mixture was filtered, and the filtrate was concentrated under reduced pressure to give a residue. The residue was added to water (1 00mL) and stirred for 10 min. The mixture was extracted with ethyl acetate (3 × 60.0 mL). The combined organic phases were washed with brine (100 mL), dried over anhydrous sodium sulfate, filtered, and concentrated under reduced pressure to afford 3-(((*tert*-butyldimethylsilyl)oxy)methyl)-5-chloro-4-methylaniline (900 mg, 3.15 mmol, 66% yield) as a yellow solid.

**Step 3:** To a solution of 3-(((*tert*-butyldimethylsilyl)oxy)methyl)-5-chloro-4-methylaniline (900 mg, 3.15 mmol, 1.00 eq) and pyridine (0.76 mL, 9.44 mmol, 3.00 eq) in acetonitrile (10.0 mL) was added phenyl chloroformate (0.43 mL, 3.46 mmol, 1.10 eq) at 0°C. The reaction was stirred at 25°C for 1 h. The mixture was concentrated to give a residue. Water (100 mL) was added, and the mixture was stirred for 10 min. The mixture was extracted with ethyl acetate (3 × 60.0 mL). The combined organic layers were washed with brine (100 mL), dried with anhydrous sodium sulfate, filtered, and concentrated under reduced pressure to give a residue. The residue was purified by silica gel column chromatography (petroleum ether/ethyl acetate = 1/0 to 10/1) to afford phenyl (3-(((*tert*-butyldimethylsilyl)oxy) methyl)-5-chloro-4-methylphenyl)carbamate (900 mg, 2.22 mmol, 70% yield) as a yellow solid.

**Step 4:** To a solution of 3-(6-(aminomethyl)-1-oxoisoindolin-2-yl)piperidine-2,6-dione hydrochloride **VI** (359 mg, 1.31 mmol, 1.00 eq, hydrochloride) and triethylamine (0.73 mL, 5.25 mmol, 4.00 eq) in dimethylformamide (5.00 mL) was added phenyl (3-(((*tert-*butyldimethylsilyl)oxy)methyl)-5-chloro-4-methylphenyl)carbamate (800 mg, 1.97 mmol, 1.50 eq). The reaction was stirred at 25°C for 1 h. The mixture was filtered, and the filtrate was concentrated under reduced pressure to give a residue. The residue was purified by reversed phase preparative HPLC to afford 1-(3-(((tert-butyldimethylsilyl)oxy)methyl)-5-chloro-4-methylphenyl)-3-((2-(2,6-dioxopiperidin-3-yl)-3-oxoisoindolin-5-yl)methyl)urea (350 mg, 598 µmol, 45% yield) as a white solid.

**Step 5:** To a solution of 1-(3-(((tert-butyldimethylsilyl)oxy)methyl)-5-chloro-4-methylphenyl)-3-((2-(2,6-dioxopiperidin-3-yl)-3-oxoisoindolin-5-yl)methyl)urea (350 mg, 598 µmol, 1.00 eq) in tetrahydrofuran (5.00 mL) was added tetrabutylammonium fluoride trihydrate (283 mg, 897 µmol, 1.50 eq). The reaction was stirred at 25°C for 2 h. Saturated aqueous ammonium chloride (20.0 mL) was added to quench the reaction, and the mixture was poured into water (60.0 mL) and stirred for 10 min. The aqueous layer was extracted with ethyl acetate (3 × 50.0 mL). The combined organic layers were washed with brine (100 mL), dried over anhydrous sodium sulfate, filtered, and concentrated under reduced pressure to give a residue. The residue was purified by reversed phase preparative HPLC to afford 1-(3-chloro-5-(hydroxymethyl)-4-methylphenyl)-3-((2-(2,6-dioxopiperidin-3-yl)-3-oxoisoindolin-5-yl)methyl)urea (180 mg, 382 µmol, 64% yield) as a white solid.

**Step 6:** To a solution of 1-(3-chloro-5-(hydroxymethyl)-4-methylphenyl)-3-((2-(2,6-dioxopiperidin-3-yl)-3- oxoisoindolin-5-yl)methyl)urea (150 mg, 318 °µmol, 1.00 eq) in dichloromethane (1.00 mL) was added manganese dioxide (110 mg, 1.27 mmol, 4.00 eq). The reaction was stirred at 25°C for 12 h. The mixture was filtered, and the filtrate was concentrated under reduced pressure to give a residue. The residue was purified by reversed phase preparative HPLC to afford **Compound 156** (37.38 mg, 72.6 µmol, 23% yield) as a white solid.

¹H NMR (400 MHz, DMSO-*d₆*) *□* = 10.98 (s, 1H), 10.20 (s, 1H), 9.21 (s, 1H), 7.94 (d, *J =* 2.3 Hz, 1H), 7.78 (d, *J =* 2.4 Hz, 1H), 7.70 - 7.64 (m, 1H), 7.62 - 7.52 (m, 2H), 7.16 - 7.02 (m, 1H), 5.12 (dd, *J* = 5.1, 13.3 Hz, 1H), 4.52 - 4.36 (m, 3H), 4.36 (br s, 1H), 2.97 - 2.82 (m, 1H), 2.69 - 2.60 (m, 1H), 2.58 - 2.55 (m, 3H), 2.47 - 2.33 (m, 1H), 2.06 - 1.96 (m, 1H). MS (ESI) m/z 469.2 [M+H]⁺

**Compound 157:** General procedure A with variant iii) was used for the preparation with a yield of 66% from compound **VI** employing phenyl (5-chloro-6-methylpyridin-3-yl)carbamate.

¹H NMR (400 MHz, DMSO-*d₆*) *□* = 10.98 (s, 1H), 9.83 (s, 1H), 8.60 (d, *J* = 2.2 Hz, 1H), 8.27 (d, *J* = 2.2 Hz, 1H), 7.66 (s, 1H), 7.56 (s, 2H), 7.40 (br s, 1H), 5.16 - 5.06 (m, 1H), 4.41 - 4.27 (m, 4H), 2.96 - 2.84 (m, 1H), 2.59 (br d, *J* = 18.2 Hz, 1H), 2.54 (s, 3H), 2.43 - 2.33 (m, 1H), 2.04 - 1.94 (m, 1H). MS (ESI) m/z 442.2 [M+H]⁺

### Scheme for the preparation of phenyl (5-chloro-6-methylpyridin-3-yl)carbamate.

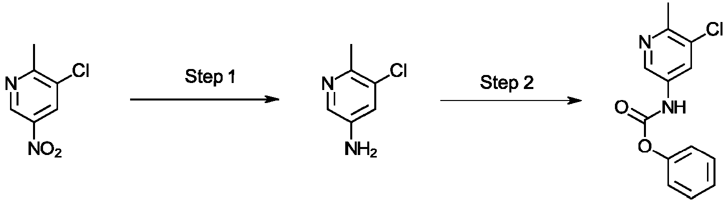

**Step 1:** A mixture of 3-chloro-2-methyl-5-nitropyridine (500 mg, 2.90 mmol, 1.00 eq), iron powder (1.13 g, 20.3 mmol, 7.00 eq) and ammonium chloride (1.08 g, 20.3 mmol, 7.00 eq) in methanol (5.00 mL) and water (5.00 mL) was stirred at 80°C for 2 h. The mixture was filtered over Celite, and the filtrate was concentrated under reduced pressure to afford 5-chloro-6-methylpyridin-3-amine (500 mg, crude) as brown oil. It was used directly in the next step.

**Step 2:** To a solution of 5-chloro-6-methylpyridin-3-amine (200 mg, 1.40 mmol, 1.00 eq) and pyridine (0.34 mL, 4.21 mmol, 3.00 eq) in acetonitrile (2.00 mL) was added phenyl chloroformate (264 µL, 2.10 mmol, 1.50 eq). The reaction was stirred at 25°C for 12 h. The mixture was concentrated under reduced pressure to give a residue. Ethyl acetate (30.0 mL) and water (50.0 mL) were added, and the organic layer was separated. The aqueous phase was extracted with ethyl acetate (3 × 100 mL). The combined organic layers were dried over sodium sulfate, filtered, and concentrated under reduced pressure to give a residue. The residue was purified by silica gel column chromatography (petroleum ether/ethyl acetate = 3 / 1) to afford phenyl (5-chloro-6-methylpyridin-3-yl)carbamate (320 mg, 1.17 mmol, 83% yield) as a light yellow solid.

**Compound 158:** General procedure A with variant iii) was used for the preparation with a yield of 57% from compound **VI** employing phenyl (2,6-dimethylpyridin-4-yl)carbamate.

¹H NMR (400 MHz, DMSO-*d₆*) *□* = 10.99 (s, 1H), 9.16 (br s, 1H), 8.19 (s, 1H), 7.66 (s, 1H), 7.57 (s, 2H), 7.16 (brt, *J* = 5.7 Hz, 1H), 7.11 (s, 2H), 5.12 (dd, *J =* 5.1, 13.3 Hz, 1H), 4.45 - 4.31 (m, 4H), 2.98 - 2.85 (m, 1H), 2.64 - 2.56 (m, 1H), 2.46 - 2.37 (m, 1H), 2.32 (s, 6H), 2.05 - 1.95 (m, 1H). MS (ESI) m/z 422.1 [M+H]⁺

### Scheme for the preparation of phenyl (2,6-dimethylpyridin-4-yl)carbamate.

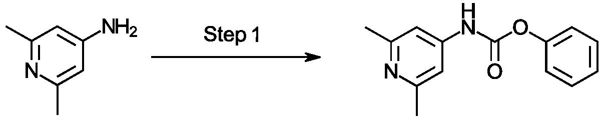

**Step** 1: To a solution of 2,6-dimethylpyridin-4-amine (1.00 g, 8.19 mmol, 1.00 eq) in acetonitrile (20.0 mL) was added pyridine (3.30 mL, 40.9 mmol, 5.00 eq) and phenyl chloroformate (1.54 mL, 12.2 mmol, 1.50 eq) at 0°C. The reaction was stirred at 25°C for 0.5 h. The mixture was concentrated under reduced pressure to give a residue. The residue was purified by reversed phase HPLC to afford phenyl (2,6-dimethylpyridin-4-yl)carbamate (600 mg, 2.48 mmol, 30% yield) as a yellow solid.

**Compound 159:** General procedure A with variant iii) was used for the preparation with a yield of 41% from compound **VI** employing phenyl (3-(difluoromethoxy)-4-methylphenyl)carbamate.

¹H NMR (400 MHz, DMSO-*d₆*) *□* = 10.99 (br s, 1H), 8.82 (s, 1H), 7.66 (s, 1H), 7.57 (s, 2H), 7.43 (s, 1H), 7.09 (t, *J* =74.0 Hz, 1H), 7.14 - 7.07 (m, 2H), 6.83 (br t, *J=* 6.0 Hz, 1H), 5.12 (dd, *J =* 5.1, 13.2 Hz, 1H), 4.49 - 4.26 (m, 4H), 3.00 - 2.84 (m, 1H), 2.64 - 2.57 (m, 1H), 2.40 (dq, *J =* 4.6, 13.2 Hz, 1H), 2.14 (s, 3H), 2.05 - 1.95 (m, 1H). MS (ESI) m/z 473.1 [M+H]⁺

### Scheme for the preparation of phenyl (3-(difluoromethoxy)-4-methylphenyl)carbamate.

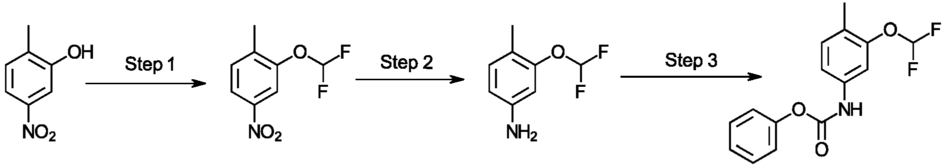

**Step 1:** To a solution of 2-methyl-5-nitrophenol (5.00 g, 32.7 mmol, 1.00 eq) and sodium 2-chloro-2,2- difluoroacetate (12.4 g, 81.6 mmol, 2.50 eq) in dimethyl formamide (50.0 mL) was added caesium carbonate (21.3 g, 65.3 mmol, 2.00 eq) in portions. The reaction was stirred at 100°C for 2 h. The mixture was diluted with water (800 mL) and extracted with ethyl acetate (3 × 100 mL). The combined organic layers were washed with brine (80.0 mL), dried over anhydrous sodium sulfate, filtered, and concentrated under reduced pressure to give a residue. The residue was purified by silica gel column chromatography (petroleum ether/ethyl acetate = 1/0 to 10/1) to afford 2-(difluoromethoxy)-1-methyl-4-nitrobenzene (5.35 g, 26.3 mmol, 81% yield) as transparent oil.

**Step 2:** To a solution of 2-(difluoromethoxy)-1-methyl-4-nitrobenzene (4.85 g, 23.8 mmol, 1.00 eq) and ammonium chloride (6.39 g, 1 19 mmol, 5.00 eq) in methanol (40.0 mL) and water (40.0 mL) was added iron powder (4.00 g, 71.6 mmol, 3.00 eq) in portions. The reaction was stirred at 80°C for 2 h. The mixture was filtered, and the filtrate was concentrated under reduced pressure to give a residue. Water (100 mL) was added, and the mixture was extracted with ethyl acetate (3 × 50.0 mL). The combined organic layers were washed with brine (20 mL) and dried over anhydrous sodium sulfate, filtered, and concentrated to give 3-(difluoromethoxy)-4-methylaniline (2.75 g, crude) as brown oil. It was used directly in the next step.

**Step 3:** To a solution of 3-(difluoromethoxy)-4-methylaniline (1.00 g, 5.78 mmol, 1.00 eq) and pyridine (1.40 mL, 17.3 mmol, 3.00 eq) in acetonitrile (10.0 mL) was added phenyl chloroformate (1.09 mL, 8.66 mmol, 1.50 eq) dropwise. The reaction was stirred at 25°C for 12 h. The mixture was diluted with water (150 mL) and extracted with ethyl acetate (3 × 50.0 mL). The combined organic layers were washed with brine (20.0 mL), dried over anhydrous sodium sulfate, filtered, and concentrated under reduced pressure to give a residue. The residue was purified by silica gel column chromatography (petroleum ether/ethyl acetate = 1/0 to 10/1) to afford phenyl (3-(difluoromethoxy)-4-methylphenyl)carbamate (1.50 g, 5.11 mmol, 89% yield) as a yellow solid.

**Compound 160:** General procedure A with variant iii) was used for the preparation with a yield of 19% from compound **VI** employing phenyl (3-chloro-4-methyl-5-((4-methylmorpholin-3-yl)methyl)phenyl)carbamate.

¹H NMR (400 MHz, DMSO-*d₆*) *□* = 10.98 (br s, 1H), 8.74 (s, 1H), 7.66 (s, 1H), 7.57 (s, 3H), 7.02 (d, *J =* 2.0 Hz, 1H), 6.82 (br t, *J* = 5.6 Hz, 1H), 5.12 (dd, *J* = 5.2, 13.2 Hz, 1H), 4.50 - 4.26 (m, 4H), 3.68 - 3.59 (m, 1H), 3.57 - 3.49 (m, 1H), 3.17 (dd, *J* = 7.8, 11.2 Hz, 1H), 3.07 (br dd, *J =* 3.4, 13.2 Hz, 1H), 2.98 - 2.84 (m, 1H), 2.72 - 2.57 (m, 3H), 2.43 - 2.37 (m, 2H), 2.34 (s, 3H), 2.29 - 2.17 (m, 5H), 2.01 (dt, *J =* 1.8, 6.2 Hz, 1H). MS (ESI) m/z 554.2 [M+H]⁺

### Scheme for the preparation of phenyl (3-chloro-4-methyl-5-((4-methylmorpholin-3-yl)methyl)phenyl)carbamate.

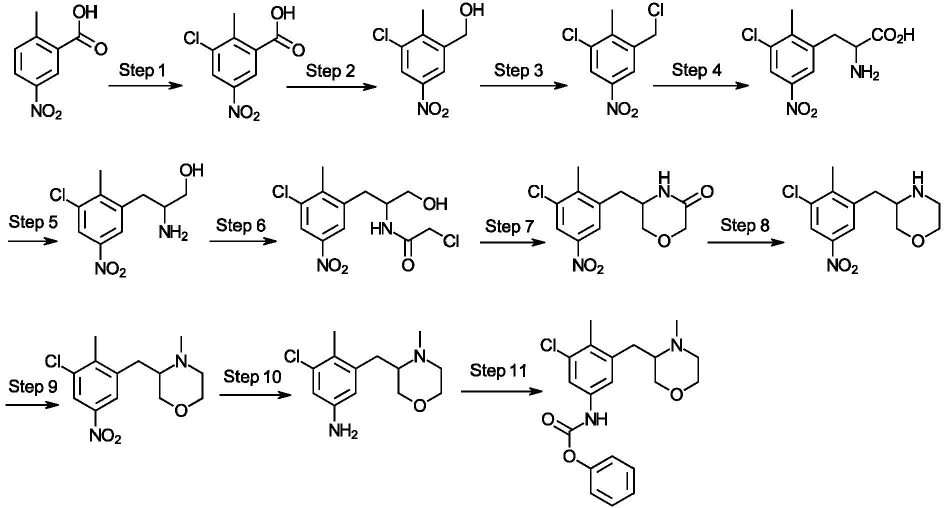

**Step 1:** A mixture of 2-methyl-5-nitrobenzoic acid (20.0 g, 110 mmol, 1.00 eq) and 1,3-dichloro-5,5- dimethylimidazolidine-2,4-dione (21.8 g, 110 mmol, 1.00 eq) in sulfuric acid (20.0 mL) was stirred at 80°C for 10 h. The mixture was poured into ice water (about 300 mL) under stirring. The resulting precipitate was collected by filtration and washed with water to afford 3-chloro-2-methyl-5-nitrobenzoic acid (24.0 g, crude) as a white solid. It was used directly in the next step.

**Step 2:** To a solution of 3-chloro-2-methyl-5-nitrobenzoic acid (24.0 g, 111 mmol, 1.00 eq) in tetrahydrofuran (200 mL) was added borane dimethyl sulfide complex (10.0 M, 22.3 mL, 2.00 eq) at 0°C. The reaction was stirred at 25°C for 10 h. Water (50.0 mL) was added to quench the reaction, and the mixture was extracted with ethyl acetate (3 × 200 mL). The combined organic layers were washed with brine (100 mL), dried over sodium sulfate, filtered, and concentrated under reduced pressure to afford (3-chloro-2-methyl-5-nitrophenyl)methanol (23.0 g, 111 mmol, crude) as a white solid. It was used directly in the next step.

**Step 3:** To a solution of (3-chloro-2-methyl-5-nitrophenyl)methanol (23.0 g, 114 mmol, 1.00 eq) in dichloromethane (200 mL) was added thionyl chloride (41.4 mL, 570 mmol, 5.00 eq). The reaction was stirred at 25°C for 10 h. The mixture was poured into ice water (50.0 mL) and extracted with dichloromethane (3 × 150 mL). The combined organic layers were washed with saturated aqueous sodium bicarbonate (2 × 50.0 mL) and brine (50.0 mL), dried over magnesium sulfate, filtered, and concentrated under reduced pressure to afford 1-chloro-3-(chloromethyl)-2-methyl-5-nitrobenzene (26.0 g, 114 mmol, crude) as a yellow oil.

**Step 4:** To a solution of sodium hydride 60% purity (1.00 g, 25.0 mmol, 1.10 eq) in dimethylformamide (50.0 mL) was added diethyl 2-acetamidomalonate (5.92 g, 27.3 mmol, 1.20 eq) at 0°C. After 5 mins, 1-chloro-3-(chloromethyl)-2-methyl-5-nitrobenzene (5.00 g, 22.7 mmol, 1.00 eq) was added. The reaction was stirred at 25°C for 10 h. Water (50.0 mL) was added to quench the reaction, and the mixture was extracted with ethyl acetate (3 × 100 mL). The combined organic layers were washed with brine (3 × 50.0 mL), dried over sodium sulfate, filtered, and concentrated under reduced pressure to afford diethyl 2-acetamido-2-(3-chloro-2-methyl-5-nitrobenzyl)malonate (9.20 g, 27.3 mmol, crude) as a yellow solid.

**Step 5:** To a solution of 2-amino-3-(3-chloro-2-methyl-5-nitrophenyl)propanoic acid (3.00 g, 11.6 mmol, 1.00 eq) in tetrahydrofuran (30.0 mL) was added borane dimethyl sulfide complex (10.0 M, 3.48 mL, 3.00 eq). The reaction was stirred at 70°C for 10 h. Methanol (20.0 mL) was added at 0°C to quench the reaction, and the mixture was concentrated under reduced pressure to afford 2-amino-3-(3-chloro-2-methyl-5-nitrophenyl) propan-1-ol (3.00 g, 11.6 mmol, crude) as a yellow oil.

**Step 6:** To a solution of 2-amino-3-(3-chloro-2-methyl-5-nitrophenyl)propan-1-ol (3.00 g, 12.3 mmol, 1.00 eq) and triethylamine (2.05 mL, 14.7 mmol, 1.20 eq) in tetrahydrofuran (30.0 mL) was added 2-chloroacetyl chloride (1.17 mL, 14.7 mmol, 1.20 eq) at 0°C. The reaction was stirred at 25°C for 1 h. Water (10.0 mL) was added to quench the reaction, and the mixture was extracted with ethyl acetate (3 × 50.0 mL). The combined organic layers were washed with brine (20.0 mL), dried over sodium sulfate, filtered, and concentrated under reduced pressure to give a residue. The residue was purified by silica gel column chromatography (petroleum ether/ethyl acetate = 1/1) to afford 2-chloro-N-(1-(3-chloro-2-methyl-5-nitrophenyl)-3-hydroxypropan-2-yl)acetamide (1.30 g, 4.05 mmol, 33% yield) as a yellow solid.

**Step 7:** To a solution of 2-chloro-N-(1-(3-chloro-2-methyl-5-nitrophenyl)-3-hydroxypropan-2-yl)acetamide (1.20 g, 3.74 mmol, 1.00 eq) in tert-butyl alcohol (3.00 mL) was added potassium tert-butoxide (839 mg, 7.47 mmol, 2.00 eq). The reaction was stirred at 100°C for 0.5 h. The mixture was concentrated under reduced pressure to give a residue. The residue was diluted with water (10.0 mL) and extracted with ethyl acetate (3 × 30.0 mL). The combined organic layers were washed with brine (20.0 mL), dried over sodium sulfate, filtered, and concentrated under reduced pressure to give a residue. The residue was purified by silica gel column chromatography (dichloromethane/methanol = 5/1) to afford 5-(3-chloro-2-methyl-5-nitrobenzyl)morpholin-3-one (0.250 g, 878 µmol, crude) as a yellow solid.

**Step 8:** To a solution of 5-(3-chloro-2-methyl-5-nitrobenzyl)morpholin-3-one (240 mg, 843 µmol, 1.00 eq) in tetrahydrofuran (3.00 mL) was added borane dimethyl sulfide complex (10.0 M, 253 µL, 3.00 eq). The reaction was stirred at 70°C for 10 h. Methanol (10.0 mL) was added to quench the reaction, and the mixture was concentrated under reduced pressure to afford 3-(3-chloro-2-methyl-5-nitrobenzyl)morpholine (0.200 g, 843 µmol, crude) as a yellow solid.

**Step 9:** To a solution of 3-(3-chloro-2-methyl-5-nitrobenzyl)morpholine (0.150 g, 554 µmol, 1.00 eq) and formaldehyde 37% purity (0.90 mL, 12.1 mmol, 21.8 eq) in methanol (2.00 mL) was added acetic acid (63.4 µL, 1.11 mmol, 2.00 eq). After 0.5 h, sodium cyanoborohydride (174 mg, 2.77 mmol, 5.00 eq) was added, and the reaction was stirred at 25°C for 10 h. Water (15.0 mL) was added to quench the reaction, and the mixture was extracted with dichloromethane (3 × 30.0 mL). The combined organic layers were washed with brine (15.0 mL), dried over sodium sulfate, filtered, and concentrated under reduced pressure to give a residue. The residue was purified by reversed phase preparative HPLC to afford 3-(3-chloro-2-methyl-5-nitrobenzyl)-4-methylmorpholine (0.140 g, 492 µmol, 89% yield) as a yellow oil.

**Step 10:** A mixture of 3-(3-chloro-2-methyl-5-nitrobenzyl)-4-methylmorpholine (0.140 g, 492 µmol, 1.00 eq), ferrous powder (137 mg, 2.46 mmol, 5.00 eq) and ammonium chloride (26.3 mg, 492 µmol, 1.00 eq) in ethanol (2.00 mL) and water (1.00 mL) was stirred at 60°C for 10 h. The mixture was concentrated under reduced pressure to give a residue. Water (15.0 mL) was added and the mixture was extracted with ethyl acetate (3 × 25.0 mL). The combined organic layers were washed with brine (10.0 mL), dried over sodium sulfate, filtered, and concentrated under reduced pressure to afford 3-chloro-4-methyl-5-((4-methylmorpholin-3-yl)methyl)aniline (0.100 g, 393 µmol, 80% yield) as a yellow oil.

**Step 11:** To a solution of 3-chloro-4-methyl-5-((4-methylmorpholin-3-yl)methyl)aniline (0.100 g, 393 µmol, 1.00 eq) and potassium carbonate (109 mg, 785 µmol, 2.00 eq) in acetone (2.00 mL) was added phenyl chloroformate (59.0 µL, 471 µmol, 1.20 eq) at 0°C. The reaction was stirred at 25°C for 10 h. The mixture was diluted with water (10.0 mL) and extracted with ethyl acetate (3 × 25.0 mL). The combined organic layers were washed with brine (10.0 mL), dried over sodium sulfate, filtered, and concentrated under reduced pressure to afford phenyl (3-chloro-4-methyl-5-((4-methylmorpholin-3-yl) methyl)phenyl)carbamate (0.140 g, 373 µmol, crude) as yellow oil.

**Compound 161:** General procedure A with variant iii) was used for the preparation with a yield of 27% from compound **VI** employing phenyl (3-chloro-5-((1-methylpyrrolidin-3-yl)methoxy)phenyl)carbamate.

¹H NMR (400 MHz, DMSO-*d₆*) *□* = 11.11 - 10.85 (m, 1H), 10.67 (br s, 1H), 9.15 (br s, 1H), 7.67 (s, 1H), 7.60 - 7.50 (m, 2H), 7.13 (br s, 1H), 7.09 (br d, *J =* 8.1 Hz, 1H), 6.57 (t, *J* = 2.1 Hz, 1H), 5.10 - 5.01 (m, 1H), 5.12 - 4.98 (m, 1H), 4.48 - 4.32 (m, 4H), 4.09 - 3.97 (m, 2H), 3.52 - 3.40 (m, 1H), 3.27 - 2.98 (m, 2H), 2.96 - 2.84 (m, 2H), 2.81 (br s, 3H), 2.68 - 2.59 (m, 1H), 2.49 - 2.34 (m, 2H), 2.31 - 2.11 (m, 1H), 2.09 - 2.00 (m, 1H), 1.99 - 1.72 (m, 1H). MS (ESI) m/z 540.3 [M+H]⁺

### Scheme for the preparation of phenyl (3-chloro-5-((1-methylpyrrolidin-3-yl)methoxy)phenyl)carbamate.

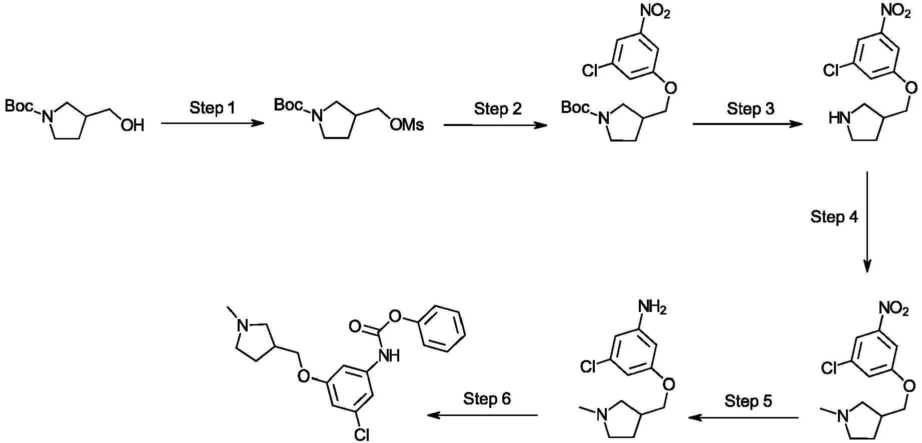

**Step 1:** To a solution of tert-butyl 3-(hydroxymethyl)pyrrolidine-1-carboxylate (3.00 g, 14.9 mmol, 1.00 eq) and triethylamine (3.77 g, 37.3 mmol, 5.19 mL, 2.50 eq) in dichloromethane (30.0 mL) at 0°C was added methylsulfamoyl chloride (1.50 mL, 19.4 mmol, 1.30 eq) dropwise under nitrogen atmosphere. The reaction was stirred at 25°C for 2 h. The mixture was diluted with ethyl acetate (100 mL) and water (150 mL). The organic layer was separated, and the aqueous layer was extracted with ethyl acetate (3 × 100 mL). The organic layers were gathered, dried over sodium sulfate, filtered, and concentrated under reduced pressure to afford tert-butyl 3-(((methylsulfonyl)oxy)methyl)pyrrolidine-1-carboxylate (4.00 g, crude) as light yellow oil.

**Step 2:** To a solution of tert-butyl 3-(((methylsulfonyl)oxy)methyl) pyrrolidine-1-carboxylate (3.00 g, 10.8 mmol, 1.00 eq) in dimethyl formamide (30.0 mL) was added 3-chloro-5-nitrophenol (2.05 g, 11.8 mmol, 1.10 eq) and cesium carbonate (10.5 g, 32.2 mmol, 3.00 eq). The reaction was stirred at 80°C for 12 h. The mixture was diluted with ethyl acetate (100 mL) and water (100 mL). The organic layer was separated, and the aqueous layer was extracted with ethyl acetate (3 × 80.0 mL). The organic layers were gathered, dried over sodium sulfate, filtered, and concentrated under reduced pressure to give a residue. The residue was purified by silica gel column chromatography (petroleum ether/ethyl acetate = 3/1) to afford tert-butyl 3-((3-chloro-5-nitrophenoxy)methyl) pyrrolidine-1-carboxylate (3.80 g, 10.7 mmol, 99 % yield) as yellow oil.

**Step 3:** To a solution of tert-butyl 3-((3-chloro-5-nitrophenoxy)methyl)pyrrolidine-1-carboxylate (1.10 g, 3.08 mmol, 1.00 eq) in ethyl acetate (5.00 mL) was added hydrochloric acid in ethyl acetate (4 M, 10 mL). The reaction was stirred at 25°C for 2 h. The mixture was concentrated under reduced pressure to afford 3-((3-chloro-5-nitrophenoxy) methyl)pyrrolidine (1.00 g, crude) as a yellow solid.

**Step 4:** To a solution of 3-((3-chloro-5-nitrophenoxy)methyl)pyrrolidine (1.50 g, 5.84 mmol, 1.00 eq) in 2,2,2-trifluoroethanol (10.0 mL) was added paraformaldehyde (0.80 mL, 29.2 mmol, 5.00 eq). The reaction was stirred at 60°C for 0.5 h. Sodium borohydride (442 mg, 11.7 mmol, 2.00 eq) was added in portions, and the reaction was stirred at 60°C for 1 h. The reaction was quenched with saturated ammonium chloride solution (10.0 mL) and concentrated under reduced pressure to give a residue. The residue was purified by reversed phase preparative HPLC to afford 3-((3-chloro-5-nitrophenoxy)methyl)-1-methylpyrrolidine (1.50 g, 4.99 mmol, 86% yield) as yellow oil.

**Step 5:** To a solution of 3-((3-chloro-5-nitrophenoxy)methyl)-1-methylpyrrolidine (1.20 g, 4.43 mmol, 1.00 eq) in methanol (6.00 mL) and water (6.00 mL) was added ammonium chloride (1.66 g, 31.0 mmol, 7.00 eq) and iron powder (1.73 g, 31.0 mmol, 7.00 eq). The reaction was stirred at 80°C for 2 h. The mixture was filtered over Celite, and the filtrate was concentrated under reduced pressure to afford 3-chloro-5-((1-methylpyrrolidin-3-yl) methoxy)aniline (1.00 g, crude) as brown oil.

**Step 6:** To a solution of phenyl chloroformate (0.28 mL, 2.24 mmol, 1.20 eq) in acetonitrile (5.00 mL) was added pyridine (0.45 mL, 5.61 mmol, 3.00 eq) and 3-chloro-5-((1-methylpyrrolidin-3-yl) methoxy)aniline (450 mg, 1.87 mmol, 1.00 eq). The reaction was stirred at 25°C for 2 h. The mixture was concentrated under reduced pressure to give a residue. The residue was purified by reversed phase preparative HPLC to afford phenyl (3-chloro-5-((1-methylpyrrolidin-3-yl) methoxy)phenyl)carbamate (630 mg, 1.64 mmol, 88% yield) as a light yellow solid.

**Compound 162:** General procedure A with variant iii) was used for the preparation with a yield of 33% from compound **VI** employing phenyl (3-chloro-5-((1-methylpyrrolidin-3-yl)oxy)phenyl)carbamate.

¹H NMR (400 MHz, DMSO-*d₆*) *□* = 11.15 (br d, *J =* 4.3 Hz, 1H), 10.98 (s, 1H), 10.63 (br s, 1H), 9.58 - 9.41 (m, 1H), 7.64 (s, 1H), 7.55 (d, *J =* 0.8 Hz, 2H), 7.25 - 7.17 (m, 1H), 7.28 - 7.13 (m, 1H), 7.12 - 7.04 (m, 1H), 6.60 (d, *J=* 1.9 Hz, 1H), 5.19 - 5.02 (m, 2H), 4.47 - 4.26 (m, 4H), 3.97 - 3.69 (m, 1H), 3.40 - 3.20 (m, 1H), 3.18 - 3.05 (m, 1H), 2.97 - 2.88 (m, 1H), 2.87 - 2.80 (m, 3H), 2.63 - 2.56 (m, 1H), 2.46 - 2.33 (m, 1H), 2.31 - 2.14 (m, 1H), 2.09 - 1.96 (m, 2H). MS (ESI) m/z 526.2 [M+H]⁺

### Scheme for the preparation of phenyl (3-chloro-5-((1-methylpyrrolidin-3-yl)oxy)phenyl)carbamate.

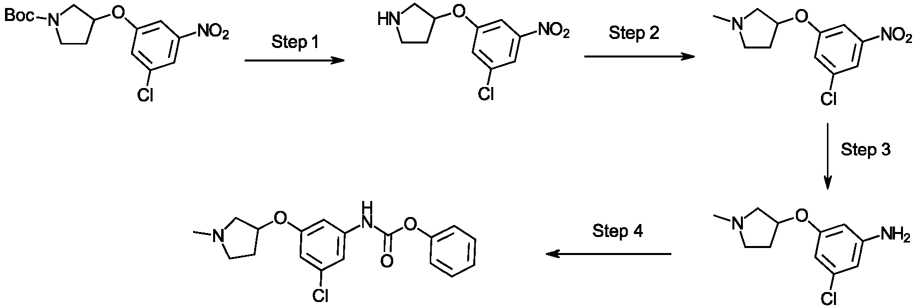

**Step 1:** To a solution of tert-butyl 3-(3-chloro-5-nitro-phenoxy)pyrrolidine-1-carboxylate (2.00 g, 5.83 mmol, 1.00 eq) in ethyl acetate (10.0 mL) was added hydrochloric acid in ethyl acetate (4 M, 20.0 mL, 13.7 eq). The reaction was stirred at 25°C for 1 h. The mixture was concentrated under reduced pressure to afford 3-(3-chloro-5-nitrophenoxy)pyrrolidine (1.40 g, 5.77 mmol, 98% yield) as a yellow oil.

**Step 2:** To a solution of 3-(3-chloro-5-nitrophenoxy)pyrrolidine (1.00 g, 4.12 mmol, 1.00 eq) in methanol (6.00 mL) was added paraformaldehyde 37% purity (6.00 mL, 80.6 mmol, 19.60 eq), acetic acid (0.23 mL, 4.11 mmol, 1.00 eq) and sodium cyanoborohydride (1.29 g, 20.6 mmol, 5.00 eq) in portions. The reaction was stirred at 25°C for 1 h. The mixture was concentrated under reduced pressure to give a residue. The residue was purified first by silica gel column chromatography (petroleum ether/ethyl acetate to ethyl acetate/methanol= 3/1 to 0/1), then by reversed phase preparative HPLC, to afford 3-(3-chloro-5-nitrophenoxy)-1-methylpyrrolidine (470 mg, 1.83 mmol, 44% yield) as yellow oil.

**Step 3:** To a solution of 3-(3-chloro-5-nitrophenoxy)-1-methylpyrrolidine (460 mg, 1.79 mmol, 1.00 eq) in methanol (15.0 mL) and water (8.00 mL) was added iron powder (300 mg, 5.37 mmol, 3.00 eq) and ammonium chloride (479 mg, 8.95 mmol, 5.00 eq) in portions. The reaction was stirred at 80°C for 1 h. The mixture was concentrated under reduced pressure to give a residue, which was diluted with water (50.0 mL) and extracted with ethyl acetate (3 × 20.0 mL). The combined organic layers were washed with water, dried over anhydrous sodium sulfate, filtered, and concentrated under reduced pressure to afford 3-chloro-5-((1-methylpyrrolidin-3-yl)oxy)aniline (300 mg, crude) as yellow oil. It was used as such in the next step.

**Step 4:** To a solution of 3-chloro-5-((1-methylpyrrolidin-3-yl)oxy)aniline (290 mg, 1.28 mmol, 1.00 eq) in acetonitrile (5.00 mL) was added pyridine (0.52 mL, 6.39 mmol, 5.00 eq) and phenyl chloroformate (0.19 mL, 1.54 mmol, 1.20 eq) in portions. The reaction was stirred at 25°C for 1 h. The mixture was concentrated under reduced pressure to give a residue, which was purified by reversed phase preparative HPLC to afford phenyl (3-chloro-5-((1-methylpyrrolidin-3-yl)oxy) phenyl) carbamate (370 mg, 1.07 mmol, 83% yield) as a white solid.

**Compound 163:** General procedure A with variant iii) was used for the preparation with a yield of 59% from compound VI employing phenyl N-(6-tert-butyl-3-pyridyl) carbamate.

¹H NMR (400 MHz, DMSO-*d₆*) *□* = 10.99 (s, 1H), 8.79 (s, 1H), 8.45 (d, *J =* 2.3 Hz, 1H), 8.23 (s, 1H), 7.86 - 7.81 (m, 1H), 7.67 (s, 1H), 7.57 (d, *J=* 1.0 Hz, 2H), 7.29 (d, *J=* 8.6 Hz, 1H), 6.91 (t, *J =* 6.0 Hz, 1H), 5.16 - 5.09 (m, 1H), 4.49 - 4.27 (m, 4H), 2.98 - 2.85 (m, 1H), 2.66 - 2.56 (m, 1H), 2.47 - 2.36 (m, 1H), 2.05 - 1.94 (m, 1H), 1.27 (s, 9H). MS (ESI) m/z 450.2 [M+H]⁺

### Scheme for the synthesis of phenyl N-(6-tert-butyl-3-pyridyl) carbamate.

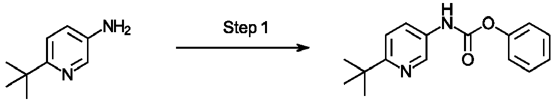

**Step 1:** To a solution of 6-(tert-butyl)pyridin-3-amine (150 mg, 998 µmol, 1.00 eq) in acetonitrile (5.00 mL) was added pyridine (0.40 mL, 4.99 mmol, 5.00 eq) and phenyl chloroformate (0.15 mL, 1.20 mmol, 1.20 eq). The reaction was stirred at 25°C for 1 h. The mixture was concentrated under reduced pressure to give a residue. The residue was purified by reverses phase preparative HPLC to afford phenyl (6-(tert-butyl)pyridin-3-yl)carbamate (230 mg, 850 µmol, 85% yield) as a yellow solid.

**Compound 164:** General procedure A with variant iii) was used for the preparation with a yield of 52% from compound VI employing phenyl(4-(trifluoromethoxy)pyridin-2-yl)carbamate.

¹H NMR (400 MHz, DMSO-*d*₆) *□* = 10.97 (br s, 1H), 9.60 (s, 1H), 8.29 (d, *J* = 5.7 Hz, 1H), 8.15 - 8.03 (m, 1H), 7.66 (s, 1H), 7.62 - 7.54 (m, 3H), 6.98 - 6.90 (m, 1H), 5.11 (dd, *J =* 5.0, 13.3 Hz, 1H), 4.51 - 4.41 (m, 3H), 4.34 - 4.27 (m, 1H), 2.95 - 2.87 (m, 1H), 2.61 (br d, *J =* 2.3 Hz, 1H), 2.45 - 2.36 (m, 1H), 2.05 - 1.95 (m, 1H). MS (ESI) m/z 478.1 [M+H]⁺

### Scheme for the preparation of phenyl(4-(trifluoromethoxy)pyridin-2-yl)carbamate.

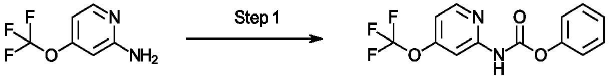

**Step 1:** To a solution of 4-(trifluoromethoxy)pyridin-2-amine (300 mg, 1.68 mmol, 1.00 eq) and pyridine (0.68 mL, 8.42 mmol, 5.00 eq) in acetonitrile (3.00 mL) was added phenyl chloroformate (0.25 mL, 2.02 mmol, 1.20 eq) dropwise at 0°C. The reaction was stirred at 25°C for 3 h. The mixture was concentrated under reduced pressure to give a residue. The residue was purified by reversed phase preparative HPLC to afford phenyl (4-(trifluoromethoxy)pyridin-2-yl)carbamate (350 mg, 1.17 mmol, 69% yield) as a white solid.

**Compound 165:** General procedure A with variant iii) was used for the preparation with a yield of 53% from compound VI employing phenyl (3-fluoro-5-(trifluoromethoxy)phenyl)carbamate.

¹H NMR (400 MHz, DMSO-*d₆*) *□* = 10.98 (s, 1H), 9.25 (s, 1H), 7.66 (s, 1H), 7.56 (d, *J=* 0.9 Hz, 2H), 7.36 - 7.30 (m, 2H), 7.06 (t, *J* = 6.0 Hz, 1H), 6.83 (br d, *J =* 9.0 Hz, 1H), 5.11 (dd, *J* = 5.0, 13.3 Hz, 1H), 4.47 - 4.38 (m, 3H), 4.34 - 4.26 (m, 1H), 2.97 - 2.85 (m, 1H), 2.60 (td, *J =* 2.0, 15.3 Hz, 1H), 2.45 - 2.35 (m, 1H), 2.04 - 1.95 (m, 1H). MS (ESI) m/z 495.2 [M+H]⁺

### Scheme for the preparation of phenyl (3-fluoro-5-(trifluoromethoxy)phenyl)carbamate.

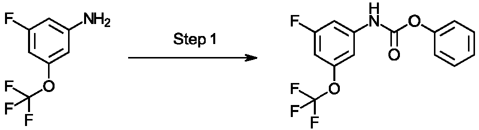

**Step 1:** To a solution of 3-fluoro-5-(trifluoromethoxy)aniline (200 mg, 1.03 mmol, 1.00 eq) in acetonitrile (10.0 mL) was added pyridine (0.25 mL, 3.10 mmol, 3.02 eq) and phenyl chloroformate (0.15 mL, 1.23 mmol, 1.20 eq) in portions at 0°C. The reaction was stirred at 25°C for 1 h. The mixture was concentrated under reduced pressure to give a residue, which was purified by reversed phase HPLC to afford phenyl (3-fluoro-5-(trifluoromethoxy)phenyl)carbamate (320 mg, 1.02 mmol, 99% yield) as a white solid.

### Protocol for Fluorescent Polarization assay

Compound activity was monitored in a fluorescence polarization (FP) homogeneous assay using 1-[5-({2-[2-(2-{[2-(2,6-dioxopiperidin-3-yl)-1,3-dioxo-2,3-dihydro-1H-isoindol-4-yl]oxy}acetamido)ethoxy]ethyl}carbamoyl)pentyl]-3,3-dimethyl-2-[(1E,3E)-5-[(2E)-1,3,3-trimethyl-5-sulfo-2,3-dihydro-1H-indol-2-ylidene]penta-1,3-dien-1-yl]-3H-indol-1-ium-5-sulfonate as a fluorescent probe. Unless otherwise stated, all reagents were purchased from Sigma Aldrich. Enzymatic reactions were conducted in Perkin-Elmer Black 384 well ProxiPlate Plus (catalogue no. 6008269) in 10 µL total volume. Full length wild-type cereblon CRBN (80.0 nM, 10 µL) was incubated in assay buffer containing 20 mM HEPES (pH 8.0), 150 NaCl, 0.5 mM TCEP and 0.05% Tween 20 in the presence or absence of compound (300 nL). Inhibitors were stored as 10 mM DMSO stocks in an inert environment (low humidity, dark, low oxygen, room temperature) using the Storage Pod System. Compounds and DMSO were dispensed using the Echo E5XX (Labcyte Inc. USA) to give concentrations from 300 to 0.937 or 3000 to 9.3 nM in a 12 data point curve. Mutant YWAA CRBN (80.0 nM, 10 µL) which does not interact with the fluorescent probe was used as a negative control for the assay. Following incubation at room temperature for 30 min, the assay was initiated by dispensing the probe to a final concentration of 5 nM (2.5 nL of a 20 µM stock) using the Echo E5XX. FP was measured after a period of 12 hours using a Pherastar plate reader (BMG Labtech, Germany) exciting at 590 nm and measuring the amount of parallel and perpendicular light at 675 nm. The FP signal was subsequently normalized to the no-compound control (i.e., DMSO). Analysis and IC50 values were derived using Dotmatics (Dotmatics UK) software.

**Table 2: IC50 values determined in the fluorescence polarization assay indicating the cereblon binding**

| **Compound** | **rFP IC50 [nM]** | **Compound** | **rFP IC50 [nM]** | **Compound** | **rFP IC50 [nM]** |
|---|---|---|---|---|---|
| **1** | 1037 | **2** | 254 | **3** | 400 |
| **4** | 483 | **5** | 339 | **6** | 467 |
| **7** | 324 | **8** | 468 | **9** | 617 |
| **10** | 763 | **11** | 1234 | **12** | 960 |
| **13** | 944 | **14** | 1415 | **15** | 902 |
| **16** | 817 | **17** | 514 | **18** | 552 |
| **19** | 576 | **20** | 550 | **21** | 622 |
| **22** | 393 | **23** | 464 | **24** | 1588 |
| **25** | 612 | **26** | 907 | **27** | 444 |
| **28** | 814 | **29** | 1145 | **30** | 580 |
| **31** | 595 | **32** | 508 | **33** | 591 |
| **34** | 481 | **35** | 602 | **36** | 546 |
| **37** | 672 | **38** | 589 | **39** | 587 |
| **40** | 566 | **41** | 590 | **42** | 589 |
| **43** | 757 | **44** | 747 | **45** | 472 |
| **46** | 1400 | **47** | 1081 | **48** | 719 |
| **49** | 867 | **50** | 479 | **51** | 417 |
| **52** | 477 | **53** | 364 | **54** | 471 |
| **55** | 497 | **56** | 434 | **57** | 373 |
| **58** | 540 | **59** | 373 | **60** | 412 |
| **61** | 381 | **62** | 441 | **63** | 492 |
| **64** | 400 | **65** | 637 | **66** | 449 |
| **67** | 284 | **68** | 290 | **69** | 381 |
| **70** | 253 | **71** | 333 | **72** | 322 |
| **73** | 211 | **74** | 196 | **75** | 284 |
| **76** | 281 | **77** | 570 | **78** | 725 |
| **79** | 317 | **80** | 515 | **81** | 919 |
| **82** | 372 | **83** | 387 | **84** | 348 |
| **85** | 214 | **86** | 203 | **87** | 278 |
| **88** | 351 | **89** | 225 | **90** | 471 |
| **91** | 315 | **92** | 304 | **93** | 248 |
| **94** | 408 | **95** | 432 | **96** | 222 |
| **97** | 301 | **98** | 404 | **99** | 367 |
| **100** | 178 | **101** | 325 | **102** | 389 |
| **103** | 272 | **104** | 234 | **105** | 295 |
| **106** | 353 | **107** | 247 | **108** | 661 |
| **109** | 163 | **110** | 302 | **111** | 240 |
| **112** | 270 | **113** | 283 | **114** | 443 |
| **115** | 230 | **116** | 139 | **117** | 152 |
| **118** | 256 | **119** | 408 | **120** | 453 |
| **121** | 243 | **122** | 657 | **123** | 570 |
| **124** | 198 | **125** | 596 | **126** | 193 |
| **127** | 261 | **128** | 302 | **129** | 350 |
| **130** | 358 | **131** | 334 | **132** | 381 |
| **133** | 252 | **134** | 394 | **135** | 477 |
| **136** | 425 | **137** | 1141 | **138** | 507 |
| **139** | 1016 | **140** | 559 | **141** | 352 |
| **142** | 594 | **143** | 3594 | **144** | 544 |
| **145** | 741 | **146** | 341 | **147** | 253 |
| **148** | 169 | **149** | 1134 | **150** | 304 |
| **151** | 883 | **152** | 284 | **153** | 1838 |
| **154** | 688 | **155** | 581 | **156** | 607 |
| **157** | 1515 | **158** | 1437 | **159** | 676 |
| **160** | 299 | **161** | 300 | **162** | 699 |
| **163** | 1467 | **164** | 1602 | **165** | 1630 |

In some embodiments, the disclosure is directed to compounds with an IC50 value of less than 1100 nM, i.e. directed to compounds 1, 2, 3, 4, 5, 6, 7, 8, 9, 10, 12, 13, 15, 16, 17, 18, 19, 20, 21, 22, 23, 26, 26, 27, 28, 30, 31, 32, 33, 34, 35, 36, 37, 38, 39, 40, 41, 42, 43, 44, 45, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 138, 139, 140, 141, 142, 144, 145, 146, 147, 148, 150, 151, 152, 154, 155, 156, 158, 159, 160, 161 and 162.

### Protocol for Immunofluorescence assay

In order to demonstrate the ability of the compounds to bind to degrade a specific protein of interest, GSPT1 was chosen and tested in an immunofluorescence assay.

CAL-51 cells were purchased from DSMZ (cat. Number ACC302), sub-cultured in 90% Dulbecco's MEM (4.5 g/L glucose, Gibco 11965) + 10% heat inactivated FBS (BioConcept, 2-01F136I) and incubated at 37°C, 5% CO₂. For the assay, imaging microtiterplate Cell Carrier 96 Ultra (Perkin Elmer 6055302) were pre-coated with Fibronectin (Sigma F085, 30µl at 0.2µg/ml) in PBS (100µl, Gibco 14190) for 45 min at room temperature, rinsed with PBS and CAL-51 cells (30K cells/well) were plated and let to adhere overnight. Cells were treated with compounds typically using a serial dilution ranging from 30 µM to 0.1 nM for 6 hours. Compounds were stored at 10 mM DMSO stocks. Vehicle (DMSO), positive (CC-885, 10 µM) and rescue controls (positive control plus 0.2 µM bortezomib) were also included atthis stage. Cells were subsequently rinsed with PBS and fixed in 10% Formalin solution (50µl, Sigma HT5011)) for 20 mins at room temperature. Following three consecutive PBS washes (100µl), cells were permeabilized in 0.1 % Triton X-100 in PBS (Sigma 93443, 50µl) for 15 mins at room temperature. Following three further PBS washes, 50µl blocking buffer (1 % BSA, Sigma A4503, in PBS) was added for 45 min for signal-to-noise reduction.

Primary antibody (human GSPT1, Sigma HPA052488) was diluted in blocking buffer (dil.1/300, 35µl/well) and incubated with the cells overnight at 4°C. After three PBS washes, Alexa-fluor 488 coupled secondary antibodies (Invitrogen, A32731 , dil.1/1000), Alexa-fluor 647-Phalloïdin (Invitrogen, A22287, dil.1/200) and DAPI (Thermo, #62248, dil.1/1000) were diluted in blocking buffer and incubated with the samples for 2 hours at room temperature. After three final PBS washes, samples were conserved in 100µl PBS in the dark, until measurement. Image acquisition was performed on the Operetta High-Content Imager (Perkin-Elmer). Fluorescence intensity of Alexa-Fluor 488 (GSPT1), Alexa-Fluor 647 (Actin) and DAPI (Nucleus) were measured. For the determination of GSPT1 DC₅₀ values, a custom algorithm implemented in the PerkinElmer image analysis software Harmony-Acapella^{®} was developed. After user-defined setting of adjustment parameters, the analysis was run identically without human intervention for all image fields. DAPI staining of the nuclei was used to determine the location of cells using standard nuclei detection modules. Segmentation artifacts were removed by threshold-based filters for area, roundness and intensity. The outline of the cells was determined analogously from the sum of the normalized, smoothed DAPI and Actin channel, starting from each nucleus. The Alexa-Fluor 488 (GSPT1) signal intensity in each cell was finally measured, in order to obtain a Mean intensity per cell. GSPT1 degradation (DC₅₀) was calculated after normalization to controls and data import in CDD vault Database, using non-linear regression.

**Table 3: Activity for GSPT1 degradation**

| Table 3 assigns each compound a code indicating the ability for GSPT1 degradation: A, B or C. According to the code, A represents a DC50 value of ≤ 100 nM, B represents a DC50 value > 100 nM and ≤ 300 nM and C represents a DC50 value of > 300 nM. | | | | | |
|---|---|---|---|---|---|
| **Compound** | **Code** | **Compound** | **Code** | **Compound** | **Code** |
| **1** | C | **2** | C | **3** | C |
| **4** | C | **5** | C | **6** | C |
| **7** | C | **8** | A | **9** | C |
| **10** | C | **11** | C | **12** | C |
| **13** | C | **14** | C | **15** | C |
| **16** | C | **17** | C | **18** | C |
| **19** | C | **20** | A | **21** | C |
| **22** | C | **23** | C | **24** | C |
| **25** | C | **26** | C | **27** | C |
| **28** | C | **29** | C | **30** | A |
| **31** | B | **32** | C | **33** | A |
| **34** | C | **35** | C | **36** | C |
| **37** | C | **38** | B | **39** | C |
| **40** | C | **41** | B | **42** | B |
| **43** | C | **44** | C | **45** | C |
| **46** | A | **47** | A | **48** | C |
| **49** | C | **50** | C | **51** | C |
| **52** | C | **53** | C | **54** | C |
| **55** | C | **56** | A | **57** | C |
| **58** | C | **59** | B | **60** | C |
| **61** | A | **62** | C | **63** | C |
| **64** | C | **65** | C | **66** | C |
| **67** | C | **68** | B | **69** | C |
| **70** | C | **71** | A | **72** | C |
| **73** | C | **74** | C | **75** | B |
| **76** | A | **77** | A | **78** | A |
| **79** | C | **80** | C | **81** | C |
| **82** | A | **83** | C | **84** | C |
| **85** | B | **86** | C | **87** | C |
| **88** | C | **89** | B | **90** | A |
| **91** | C | **92** | C | **93** | C |
| **94** | C | **95** | C | **96** | C |
| **97** | C | **98** | C | **99** | C |
| **100** | C | **101** | B | **102** | C |
| **103** | C | **104** | C | **105** | C |
| **106** | B | **107** | C | **108** | C |
| **109** | B | **110** | C | **111** | C |
| **112** | C | **113** | C | **114** | C |
| **115** | B | **116** | C | **117** | C |
| **118** | A | **119** | A | **120** | C |
| **121** | B | **122** | A | **123** | C |
| **124** | C | **125** | A | **126** | C |
| **127** | A | **128** | C | **129** | A |
| **130** | C | **131** | C | **132** | A |
| **133** | C | **134** | C | **135** | C |
| **136** | C | **137** | C | **138** | A |
| **139** | A | **140** | A | **141** | A |
| **142** | A | **143** | B | **144** | A |
| **145** | A | **146** | C | **147** | C |
| **148** | C | **149** | C | **150** | C |
| **151** | A | **152** | C | **153** | C |
| **154** | C | **155** | B | **156** | C |
| **157** | C | **158** | C | **159** | C |
| **160** | A | **161** | C | **162** | C |
| **163** | A | **164** | C | **165** | C |

In some embodiments, the compounds of any of formula I to IV exhibits a DC50 value 300 nM or less, i.e. compounds with code A and B. In some embodiments the compound is selected from the group consisting of 8, 20, 30, 31, 33, 38, 41, 42, 46, 47, 56, 59, 61, 68, 71, 75, 76, 77, 78, 82, 85, 89, 90, 101, 106, 109, 115, 118, 119, 121, 122, 125, 127, 129, 132, 138, 139, 140, 141, 142, 143, 144, 145, 151, 155, 160 and 163:

| **Compound** | **DC50 [nM]** | **Compound** | **DC50 [nM]** | **Compound** | **DC50 [nM]** |
|---|---|---|---|---|---|
| **8** | 82 | **20** | 40 | **30** | 50 |
| **31** | 106 | **33** | 65 | **38** | 120 |
| **41** | 172 | **42** | 253 | **46** | 98 |
| **47** | 30 | **56** | 34 | **59** | 124 |
| **61** | 92 | **68** | 200 | **71** | 8 |
| **75** | 109 | **76** | 46 | **77** | 96 |
| **78** | 84 | | | | |
| **82** | 22 | **85** | 274 | **89** | 198 |
| **90** | 23 | **101** | 224 | **106** | 188 |
| **109** | 246 | **115** | 299 | **118** | 68 |
| **119** | 71 | **121** | 150 | **122** | 36 |
| **125** | 42 | **127** | 45 | **129** | 37 |
| **132** | 34 | **138** | 17 | **139** | 59 |
| **140** | 12 | **141** | 29 | **142** | 17 |
| **143** | 119 | **144** | 80 | **145** | 36 |
| **151** | 31 | **155** | 176 | **160** | 14 |
| **163** | 30 | | | | |

In some embodiments, the compounds of any of formula I to IV exhibits a DC50 value of 100 nM or less, i.e. compounds with code A. In some embodiments the compound is selected from the group consisting of 8, 20, 30, 33, 46, 47, 56,61, 71, 76, 77, 78, 82, 90, 118, 119, 122, 125, 127, 129, 132, 138, 139, 140, 141, 142, 144, 145, 151,160and 163:

| **Compound** | **DC50 [nM]** | **Compound** | **DC50 [nM]** | **Compound** | **DC50 [nM]** |
|---|---|---|---|---|---|
| **8** | 82 | **20** | 40 | **30** | 50 |
| **33** | 65 | **46** | 98 | **47** | 30 |
| **56** | 34 | **61** | 92 | **71** | 8 |
| **76** | 46 | **77** | 96 | **78** | 84 |
| **82** | 22 | **90** | 23 | **118** | 68 |
| **119** | 71 | **122** | 36 | **125** | 42 |
| **127** | 45 | **129** | 37 | **132** | 34 |
| **138** | 17 | **139** | 59 | **140** | 12 |
| **141** | 29 | **142** | 17 | **144** | 80 |
| **145** | 36 | **151** | 31 | **160** | 14 |
| **163** | 30 | | | | |

### 3D spheroid experiments - HMEC

Human mammary epithelial cells (HMEC) were engineered to express either c-myc tagged with EGFP or EGFP alone (non myc) (analogous but distinct from Kessler JD et al, Science. 2012 Jan 20; 335(6066):348-53. doi: 10.1126/science.1212728; Hsu TY et al, Nature. 2015 Sep 17; 525(7569):384-8. doi: 10.1038/nature14985). The engineered cells were seeded at 1000 (myc) or 4000 (non myc) cells/well in a 384 ultra-low attachment plate in a total volume of 40µL HMEC cell culture media (DMEM/F1 2 + 10% HI-FBS + 15mM HEPES + 0.5ug/ml Hydrocortisone + 10ug/ml Insulin + 20ng/ml EGF). Plates were spun at 1200 rpm for 5 minutes at room temperature to ensure that cells have gathered in the middle of the well and incubated at 37°C for 48hrs before firing with compounds. On Day 2 (48h post seeding) cells were imaged (brightfield and EGFP fluorescence) using the Celigo imaging cytometer prior to firing compounds. For primary screens, compounds were added at three concentrations (1.25, 10 and 30uM) in a volume of 120nL using the ECHO acoustic dispenser and spun at 2000rpm for 2 minutes at room temperature before incubating at 37°C for 5 days. For counter-screen and establishment of IC50 concentrations, a 12-point dose was prepared starting from 30uM with 3-fold dilutions and added to cells in a volume of 120nL using the ECHO acoustic dispenser and plates spun at 2000rpm for 2 minutes at room temperature before incubating at 37°C for 5 days. On Day 7, prior to measuring cell viability using CellTiterGlo3D, cells were imaged (brightfield and EGFP fluorescence) on the Celigo imaging cytometer. The CellTiterGlo3D reagent is added at 30uL/well and incubated at room temperature for 30mins. After 30mins of incubation, luminescence readings were recorded using the Perkin Elmer EnVision reader.

**Table 4: 3D spheroid assay**

| Table 4 assigns each compound a code indicating the EC50 value in the myc-HMEC assay as well as in the non myc-HMEC assay: D, E, F or G. According to the code, D represents an EC50 value of ≤ 400 nM in the myc-HMEC assay, E represents an EC50 value > 400 nM and ≤ 2000 nM in the myc-HMEC assay, F represents an EC50 value of > 2000 nM in the myc-HMEC assay and G represents an EC50 value of > 10000nM in the non myc-HMEC assay.. | | | | | | | | |
|---|---|---|---|---|---|---|---|---|
| **Compound** | **Code** | **Code** | **Compound** | **Code** | **Code** | **Compound** | **Code** | **Code** |
| **1** | F | **G** | **2** | F | **G** | **3** | F | G |
| **4** | F | **G** | **5** | F | **G** | **6** | F | G |
| **7** | F | **G** | **8** | D | **G** | **9** | F | G |
| **10** | F | **G** | **11** | F | **G** | **12** | F | G |
| **13** | F | **G** | **14** | F | **G** | **15** | F | G |
| **16** | F | **G** | **17** | F | **G** | **18** | F | G |
| **19** | F | **G** | **20** | E | **G** | **21** | F | G |
| **22** | F | **G** | **23** | F | **G** | **24** | F | G |
| **25** | F | **G** | **26** | F | **G** | **27** | F | G |
| **28** | F | **G** | **29** | F | **G** | **30** | D | G |
| **31** | E | **G** | **32** | F | **G** | **33** | D | G |
| **34** | F | **G** | **35** | F | **G** | **36** | F | G |
| **37** | F | **G** | **38** | D | **G** | **39** | F | G |
| **40** | F | **G** | **41** | E | **G** | **42** | E | G |
| **43** | F | **G** | **44** | F | **G** | **45** | F | G |
| **46** | E | **G** | **47** | E | **G** | **48** | F | G |
| **49** | F | **G** | **50** | F | **G** | **51** | F | G |
| **52** | F | **G** | **53** | F | **G** | **54** | F | G |
| **55** | F | **G** | **56** | D | **G** | **57** | F | G |
| **58** | F | **G** | **59** | E | **G** | **60** | F | G |
| **61** | D | **G** | **62** | F | **G** | **63** | F | G |
| **64** | F | **G** | **65** | F | **G** | **66** | F | G |
| **67** | F | **G** | **68** | E | **G** | **69** | F | G |
| **70** | F | **G** | **71** | D | **G** | **72** | F | G |
| **73** | F | **G** | **74** | F | **G** | **75** | | G |
| **76** | | **G** | **77** | | **G** | **78** | | G |

In some embodiments, the compounds of any of formula I to IV exhibits an EC50 value of 2000 nM or less, i.e. compounds with code D and E. In some embodiments the compound is selected from the group consisting of 8, 20, 30, 31, 33, 38, 41, 42, 46, 47, 56, 59, 61, 68, and 71 :

| **Compound** | **EC50 [nM]** | **Compound** | **EC50 [nM]** | **Compound** | **EC50 [nM]** |
|---|---|---|---|---|---|
| **8** | 220 | **20** | 555 | **30** | 359 |
| **31** | 457 | **33** | 250 | **38** | 161 |
| **41** | 1095 | **42** | 1721 | **46** | 469 |
| **47** | 544 | **56** | 228 | **59** | 589 |
| **61** | 92 | **68** | 458 | **71** | 39 |

In some embodiments, the compounds of any of formula I to IV exhibits an EC50 value of 400 nM or less, i.e. compounds with code D. In some embodiments the compound is selected from the group consisting of 8, 30, 33, 38, 56, 61 and 71 :

| **Compound** | **EC50 [nM]** | **Compound** | **EC50 [nM]** | **Compound** | **EC50 [nM]** |
|---|---|---|---|---|---|
| **8** | 220 | **20** | 555 | **30** | 359 |
| **31** | 457 | **33** | 250 | **38** | 161 |
| **41** | 1095 | **42** | 1721 | **46** | 469 |
| **47** | 544 | **56** | 228 | **59** | 589 |
| **61** | 92 | **68** | 458 | **71** | 39 |

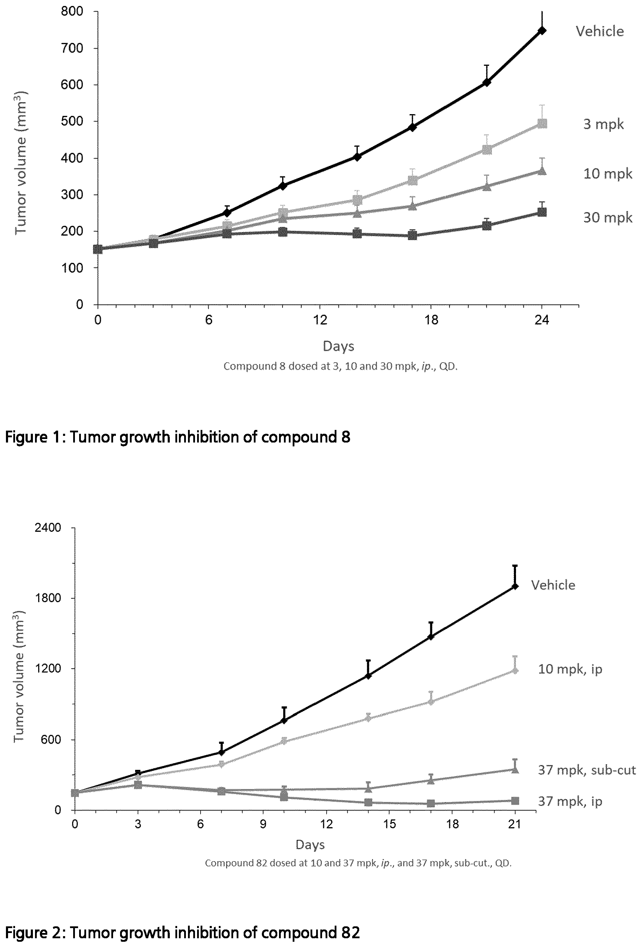

### In vivo efficacy study - Tumor growth inhibition (Fig. 1)

CAL51 cells (DSMZ-ACC-302) were maintained *in vitro* in DMEM medium supplemented with 20% heat inactivated FBS at 37°C in an atmosphere of 5% CO₂ in air. Cells (5 millions) resuspended in 0.2 mL of PBS with Matrigel (50:50) were inoculated into female SCID beige mice and allowed to grow to 150 mm³ in size. Mice were dosed daily *i.p.* with vehicle or Compound 8 (3, 10 and 30 mg per kilogram). Compound formulations were prepared fresh daily in 0.5% MC4000 and 0.2% Tween80. Mice were dosed for 24 days and tumor volumes measured every 3 days.

### In vivo efficacy study - Tumor growth inhibition in MDA-MB-213 model (Fig 2)

MDA-MB-231 cells were maintained *in vitro* in DMEM medium supplemented with 20% heat inactivated FBS at 37°C in an atmosphere of 5% CO₂ in air. Cells (10 millions) resuspended in 0.2 mL of PBS with Matrigel (50:50) were inoculated into female BALB/c nude mice and allowed to grow to 150 mm³ in size. Mice were dosed daily *i.p.* with vehicle or Compound 82 (10 and 37 mg per kilogram *i.p.* or 37 mg per kilogram *sub-cut*). Compound formulations were prepared fresh daily for *i.p.* in 0.5% MC4000 and 0.2% Tween80 and for *sub-cut* in 5% DMSO / 75% (40% HP-□-CD in 0.1M HCl) / 20% purified water. Mice were dosed for 24 days and tumor volumes measured every 3 days.

### CK1 alpha/lkaros/Aiolos/ZFP91 Selectivity determination by Western blot assay

MM1S cells were purchased from ATCC (cat. Number CRL-2974), sub-cultured in 90% RPMI 1640 with 10% FBS, supplemented with 1x P/S and incubated at 37°C, 5% CO2. Compounds were stored as 10 mM DMSO stock. For the assay, MM1S cells (3 million cells/well) were plated in 6-well plates and incubated over night. Cells were treated with respective compounds using a serial dilution: 0.3 µM, 3 µM and 30 µM as well as a vehicle only (DMSO) control for 6 hours. Media with suspension cells was subsequently transferred to 15 mL conical tubes, wells rinsed twice with ice-cold PBS and merged with cell suspension in respective 15 mL conical tube. Cells were spinned down, supernatant aspirated, pellets resuspended in ice-cold PBS and transferred to microtubes. Cells were spinned down, supernatant aspirated and pellets resuspended in 120 µL RIPA lysis buffer supplemented with protease and phosphatase inhibitors. Cell lysates were incubated on ice for 20 minutes followed by centrifugation at > 20,000xg for 5 min. Supernatants were transferred to fresh microtubes and stored at -80°C. Total protein concentration was determined using a BCA assay with a BSA standard curve and concentration of all samples was adjusted to 1 mg/mL. 25 µL 4x LDS sample buffer supplemented with 100 mM DTT was added to 75 µL sample. Samples were centrifuged (8,000xg, 1 min) and incubated at 95°C for 5 min followed by another centrifugation step (8,000xg, 1 min). 20 µL of each sample was loaded on a 4-12% gel alongside a protein molecular weight marker. Gels were run in the presence of MOPS buffer at 80 Volts for 30 min, followed by 120 Volts for 1.5 h and proteins subsequently transferred onto nitrocellulose membranes at 20 Volts for 7 min using an iBlot2 Gel Transfer Device. Membranes were then cut horizontally into two pieces, covering 80 - 50 kDa and 50 - 25 kDa. Blocking the membranes was performed by gently shaking in 5% (w/v) skim milk in TSB-T for 1hr at room temperature. All primary antibodies were used at a 1/1000 in 5% (w/v) BSA dissolved in TBST and incubated with membranes over night at 4°C. After three washes with 1x TBST for 5 min, HRP-coupled secondary antibodies diluted in 5% (w/v) BSA/TBST (Goat Anti-Rb IgG, dil. 1/10,000; Goat Anti-Mouse IgG, dil. 1/5000) were added for 1hr at room temperature. After three washes with 1x TBST (5 minutes each), membranes were incubated with ECL reagent for 1 min at room temperature. Chemiluminescence signals were then detected using a LAS-4000 system with default settings and signals quantified using Image Studio Lite software (version 5.2). Membrane parts previously incubated with antibody against CK1alpha were stripped off antibodies by incubating with stripping buffer for 30 minutes followed by three washed with TBST (5 min each), blocking with 5% (w/v) skim milk for 1hr, and incubation with primary antibody against GAPDH overnight at 4°C. Subsequent washes, incubation with secondary antibody and signal acquisition were performed as described above.

**Table 5: Selectivity for relevant Zincfinger proteins:**

| Table 5 assigns each compound a code indicating the ability for the degradation of IKZF1, IKZF3, CK1 alpha and ZFP91: A, B, C, D, E or F. According to the code, A represents a no degradation observed at 30 µM, B represents trace degradation at 30 µM (below 20%), C represents weak degradation at 30 µM (below 50%), D represents degradation at 30 µM (>90%), E represents degradation at 3 µM (>90%) and F represents degradation at 0.3 µM (>90%). | | | | | |
|---|---|---|---|---|---|
| **Compound** | **IKZF1** | **IKZF3** | **CK1 alpha** | **ZFP91** | **GSPT1** |
| **8** | A | **B** | A | **B** | F |
| **75** | A | **A** | A | **A** | E |
| **82** | A | **A** | A | **A** | F |
| **85** | A | **A** | B | **A** | E |
| **87** | A | **A** | A | **A** | E |
| **105** | B | **A** | A | **B** | E |
| **132** | A | **A** | C | **C** | F |
| **138** | C | **B** | A | **A** | F |

## Claims

1. A compound or pharmaceutically acceptable salts or stereoisomers thereof of formula I 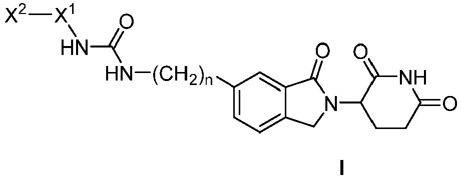 wherein
X¹ is linear or branched C₁₋₆ alkyl, C₃₋₆ cycloalkyl, -C₁₋₆ alkyl C₃₋₆ cycloalkyl, C₆₋₁₀ aryl, 5-10 membered heteroaryl, C₁₋₆ alkyl C₆₋₁₀ aryl, C₁₋₆ alkyl 5-10 membered heteroaryl, wherein X¹ is unsubstituted or substituted with one or more of halogen, linear or branched C₁₋₆ alkyl, linear or branched C₁₋₆ heteroalkyl, CF₃, CHF₂, -O-CHF₂, -O-(CH₂)₂-OMe, OCF₃, C₁₋₆ alkylamino, -CN, -N(H)C(O)-C₁₋₆alkyl, -OC(O)-C₁₋₆alkyl, -OC(O)-C₁₋₄alkylamino, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy;
X² is hydrogen, C₆₋₁₀ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄alkyl-(4-8 membered heterocycloalkyl), -OC(O)-C₁₋₄alkyl-4-8 membered heterocycloalkyl or C₆ aryloxy, wherein X² is unsubstituted or substituted with one or more of linear or branched C₁₋₆ alkyl, NH₂, NMe₂ or 5-6 membered heterocycloalkyl;
n is o, 1 or 2.

2. The compound of claim 1 or pharmaceutically acceptable salts or stereoisomers thereof,
wherein
X¹ is linear or branched C₁₋₄ alkyl, C₃₋₆ cycloalkyl, -C₁₋₄ alkyl C₃₋₆ cycloalkyl, C₆ aryl, 5-10 membered heteroaryl, C₁₋₆ alkyl C₆ aryl, C₁₋₆ alkyl 5-10 membered heteroaryl, wherein X¹ is unsubstituted or substituted with one or more of halogen, linear or branched C₁₋₄ alkyl, linear or branched C₁₋₆ heteroalkyl, CF₃, CHF₂, -O-CHF₂, -O-(CH₂)₂-OMe, OCF₃, C₁₋₆ alkylamino,-CN, -N(H)C(O)-C₁₋₆alkyl, -OC(O)-C₁₋₆alkyl, -OC(O)-C₁₋₄alkylamino, -C(O)O-C₁₋₆alkyl, -COOH, -CHO, -C₁₋₆alkylC(O)OH, -C₁₋₆alkylC(O)O-C₁₋₆alkyl, NH₂, C₁₋₆ alkoxy or C₁₋₆ alkylhydroxy; and
X² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein X² is unsubstituted or substituted with one or more of linear or branched C₁₋₆ alkyl, NH₂, NMe₂ or 5-6 membered heterocycloalkyl; and wherein n is o, 1, or 2.

3. The compound of claims 1 or 2, wherein n is 1 or 2, preferably 1.

4. The compound of any preceding claim or pharmaceutically acceptable salts or stereoisomers thereof of formula II 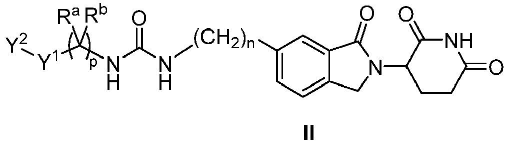 wherein
n is 1 or 2;
p is 0, 1, 2, 3, 4, 5, 6;
Y¹ is hydrogen, C₆ aryl, 5- 10 membered heteroaryl C₃₋₆ cycloalkyl, wherein Y¹ is unsubstituted or substituted with one or more of halogen, preferably F, Cl or Br, linear or branched C₁₋₄ alkyl, linear or branched C₁₋₆ heteroalkyl, CF₃, CHF₂, -O-CHF₂, -O-(CH₂)₂-OMe, OCF₃, -CN, -N(H)C(O)-C₁₋₆alkyl, -OC(O)-C₁₋₆alkyl, -OC(O)-C₁₋₄heteroalkyl, -C(O)O-C₁₋₆alkyl, -COOH, -CHO, -C₁₋₆alkylC(O)OH, -C₁₋₆alkylC(O)O-C₁₋₆alkyl, NH₂, or C₁₋₆ alkylhydroxy; with the proviso that when p is o, Y¹ is not hydrogen;
Y² is hydrogen, C₆ aryl, 5-10 membered heteroaryl, -O-(5-10 membered heteroaryl), 4-8 membered heterocycloalkyl, C₁₋₄ alkyl 4-8 membered heterocycloalkyl, -O-(4-8 membered heterocycloalkyl), -O-C₁₋₄ alkyl-(4-8 membered heterocycloalkyl), -OC(O)-C₁₋₄alkyl-4-8 membered heterocycloalkyl or C₆ aryloxy, wherein Y² is unsubstituted or substituted with one or more of linear or branched C₁₋₆ alkyl, NH₂, NMe₂ or 5-6 membered heterocycloalkyl;
R^{a}, R^{b} each are independently selected from hydrogen and linear or branched C₁₋₄ alkyl, preferably hydrogen and methyl.

5. The compound of any of the preceding claims or pharmaceutically acceptable salts or stereoisomers thereof, of formula IIIa', IIIb' or IIIc 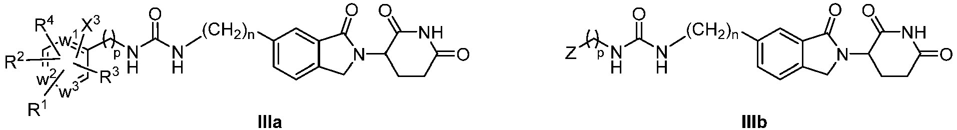 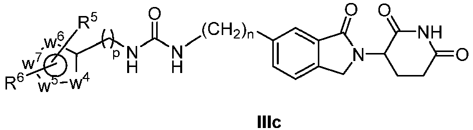 wherein
n is 1 or 2
p is 0, 1 or 2
one of w¹, w² or w³ is selected from C and N, and the other two of w¹, w² or w³ are C;
one or two of w⁴, w⁵, w⁶, w⁷ is selected from C, O, N, NMe, NH, or S while two or three of w⁴, w⁵, w⁶ and w⁷ are C;
R¹, R² , R³, R⁴ each are independently selected from hydrogen, linear or branched C₁₋₆ alkyl, C₁₋₆ heteroalkyl, C₆ aryl, preferably phenyl, CF₃, CHF₂, -O-CHF₂ -O-(CH₂)₂-OMe, OCF₃, -CN, -N(H)C(O)-C₁₋₆alkyl, -OC(O)-C₁₋₄alkylamino, -OC(O)-C₁₋₆alkyl, -C(O)O-C₁₋₆alkyl,-COOH, -CHO, -C₁₋₆alkylC(O)OH, -C₁₋₆alkylC(O)O-C₁₋₆alkyl, NH₂, C₁₋₄ alkylhydroxy, halogen, preferably F, Cl, Br, more preferably F or Cl; and/or two of R¹, R², R³, R⁴ form together a 5-6 membered heterocycloalkyl or a 5-6 membered heteroaryl;
R⁵, R⁶ each are independently selected from hydrogen, linear or branched C₁₋₄ alkyl, CF₃, CHF₂, halogen, preferably F, Cl, Br, more preferably F or Cl
X³ is absent, hydrogen or 4-8 membered heterocycloalkyl, C₁₋₄ alkyl 4-8 membered heterocycloalkyl, -O-(4-8 membered heterocycloalkyl), -C₁₋₄ alkoxy-(4-8 membered heterocycloalkyl), 5-10 membered heteroaryl, -O-(5-10 membered heteroaryl),-OC(O)-C₁₋₄alkyl-4-8 membered heterocycloalkyl, wherein X³ is unsubstituted or substituted with one or more of linear or branched C₁₋₆ alkyl, NH₂, NMe₂ or 5-6 membered heterocycloalkyl;
Z is linear or branched C₁₋₆ alkyl or C₃₋₆ cycloalkyl or C₁₋₄ alkoxy, wherein Z is unsubstituted or substituted with C₁₋₄ alkyl.

6. The compound of claims 4 or 5 or pharmaceutically acceptable salts or stereoisomers thereof, wherein n is 1.

7. The compound of claims 5 or 6 or pharmaceutically acceptable salts or stereoisomers thereof, wherein p is 0 or 1.

8. The compound of any of the preceding claims or pharmaceutically acceptable salts or stereoisomers thereof, of formula IV, V, VI and VII 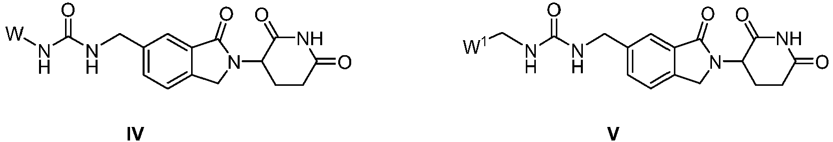 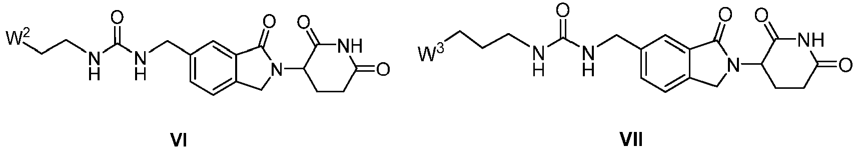 wherein
W, W¹, W², W³ are independently selected from

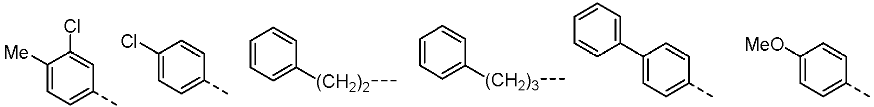

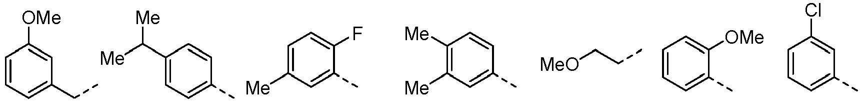

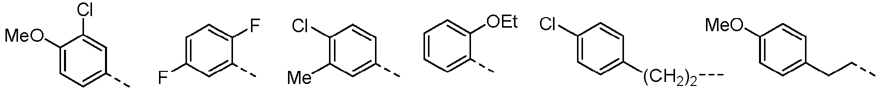

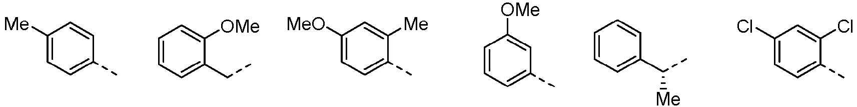

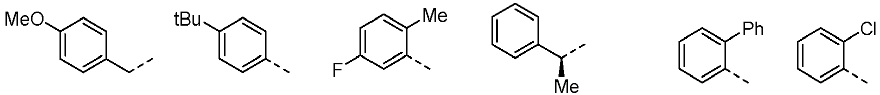

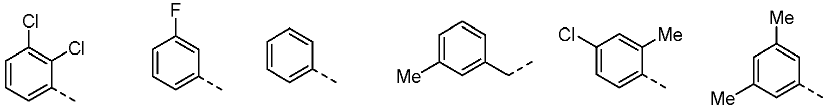

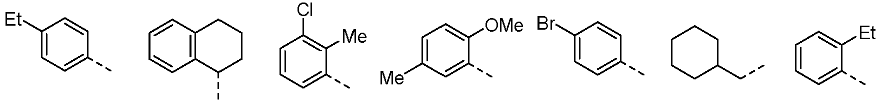

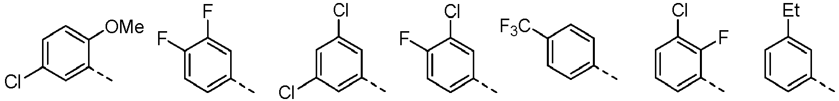

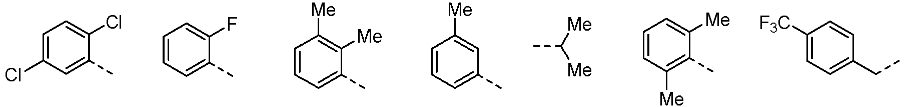

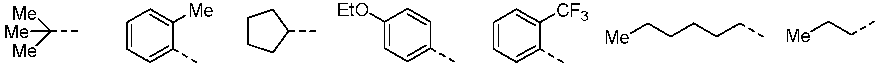

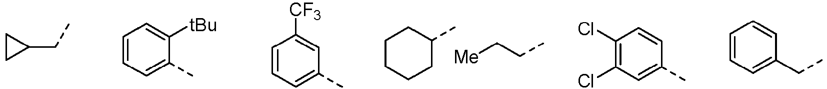

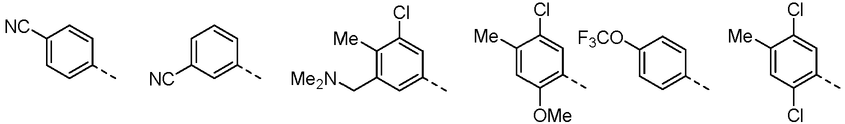

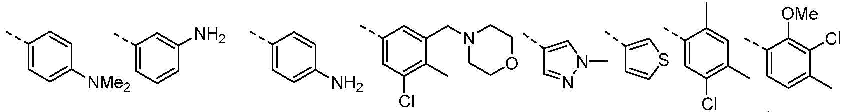

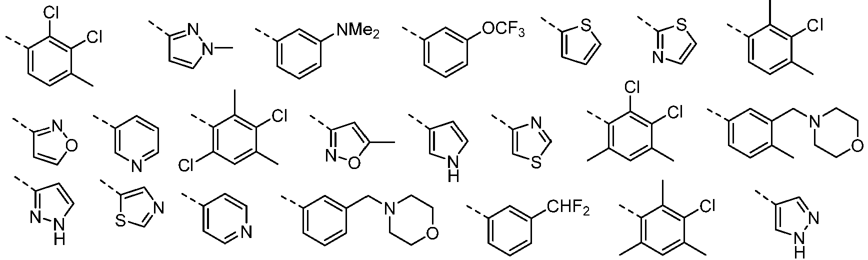

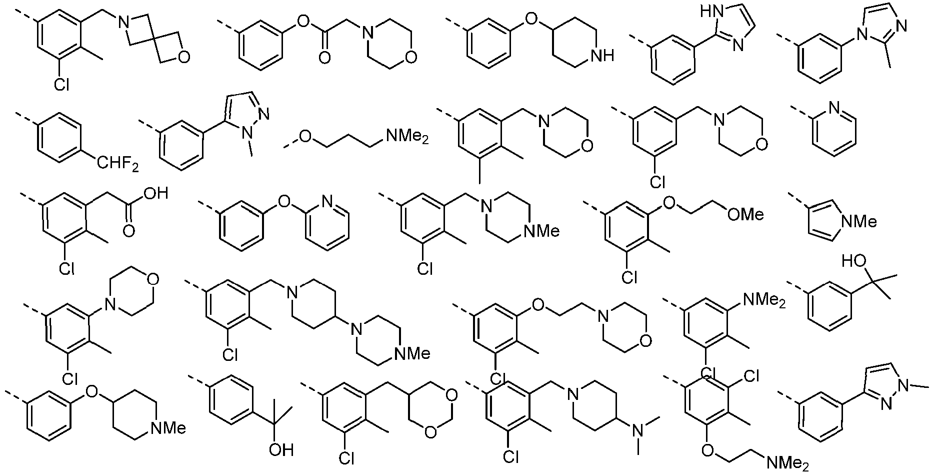

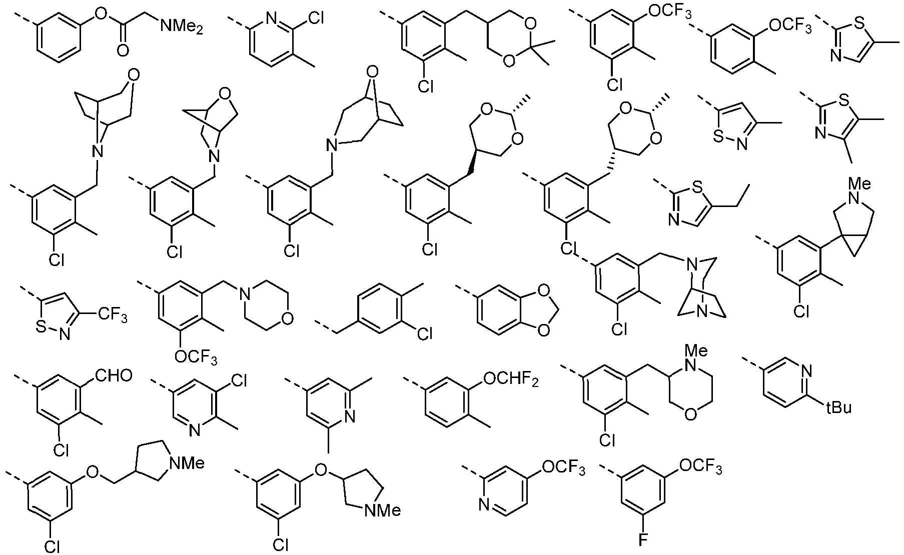

9. The compound of claim 8 or pharmaceutically acceptable salts or stereoisomers thereof, wherein W is

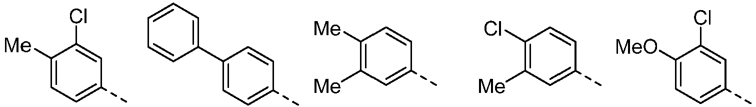

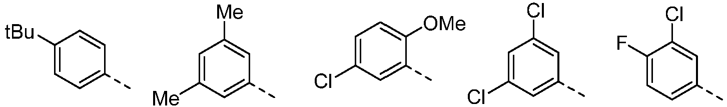

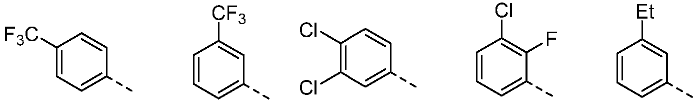

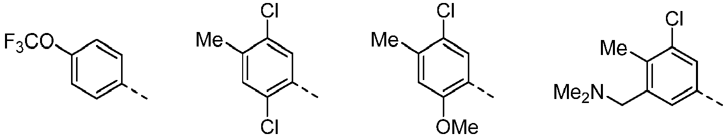

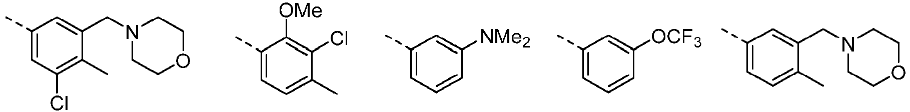

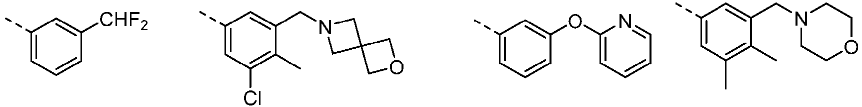

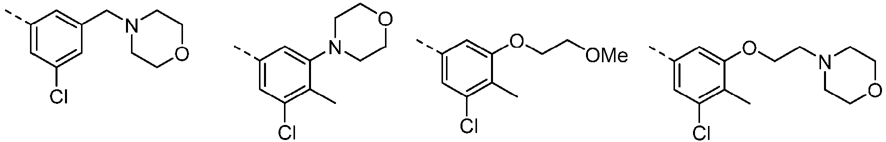

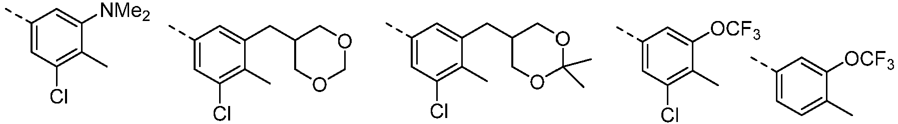

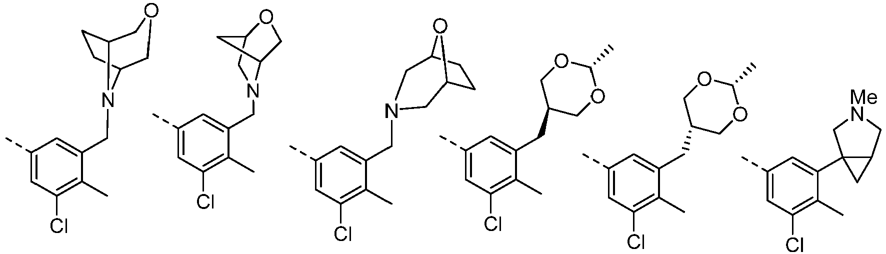

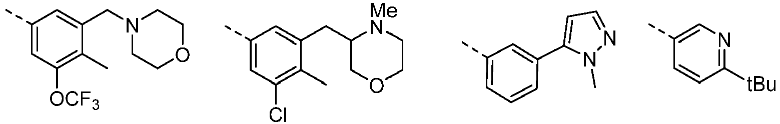

10. A composition comprising a compound according to any one of claims 1-9 or pharmaceutically acceptable salts or stereoisomers thereof.

11. The composition of claim 10, further comprising a pharmaceutically acceptable carrier and/or a second therapeutically active agent.

12. A compound of claims 1 to 9 or a composition of any one of claims 10-11 for use in therapy.

13. A compound of claims 1 to 9 or a composition of any one of claims 10-11 for use in a method of treatment of a disease associated with GSPT1.

14. A compound of claims 1 to 9 or a composition of any one of claims 10-11 for use in a method of treatment of cancer, in particular breast cancer.

15. The compound or composition for use of claim 14, wherein the compound according to any one of claims 1-9 or pharmaceutically acceptable salts or stereoisomers thereof binds to the cereblon.

## Patentansprüche

1. Verbindung oder pharmazeutisch unbedenkliche Salze oder Stereoisomere davon der Formel I 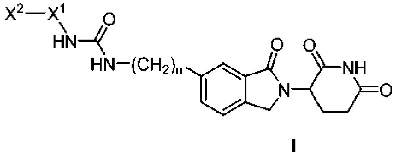 wobei
X¹ für ein lineares oder verzweigtes C₁₋₆-Alkyl, C₃₋₆-Cycloalkyl, -C₁₋₆-Alkyl-C₃₋₆-Cycloalkyl, C₆₋₁₀-Aryl, 5-10-gliedriges Heteroaryl, C₁₋₆-Alkyl-C₆₋₁₀-aryl, C₁₋₆-Alkyl-5-10-gliedriges-heteroaryl steht, wobei X¹ unsubstituiert oder mit einem oder mehreren aus Halogen, linearem oder verzweigtem C₁₋₆-Alkyl, linearem oder verzweigtem C₁₋₆-Heteroalkyl, CF₃, CHF₂, -O-CHF₂, -O-(CH₂)₂-OMe, OCF₃, C₁₋₆-Alkylamino, -CN, -N(H)C(O)C₁₋₆-Alkyl, -OC(O)-C₁₋₆-Alkyl,-OC(O)-C₁₋₄Alkylamino, -C(O)O-C₁₋₆Alkyl, -COOH, -CHO, -C₁₋₆Alkyl-C(O)OH, -C₁₋₆-Alkyl-C(O)O-C₁₋₆alkyl, NH₂, C₁₋₆-Alkoxy oder C₁₋₆-Alkylhydroxy substituiert ist;
X² für Wasserstoff, C₆₋₁₀-Aryl, 5-10-gliedriges Heteroaryl, -O-(5-10-gliedriges-Heteroaryl), 4-8-gliedriges Heterocycloalkyl, C₁₋₄-Alkyl-4-8-gliedriges-heterocycloalkyl, -O-(4-8-gliedriges-heterocycloalkyl), -O-C₁₋₄-Alkyl-(4-8-gliedriges-heterocycloalkyl), -OC(O)-C₁₋₄-Alkyl-4-8-gliedriges-heterocycloalkyl oder C₆-Aryloxy steht, wobei X² unsubstituiert oder mit einem oder mehreren aus linearem oder verzweigtem C₁₋₆-Alkyl, NH₂, NMe₂ oder 5-6-gliedrigem Heterocycloalkyl substituiert ist;
n für 0, 1 oder 2 steht.

2. Verbindung nach Anspruch 1 oder pharmazeutisch unbedenkliche Salze oder Stereoisomere davon, wobei
X¹ für lineares oder verzweigtes C₁₋₄-Alkyl, C₃₋₆-Cycloalkyl, -C₁₋₄-Alkyl-C₃₋₆-cycloalkyl, C₆-Aryl, 5-10-gliedriges Heteroaryl, C₁₋₆-Alkyl-C₆-aryl, C₁₋₆-Alkyl-5-10-gliedriges-heteroaryl, wobei X¹ unsubstituiert oder mit einem oder mehreren aus Halogen, linearem oder verzweigtem C₁₋₄-Alkyl, linearem oder verzweigtem C₁₋₆-Heteroalkyl, CF₃, CHF₂, -O-CHF₂-O- (CH₂)₂-OMe, OCF₃, C₁₋₆-Alkylamino, -CN, -N(H)C(O)₁₋₆-Alkyl, -OC(O)-C₁₋₆-Alkyl,-OC(O)₁₋₄-Alkylamino, -C(O)O-C₁₋₆-Alkyl, -COOH, -CHO, -C₁₋₆Alkyl-C(O)OH, -C₁₋₆-Alkyl-C(O)O-C₁₋₆alkyl, NH₂, C₁₋₆-Alkoxy oder C₁₋₆-Alkylhydroxy substituiert ist; und
X² für Wasserstoff, C₆-Aryl, 5-10-gliedriges Heteroaryl, -O-(5-10-gliedriges Heteroaryl), 4-8-gliedriges Heterocycloalkyl, C₁₋₄-Alkyl-4-8-gliedriges Heterocycloalkyl, -O-(4-8-gliedriges-Heterocycloalkyl), -O-C₁₋₄-Alkyl-(4-8-gliedriges-Heterocycloalkyl), -OC(O)-C₁₋₄-Alkyl-4-8-gliedriges-Heterocycloalkyl oder C₆-Aryloxy steht, wobei X² unsubstituiert oder mit einem oder mehreren aus linearem oder verzweigtem C₁₋₆-Alkyl, NH₂, NMe₂ oder 5-6-gliedrigem Heterocycloalkyl substituiert ist; und wobei n für 0, 1 oder 2 steht.

3. Verbindung nach Anspruch 1 oder 2, wobei n für 1 oder 2, vorzugsweise 1 steht.

4. Verbindung nach einem der vorhergehenden Ansprüche oder pharmazeutisch unbedenkliche Salze oder Stereoisomere davon der Formel II 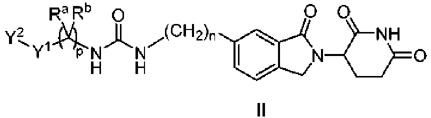 wobei
n für 1 oder 2 steht;
p für 0, 1, 2, 3, 4, 5, 6 steht;
Y¹ für Wasserstoff, C₆-Aryl, 5-10-gliedriges Heteroaryl, C₃₋₆-Cycloalkyl steht, wobei Y¹ unsubstituiert oder mit einem oder mehreren aus Halogen, vorzugsweise F, Cl oder Br, linearem oder verzweigtem C₁₋₄-Alkyl, linearem oder verzweigtem C₁₋₆-Heteroalkyl, CF₃, CHF₂, -O-CHF₂, -O-(CH₂)₂-OMe, OCF₃, -CN, -N(H)C(O)-C₁₋₆-Alkyl,-OC(O)-C₁₋₆-Alkyl, -OC(O)-C₁₋₄-Heteroalkyl, -C(O)O-C₁₋₆-Alkyl, -COOH, -CHO, -C₁₋₆-Alkyl-C(O)OH, -C₁₋₆-Alkyl-C(O)O-C₁₋₆-alkyl, NH₂ oder C₁₋₆-Alkylhydroxy substituiert ist; mit der Maßgabe, dass wenn p für o steht, Y¹ nicht für Wasserstoff steht;
Y² für Wasserstoff, C₆-Aryl, 5-10-gliedriges Heteroaryl, -O-(5-10-gliedriges-Heteroaryl), 4-8-gliedriges Heterocycloalkyl, C₁₋₄-Alkyl-4-8-gliedriges-Heterocycloalkyl, -O-(4-8-gliedriges-Heterocycloalkyl), -O-C₁₋₄-Alkyl-(4-8-gliedriges-Heterocycloalkyl), -OC(O)-C₁₋₄-Alkyl-4-8-gliedriges-Heterocycloalkyl oder C₆-Aryloxy steht, wobei Y² unsubstituiert oder mit einem oder mehreren aus linearem oder verzweigtem C₁₋₆-Alkyl, NH₂, NMe₂ oder 5-6-gliedrigem Heterocycloalkyl substituiert ist;
R^{a}, R^{b} jeweils unabhängig aus Wasserstoff und linearem oder verzweigtem C₁₋₄-Alkyl, vorzugsweise Wasserstoff und Methyl ausgewählt sind.

5. Verbindung nach einem der vorhergehenden Ansprüche oder pharmazeutisch unbedenkliche Salze oder Stereoisomere davon der Formel IIIa', IIIb' oder IIIc 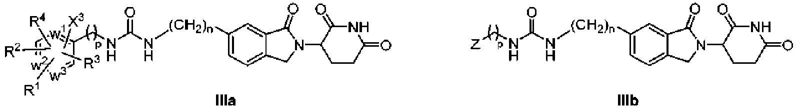 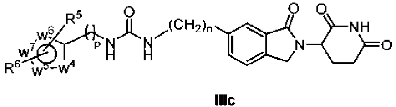 wobei
n für 1 oder 2 steht;
p für 0, 1 oder 2 steht;
eines aus w¹, w² oder w³ aus C und N ausgewählt ist, und die anderen zwei aus w¹, w² oder w³ für C stehen;
eines oder zwei aus w⁴, w⁵, w⁶, w⁷ aus C, O, N, NMe, NH oder S ausgewählt ist/sind, während zwei oder drei aus w⁴, w⁵, w⁶ und w⁷ für C stehen;
R¹, R², R³, R⁴ jeweils unabhängig aus Wasserstoff, linearem oder verzweigtem C₁₋₆-Alkyl, C₁₋₆-Heteroalkyl, C₆-Aryl, vorzugsweise Phenyl, CF₃, CHF₂, -O-CHF₂-O-(CH₂)₂-OMe, OCF₃, -CN, -N(H)C(O)-C₁₋₆-Alkyl, -OC(O)-C₁₋₄-Alkylamino,-OC(O)-C₁₋₆-Alkyl, -C(O)O-C₁₋₆-Alkyl, -COOH, -CHO, -C₁₋₆-Alkyl-C(O)OH, -C₁₋₆-Alkyl-C(O)O-C₁₋₆alkyl, NH₂, C₁₋₄-Alkylhydroxy, Halogen, vorzugsweise F, Cl, Br, besonders bevorzugt F oder Cl ausgewählt sind; und/oder zwei von R¹, R², R³, R⁴ zusammen ein 5-6-gliedriges Heterocycloalkyl oder ein 5-6-gliedriges Heteroaryl bilden;
R⁵, R⁶ jeweils unabhängig aus Wasserstoff, linearem oder verzweigtem C₁₋₄-Alkyl, CF₃, CHF₂, Halogen, vorzugsweise F, Cl, Br, besonders bevorzugt F oder Cl ausgewählt sind, X³ fehlt, für Wasserstoff oder 4-8-gliedriges Heterocycloalkyl, C₁₋₄-Alkyl-4-8-gliedriges-heterocycloalkyl, -O-(4-8-gliedriges-heterocycloalkyl), -C₁₋₄-Alkoxy-(4-8-gliedriges heterocycloalkyl), 5-10-gliedriges Heteroaryl, -O-(5-10-gliedriges-heteroaryl), -OC(O)-C₁₋₄-Alkyl-4-8-gliedriges-heterocycloalkyl, wobei X³ unsubstituiert oder mit einem oder mehreren aus linearem oder verzweigtem C₁₋₆-Alkyl, NH₂, NMe₂ oder 5-6-gliedrigem Heterocycloalkyl substituiert ist;
Z für lineares oder verzweigtes C₁₋₆-Alkyl oder C₃₋₆-Cycloalkyl oder C₁₋₄-Alkoxy steht, wobei Z unsubstituiert oder mit C₁₋₄-Alkyl substituiert ist.

6. Verbindung nach Anspruch 4 oder 5 oder pharmazeutisch unbedenkliche Salze oder Stereoisomere davon, wobei n für 1 steht.

7. Verbindung nach Anspruch 5 oder 6 oder pharmazeutisch unbedenkliche Salze oder Stereoisomere davon, wobei p für 0 oder 1 steht.

8. Verbindung nach einem der vorhergehenden Ansprüche oder pharmazeutisch unbedenkliche Salze oder Stereoisomere davon der Formel IV, V, VI und VII 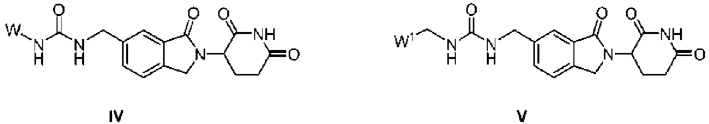 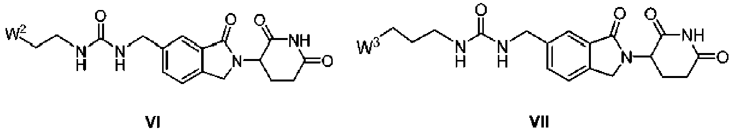 wobei
W, W¹, W², W³ unabhängig aus Folgendem ausgewählt sind

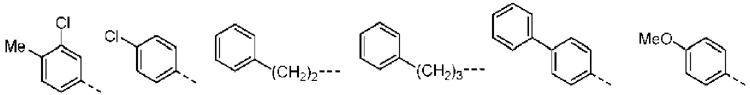

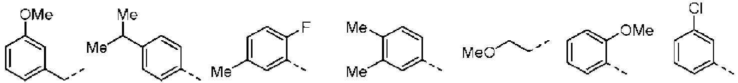

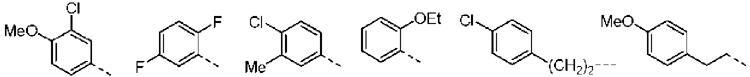

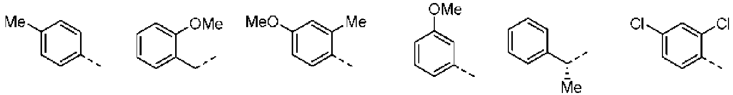

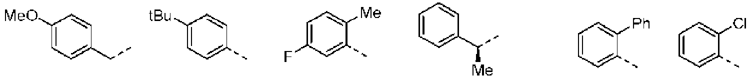

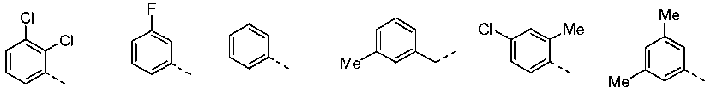

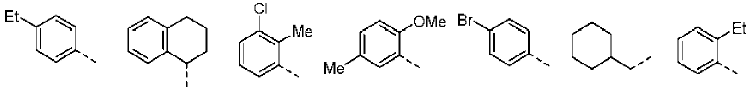

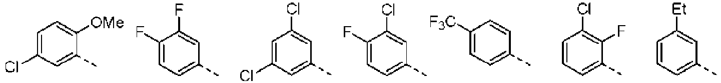

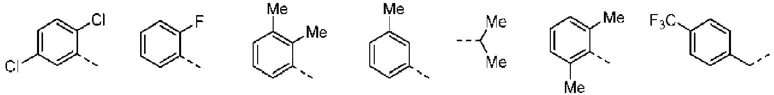

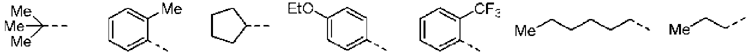

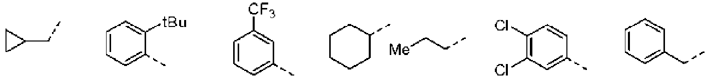

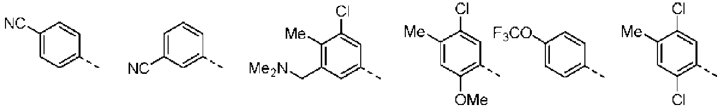

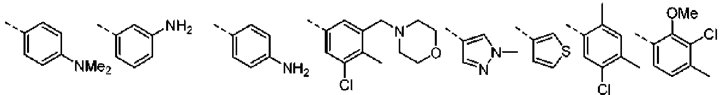

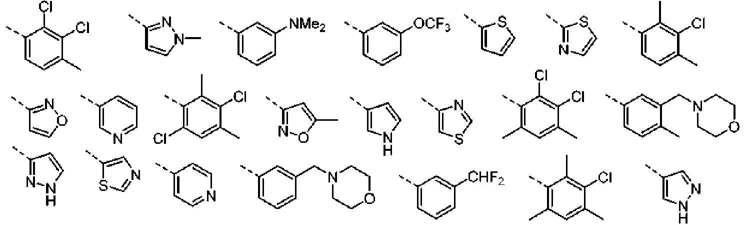

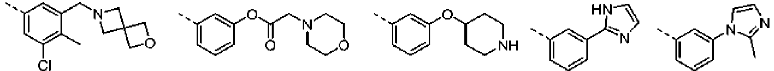

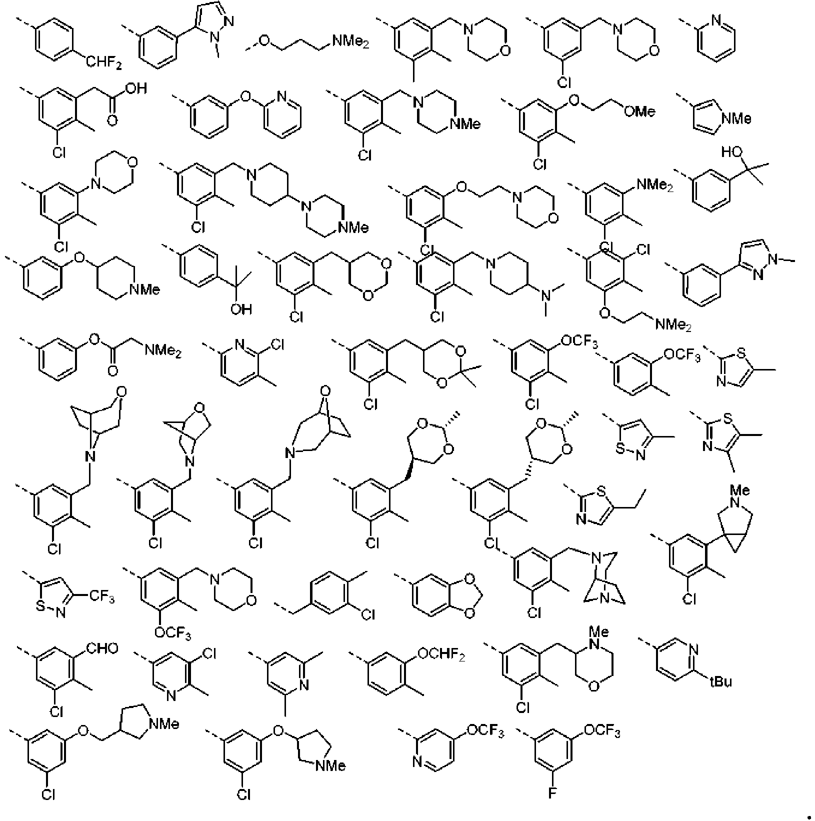

9. Verbindung nach Anspruch 8 oder pharmazeutisch unbedenkliche Salze oder Stereoisomere davon, wobei W für Folgendes steht:

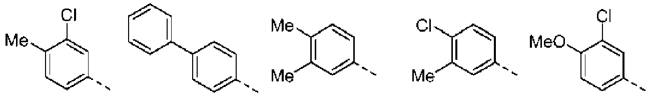

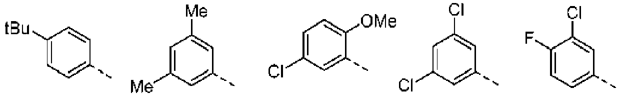

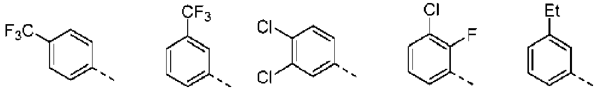

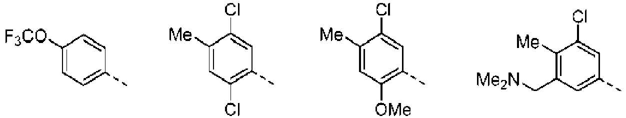

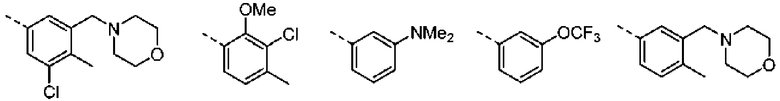

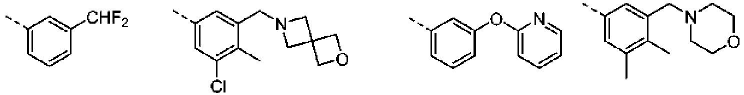

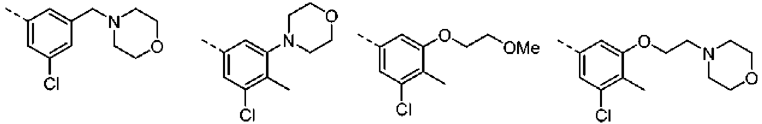

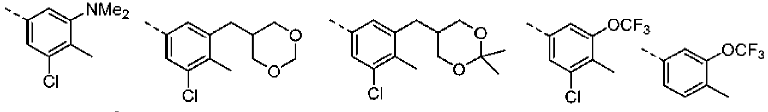

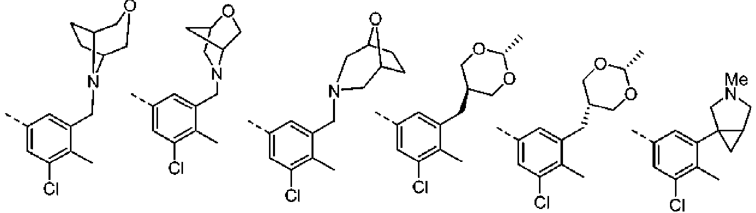

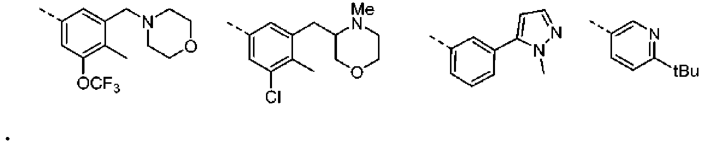

10. Zusammensetzung, umfassend eine Verbindung nach einem der Ansprüche 1-9 oder pharmazeutisch unbedenkliche Salze oder Stereoisomere davon.

11. Zusammensetzung nach Anspruch 10, ferner umfassend einen pharmazeutisch unbedenklichen Träger und/oder einen zweiten therapeutisch aktiven Stoff.

12. Verbindung nach den Ansprüchen 1 bis 9 oder Zusammensetzung nach einem der Ansprüche 10-11 zur Verwendung bei der Therapie.

13. Verbindung nach den Ansprüchen 1 bis 9 oder Zusammensetzung nach einem der Ansprüche 10-11 zur Verwendung bei einem Verfahren zur Behandlung einer mit GSPT1 assoziierten Krankheit.

14. Verbindung nach den Ansprüchen 1 bis 9 oder Zusammensetzung nach einem der Ansprüche 10-11 zur Verwendung bei einem Verfahren zur Behandlung von Krebs, insbesondere Brustkrebs.

15. Verbindung oder Zusammensetzung zur Verwendung nach Anspruch 14, wobei die Verbindung nach einem der Ansprüche 1-9 oder pharmazeutisch unbedenkliche Salze oder Stereoisomere davon an das Cereblon bindet.

## Revendications

1. Composé ou sels ou stéréoisomères pharmaceutiquement acceptables de celui-ci de formule I 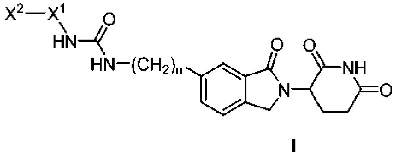
X¹ étant C₁₋₆ alkyle linéaire ou ramifié, C₃₋₆ cycloalkyle, -C₁₋₆ alkyl-C₃₋₆ cycloalkyle, C₆₋₁₀ aryle, hétéroaryle à 5 à 10 chaînons, C₁₋₆ alkyl-C₆₋₁₀ aryle, C₁₋₆ alkyle hétéroaryle à 5 à 10 chaînons, X¹ étant non substitué ou substitué par un ou plusieurs parmi halogène, C₁₋₆ alkyle linéaire ou ramifié, C₁₋₆ hétéroalkyle linéaire ou ramifié, CF₃, CHF₂, -O-CHF₂, -O-(CH₂)₂-OMe, OCF₃, C₁₋₆ alkylamino, -CN, -N(H)C(O)C₁₋₆alkyle, -OC(O)-C₁₋₆alkyle, -OC(O)-C₁₋₄alkylamino, -C(O)O-C₁₋₆alkyle, -COOH, -CHO, -C₁₋₆alkylC(O)OH, -C₁₋₆alkylC(O)O-C₁₋₆alkyle, NH₂, C₁₋₆ alcoxy ou C₁₋₆ alkylhydroxy ;
X² étant hydrogène, C₆₋₁₀ aryle, hétéroaryle à 5 à 10 chaînons, -O-(hétéroaryle à 5 à 10 chaînons), hétérocycloalkyle à 4 à 8 chaînons, C₁₋₄ alkyle hétérocycloalkyle à 4 à 8 chaînons, -O-(hétérocycloalkyle à 4 à 8 chaînons), -O-C₁₋₄ alkyl-(hétérocycloalkyle à 4 à 8 chaînons), -OC(O)-C₁₋₄alkyl-hétérocycloalkyle à 4 à 8 chaînons ou C₆ aryloxy, X² étant non substitué ou substitué par un ou plusieurs parmi C₁₋₆ alkyle linéaire ou ramifié, NH₂, NMe₂ ou hétérocycloalkyle à 5 à 6 chaînons ;
n étant 0, 1 ou 2.

2. Composé selon la revendication 1 ou sels ou stéréoisomères pharmaceutiquement acceptables de celui-ci,
X¹ étant C₁₋₄ alkyle linéaire ou ramifié, C₃₋₆ cycloalkyle, -C₁₋₄ alkyl-C₃₋₆ cycloalkyle, C₆ aryle, hétéroaryle à 5 à 10 chaînons, C₁₋₆ alkyl-C₆ aryle, C₁₋₆ alkyle hétéroaryle à 5 à 10 chaînons, X¹ étant non substitué ou substitué par un ou plusieurs parmi halogène, C₁₋₄ alkyle linéaire ou ramifié, C₁₋₆ hétéroalkyle linéaire ou ramifié, CF₃, CHF₂, -O-CHF₂, -O- (CH₂)₂-OMe, OCF₃, C₁₋₆ alkylamino, -CN,-N(H)C(O)₁₋₆alkyle, -OC(O)-C₁₋₆alkyle, -OC(O)₁₋₄alkylamino, -C(O)O-C₁₋₆alkyle, -COOH, -CHO, -C₁₋₆alkylC(O)OH, -C₁₋₆alkylC(O)O-C₁₋₆alkyle, NH₂, C₁₋₆ alcoxy ou C₁₋₆ alkylhydroxy ; et
X² étant hydrogène, C₆ aryle, hétéroaryle à 5 à 10 chaînons, -O-(hétéroaryle à 5 à 10 chaînons), hétérocycloalkyle à 4 à 8 chaînons, C₁₋₄ alkyle hétérocycloalkyle à 4 à 8 chaînons, -O-(hétérocycloalkyle à 4 à 8 chaînons), -O-C₁₋₄ alkyl-(hétérocycloalkyle à 4 à 8 chaînons), -OC(O)-C₁₋₄alkyl-hétérocycloalkyle à 4 à 8 chaînons ou C₆ aryloxy, X² étant non substitué ou substitué par un ou plusieurs parmi C₁₋₆ alkyle linéaire ou ramifié, NH₂, NMe₂ ou hétérocycloalkyle à 5 à 6 chaînons ; et n étant 0, 1, ou 2.

3. Composé selon les revendications 1 ou 2, n étant 1 ou 2, de préférence 1.

4. Composé selon l'une quelconque des revendications précédentes ou sels ou stéréoisomères pharmaceutiquement acceptables de celui-ci de formule II 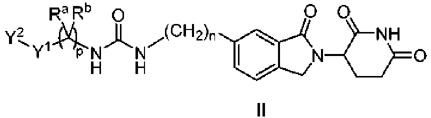
n étant 1 ou 2 ;
p étant 0, 1, 2, 3, 4, 5, 6 ;
Y¹ étant hydrogène, C₆ aryle, hétéroaryle à 5 à 10 chaînons-C₃₋₆ cycloalkyle, Y¹ étant non substitué ou substitué par un ou plusieurs parmi halogène, de préférence F, Cl ou Br, C₁₋₄ alkyle linéaire ou ramifié, C₁₋₆ hétéroalkyle linéaire ou ramifié, CF₃, CHF₂, -O-CHF₂, -O-(CH₂)₂-OMe, OCF₃, -CN, -N(H)C(O)-C₁₋₆alkyle, -OC(O)-C₁₋₆alkyle, -OC(O)-C₁₋₄hétéroalkyle, -C(O)O-C₁₋₆alkyle,-COOH, -CHO, -C₁₋₆alkylC(O)OH, -C₁₋₆alkylC(O)O-C₁₋₆alkyle, NH₂, ou C₁₋₆ alkylhydroxy ; à la condition que lorsque p est o, Y¹ n'est pas hydrogène ;
Y² étant hydrogène, C₆ aryle, hétéroaryle à 5 à 10 chaînons, -O-(hétéroaryle à 5 à 10 chaînons), hétérocycloalkyle à 4 à 8 chaînons, C₁₋₄ alkyle hétérocycloalkyle à 4 à 8 chaînons, -O-(hétérocycloalkyle à 4 à 8 chaînons), -O-C₁₋₄ alkyl-(hétérocycloalkyle à 4 à 8 chaînons), -OC(O)-C₁₋₄alkyl-hétérocycloalkyle à 4 à 8 chaînons ou C₆ aryloxy, Y² étant non substitué ou substitué par un ou plusieurs parmi C₁₋₆ alkyle linéaire ou ramifié, NH₂, NMe₂ ou hétérocycloalkyle à 5 à 6 chaînons ;
R^{a}, R^{b} étant chacun indépendamment choisis parmi hydrogène et C₁₋₄ alkyle linéaire ou ramifié, de préférence hydrogène et méthyle.

5. Composé selon l'une quelconque des revendications précédentes ou sels ou stéréoisomères pharmaceutiquement acceptables de celui-ci, de formule IIIa', IIIb' ou IIIc 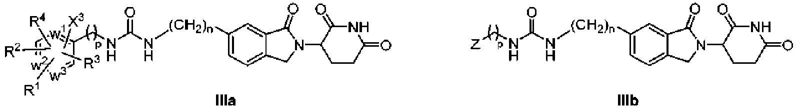 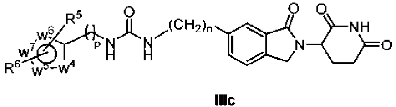
n étant 1 ou 2
p étant 0, 1 ou 2
l'un parmi w¹, w² ou w³ étant choisi parmi C et N, et les deux autres parmi w¹, w² ou w³ étant C ;
l'un ou les deux parmi W⁴, w⁵, w⁶, w⁷ étant choisi (s) parmi C, O, N, NMe, NH, ou S tandis que deux ou trois parmi w⁴, w⁵, w⁶ et w⁷ étant C ;
R¹, R², R³, R⁴ étant chacun indépendamment choisis parmi hydrogène, C₁₋₆ alkyle linéaire ou ramifié, C₁₋₆ hétéroalkyle, C₆ aryle, de préférence phényle, CF₃, CHF₂, -O-CHF₂ -O-(CH₂)₂-OMe, OCF₃, -CN, -N(H)C(O)-C₁₋₆alkyle,-OC(O)-C₁₋₄alkylamino, -OC(O)-C₁₋₆alkyle, -C(O)O-C₁₋₆alkyle, -COOH, -CHO, -C₁₋₆alkylC(O)OH, -C₁₋₆alkylC(O)O-C₁₋₆alkyle, NH₂, C₁₋₄ alkylhydroxy, halogène, de préférence F, Cl, Br, plus préférablement F ou Cl ; et/ou deux parmi R¹, R², R³, R⁴ formant ensemble un hétérocycloalkyle à 5 à 6 chaînons ou un hétéroaryle à 5 à 6 chaînons ;
R⁵, R⁶ étant chacun indépendamment choisis parmi hydrogène, C₁₋₄ alkyle linéaire ou ramifié, CF₃, CHF₂, halogène, de préférence F, Cl, Br, plus préférablement F ou Cl
X³ étant absent, hydrogène ou hétérocycloalkyle à 4 à 8 chaînons, C₁₋₄ alkyle hétérocycloalkyle à 4 à 8 chaînons, -O-(hétérocycloalkyle à 4 à 8 chaînons), -C₁₋₄ alcoxy-(hétérocycloalkyle à 4 à 8 chaînons), hétéroaryle à 5 à 10 chaînons, -O-(hétéroaryle à 5 à 10 chaînons),-OC(O)-C₁₋₄alkyl-hétérocycloalkyle à 4 à 8 chaînons, X³ étant non substitué ou substitué par un ou plusieurs parmi C₁₋₆ alkyle linéaire ou ramifié, NH₂, NMe₂ ou hétérocycloalkyle à 5 à 6 chaînons ;
Z étant C₁₋₆ alkyle linéaire ou ramifié ou C₃₋₆ cycloalkyle ou C₁₋₄ alcoxy, Z étant non substitué ou substitué par C₁₋₄ alkyle.

6. Composé selon les revendications 4 ou 5 ou sels ou stéréoisomères pharmaceutiquement acceptables de celui-ci, n étant 1.

7. Composé selon les revendications 5 ou 6 ou sels ou stéréoisomères pharmaceutiquement acceptables de celui-ci, p étant 0 ou 1.

8. Composé selon l'une quelconque des revendications précédentes ou sels ou stéréoisomères pharmaceutiquement acceptables de celui-ci, de formule IV, V, VI et VII 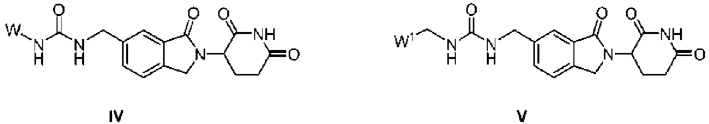 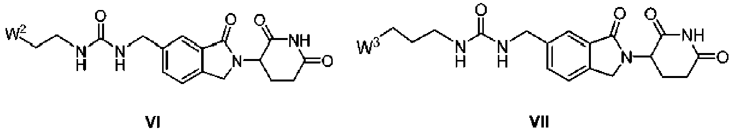 W, W¹, W², W³ étant choisis indépendamment parmi

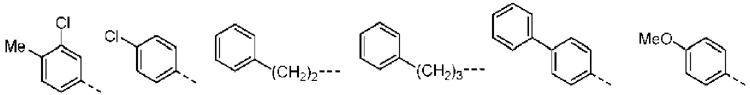

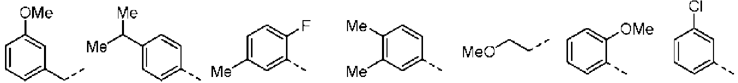

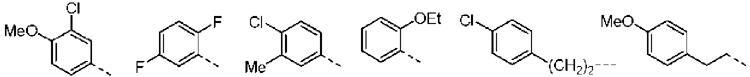

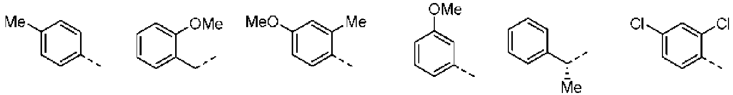

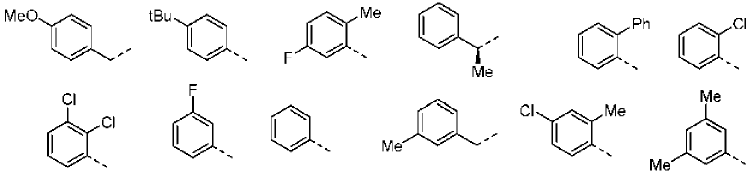

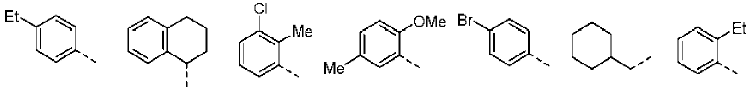

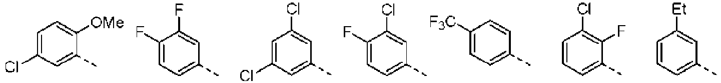

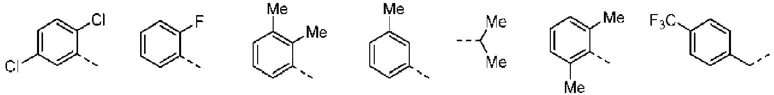

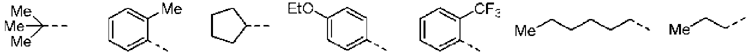

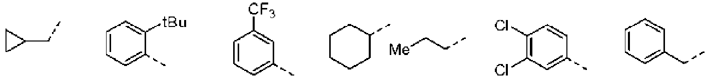

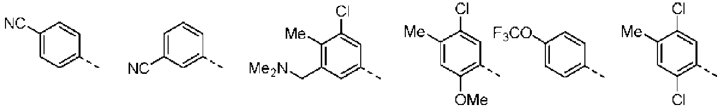

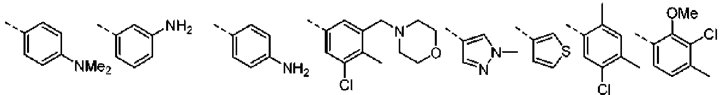

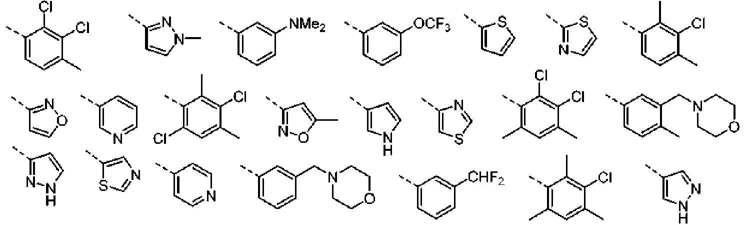

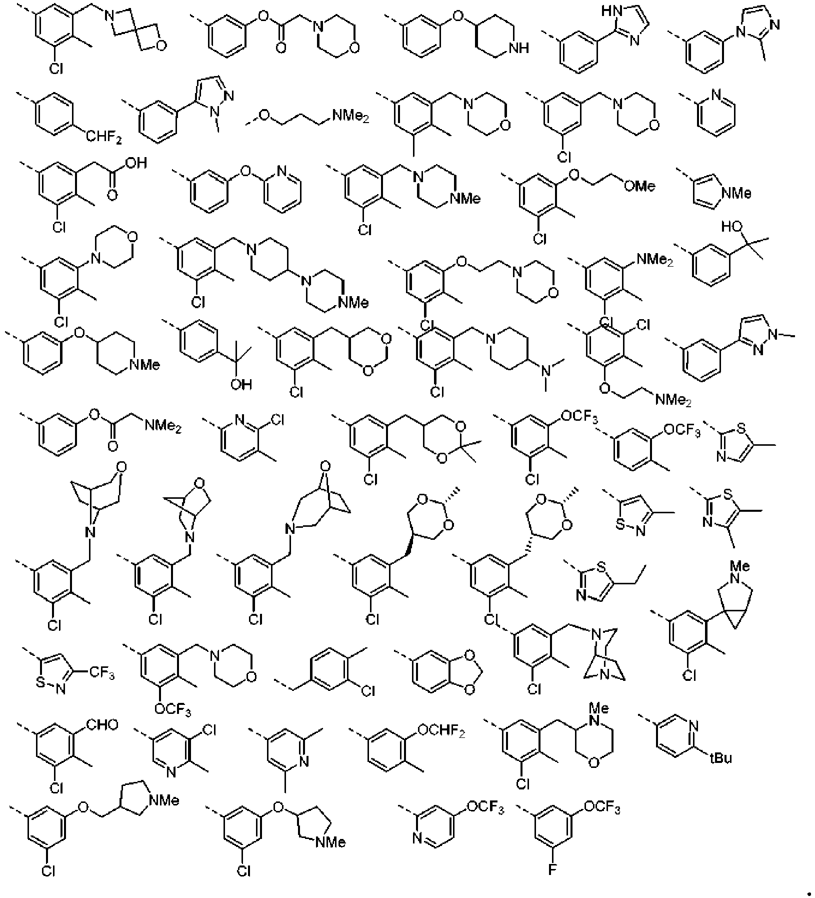

9. Composé selon la revendication 8, ou sels ou stéréoisomères pharmaceutiquement acceptables de celui-ci, W étant

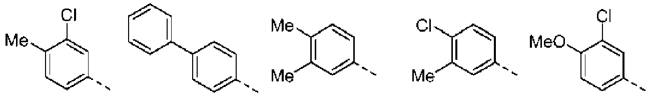

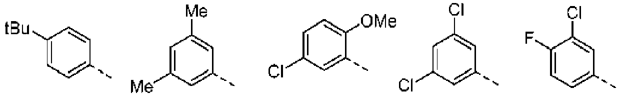

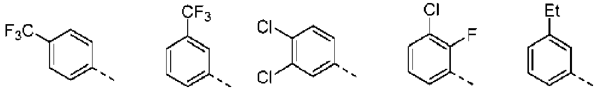

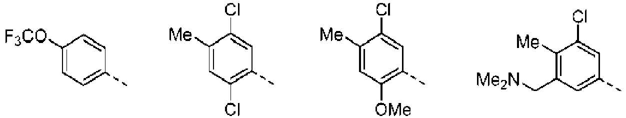

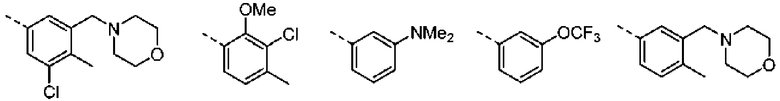

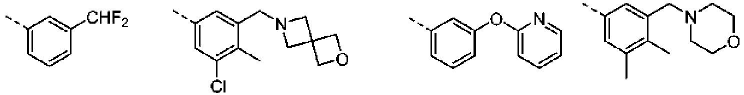

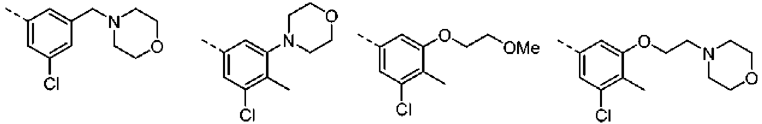

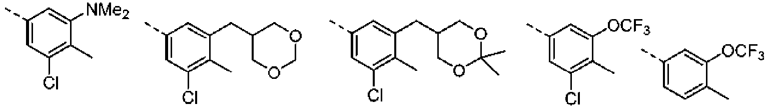

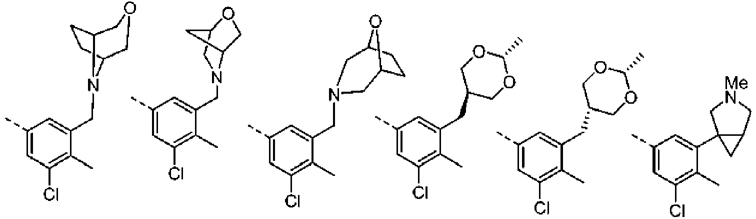

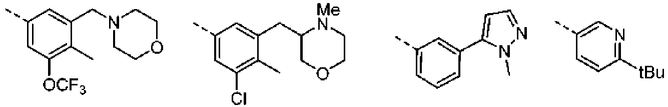

10. Composition comprenant un composé selon l'une quelconque des revendications 1-9 ou des sels ou stéréoisomères pharmaceutiquement acceptables de celui-ci.

11. Composition selon la revendication 10, comprenant en outre un support pharmaceutiquement acceptable et/ou un second agent thérapeutiquement actif.

12. Composé selon les revendications 1 à 9 ou composition selon l'une quelconque des revendications 10-11 à utiliser en thérapie.

13. Composé selon les revendications 1 à 9 ou composition selon l'une quelconque des revendications 10-11 à utiliser dans un procédé de traitement d'une maladie associée à GSPT1.

14. Composé selon l'une quelconque des revendications 1 à 9 ou composition selon l'une quelconque des revendications 10-11 à utiliser dans un procédé de traitement d'un cancer, en particulier d'un cancer du sein.

15. Composé ou composition pour utilisation selon la revendication 14, le composé selon l'une quelconque des revendications 1-9 ou des sels ou stéréoisomères pharmaceutiquement acceptables de celui-ci se liant au céréblon.
